(19) Europäisches Patentamt / European Patent Office / Office européen des brevets

(11) EP 3 397 773 B1

(12) **EUROPÄISCHE PATENTSCHRIFT**

(45) Veröffentlichungstag und Bekanntmachung des Hinweises auf die Patenterteilung:
**28.04.2021 Patentblatt 2021/17**

(21) Anmeldenummer: **16826049.5**

(22) Anmeldetag: **22.12.2016**

(51) Int Cl.:
***C12Q 1/68*** *(2018.01)*

(86) Internationale Anmeldenummer:
**PCT/EP2016/082414**

(87) Internationale Veröffentlichungsnummer:
**WO 2017/114754 (06.07.2017 Gazette 2017/27)**

(54) **VERFAHREN UND MITTEL ZUR DIAGNOSTIK VON TUMOREN**

METHOD AND MEANS FOR DIAGNOSING TUMORS

PROCÉDÉS ET MOYENS DE DIAGNOSTIC DE TUMEURS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.12.2015 DE 102015226843**
**31.08.2016 DE 102016216438**

(43) Veröffentlichungstag der Anmeldung:
**07.11.2018 Patentblatt 2018/45**

(73) Patentinhaber: **Technische Universität Dresden**
**01069 Dresden (DE)**

(72) Erfinder:
- **MENSCHIKOWSKI, Mario**
  **01454 Radeberg OT Liegau-Augustusbad (DE)**
- **HAGELGANS, Albert**
  **01307 Dresden (DE)**
- **SIEGERT, Gabriele**
  **01259 Dresden (DE)**

(74) Vertreter: **Kailuweit & Uhlemann Patentanwälte Partnerschaft mbB**
**Bamberger Straße 49**
**01187 Dresden (DE)**

(56) Entgegenhaltungen:
**WO-A1-2012/092259 WO-A1-2013/033714**
**WO-A1-2013/041731 WO-A1-2013/192351**
**US-A1- 2010 233 707 US-A1- 2013 022 974**

- **NICHOLAS REDSHAW ET AL: "Quantification of epigenetic biomarkers: an evaluation of established and emerging methods for DNA methylation analysis", BMC GENOMICS, BIOMED CENTRAL LTD, LONDON, UK, Bd. 15, Nr. 1, 23. Dezember 2014 (2014-12-23), Seite 1174, XP021211806, ISSN: 1471-2164, DOI: 10.1186/1471-2164-15-1174**
- **WOJDACZ TOMASZ K ET AL: "A new approach to primer design for the control of PCR bias in methylation studies", BMC RESEARCH NOTES, BIOMED CENTRAL LTD, GB, Bd. 1, Nr. 1, 28. Juli 2008 (2008-07-28), Seite 54, XP021045897, ISSN: 1756-0500, DOI: 10.1186/1756-0500-1-54**
- **TOMASZ WOJDACZ ET AL: "Reversal of PCR bias for improved sensitivity of the DNA methylation melting curve assay", BIOTECHNIQUES RAPID DISPATCHES, Bd. 41, Nr. 3, 1. September 2006 (2006-09-01), Seiten 274-278, XP055352939, US ISSN: 0736-6205, DOI: 10.2144/000112240**
- **TOMASZ WOJDACZ ET AL: "Primer designs versus PCR bias in methylation independent PCR amplifications", EPIGENETICS, Bd. 4, Nr. 4, 16. Mai 2009 (2009-05-16), Seiten 231-234, XP055023508, DOI: 10.4161/epi.9020**
- **STEVEN T OKINO ET AL: "Chromatin Changes on the GSTP1 Promoter Associated With its Inactivation in Prostate Cancer", MOLECULAR CARCINOGENESIS, JOHN WILEY & SONS, INC, US, Bd. 46, Nr. 10, 1. Oktober 2007 (2007-10-01), Seiten 839-846, XP008116184, ISSN: 0899-1987, DOI: 10.1002/MC.20313 [gefunden am 2007-04-05]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **MARIO MENSCHIKOWSKI ET AL: "Aberrant methylation of the M-type phospholipase A2 receptor gene in leukemic cells", BMC CANCER, BIOMED CENTRAL, LONDON, GB, Bd. 12, Nr. 1, 5. Dezember 2012 (2012-12-05), Seite 576, XP021139013, ISSN: 1471-2407, DOI: 10.1186/1471-2407-12-576**
- **DATABASE Geneseq [Online] 9. September 2004 (2004-09-09), "PCR primer amplifies promoter CpG islands of cancer related genes Seq86.", XP55353581, gefunden im EBI accession no. GSN:ADQ77404 Database accession no. ADQ77404**
- **DATABASE Geneseq [Online] 23. Juli 2009 (2009-07-23), "Methylation detecting PCR primer, SEQ ID 122.", XP55353586, gefunden im EBI accession no. GSN:AXB24985 Database accession no. AXB24985**
- **DATABASE Geneseq [Online] 7. April 2016 (2016-04-07), "Human RASSF1A gene specific probe/RASS P1, SEQ ID 22.", XP55353595, gefunden im EBI accession no. GSN:BCM21582 Database accession no. BCM21582**
- **DATABASE Geneseq [Online] 15. Juli 2015 (2015-07-15), "Human RASSF1A gene specific probe/RASS P2, SEQ ID 23.", XP55353608, gefunden im EBI accession no. GSN:BCM21583 Database accession no. BCM21583**
- **WOJDACZ TOMASZ K ET AL: "A new approach to primer design for the control of PCR bias in methylation studies", BMC RESEARCH NOTES, BIOMED CENTRAL LTD, GB, vol. 1, no. 1, 28 July 2008 (2008-07-28), page 54, XP021045897, ISSN: 1756-0500, DOI: 10.1186/1756-0500-1-54**
- **MENSCHIKOWSKI MARIO ET AL: "Identification of rare levels of methylated tumor DNA fragments using an optimized bias based pre-amplification-digital droplet PCR (OBBPA-ddPCR).", ONCOTARGET 16 NOV 2018, vol. 9, no. 90, 16 November 2018 (2018-11-16), pages 36137-36150, ISSN: 1949-2553**

SEQUENCE LISTING

<110> Technische Universität Dresden

<120> Verfahren und Mittel zur Diagnostik von Tumoren

<130> 00017P0297DEWO

<150> 10 2015 226 843.8
<151> 2015-12-30

<150> 10 2016 216 438.4
<151> 2016-08-31

<160> 184

<170> PatentIn version 3.5

<210> 1
<211> 20
<212> DNA
<213> Artificial Sequence

<220>
<223> Forward Primer for PLA2R1 (168 bp amplificate)

<400> 1
ggggtaagga aggtggagat 20

<210> 2
<211> 27
<212> DNA
<213> Artificial Sequence

<220>
<223> Reverse Primer for PLA2R1 (168 bp amplificate)

<400> 2
acaaaccacc taaattctaa taaacac 27

<210> 3
<211> 22
<212> DNA
<213> Artificial Sequence

<220>
<223> Forward Primer for RASSF1A (117 bp amplificate)

<400> 3
gtttgttagc gtttaaagtt ag 22

<210> 4
<211> 18
<212> DNA
<213> Artificial Sequence

<220>
<223> Reverse Primer for RASSF1A (117 bp amplificate)

<400> 4
aatacgaccc ttcccaac 18

<210> 5
<211> 21
<212> DNA
<213> Artificial Sequence

<220>
<223> Forward Primer for GSTP1 (120 bp amplificate)

<400> 5
gtgaagcggg tgtgtaagtt t 21

<210> 6
<211> 23
<212> DNA
<213> Artificial Sequence

<220>
<223> Reverse Primer for GSTP1 (120 bp amplificate)

<400> 6
taaacaaaca acaaaaaaaa aac 23

<210> 7
<211> 18
<212> DNA
<213> Artificial Sequence

<220>
<223> Forward Primer for PLA2R1 (133 bp amplificate)

<400> 7
ggaaggtgga gattacgg 18

<210> 8
<211> 20
<212> DNA
<213> Artificial Sequence

<220>
<223> Reverse Primer for PLA2R1 (133 bp amplificate)

<400> 8
gcgaatttac aacgaacaac 20

<210> 9
<211> 23
<212> DNA
<213> Artificial Sequence

<220>
<223> Reverse Primer for PLA2R1 (150 bp amplificate)

<400> 9
aataaacacc gcgaatttac aac 23

<210> 10
<211> 23
<212> DNA
<213> Artificial Sequence

<220>
<223> Reverse Primer for PLA2R1 (161 bp amplificate)

<400> 10
acctaaattc taataaacac cgc 23

<210> 11
<211> 22
<212> DNA
<213> Artificial Sequence

<220>
<223> Reverse Primer for PLA2R1 (160 bp amplificate)

<400> 11
cctaaattct aataaacacc gc 22

<210> 12
<211> 19
<212> DNA
<213> Artificial Sequence

<220>
<223> Forward Primer for GSTP1 (171 bp amplificate)

<400> 12
gttcggttaa tatggtgaa 19

<210> 13
<211> 22
<212> DNA
<213> Artificial Sequence

<220>
<223> Reverse Primer for GSTP1 (171 bp amplificate)

<400> 13
acccaaacta aaatacaata ac 22

<210> 14
<211> 22
<212> DNA
<213> Artificial Sequence

<220>
<223> Forward Primer for AOX1 (180 bp amplificate)

<400> 14
tgggttggat tttaggtttt ag 22

<210> 15
<211> 18
<212> DNA
<213> Artificial Sequence

<220>
<223> Reverse Primer for AOX1 (180 bp amplificate)

<400> 15
ctcaccttac gaccgttc 18

<210> 16
<211> 18
<212> DNA
<213> Artificial Sequence

<220>
<223> Forward Primer for SERPINE1 (123 bp amplificate)

<400> 16
agagcgttgt taagaaga 18

<210> 17
<211> 22
<212> DNA
<213> Artificial Sequence

<220>
<223> Reverse Primer for SERPINE1 (123 bp amplificate)

<400> 17
ctcctaccta aaattctcaa aa 22

<210> 18
<211> 23
<212> DNA
<213> Artificial Sequence

<220>
<223> Forward Primer for AOX1 (138 bp amplificate)

<400> 18
gttggatttt aggttttagt aag 23

<210> 19
<211> 20
<212> DNA
<213> Artificial Sequence

<220>
<223> Reverse Primer for AOX1 (138 bp amplificate)

<400> 19
gcccgatcca ttataatatc 20

<210> 20
<211> 21
<212> DNA
<213> Artificial Sequence

<220>
<223> Probe for PLA2R1 (3 CpG) methylated

<400> 20
cccaactact ccgcgacgca a 21

<210> 21
<211> 24
<212> DNA

<213> Artificial Sequence

<220>
<223> Probe for PLA2R1 (3 CpG) non-methylated

<400> 21
aacccaacta ctccacaaca caaa 24

<210> 22
<211> 22
<212> DNA
<213> Artificial Sequence

<220>
<223> Probe for PLA2R1 (4 CpG) methylated

<400> 22
caactactcc gcgacgcaaa cg 22

<210> 23
<211> 26
<212> DNA
<213> Artificial Sequence

<220>
<223> Probe for PLA2R1 (4 CpG) non-methylated

<400> 23
aacccaacta ctccacaaca caaaca 26

<210> 24
<211> 23
<212> DNA
<213> Artificial Sequence

<220>
<223> Probe for RASSF1A (3 CpG) methylated

<400> 24
cgcccaacga ataccaactc ccg 23

<210> 25
<211> 26
<212> DNA
<213> Artificial Sequence

<220>
<223> Probe for RASSF1A (3 CpG) non- methylated

<400> 25
cacccaacaa ataccaactc ccacaa 26

<210> 26
<211> 24
<212> DNA
<213> Artificial Sequence

<220>

<223> Probe for PLA2R1 (3 CpG) non- methylated

<400> 26
tttgtgttgt ggagtagttg ggtt 24

<210> 27
<211> 22
<212> DNA
<213> Artificial Sequence

<220>
<223> Probe for PLA2R1 (4 CpG) methylated

<400> 27
cgtttgcgtc gcggagtagt tg 22

<210> 28
<211> 26
<212> DNA
<213> Artificial Sequence

<220>
<223> Probe for PLA2R1 (4 CpG) non-methylated

<400> 28
tgtttgtgtt gtggagtagt tgggtt 26

<210> 29
<211> 23
<212> DNA
<213> Artificial Sequence

<220>
<223> Probe for RASSF1A (3 CpG) methylated

<400> 29
cgggagttgg tattcgttgg gcg 23

<210> 30
<211> 26
<212> DNA
<213> Artificial Sequence

<220>
<223> Probe for RASSF1A (3 CpG) non-methylated

<400> 30
ttgtgggagt tggtatttgt tgggtg 26

<210> 31
<211> 25
<212> DNA
<213> Artificial Sequence

<220>
<223> Probe for RASSF1A (4 CpG) methylated

<400> 31

cgcgggagtt ggtattcgtt gggcg 25

<210> 32
<211> 29
<212> DNA
<213> Artificial Sequence

<220>
<223> Probe for RASSF1A (4 CpG) non-methylated

<400> 32
gagttgtggg agttggtatt tgttgggtg 29

<210> 33
<211> 20
<212> DNA
<213> Artificial Sequence

<220>
<223> Probe for GSTP1 (3 CpG) methylated

<400> 33
gttgcgtata tttcgttgcg 20

<210> 34
<211> 20
<212> DNA
<213> Artificial Sequence

<220>
<223> Probe for GSTP1 (3 CpG) non-methylated

<400> 34
gttgtgtata ttttgttgtg 20

<210> 35
<211> 20
<212> DNA
<213> Artificial Sequence

<220>
<223> Probe for GSTP1 (4 CpG) methylated

<400> 35
gtttcggcgc gttagttcgt 20

<210> 36
<211> 20
<212> DNA
<213> Artificial Sequence

<220>
<223> Probe for GSTP1 (4 CpG) non-methylated

<400> 36
gttttggtgt gttagtttgt 20

<210> 37

<211> 24
<212> DNA
<213> Artificial Sequence

<220>
<223> Probe for AOX1 (3 CpG) methylated

<400> 37
actcgaacgc ccgatccatt ataa 24

<210> 38
<211> 27
<212> DNA
<213> Artificial Sequence

<220>
<223> Probe for AOX1 (3 CpG) non-methylated

<400> 38
acaactcaaa cacccaatcc attataa 27

<210> 39
<211> 24
<212> DNA
<213> Artificial Sequence

<220>
<223> Probe for AOX1 (4 CpG) methylated

<400> 39
cgctaattcg aaaacccgaa acga 24

<210> 40
<211> 24
<212> DNA
<213> Artificial Sequence

<220>
<223> Probe for AOX1 (4 CpG) non-methylated

<400> 40
cactaattca aaaacccaaa acaa 24

<210> 41
<211> 26
<212> DNA
<213> Artificial Sequence

<220>
<223> Probe for AOX1 (5 CpG) methylated

<400> 41
cgcgctaatt cgaaaacccg aaacga 26

<210> 42
<211> 26
<212> DNA
<213> Artificial Sequence

<220>
<223> Probe for AOX1 (5 CpG) non-methylated

<400> 42
cacactaatt caaaaaccca aaacaa 26

<210> 43
<211> 27
<212> DNA
<213> Artificial Sequence

<220>
<223> Probe for SERPINE1 (4 CpG) methylated

<400> 43
cgattaacga ttcgtcctac tctaacg 27

<210> 44
<211> 27
<212> DNA
<213> Artificial Sequence

<220>
<223> Probe for SERPINE1 (4 CpG) non-methylated

<400> 44
caattaacaa ttcatcctac tctaaca 27

<210> 45
<211> 24
<212> DNA
<213> Artificial Sequence

<220>
<223> Probe for GSTP1 (3 CpG) methylated

<400> 45
cgatctcgac gactcactac aacc 24

<210> 46
<211> 26
<212> DNA
<213> Artificial Sequence

<220>
<223> Probe for GSTP1 (3 CpG) non-methylated

<400> 46
caatctcaac aactcactac aacctc 26

<210> 47
<211> 24
<212> DNA
<213> Artificial Sequence

<220>
<223> Probe for GSTP1 (4 CpG) methylated

<400> 47
cgcgatctcg acgactcact acaa 24

<210> 48
<211> 27
<212> DNA
<213> Artificial Sequence

<220>
<223> Probe for GSTP1 (4 CpG) non-methylated

<400> 48
cacaatctca acaactcact acaacct 27

<210> 49
<211> 24
<212> DNA
<213> Artificial Sequence

<220>
<223> Probe for GSTP1 (3 CpG) methylated

<400> 49
ggttgtagtg agtcgtcgag atcg 24

<210> 50
<211> 26
<212> DNA
<213> Artificial Sequence

<220>
<223> Probe for GSTP1 (3 CpG) non-methylated

<400> 50
gaggttgtag tgagtcgtcg agatcg 26

<210> 51
<211> 24
<212> DNA
<213> Artificial Sequence

<220>
<223> Probe for GSTP1 (4 CpG) methylated

<400> 51
ttgtagtgag tcgtcgagat cgcg 24

<210> 52
<211> 27
<212> DNA
<213> Artificial Sequence

<220>
<223> Probe for GSTP1 (4 CpG) non-methylated

<400> 52
aggttgtagt gagtcgtcga gatcgcg 27

<210> 53
<211> 20
<212> DNA
<213> Artificial Sequence

<220>
<223> Forward-Primer for PLA2R1 (150bp amplificate)

<400> 53
ggggtaagga aggtggagat 20

<210> 54
<211> 25
<212> DNA
<213> Artificial Sequence

<220>
<223> Probe for RASSF1A (4 CpG) methylated

<400> 54
cgcccaacga ataccaactc ccgcg 25

<210> 55
<211> 29
<212> DNA
<213> Artificial Sequence

<220>
<223> Probe for RASSF1A (4 CpG) non-methylated

<400> 55
cacccaacaa ataccaactc ccacaactc 29

<210> 56
<211> 20
<212> DNA
<213> Artificial Sequence

<220>
<223> Probe for GSTP1 (3 CpG) methylated

<400> 56
cgcaacgaaa tatacgcaac 20

<210> 57
<211> 20
<212> DNA
<213> Artificial Sequence

<220>
<223> Probe for GSTP1 (3 CpG) non-methylated

<400> 57
cacaacaaaa tatacacaac 20

<210> 58
<211> 20
<212> DNA

<213> Artificial Sequence

<220>
<223> Probe for GSTP1 (4 CpG) methylated

<400> 58
acgaactaac gcgccgaaac 20

<210> 59
<211> 20
<212> DNA
<213> Artificial Sequence

<220>
<223> Probe for GSTP1 (4 CpG) non-methylated

<400> 59
acaaactaac acaccaaaac 20

<210> 60
<211> 21
<212> DNA
<213> Artificial Sequence

<220>
<223> Probe for GSTP1 (3 CpG) methylated

<400> 60
ttgcgtcgcg gagtagttgg g 21

<210> 61
<211> 20
<212> DNA
<213> Artificial Sequence

<220>
<223> Forward Primer for RASSF1A (124 bp amplificate)

<400> 61
gcgtttgtta gcgtttaaag 20

<210> 62
<211> 18
<212> DNA
<213> Artificial Sequence

<220>
<223> Reverse Primer for RASSF1A (124 bp amplificate)

<400> 62
aaccgaatac gacccttc 18

<210> 63
<211> 23
<212> DNA
<213> Artificial Sequence

<220>

<223> Forward Primer for AOX1 (138 bp amplificate)

<400> 63
gttggatttt aggttttagt aag 23

<210> 64
<211> 20
<212> DNA
<213> Artificial Sequence

<220>
<223> Reverse Primer for AOX1 (138 bp amplificate)

<400> 64
gcccgatcca ttataatatc 20

<210> 65
<211> 24
<212> DNA
<213> Artificial Sequence

<220>
<223> Forward Primer for AOX1 (135 bp amplificate)

<400> 65
ggattttagg ttttagtaag tttc 24

<210> 66
<211> 22
<212> DNA
<213> Artificial Sequence

<220>
<223> Reverse Primer for AOX1 (135 bp amplificate)

<400> 66
gcccgatcca ttataatatc cg 22

<210> 67
<211> 24
<212> DNA
<213> Artificial Sequence

<220>
<223> Forward Primer for AOX1 (134 bp amplificate)

<400> 67
gattttaggt tttagtaagt ttcg 24

<210> 68
<211> 21
<212> DNA
<213> Artificial Sequence

<220>
<223> Forward Primer for SERPINE1 (119 bp amplificate)

<400> 68

cgttgttaag aagatttata c 21

<210> 69
<211> 23
<212> DNA
<213> Artificial Sequence

<220>
<223> Reverse Primer for SERPINE1 (119 bp amplificate)

<400> 69
taaacccgaa ataaaaaatt aaa 23

<210> 70
<211> 19
<212> DNA
<213> Artificial Sequence

<220>
<223> Forward Primer for Thrombomodulin (125 bp amplificate)

<400> 70
ggtcgattcg tatgttaga 19

<210> 71
<211> 18
<212> DNA
<213> Artificial Sequence

<220>
<223> Reverse Primer for Thrombomodulin (125 bp amplificate)

<400> 71
aaccgtaccg aaacaaaa 18

<210> 72
<211> 19
<212> DNA
<213> Artificial Sequence

<220>
<223> Forward Primer for Thrombomodulin (144 bp amplificate)

<400> 72
gtttgggttg ggacggata 19

<210> 73
<211> 20
<212> DNA
<213> Artificial Sequence

<220>
<223> Reverse Primer for Thrombomodulin (144 bp amplificate)

<400> 73
aaaaaccaaa accccaaaca 20

<210> 74

<211> 20
<212> DNA
<213> Artificial Sequence

<220>
<223> Forward Primer for Thrombomodulin (166 bp amplificate)

<400> 74
gtttggggtt ttggtttttg 20

<210> 75
<211> 20
<212> DNA
<213> Artificial Sequence

<220>
<223> Reverse Primer for Thrombomodulin (166 bp amplificate)

<400> 75
gcaatccgtc gcaaatctaa 20

<210> 76
<211> 20
<212> DNA
<213> Artificial Sequence

<220>
<223> Reverse Primer for Thrombomodulin (165 bp amplificate)

<400> 76
caatccgtcg caaatctaac 20

<210> 77
<211> 24
<212> DNA
<213> Artificial Sequence

<220>
<223> Probe for AOX1 (4 CpG) methylated

<400> 77
cgctaattcg aaaacccgaa acga 24

<210> 78
<211> 24
<212> DNA
<213> Artificial Sequence

<220>
<223> Probe for AOX1 (4 CpG) non-methylated

<400> 78
cactaattca aaaacccaaa acaa 24

<210> 79
<211> 27
<212> DNA
<213> Artificial Sequence

<220>
<223> Probe for AOX1 (4 CpG) non-methylated

<400> 79
cactaattca aaaacccaaa acaaaaa 27

<210> 80
<211> 21
<212> DNA
<213> Artificial Sequence

<220>
<223> Probe for Thrombomodulin (3CpG) methylated

<400> 80
acgccgataa cgacaacctc t 21

<210> 81
<211> 24
<212> DNA
<213> Artificial Sequence

<220>
<223> Probe for Thrombomodulin (3CpG) non-methylated

<400> 81
aaaaagcaga taaagacaac ctct 24

<210> 82
<211> 25
<212> DNA
<213> Artificial Sequence

<220>
<223> Probe for Thrombomodulin (4CpG) methylated

<400> 82
ccgactacga ctctacgaat acgaa 25

<210> 83
<211> 29
<212> DNA
<213> Artificial Sequence

<220>
<223> Probe for Thrombomodulin (4CpG) non-methylated

<400> 83
cagactaaga ctctaagaat aagaaaaac 29

<210> 84
<211> 24
<212> DNA
<213> Artificial Sequence

<220>
<223> Probe for AOX1 (4 CpG) methylated

<400> 84
tcgtttcggg ttttcgaatt agcg 24

<210> 85
<211> 24
<212> DNA
<213> Artificial Sequence

<220>
<223> Probe for AOX1 (4 CpG) non-methylated

<400> 85
ttgttttggg tttttgaatt agtg 24

<210> 86
<211> 27
<212> DNA
<213> Artificial Sequence

<220>
<223> Probe for AOX1 (4 CpG) non-methylated

<400> 86
tttttgtttt gggtttttga attagtg 27

<210> 87
<211> 21
<212> DNA
<213> Artificial Sequence

<220>
<223> Probe for Thrombomodulin (3CpG) methylated

<400> 87
agaggttgtc gttatcggcg t 21

<210> 88
<211> 21
<212> DNA
<213> Artificial Sequence

<220>
<223> Probe for Thrombomodulin (3CpG) non-methylated

<400> 88
agaggttgtt gttattggtg t 21

<210> 89
<211> 25
<212> DNA
<213> Artificial Sequence

<220>
<223> Probe for Thrombomodulin (4CpG) methylated

<400> 89
ttcgtattcg tagagtcgta gtcgg 25

<210> 90
<211> 25
<212> DNA
<213> Artificial Sequence

<220>
<223> Probe for Thrombomodulin (4CpG) non-methylated

<400> 90
tttgtatttg tagagttgta gttgg 25

<210> 91
<211> 21
<212> DNA
<213> Artificial Sequence

<220>
<223> Forward Primer for GSTP1 (114 bp amplificate)

<400> 91
cgtagcggtt ttagggaatt t 21

<210> 92
<211> 20
<212> DNA
<213> Artificial Sequence

<220>
<223> Reverse Primer for GSTP1 (114 bp amplificate)

<400> 92
tccccaacga aacctaaaaa 20

<210> 93
<211> 20
<212> DNA
<213> Artificial Sequence

<220>
<223> Forward Primer for GSTP1 (116 bp amplificate)

<400> 93
atcgtagcgg ttttagggaa 20

<210> 94
<211> 20
<212> DNA
<213> Artificial Sequence

<220>
<223> Reverse Primer for GSTP1 (116 bp amplificate)

<400> 94
tccccaacga aacctaaaaa 20

<210> 95
<211> 21
<212> DNA

<213> Artificial Sequence

<220>
<223> Forward Primer for GSTP1 (129 bp amplificate)

<400> 95
tgtaagtttc gggatcgtag c 21

<210> 96
<211> 20
<212> DNA
<213> Artificial Sequence

<220>
<223> Reverse Primer for GSTP1 (129 bp amplificate)

<400> 96
tccccaacga aacctaaaaa 20

<210> 97
<211> 20
<212> DNA
<213> Artificial Sequence

<220>
<223> Forward Primer for GSTP1 (132 bp amplificate)

<400> 97
gtgtgtaagt ttcgggatcg 20

<210> 98
<211> 20
<212> DNA
<213> Artificial Sequence

<220>
<223> Reverse Primer for GSTP1 (132 bp amplificate)

<400> 98
tccccaacga aacctaaaaa 20

<210> 99
<211> 24
<212> DNA
<213> Artificial Sequence

<220>
<223> Forward Primer for AOX1 (171 bp amplificate)

<400> 99
ttttaattaa ggtttttttc gtcg 24

<210> 100
<211> 21
<212> DNA
<213> Artificial Sequence

<220>

<223> Reverse Primer for AOX1 (171 bp amplificate)

<400> 100
cccgatccat tataatatcc g 21

<210> 101
<211> 23
<212> DNA
<213> Artificial Sequence

<220>
<223> Forward Primer for Thrombomodulin (160 bp amplificate)

<400> 101
tttgtgtttt tttgtttcgg tac 23

<210> 102
<211> 20
<212> DNA
<213> Artificial Sequence

<220>
<223> Reverse Primer for Thrombomodulin (160 bp amplificate)

<400> 102
cacccgacta cgactctacg 20

<210> 103
<211> 20
<212> DNA
<213> Artificial Sequence

<220>
<223> Reverse Primer for Thrombomodulin (159 bp amplificate)

<400> 103
acccgactac gactctacga 20

<210> 104
<211> 21
<212> DNA
<213> Artificial Sequence

<220>
<223> Forward Primer for RASSF1A (86 bp amplificate)

<400> 104
tttagtttgg attttggggg a 21

<210> 105
<211> 21
<212> DNA
<213> Artificial Sequence

<220>
<223> Reverse Primer for RASSF1A (86 bp amplificate)

<400> 105

ctaactttaa acgctaacaa a 21

<210> 106
<211> 19
<212> DNA
<213> Artificial Sequence

<220>
<223> Forward Primer for RASSF1A (82 bp amplificate)

<400> 106
gtttggattt tgggggagc 19

<210> 107
<211> 20
<212> DNA
<213> Artificial Sequence

<220>
<223> Reverse Primer for RASSF1A (82 bp amplificate)

<400> 107
actttaaacg ctaacaaacg 20

<210> 108
<211> 19
<212> DNA
<213> Artificial Sequence

<220>
<223> Forward Primer for RASSF1A (82 bp amplificate)

<400> 108
tttggatttt gggggagcg 19

<210> 109
<211> 20
<212> DNA
<213> Artificial Sequence

<220>
<223> Reverse Primer for RASSF1A (82 bp amplificate)

<400> 109
cgctaacttt aaacgctaac 20

<210> 110
<211> 22
<212> DNA
<213> Artificial Sequence

<220>
<223> Forward Primer for RASSF1A (86 bp amplificate)

<400> 110
tttagtttgg attttggggg ag 22

<210> 111

<211> 23
<212> DNA
<213> Artificial Sequence

<220>
<223> Reverse Primer for RASSF1A (86 bp amplificate)

<400> 111
cgctaacttt aaacgctaac aaa 23

<210> 112
<211> 26
<212> DNA
<213> Artificial Sequence

<220>
<223> Probe for AOX1 (5 CpG) methylated

<400> 112
cgcgctaatt cgaaaacccg aaacga 26

<210> 113
<211> 26
<212> DNA
<213> Artificial Sequence

<220>
<223> Probe for AOX1 (5 CpG) non-methylated

<400> 113
cacactaatt caaaaaccca aaacaa 26

<210> 114
<211> 29
<212> DNA
<213> Artificial Sequence

<220>
<223> Probe for AOX1 (5 CpG) non-methylated

<400> 114
cacactaatt caaaaaccca aaacaaaaa 29

<210> 115
<211> 20
<212> DNA
<213> Artificial Sequence

<220>
<223> Probe for RASSF1A (4 CpG) methylated

<400> 115
cgcgaaccga acgaaaccac 20

<210> 116
<211> 20
<212> DNA
<213> Artificial Sequence

<220>
<223> Probe for RASSF1A (4 CpG) non-methylated

<400> 116
cacaaaccaa acaaaaccac 20

<210> 117
<211> 23
<212> DNA
<213> Artificial Sequence

<220>
<223> Probe for RASSF1A (4 CpG) non-methylated

<400> 117
cacaaaccaa acaaaaccac aaa 23

<210> 118
<211> 26
<212> DNA
<213> Artificial Sequence

<220>
<223> Probe for RASSF1A (4 CpG) non-methylated

<400> 118
aaacacaaac caaacaaaac cacaaa 26

<210> 119
<211> 26
<212> DNA
<213> Artificial Sequence

<220>
<223> Probe for AOX1 (5CpG) methylated

<400> 119
tcgtttcggg ttttcgaatt agcgcg 26

<210> 120
<211> 26
<212> DNA
<213> Artificial Sequence

<220>
<223> Probe for AOX1 (5CpG) non-methylated

<400> 120
ttgttttggg tttttgaatt agtgtg 26

<210> 121
<211> 29
<212> DNA
<213> Artificial Sequence

<220>
<223> Probe for AOX1 (5CpG) non-methylated

<400> 121
ttttgtttt gggtttttga attagtgtg 29

<210> 122
<211> 20
<212> DNA
<213> Artificial Sequence

<220>
<223> Probe for RASSF1A (4CpG) methylated

<400> 122
gtggtttcgt tcggttcgcg 20

<210> 123
<211> 20
<212> DNA
<213> Artificial Sequence

<220>
<223> Probe for RASSF1A (4CpG) non-methylated

<400> 123
gtggttttgt ttggtttgtg 20

<210> 124
<211> 23
<212> DNA
<213> Artificial Sequence

<220>
<223> Probe for RASSF1A (4CpG) non-methylated

<400> 124
tttgtggttt tgtttggttt gtg 23

<210> 125
<211> 26
<212> DNA
<213> Artificial Sequence

<220>
<223> Probe for RASSF1A (4CpG) non-methylated

<400> 125
tttgtggttt tgtttggttt gtgttt 26

<210> 126
<211> 19
<212> DNA
<213> Artificial Sequence

<220>
<223> Forward Primer for Thrombomodulin (125 bp amplificate)

<400> 126
ggtcgattcg tatgttaga 19

<210> 127
<211> 18
<212> DNA
<213> Artificial Sequence

<220>
<223> Reverse Primer for Thrombomodulin(125 bp amplificate)

<400> 127
aaccgtaccg aaacaaaa 18

<210> 128
<211> 19
<212> DNA
<213> Artificial Sequence

<220>
<223> Forward Primer for Thrombomodulin (83 bp amplificate)

<400> 128
tagcggtaag aagtgtttg 19

<210> 129
<211> 18
<212> DNA
<213> Artificial Sequence

<220>
<223> Forward Primer for Thrombomodulin (70 bp amplificate)

<400> 129
tacggttttg tcgtagtg 18

<210> 130
<211> 20
<212> DNA
<213> Artificial Sequence

<220>
<223> Reverse Primer for Thrombomodulin (70 bp amplificate)

<400> 130
cccaaacata ttacccaaac 20

<210> 131
<211> 18
<212> DNA
<213> Artificial Sequence

<220>
<223> Forward Primer for Thrombomodulin (113 bp amplificate)

<400> 131
ggagaggttg tcgttatc 18

<210> 132
<211> 20
<212> DNA

<213> Artificial Sequence

<220>
<223> Reverse Primer for Thrombomodulin (113 bp amplificate)

<400> 132
cccaaacata ttacccaaac 20

<210> 133
<211> 18
<212> DNA
<213> Artificial Sequence

<220>
<223> Reverse Primer for Thrombomodulin (115 bp amplificate)

<400> 133
accccaaaca tattaccc 18

<210> 134
<211> 18
<212> DNA
<213> Artificial Sequence

<220>
<223> Forward Primer for Thrombomodulin (99 bp amplificate)

<400> 134
gtcgagtacg attgtttc 18

<210> 135
<211> 21
<212> DNA
<213> Artificial Sequence

<220>
<223> Reverse Primer for Thrombomodulin (99 bp amplificate)

<400> 135
acgcactatc attaaataac c 21

<210> 136
<211> 18
<212> DNA
<213> Artificial Sequence

<220>
<223> Forward Primer for Thrombomodulin (97 bp amplificate)

<400> 136
cggtggttgt cgatgtta 18

<210> 137
<211> 18
<212> DNA
<213> Artificial Sequence

<220>

<223> Reverse Primer for Thrombomodulin (97 bp amplificate)

<400> 137
ccgcaaccga ataacaac 18

<210> 138
<211> 18
<212> DNA
<213> Artificial Sequence

<220>
<223> Forward Primer for Thrombomodulin (125 bp amplificate)

<400> 138
ttgcggggtt atttaatg 18

<210> 139
<211> 19
<212> DNA
<213> Artificial Sequence

<220>
<223> Reverse Primer for Thrombomodulin (125 bp amplificate)

<400> 139
caaccgaata acaactaca 19

<210> 140
<211> 19
<212> DNA
<213> Artificial Sequence

<220>
<223> Forward Primer for Septin-9 (72 bp amplificate)

<400> 140
gcgattcgtt gtttattag 19

<210> 141
<211> 18
<212> DNA
<213> Artificial Sequence

<220>
<223> Reverse Primer for Septin-9 (72 bp amplificate)

<400> 141
atccgaaata atcccatc 18

<210> 142
<211> 20
<212> DNA
<213> Artificial Sequence

<220>
<223> Forward Primer for Septin-9 (169 bp amplificate)

<400> 142

cggttagttt tgtattgtag 20

<210> 143
<211> 19
<212> DNA
<213> Artificial Sequence

<220>
<223> Forward Primer for Septin-9 (94 bp amplificate)

<400> 143
cggggttgtt ttgtttaag 19

<210> 144
<211> 18
<212> DNA
<213> Artificial Sequence

<220>
<223> Reverse Primer for Septin-9 (94 bp amplificate)

<400> 144
ccaacaccga caatcaaa 18

<210> 145
<211> 21
<212> DNA
<213> Artificial Sequence

<220>
<223> Probe for Thrombomodulin (3CpG) methylated

<400> 145
acgccgataa cgacaacctc t 21

<210> 146
<211> 21
<212> DNA
<213> Artificial Sequence

<220>
<223> Probe for Thrombomodulin (3CpG) non-methylated

<400> 146
aagcagataa agacaacctc t 21

<210> 147
<211> 20
<212> DNA
<213> Artificial Sequence

<220>
<223> Probe for Thrombomodulin (5CpG) methylated

<400> 147
cgccgcgtac aaacgccgaa 20

<210> 148

<211> 20
<212> DNA
<213> Artificial Sequence

<220>
<223> Probe for Thrombomodulin (5CpG) non-methylated

<400> 148
agcagagtac aaaagcagaa 20

<210> 149
<211> 20
<212> DNA
<213> Artificial Sequence

<220>
<223> Probe for Thrombomodulin (5CpG) methylated

<400> 149
aacgcgccgc gtacaaacgc 20

<210> 150
<211> 20
<212> DNA
<213> Artificial Sequence

<220>
<223> Probe for Thrombomodulin (5CpG) non-methylated

<400> 150
aaagagcaga gtacaaaagc 20

<210> 151
<211> 23
<212> DNA
<213> Artificial Sequence

<220>
<223> Probe for Thrombomodulin (3CpG) methylated

<400> 151
cgcaatccgt cgcaaatcta act 23

<210> 152
<211> 23
<212> DNA
<213> Artificial Sequence

<220>
<223> Probe for Thrombomodulin (3CpG) non-methylated

<400> 152
agcaatcagt agcaaatcta act 23

<210> 153
<211> 20
<212> DNA
<213> Artificial Sequence

<220>
<223> Probe for Thrombomodulin (5CpG) methylated

<400> 153
aacgccgacg accaacgccg 20

<210> 154
<211> 20
<212> DNA
<213> Artificial Sequence

<220>
<223> Probe for Thrombomodulin (5CpG) non-methylated

<400> 154
aaagcagaag accaaagcag 20

<210> 155
<211> 20
<212> DNA
<213> Artificial Sequence

<220>
<223> Probe for Thrombomodulin (4CpG) methylated

<400> 155
aaaacgccga cgaccaacgc 20

<210> 156
<211> 20
<212> DNA
<213> Artificial Sequence

<220>
<223> Probe for Thrombomodulin (4CpG) non-methylated

<400> 156
aaaaagcaga agaccaaagc 20

<210> 157
<211> 21
<212> DNA
<213> Artificial Sequence

<220>
<223> Probe for Thrombomodulin (4CpG) methylated

<400> 157
aaaacgccga cgaccaacgc c 21

<210> 158
<211> 21
<212> DNA
<213> Artificial Sequence

<220>
<223> Probe for Thrombomodulin (4CpG) non-methylated

<400> 158
aaaaagcaga agaccaaagc c 21

<210> 159
<211> 25
<212> DNA
<213> Artificial Sequence

<220>
<223> Probe for Septin-9 (4CpG) methylated

<400> 159
cgttaaccgc gaaatccgac ataat 25

<210> 160
<211> 25
<212> DNA
<213> Artificial Sequence

<220>
<223> Probe for Septin-9 (4CpG) non-methylated

<400> 160
agttaacaga gaaatcagac ataat 25

<210> 161
<211> 27
<212> DNA
<213> Artificial Sequence

<220>
<223> Probe for Septin-9 (4CpG) methylated

<400> 161
cgttaaccgc gaaatccgac ataataa 27

<210> 162
<211> 27
<212> DNA
<213> Artificial Sequence

<220>
<223> Probe for Septin-9 (4CpG) non-methylated

<400> 162
agttaacaga gaaatcagac ataataa 27

<210> 163
<211> 21
<212> DNA
<213> Artificial Sequence

<220>
<223> Probe for Septin-9 (2CpG) methylated

<400> 163
aaacgcacgc actcacaaac t 21

<210> 164
<211> 21
<212> DNA
<213> Artificial Sequence

<220>
<223> Probe for Septin-9 (2CpG) non-methylated

<400> 164
aaaagcaagc actcacaaac t 21

<210> 165
<211> 21
<212> DNA
<213> Artificial Sequence

<220>
<223> Probe for Thrombomodulin (3CpG) methylated

<400> 165
agaggttgtc gttatcggcg t 21

<210> 166
<211> 21
<212> DNA
<213> Artificial Sequence

<220>
<223> Probe for Thrombomodulin (3CpG) non-methylated

<400> 166
agaggttgtc tttatctgct t 21

<210> 167
<211> 20
<212> DNA
<213> Artificial Sequence

<220>
<223> Probe for Thrombomodulin (5CpG) methylated

<400> 167
ttcggcgttt gtacgcggcg 20

<210> 168
<211> 20
<212> DNA
<213> Artificial Sequence

<220>
<223> Probe for Thrombomodulin (5CpG) non-methylated

<400> 168
ttctgctttt gtactctgct 20

<210> 169
<211> 20
<212> DNA

<213> Artificial Sequence

<220>
<223> Probe for Thrombomodulin (5CpG) methylated

<400> 169
gcgtttgtac gcggcgcgtt 20

<210> 170
<211> 20
<212> DNA
<213> Artificial Sequence

<220>
<223> Probe for Thrombomodulin (5CpG) non-methylated

<400> 170
gcttttgtac tctgctcttt 20

<210> 171
<211> 23
<212> DNA
<213> Artificial Sequence

<220>
<223> Probe for Thrombomodulin (3CpG) methylated

<400> 171
agttagattt gcgacggatt gcg 23

<210> 172
<211> 23
<212> DNA
<213> Artificial Sequence

<220>
<223> Probe for Thrombomodulin (3CpG) non-methylated

<400> 172
agttagattt gctactgatt gct 23

<210> 173
<211> 20
<212> DNA
<213> Artificial Sequence

<220>
<223> Probe for Thrombomodulin (5CpG) methylated

<400> 173
cggcgttggt cgtcggcgtt 20

<210> 174
<211> 20
<212> DNA
<213> Artificial Sequence

<220>

<223> Probe for Thrombomodulin (5CpG) non-methylated

<400> 174
ctgctttggt cttctgcttt 20

<210> 175
<211> 20
<212> DNA
<213> Artificial Sequence

<220>
<223> Probe for Thrombomodulin (4CpG) methylated

<400> 175
gcgttggtcg tcggcgtttt 20

<210> 176
<211> 20
<212> DNA
<213> Artificial Sequence

<220>
<223> Probe for Thrombomodulin (4CpG) non-methylated

<400> 176
gctttggtct tctgcttttt 20

<210> 177
<211> 21
<212> DNA
<213> Artificial Sequence

<220>
<223> Probe for Thrombomodulin (4CpG) methylated

<400> 177
ggcgttggtc gtcggcgttt t 21

<210> 178
<211> 21
<212> DNA
<213> Artificial Sequence

<220>
<223> Probe for Thrombomodulin (4CpG) non-methylated

<400> 178
ggctttggtc ttctgctttt t 21

<210> 179
<211> 25
<212> DNA
<213> Artificial Sequence

<220>
<223> Probe for Septin-9 (4CpG) methylated

<400> 179

attatgtcgg atttcgcggt taacg 25

<210> 180
<211> 25
<212> DNA
<213> Artificial Sequence

<220>
<223> Probe for Septin-9 (4CpG) non-methylated

<400> 180
attatgtctg atttctctgt taact 25

<210> 181
<211> 27
<212> DNA
<213> Artificial Sequence

<220>
<223> Probe for Septin-9 (4CpG) methylated

<400> 181
ttattatgtc ggatttcgcg gttaacg 27

<210> 182
<211> 27
<212> DNA
<213> Artificial Sequence

<220>
<223> Probe for Septin-9 (4CpG) non-methylated

<400> 182
ttattatgtc tgatttctct gttaact 27

<210> 183
<211> 21
<212> DNA
<213> Artificial Sequence

<220>
<223> Probe for Septin-9 (2CpG) methylated

<400> 183
agtttgtgag tgcgtgcgtt t 21

<210> 184
<211> 20
<212> DNA
<213> Artificial Sequence

<220>
<223> Probe for Septin-9 (2CpG) non-methylated

<400> 184
agtttgtgag tgctgctttt 20

## Beschreibung

Die Erfindung betrifft Verfahren und Mittel zur Diagnostik von Tumoren, insbesondere zur Frühdiagnostik (Vorsorge) und zur Unterscheidung von benignen und malignen Tumoren mittels PCR, insbesondere in Körperflüssigkeiten Für die Prognose von Krebserkrankungen, wie Prostata- und Brustkrebs wie auch anderen soliden Tumoren, ist der Zeitpunkt der Diagnosestellung von entscheidender Bedeutung. So hängen die 5- bzw. 10-Jahresüberlebensraten stark vom Tumorstadium ab, in dem die Erkrankung entdeckt wurde. Eine zu spät gestellte Diagnostik einer Tumorerkrankung ist oftmals mit bereits auftretenden Metastasen assoziiert. Für eine rechtzeitige Diagnostik von malignen Entartungen sind deshalb Biomarker erforderlich, die eine solche Entartung mit hoher diagnostischer Sensitivität und Spezifität frühzeitig anzeigen. Die bisher bekannten und in der klinisch-chemischen Diagnostik eingesetzten Tumormarker haben sich bisher nur für die Verlaufskontrolle von Tumorpatienten nach therapeutischen Interventionen bewährt, sie erlauben jedoch bis auf wenige Ausnahmen keinen Einsatz in der Frühdiagnostik (Screening bzw. Vorsorge) von Tumorerkrankungen. Hier sind die diagnostischen Sensitivitäten und Spezifitäten noch unzureichend, um mit Hilfe dieser Biomarker Tumorerkrankte von Gesunden, insbesondere im frühen Entwicklungsstadium, sicher zu unterscheiden.

Tumorzellen unterscheiden sich neben sequenzabhängigen durch sequenzunabhängige epigenetische Veränderungen der DNA, wozu Hypermethylierungen zählen. Diese Tumorspezifischen Veränderungen in der DNA-Methylierung lassen sich als neue Krebsmarker nutzen (z. B. WO2012007462A1, WO2013064163A1, US20130022974, US20110301050).

Der Nachweis dieser veränderten Methylierungsmuster von selektiven DNA-Abschnitten in Blut-, Urin- oder anderen menschlichen Körperflüssigkeiten bzw. Abstrichen und histologischen Präparaten ist bisher jedoch nur mit einer ungenügenden analytischen Sensitivität möglich. Die Bestimmung des Methylierungsgrades unterschiedlicher Zielgene erfolgt bisher hauptsächlich mit Hilfe PCR-basierter Verfahren, die sich mit Ausnahme der methylierungssensitiven Restriktionsenzymanalyse (MSRE-PCR) einer Bisulfit-Vorbehandlung von genomischer DNA anschließen. Die Nachweisgrenzen der einzelnen Methoden für die Detektion methylierter DNA sind unterschiedlich. Die Direct-BSP (Sequenzierung nach Sanger) weist eine Sensitivität von 10-20 % auf, während Pyrosequencing und MALDI-TOF-Massenspektrometrie-basierte Verfahren Sensitivitäten von ca. 5% erreichen [1,2]. MSP (methylation specific PCR), MethyLight, SMART-MSP (Sensitive Melting Analysis after Real Time-Methylation Specific PCR) und MS-HRM (methylation-sensitive high-resolution melting) verfügen über eine Nachweisempfindlichkeit zwischen 0,1 %-1,0 % [3-6]. Redshaw et al vergleichen fünf Methoden zur Quatifzierung der DNA-Methylierung [21]. Als ein wesentlicher Nachteil bisheriger PCR-basierter Methoden wird die sogenannte PCR-Bias diskutiert, ein Phänomen, wodurch methylierte und nicht-methylierte DNA-Stränge mit unterschiedlicher Effizienz vervielfältigt werden [3,4, 23].

Ein weiteres Problem bisheriger Nachweisverfahren für DNA-Methylierungen stellen falsch-positive Ergebnisse dar, welche aufgrund von unvollständiger Bisulfit-Umwandlung und unspezifischem Primer-Annealing insbesondere bei Verwendung methylspezifischer Primer entstehen können [4]. Außerdem ist mit keiner der genannten Methoden ein sensitiver und vor allem quantitativer Nachweis heterogen methylierter DNA-Fragmente, sogenannte Epi-Allele, möglich. Eine neue Technik stellt die digitale PCR dar (WO2012092259A1). Bei dieser Technik tritt keine PCR-Bias auf, da jedes DNA-Molekül in einem eigenen Reaktionskompartiment vervielfältigt wird [7, 8]. Abhängig von der zur Verfügung stehenden DNA-Menge und dem Assay-Design wurden für die dPCR Sensitivitäten von bis zu 0,001 % beschrieben [9]. Ein weiterer Vorteil dieser Technik beruht darin, dass auch ohne Verwendung von Kalibratoren absolute Werte erzielt werden [9].

WO2013192351A1 beschreibt ein Verfahren zum Mixen von Droplets. WO2013033714A1 beschreibt einen speziellen digitalen PCR Assay.

Ein kommerzieller Test, der auf dem Nachweis zellulärer epigenetischer Veränderungen wie der DNA-Methylierung als frühes und typisches Merkmal maligner Veränderungen beruht, existiert bisher nur für die Diagnostik des kolorektalen Karzinoms mit der Bestimmung des methylierten *SEPT9* [10], von Gliomen durch *MGMT* und Lungen-Karzinomen durch *SHOX2*-Bestimmungen [11]. Der SEPT9-Test (Epigenomics AG, Berlin, DE) weist eine diagnostische Sensitivität von 72% und eine diagnostische Spezifität von 90% beim Nachweis eines Kolonkarzinoms auf, d.h. 10% der untersuchten Individuen wird ein pathologischer Befund zugeordnet, obwohl kein Kolonkarzinom vorliegt [10]. Okino ST et al. postulieren, dass Veränderungen im Methylierungsmuster des GSTP1_Promoters mit dessen Inaktivierung bei Prostatata Krebs assoziert sind [22]. Für das GS*TP1*-Gen ist der LightMix Kit GSTP1 (Epigenomics, Berlin, DE) kommerziell erhältlich, doch auf Grund einer fehlenden hohen analytischen Sensitivität fand dieser Test in Verbindung mit der PSA-Bestimmung bisher keine breite klinisch-chemische Anwendung in der Diagnostik von Prostatakarzinomen. Der von MDxHealth vertriebene ConfimMDx-Test zum Nachweis bzw. Ausschluss eines Prostatakarzinoms in Prostata-Gewebebiopsieproben weist eine diagnostische Sensitivität von 68% und eine diagnostische Spezifität von 64% für den Nachweis von Prostatatumorzellen in den untersuchten Geweben auf, d.h. für 36% der untersuchten Gewebeproben werden pathologische Befunde erstellt, obwohl kein Prostata-Karzinom vorliegt [12]. In WO2013041731A1 werden mehrere potentiele Markergene zur Unterscheidung zwischen gutartigen und bösartigen Prostata-Tumoren offenbart. In US20100233707A1 wird ein Test zur Untersuchung von Lungenkrebs durch Detektion des Methylierungsmusters des BAX Promoters beschrieben.

Wie wichtig die Etablierung neuer Biomarker und die Bereitstellung entsprechender kommerzieller Testkits z. B. für die Diagnostik des Prostatakarzinoms (PCa) ist, zeigt sich in der hohen Anzahl (mehr als 150.000) der allein in Deutschland jährlich durchgeführten Prostatabiopsien, die auf Grund von PSA-Bestimmungen indiziert sind und bei denen nur in ca. 25% der untersuchten Fälle Tumoren nachgewiesen werden [13, 14]. Die Prostatastanzbiopsie stellt ein invasives diagnostisches Verfahren dar, das in etwa 5 % der durchgeführten Biopsien mit behandlungsbedürftigen Nebenwirkungen wie Blutungen und Entzündungen einhergeht [13, 14]. Zudem werden nicht alle PCa mit Hilfe einer einmaligen Gewebebiopsie erkannt, so dass Wiederholungsuntersuchungen erforderlich sind, auch schließt ein Negativbefund das Vorliegen eines PCa nicht sicher aus [13, 14].

Aufgabe der Erfindung ist es ein verbessertes Verfahren zur Diagnostik von Tumoren sowie ein Kit zu dessen Durchführung anzugeben, welches sich insbesondere zur Frühdiagnostik (Vorsorge) und zur Unterscheidung von benignen und malignen Tumoren mittels PCR, insbesondere in Körperflüssigkeiten, eignet.

Die Aufgabe wird gelöst durch ein Verfahren gemäß Anspruch 1 zur Diagnose einer Tumorerkrankung in einer isolierten Probe mit den Schritten

a) Bisulfit-Umwandlung der DNA in der Probe (Umwandlung nicht-methylierter Cytosine in Uracil),

b) (Prä-)Amplifikation von methylierten und nicht-methylierten DNA-Sequenzen mittels PCR, wobei bevorzugt die methylierten DNA-Sequenzen (wenn sie Tumor-spezifisch sind) stärker amplifiziert werden als die nicht-methylierten DNA-Sequenzen bzw. alternativ die nicht-methylierten DNA-Sequenzen (wenn sie Tumor-spezifisch sind) gegenüber methylierter DNA-Sequenzen und nachfolgend

c) Quantifizierung der prä-amplifizierten methylierten und nicht-methylierten DNA mittels digitaler PCR (dPCR), wobei in der dPCR die eingesetzte Anzahl von prä-amplifizierten DNA-Kopien bevorzugt oberhalb der normalen Poisson-Verteilung liegt.

Der Schritt b) beinhaltet eine Präamplifikation genomischer DNA mittels einer optimierten Bias-basierten PCR, wobei die Bias bevorzugt zugunsten der methylierten oder alternativ zugunsten der nicht-methylierten Sequenzen besteht (je nach der Spezifität für Tumor-DNA). Dieser Schritt wird daher nachfolgend auch Bias-basierte PCR-Amplifikation (BBPA) genannt. In diesem Schritt werden genomische DNA-Sequenzen, die bekanntermaßen in bösartigen Tumoren methyliert sind (nachfolgend Zielgene oder Zielsequenzen genannt) mittels spezifischer Primerpaare jeweils so amplifiziert, dass auch korrespondierende nicht-methylierte Sequenzen (DNA aus gesunden Zellen) mitamplifiziert werden. Überraschend hat sich gezeigt, dass dadurch gegenüber einer (herkömmlichen) methyl-spezifischen PCR (MS-PCR) mit methyl-spezifischen Primern (MSP) im erfindungsgemäßen Verfahren die Anzahl falsch positiver Ergebnisse deutlich reduziert wird. Für jedes Primerpaar wird dazu bevorzugt zunächst die Bias in Abhängigkeit der $MgCl_2$-Konzentration, Annealingtemperatur und Zyklenanzahl bestimmt. Auf diese Weise werden bei der Unterscheidung zwischen Gesund und Tumorerkrankt deutlich höhere diagnostische Spezifitäten erreicht, ohne dass die analytische Sensitivität vermindert wird. Eine hohe diagnostische Spezifität, d.h. eine niedrige Rate von falsch-positiven Befunden, ist entscheidend, ob ein Testverfahren für ein Screening bzw. Vorsorge von Tumorerkankungen eingesetzt werden kann. Vorteilhaft wird im erfindungsgemäßen Verfahren auch teilweise-methylierte DNA (sogenannte heterogen methylierte Epi-Allele) mit amplifiziert. In Schritt b) (Prä-Amplifikation) werden die Reaktionsbedingungen wie $MgCl_2$-Konzentration und Annealingtemperatur bevorzugt so gewählt, dass unabhängig davon, wieviel nicht-methylierte Sequenzen in der zu untersuchenden Probe enthalten sind, zum überwiegenden Anteil methylierte Sequenzen vervielfältigt und bei Abwesenheit methylierter DNA-Sequenzen ausschließlich nicht-methylierte Sequenzen vervielfältigt werden.

Der Begriff Zielgen (target of interest) oder Zielsequenz schließt dabei auch regulatorische Sequenzen mit ein, die außerhalb eines ORFs (open reading-frame) liegen.

Vorteilhaft hat sich gezeigt, dass im erfindungsgemäßen Verfahren die bevorzugte Präamplifikation methylierter Sequenzen auch in Proben möglich ist, in denen eine hohe Hintergrund-DNA (nicht-methylierte DNA) vorliegt. Diese stört im erfindungsgemäßen Verfahren nicht. Damit eignet sich das erfindungsgemäße Verfahren auch für die Analyse von Tumor-DNA in Körperflüssigkeiten, Abstrichen oder Gewebeproben (free circulating Tumor DNA - fc-Tumor DNA).

In der Regel sind die methylierten DNA-Sequenzen Tumor-spezifisch und werden daher in Schritt b) stärker amplifiziert als die nicht-methylierten DNA-Sequenzen. Dies ist z. B. bei den nachfolgend bevorzugt untersuchten Zielgenen PLA2R1, RASSF1A, GSTP1, AOX1, SERPINE-1 und Thrombomodulin der Fall. Es gibt allerdings auch Fälle, wie z.B. beim MGMT-Gen beim Glioblastom [15], in denen die nicht-methylierten DNA-Sequenzen Tumor-spezifisch sind und die methylierten DNA-Sequenzen in gesundem Gewebe vorkommen. In diesen Fällen werden in Schritt b) bevorzugt die nicht-methylierten DNA-Sequenzen stärker amplifiziert als die methylierten DNA-Sequenzen

In Schritt c) erfolgt eine Quantifizierung mittels digitaler PCR - nachfolgend auch dPCR genannt. In den bekannten Methoden der digitalen PCR erfolgt eine Grenzverdünnung der eingesetzten DNA derart, dass in einer maximalen Anzahl von Kompartimenten kein oder genau ein DNA-Molekül vorliegt (Poisson-Verteilung). Die Erfinder haben nun überraschend herausgefunden, dass eine Erhöhung der in der dPCR eingesetzten DNA-Menge über die Poissonverteilung hinaus (z. B. werden bei 10.000 Kompartimenten mehr als 80.000 DNA-Kopien in der dPCR, d. h. mit einem KPK [Kopien pro Kompartiment]-Wert > 8 analysiert), die Spezifität und die Unterscheidung zwischen gesund und bösartiger Tumorerkrankung deutlich verbessert. Nach dem Stand der Technik ist für die dPCR eine Poissonverteilung vorliegend, wenn die KPK < 8 beträgt, da ansonsten keine Kompartimente ohne DNA-Kopien als Grundlage für die Berechnungen vorliegen [9].

Durch die Kombination der Schritte b) und c) - nachfolgend auch BBPA-dPCR genannt - werden einerseits vorteilhaft deutlich höhere analytische und diagnostische Sensitivitäten erreicht, andererseits ermöglicht das erfindungsgemäße Verfahren überraschend eine deutlich zuverlässigere Aussage, ob ein bösartiger Tumor vorliegt oder nicht. Damit eignet sich das Verfahren für das Früherkennungsscreening (Vorsorge). Weiter vorteilhaft ermöglicht das erfindungsgemäße Verfahren eine Unterscheidung gutartiger (benigner) von bösartigen (malignen) Tumoren und den Nachweis einer minimalen Resterkrankung (MRD) und damit der Therapieüberwachung und Verlaufskontrolle. Das Prinzip des erfindungsgemäßen Verfahrens ist anhand von Ausführungsformen in Fig. 1 zusammengefasst dargestellt.

Bei Voruntersuchungen der Erfinder stellte sich überraschend heraus, dass bei Wahl einer optimalen Annealingtemperatur in der BBPA der in der anschließenden dPCR bestimmte Methylierungsgrad der untersuchten Zielgene in Proben gesunder Probanden mit steigender PCR-Zyklenanzahl der BBPA in Richtung 0 %, der von Patientinnen mit Mammakarzinomen und Patienten mit Prostatakarzinomen in Richtung 100% sich verschob, wenn bei einer entsprechenden Annealingtemperatur eine passende $MgCl_2$-Konzentration und Zyklenanzahl in der BBPA gewählt wurde (Fig. 2-4, 7 und 8, Tabellen 2-5). Dieses unerwartete Phänomen ist noch nicht vollständig erklärt, doch kann dieses Phänomen für eine deutlich verbesserte Unterscheidung zwischen Gesund, d. h. ohne fc-Tumor-DNA-Nachweis und Krank, d. h. mit entsprechendem Nachweis von fcT-DNA verwendet werden. Solch eine stringente Unterscheidung gestattet im Prinzip eine Ja/Nein-Antwort auf die Frage, ob eine Tumorspezifische DNA und damit eine Tumorerkrankung vorliegt oder nicht. Dieses Verfahren ermöglicht prinzipiell den spezifischen Nachweis nur eines einzelnen Tumor-DNA-Moleküls vor einem großen Hintergrund normaler DNA.

Umso höher der Anteil methylierter Sequenzen, umso höher ist die Wahrscheinlichkeit, dass eine bösartige Tumorerkrankung, insbesondere im fortgeschrittenen Stadium, vorliegt.

Nach dem Stand der Technik sind die digitale PCR allein als auch die bisher bekannten PCR-basierten Techniken (MS-qPCR oder PCR mit anschließender Schmelzkurvenanalyse [MS-HRM]) allein nicht in der Lage, Tumorerkrankte sicher von Gesunden (ohne Tumorerkrankung) an Hand von Proben aus Körperflüssigkeiten ("liquid-biopsy"-Untersuchungen) zu unterscheiden. So kann bei Ergebnissen, d.h. dem Anteil von fcT-DNA im Verhältnis zu dem Anteil normaler Wildtyp-DNA zwischen Gesunden und Tumorerkrankten, die dicht beieinander liegen und nicht sicher unterschieden werden können, mittels MS-HRM oder dPCR allein nicht weiter differenziert werden mit Ausnahme, mehr DNA in die Untersuchungen einzusetzen. Im Fall der Nutzung von *Liquid-Biopsy*-Materialien, wie z.B. Serum, Plasma, Urin, Liquor cerebrospinalis oder Abstrichen ist das jedoch oftmals nicht möglich. Im Gegensatz dazu liefert die erfindungsgemäße BBPA-dPCR-Technik die Möglichkeit durch Erhöhung der Zyklenanzahl in der BBPA die Stringenz bei der Unterscheidung zwischen Gesund und Krank entsprechend zu erhöhen. Das ist besonders bedeutsam bei der Unterscheidung benigner Hyperplasien, z. B. der benignen Hyperplasie der Prostata (BPH) und malignen Erkrankungen wie dem Prostatakarzinom, wie in den Ausführungsbeispielen an Hand von Zellkulturen und Serumproben von Patienten mit Prostatakarzinom gezeigt werden konnte.

Bevorzugt werden in Schritt b) die methylierten und nicht-methylierten DNA-Sequenzen der Zielgene mit Hilfe einer entsprechend hohen Anzahl von PCR-Zyklen (bevorzugt 10 bis 50 Zyklen) vervielfältigt und anschließend direkt oder nach einer geringfügen Vorverdünnung der Amplifikate im Fall einer hohen Zyklenanzahl bei der BBPA in der dPCR (Schritt c) quantifiziert.

Nachfolgend werden die einzelnen Schritte des Verfahrens und bevorzugte Ausgestaltungen näher erläutert: Vor **Schritt a)** erfolgt bevorzugt eine DNA-Isolierung (Schritt a')) mit bekannten Methoden. Die Durchführung der Bisulfitumwandlung ist dem Fachmann ebenfalls bekannt. Für beide Schritte kann sich der Fachmann kommerziell erhältlicher Kits bedienen.

Die isolierte Probe kann prinzipiell eine Gewebeprobe sein. Vorteilhaft eignet sich das erfindungsgemäße Verfahren aber insbesondere zum Nachweis von methylierter Tumor-DNA in Körperflüssigkeiten ("liquid biopsy"), wie z. B. Vollblut, Serum, Plasma, Urin, Liquor (cerebrospinal fluid), Sputum, Bronchiallavage, Spermaflüssigkeit, Brustdrüsensekret, Scheidensekret (Abstrich) oder Lymphe, besonders bevorzugt Serum, Plasma oder Urin.

In **Schritt b)** (BBPA) werden methylierte und korrespondierende nicht-methylierte DNA-Sequenzen desselben Genabschnittes mittels PCR so amplifiziert, dass die methylierten DNA-Sequenzen (wenn sie spezifisch für Tumoren sind) oder nicht-methylierte DNA-Sequenzen (wenn sie spezifisch für Tumoren sind) stärker amplifiziert werden.

Entgegen dem Stand der Technik, in dem Primerpaare mit maximal 2 CpG-Stellen, in einzelnen Fällen mit 3 CpG-Stellen empfohlen wird, die sich alle so nah wie möglich am 5'-Ende, nicht aber am 3'-Ende der Primersequenzen befinden sollen [4, 16-19], können erfindungsgemäß, insbesondere in Kombination mit geeigneten $MgCl_2$-Konzentrationen und Annealingtemperaturen, Primer in der Prä-Amplifikation eingesetzt werden, die zusammen bis zu sieben CpG-Stellen in ihren Sequenzen enthalten. Die CpG-Stellen können dabei über die gesamte Primersequenz verteilt sein, bevorzugt in der Nähe des 3'-Endes bis hin, dass ein Cytosin einer CpG-Dinukleotidsequenz sich direkt am 3'-Ende befindet. Auf diese Weise können Tumor-DNA deutlich sensitiver und gleichzeitig spezifischer nachgewiesen werden, d.h. ohne dass falsch positive Signale verstärkt entstehen, auch wenn ein hoher Anteil normaler DNA (Wildtyp-DNA) in der zu untersuchenden Probe vorliegt, was in menschlichen Körperflüssigkeiten, Abstrichen oder Gewebeproben häufig der Fall ist. Darüber hinaus lassen sich auf diese Weise auch Primer für CpG-reiche Gensequenzen konstruieren, mit deren Hilfe diese Gensequenzen ansonsten hätten nicht untersucht werden können.

Da die Primer und insbesondere auch die Reaktionsbedingungen (Annealingtemperatur immer bei entsprechend optimierter $MgCl_2$-Konzentration im Reaktionspuffer) so ausgewählt sind, dass sie sowohl methylierte als auch nicht-methylierte DNA-Sequenzen amplifizieren, d.h. sie sind methylierungsunabhängig (MIP, methylation independent primer), kommt es bei den optimierten Reaktionsbedingungen zu einer selektiven Konkurrenzreaktion der Primer um methylierte und nicht-methylierte DNA-Moleküle, wodurch im Gegensatz zur Nutzung methylierungsspezifischer Primer (MSP) das Auftreten falsch positiver Signale vermieden wird. Neben der erhöhten Spezifität können auf diese Weise methylierte DNA-Kopien vor einem großen Hintergrund nicht-methylierter DNA-Kopien wesentlich sensitiver und spezifischer bestimmt werden. Zudem werden neben homogen methylierten auch heterogen methylierte DNA-Fragmente (sogenannte Epi-Allele) quantifiziert, wodurch eine stringentere Differenzierung zwischen Tumorerkrankung und Gesund möglich wird.

Bevorzugt werden die PCR-Bedingungen (insbesondere Primersequenzen, Magnesiumkonzentration, Annealingtemperatur und insbesondere auch die Zyklenanzahl) so eingestellt, dass die Bias zugunsten der methylierten DNA-Sequenzen optimiert wird, ohne dabei nicht-methylierte DNA-Sequenzen nicht mit zu amplifizieren.

Bevorzugt werden in Schritt b) für die PCR Magnesiumkonzentrationen (Endkonzentrationen) von 0,5 bis 15 mmol/l, vorzugsweise 1 bis 10 mmol/l, besonders bevorzugt 2 bis 5 mmol/l, insbesondere 2 bis 4 mmol/l, besonders bevorzugt 2,5 bis 3,5 mmol/l gewählt.

Die Erfinder haben herausgefunden, dass die Bias durch eine hohe Annealingtemperatur und hohen 5'-CG-3'-Gehalt zugunsten der methylierten DNA-Sequenzen verschoben wird. Vorteilhaft kann die Spezifität und Sensitivität des Verfahrens bei einer entsprechend aufeinander abgestimmten Wahl der Annealingtemperatur, $MgCl_2$-Konzentration, Zyklenanzahl und des Primerdesigns so erhöht werden kann, dass ein Tumorfrühscreening (Vorsorge) und der Nachweis einer minimalen Resterkrankung (MRD) anhand einer *Liquid Biopsy* möglich ist. Sollen methylierte gegenüber nicht-methylierten Sequenzen bevorzugt prä-amplifiziert werden, werden bevorzugt neben den 5'-CG-3'-enthaltenen Primersequenzen hohe Annealingtemperaturen und erniedrigte $MgCl_2$-Konzentrationen (hier allerdings nur soweit erniedrigt, dass nicht-methylierte DNA-Sequenzen noch geringfügig mit prä-amplifiziert und dadurch falsch-positive Signale vermieden werden) gewählt werden. Sollen nicht-methylierte gegenüber methylierten Sequenzen bevorzugt prä-amplifiziert werden, können neben dem Design der Primer (möglichst keine oder maximal zwei 5'-CG-3'-enthaltene Sequenz) die Annealingtemperatur und $MgCl_2$-Konzentration nach Voruntersuchungen, wie sie im Folgenden beschrieben sind, entsprechend verändert werden.

Die Primer werden in Schritt b) bevorzugt so gewählt, dass sie keine bis sieben 5'-CG-3' Dinukleotidsequenzen pro Primerpaar, bevorzugt zwei bis sechs, vorzugsweise drei bis fünf, besonders bevorzugt ein bis maximal drei, weiter bevorzugt ein oder zwei 5'-CG-3' Dinukleotidsequenzen pro Primerpaar, enthalten.

Überraschend hat sich auch gezeigt, dass die diagnostische Sensitivität des Verfahrens weiter erhöht wird, d.h. die Anzahl der pathologischen Befunde bei tatsächlich vorliegender Tumorerkrankung steigt, wenn pro Zielgen 2 oder mehrere (unterschiedliche) Primerpaare in der Prä-Amplifikation separat oder gleichzeitig eingesetzt werden, die bevorzugt alle die Sequenz einschließen, die durch die Sonden in der dPCR detektiert werden.

Die Annealingtemperaturen sind vorzugsweise über 40°C, insbesondere über 45°C, und liegen bevorzugt zwischen 50 und 72°C, vorzugsweise 53 bis 70°C, besonders bevorzugt bis 63°C. Die optimalen Annealingtemperaturen werden wie auch die $MgCl_2$-Konzentrationen und die optimalen Zyklenanzahl bevorzugt für jedes Primer-Paar empirisch ermittelt.

Die Optimierung der Bias zugunsten der methylierten DNA-Sequenzen erfolgt bevorzugt durch empirische Anpassung der Primersequenzen und Annealingtemperaturen, bevorzugt gemeinsam mit der $MgCl_2$-Konzentration, entlang der oben genannten Auswahlregeln. Für jedes Zielgen bzw. jede Zielsequenz werden die PCR-Bedingungen (insbesondere Primerauswahl und Annealingtemperatur gemeinsam mit der $MgCl_2$-Konzentration und Zyklenanzahl) bevorzugt mit einer Probe mit bekanntem Verhältnis methylierte DNA/ nicht-methylierte DNA bestimmt (Fig. 34-37 und Tabellen 21 und 22). Erreicht man bei Primern ohne 5'-CG-3' Dinukleotidsequenz keine Bias oder nur bei hohen Annealingtemperaturen (z. B. über 70°C) wird bevorzugt eine 5'-CG-3' Dinukleotidsequenz eingefügt, wenn das nicht ausreicht, insgesamt maximal zwei, drei oder vier 5'-CG-3' Dinukleotidsequenzen (d. h. jeweils eine 5'-CG-3' Dinukleotidsequenz in die Forward- und Reverse-Primer-Sequenz oder zwei bis vier 5'-CG-3' Dinukleotidsequenz in die Forward- oder Reverse-Primer-Sequenz eingefügt). Bei niedrigen $MgCl_2$-Konzentrationen (z.B. unterhalb von 1,0 mmol/l) werden bevorzugt drei 5'-CG-3' Dinukleotidsequenzen eingefügt, wenn das nicht ausreicht, insgesamt maximal acht, bevorzugt maximal sieben, 5'-CG-3' Dinukleotidsequenzen pro Primerpaar. Dabei spielt es keine Rolle, dass die 5'-CG-3' Dinukleotidsequenzen sich am 5'-Ende der Primer befinden, wie es in der bisherigen Literatur empfohlen wurde [4, 16-19].

Ganz im Gegenteil lässt sich die Sensitivität der Tests an Proben mit einem hohen Hintergrund normaler Wild-Typ-DNA mit Primern erhöhen, bei denen die 5'-CG-3' Dinukleotidsequenzen sich besonders in der Nähe bzw. direkt am 3'-Ende der Primer befinden [Fig. 38-45, Tabellen 27-30]. Neben einer höheren analytischen Sensitivität lassen sich auf diese Weise vorteilhaft auch Primer für Genabschnitte konstruieren, die durch eine hohe Dichte an 5'-CG-3' Dinukleotidsequenzen charakterisiert sind und die sich bisher - entsprechend den bisherigen Literaturempfehlungen [4, 16-19] - einer Untersuchung entzogen haben.

Die Anzahl der PCR-Zyklen im Schritt b) ist insbesondere abhängig von der Ausgangskonzentration der DNA in der zu untersuchenden Probe. Je nach zur Verfügung stehender DNA-Menge werden Zyklenzahlen von 5 bis 50, bevorzugt 10 bis 50, besonders bevorzugt mindestens 15 und/oder bis 40 gewählt.

Das erfindungsgemäße Verfahren ist prinzipiell für jedes zu untersuchende Ziel-Gen (*target of interest*) anwendbar. Bevorzugt werden im erfindungsgemäßen Verfahren DNA-Sequenzen von drei bis fünf, bevorzugt bis sechs Zielgenen auf den Methylierungsgrad analysiert. Bevorzugt wird dazu Schritt b) als Multiplex-PCR durchgeführt, d. h. die Primerpaare für die Prä-Amplifikation der Zielsequenzen werden so aufeinander abgestimmt, dass sie eine Annealingtemperatur in der gleichen Größenordnung aufweisen und nicht miteinander hybridisieren. Bevorzugt werden die Primerpaare für die Präamplifikation der Zielsequenzen in Schritt so gewählt, dass bei der gleichen Annealingtemperatur, $MgCl_2$-Konzentration und Zyklenanzahl die höchsten analytischen und diagnostischen Sensitivitäten und Spezifitäten resultieren.

Vorteile des 2-stufigen Verfahrens (BBPA-dPCR) bestehen darin, dass das Verfahren eine wesentlich höhere diagnostische Sensitivität und insbesondere auch Spezifität gegenüber bisherigen Verfahren wie dPCR, MSP oder MS-HRM aufweist, wodurch prinzipiell ein einzelnes Tumor-DNA-Molekül vor einem großen Hintergrund normaler DNA (Wildtyp-DNA) detektiert werden kann. Dadurch können neben Plasma auch Serumproben untersucht werden, ohne eine spezielle Prä-Analytik beachten zu müssen. Die analytische und diagnostische Sensitivität des neuen Verfahrens ist letztendlich nur dadurch limitiert, dass in der zu untersuchenden Probe tatsächlich ein einzelnes Tumor-DNA-Molekül mit der zu untersuchenden Zielgensequenz vorliegt und in die Prä-Amplifikation überführt werden kann.

Durch die gleichzeitige Prä-Amplifikation nicht-methylierter DNA-Fragmente, wenn auch durch eine deutlich geringere Effizienz gegenüber methylierten DNA-Sequenzen oder, wie sich überraschend zeigte, mit einer höheren Effizienz gegenüber methylierten DNA-Sequenzen in Proben von Probanden ohne Tumorerkrankung, werden falsch positive Signale vermieden. Dies beruht vermutlich auf einer Konkurrenzreaktion der Primer um die Zielsequenzen. Des Weiteren können trotz der PCR-Bias die ermittelten relativen Methylierungsgrade der Zielgene zwischen einzelnen Patientenproben miteinander verglichen werden, da die Höhe der Bias bei konstanten PCR-Bedingungen (konstante Annealingtemperatur, $MgCl_2$-Konzentration und Zyklenanzahl) für jede untersuchte Probe gleich hoch ist. Durch die bereits genannten Vorteile und der erzielten hohen diagnostischen Spezifitäten des neuen Verfahrens ist auch eine Unterscheidung zwischen benignen Hyperplasien und malignen Erkrankungen möglich. Bevorzugt ist das in der Differentialdiagnostik der benignen Prostatahyperplasie (BPH), Prostatitis und Prostatakarzinom (PCa)-Erkrankungen von Interesse, da auf Grund erhöhter PSA-Werte (der kritische Bereich liegt zwischen 2,0-15,0 mg/ml, Referenzbereich liegt bei < 2,5-4,0 mg/ml) die Indikation für eine Prostatagewebebiopsie gestellt werden muss.

Umso höher der Anteil methylierter Sequenzen (insbesondere wenn diese spezifisch für Tumor-DNA sind), umso höher ist die Wahrscheinlichkeit, dass eine fortgeschrittene bösartige Tumorerkrankung vorliegt. Sind nicht-methylierte Sequenzen Tumor-spezifisch, verhält es sich genau umgekehrt.

In Abhängigkeit von den gewählten Primern und PCR-Bedingungen (Annealingtemperatur, Magnesiumchloridkonzentration und Anzahl der Zyklen) in der Prä-Amplifikation werden die prozentualen Anteile (fraktionale Häufigkeit) an methylierten Sequenzkopien bestimmt, die als Referenzbereich (Normalbereich Gesunder) für die Abwesenheit einer malignen Erkrankung sprechen. In den Ausführungsbeispielen sprechen z. B. bevorzugt jeweils ein Anteil methylierte Sequenzen > 2 % bei PLA2R1 (bevorzugt 168 bp Fragment), > 0,1 % bei RASSF1A (bevorzugt 117 bp Fragment), > 2 % bei GSTP1 (bevorzugt 120 bp Fragment) und > 0,05 % bei GSTP1 (bevorzugt 116 bp Fragment) für eine bösartige Tumorerkrankung. Diese Grenzen (*cut-off*-Werte) zwischen Gesund und Krank werden für jede Zielsequenz (und konkreten PCR-Bedingungen) durch Mitführen von Kontrollen für Gesund (z. B. DNA isoliert aus gesunden Epithelzellen der Prostata (PrEC), der Mamma (HMEC, MCF10A-Zelllinie) oder anderen zu untersuchenden Geweben und Krank (z. B. DNA isoliert aus maligen LNCAP-, PC-3 und DU145-Zellen der Prostata, aus malignen MCF-7, Cal-51, UACC-812, BT-474, MDA-MB-453 und MDA-MB231-Zellen der Mamma) bestimmt.

Als tumorerkrankte Patienten werden bevorzugt Patienten klassifiziert, die für mindestens ein, bevorzugt zwei, oder auch mehr Zielgensequenzen erhöhte Werte für homogen methylierte oder heterogen methylierte Epi-Allele aufweisen.

In **Schritt c)** erfolgt eine Quantifizierung mittels digitaler PCR, wobei im Gegensatz zur herkömmlichen dPCR die eingesetzte Anzahl von prä-amplifizierten DNA-Kopien außerhalb der normalen Poisson-Verteilung liegt.

Überraschend führt eine derartige "Überladung" der digitalen PCR mit DNA-Kopiemengen über einen KPK-Wert von 8 hinaus zur Erhöhung der Spezifität und damit deutlich verbesserten Unterscheidung zwischen Gesund (ohne Tumorerkrankung) und bösartiger Tumorerkrankung (siehe Tabelle 6 gemeinsam mit Tabelle 4 und 5). So ergab die in die dPCR eingesetzte Probenmenge gesunder Probanden einen KPK-Wert von 560 und für die an Prostatakarzinomen erkrankten Patienten einen KPK-Wert von 1938, bei der die beste Unterscheidung (d.h. ein möglichst niedriger Wert für Gesund bei möglichst hohem Wert für Krank) zwischen Gesund und Krank erzielt wurde (Tabelle 5).

Bevorzugt wird dazu die in Schritt b) präamplifizierte DNA unverdünnt oder leicht verdünnt (z. B. bis zu 1:1000 nach 50 Zyklen) auf die Kompartimente für die digitale PCR verteilt. Bevorzugt wird eine DNA-Menge in die digitale PCR eingesetzt, so dass in jedem Kompartiment mindestens ein DNA-Molekül vorliegt, bevorzugt mindestens fünf Moleküle vorliegen. Bevorzugt liegt die eingesetzte DNA-Menge doppelt so hoch wie die nach der Poisson-Verteilung und -Statistik erlaubt ist. Bevorzugt liegen pro Kompartiment gemittelt mindestens 10, vorzugsweise mindestens 20, weiter bevorzugt mindestens 50 DNA-Moleküle, besonders bevorzugt mindestens 100 DNA-Moleküle vor. Diese Kopienzahl pro Kompartiment (KPK) bezeichnet dabei die gemittelte Anzahl (arithmetisches Mittel) dsDNA-Moleküle pro Kompartiment.

Die in der dPCR einzusetzende Kopienzahl der prä-amplifizierten DNA lässt sich wie folgt berechnen:

$$\text{Kopienanzahl} = \text{KPK} * \text{Anzahl der Kompartimente:}$$

Demnach ergibt sich beispielweise bei einer bevorzugten KPK von 8 und 5.000 Kompartimenten eine benötigte DNA-Kopienanzahl von 40.000, bei 100.000 Kompartimenten entsprechend 800.000. Bei einer weiter bevorzugten KPK von 50 müssen bei 100.000 Kompartimenten 5 x $10^6$ Kopien in die dPCR eingetragen werden. Bei Verwendung z.B. des RainDrop Digitalen Systems (RainDance-Technologies) mit bis zu 1 Million Kompartimenten (Tröpfchen) werden somit bei einer bevorzugten KPK von 10 in der dPCR 1 x $10^7$ DNA-Kopien und bei einer weiter bevorzugten KPK von 50 in der dPCR 5 x $10^7$ DNA-Kopien eingesetzt.

Die Quantifizierung der methylierten und nicht-methylierten DNA-Sequenzen erfolgt in Schritt c) mittels Sonden, die spezifisch mit methylierten bzw. den korrespondieren nicht-methylierten Bereichen der prä-amplifizierten Zielsequenzen hybridisieren.

Bevorzugt weisen die Sonden für die methylierten DNA-Sequenzen insgesamt für jedes Zielgen mindestens drei, bevorzugt mindestens vier, und bis zu acht, bevorzugt bis zu sieben, 5'-CG-3' Dinukleotide auf. Die Sonden für die nicht-methylierten DNA-Sequenzen weisen bevorzugt insgesamt für jedes Gen mindestens drei, bevorzugt vier, bis zu acht, bevorzugt bis zu sieben, 5'-CA-3' Dinukleotide auf. Alternativ kann mit den Sonden der komplementäre Strang im amplifizierten DNA-Doppelstrangmolekül detektiert werden. Dies erfolgt entweder separat oder gemeinsam mit der Sonde für den kodierenden Strang (*coding strand*). Zur Detektion des komplementären Strang weisen die Sonden für die nicht-methylierten DNA-Sequenzen bevorzugt drei, bevorzugt vier, bis sieben 5'-TG-3' Dinukleotide (anstelle den 5'-CA-3' Dinukleotiden) auf. Die Anzahl der 5'-CG-3' Dinukleotide in den Sonden für die methylierten DNA-Sequenzen korrespondiert bevorzugt zu der Anzahl der 5'-CA-3' Dinukleotide (bzw. 5'-TG-3' Dinukleotide) in den Sonden für die nicht-methylierten DNA-Sequenzen desselben Zielgens. Die oben genannte Anzahl der 5'-CG-3' bzw. 5'-CA-3' (oder 5'-TG-3') Dinukleotide ist pro Zielgen entweder in einer Sonde enthalten oder auf mehrere Sonden verteilt (insbesondere wenn aufgrund der Sequenz des Zielgens nicht eine Sonde designed werden kann, welche alle Methylierungsstellen umfasst). Im Fall von zwei Sonden für ein Zielgen enthalten beide Sonden für die methylierten Sequenzen bevorzugt jeweils drei oder vier 5'-CG-3' Dinukleotide und beide Sonden für die nicht-methylierten Sequenzen bevorzugt drei oder vier 5'-CA-3' (oder 5'-TG-3') Dinukleotide.

Die digitale PCR wird ansonsten nach den gängigen Methoden durchgeführt und quantifiziert. Die Kompartimente sind bevorzugt Öltröpfchen (droplet digital PCR - ddPCR). Alternativ bevorzugt sind die Kompartimente auf einem Chip angeordnet. In der digitalen PCR werden jedoch bevorzugt mehr als ein Amplifikationszyklus durchgeführt, vorzugsweise mindestens 5, bevorzugt mindestens 15, weiter bevorzugt 20 bis 50, besonders bevorzugt 30 bis 45, weiter bevorzugt bis zu 40. Zur Quantifizierung werden die Kompartimente ausgezählt, in denen eine Amplifikation stattgefunden hat und mit denen die Sonden für die methylierten bzw. nicht-methylierten DNA-Sequenzen hybridisieren. Als Primer für die dPCR können jeweils dieselben Primer eingesetzt werden wie für die BBPA in Schritt b). Alternativ und bevorzugt werden "nested" Primer gewählt, die an anderen Stellen der präamplifizierten Sequenz anbinden. Eine nested PCR kann die Spezifizität weiter erhöhen. Die Auswahl der Primer kann hier nach den üblichen Regeln erfolgen und muss hier nicht zwingend den in Schritt b) erwähnten Auswahlregeln genügen. Bevorzugt erfolgt die Auswahl jedoch nach den gleichen Regeln wie in Schritt b).

Unter einer digitalen PCR (dPCR) im Sinne der Erfindung wird somit eine PCR in einer großen Anzahl getrennter Kompartimente, bevorzugt mit einem Volumen im Picoliterbereich oder im Nanobereich verstanden. Die dPCR zeichnet sich dadurch aus, dass die Quantifizierung der Kompartimente nach einem digitalen Ergebnis (Amplifikation: ja oder nein) erfolgt. Durch Auszählen einer großen Anzahl von Reaktionskompartimenten (Highthroughput screening mit bevorzugt 10.000 bis 100.000 Kompartimenten pro PCR) wird eine statistische Signifikanz erreicht. Der Anteil an Reaktionsräumen mit erfolgter Amplifikation ist proportional zur eingesetzten DNA-Menge der amplifizierten DNA-Sequenz, was zur Mengenbestimmung verwendet wird.

Die Sonden sind bevorzugt fluoreszenzmarkiert, wobei für die Sonden für methylierte und nicht methylierte-Sequenzen bevorzugt mit unterschiedlichen Fluoreszenzmarkern versehen sind. Der Fluoreszenzlabel ist bevorzugt an einem Ende der Sonde (bevorzugt dem 5'-Ende) angebracht. Am anderen Ende befindet sich bevorzugt ein zum Fluoreszenzlabel passender Quencher, der das Fluoreszenzsignal unterdrückt. Durch Hybridisierung oder nach Hybridisierung und anschließender Polymerasewirkung mit der amplifizierten DNA wird der Quencheffekt aufgehoben und das Fluoreszenzsignal detektierbar. Derartige Fluoreszenzlabel und Quencher sind dem Fachmann gut bekannt und für beliebige Sondensequenzen kommerziell erhältlich.

Wenn entsprechende Mehrfarben-Fluoreszenzdetektionssysteme zur Verfügung stehen, wird die digitale PCR bevorzugt ebenfalls als Multiplex-PCR durchgeführt. Zur Quantifizierung werden bevorzugt für jedes amplifizierte Zielgen unterschiedlich farbige Sonden eingesetzt. Die Sonden werden hier bevorzugt so designed, dass sie eine vergleichbare Hybridisierungstemperatur aufweisen.

Alternativ werden die Sonden in separaten Ansätzen in der dPCR eingesetzt und ausgewertet. Das trifft auch zu, wenn die Sonden unterschiedliche Hybridisierungstemperaturen aufweisen.

Wenn pro Zielgen mehrere Sonden eingesetzt werden, können diese dieselben Fluoreszenzmarker aufweisen und das Signal integriert werden. Alternativ kommen die beiden oben für die Multiplex-PCR beschriebenen Alternativen zum Einsatz.

Bevorzugt werden zur Prä-Amplfikation in Schritt b) Primer eingesetzt, welche methylierte und nicht-methylierte DNA-Sequenzen der Gene PLA2R1, RASSF1A und GSTP1 amplifizieren (wobei methylierte DNA stärker amplifiziert wird als nicht-methylierte DNA). In Schritt c) wird die Methylierung dieser Zielgene quantifiziert.

Die Auswahl dieser drei Zielgene ermöglicht wie in den Ausführungsbeispielen gezeigt (s. insbesondere Tabellen 7-11) vorteilhaft eine Unterscheidung von Gesund und Krank für alle getesteten Tumore (Prostatakarzinom, Mammakarzinom, Ovarialkarzinom und Nierenzellkarzinom).

Für die Prä-Amplifikation von methylierten und nicht-methylierten DNA-Sequenzen der Gene PLA2R1 (Phospolipase A2 Rezeptor 1, HGNC Acc. HGNC 9042, Ensembl: ENSG00000153246), RASSF1A (Rass association (RalGDS/AF-6) domain family member 1, HGNC Acc. HGNC 9882, Ensembl: ENSG00000068028) und GSTP1 (Gluthathione S-Transferase pi 1, HGNC Acc HGNC 4638, Ensembl: ENSG00000084207)) werden in Schritt b) bevorzugt folgende Primerpaare eingesetzt:

| Target | Forward (5'→3') | SEQ ID | Reverse (5'→3') | SEQ ID |
|---|---|---|---|---|
| *PLA2R1* (168bp) | GGGGTAAGGAAGGTGGAGAT | 1 | ACAAACCACCTAAATTCTAATAAACAC | 2 |
| *RASSF1A* (117bp) | GTTTGTTAG<u>CG</u>TTTAAAGTTAG | 3 | AATA<u>CG</u>ACCCTTCCCAAC | 4 |
| *GSTP1* (120bp) | GTGAAG<u>CG</u>GGTGTGTAAGTTT | 5 | TAAACAAACAACAAAAAAAAAAC | 6 |

Optional wird anstelle des PLA2R1 (168 bp) Forward-Primers (SEQ ID No. 1) der Forward-Primer gemäß SEQ ID No. 7 und/oder 53 und/oder anstelle des Reverse-Primers (SEG ID NO. 2) einer der folgenden Reverse-Primer (SEQ ID No. 8, 9, 10, oder 11) eingesetzt:

| Target | Forward (5'→3') | SEQ ID | Reverse (5'→3') | SEQ ID |
|---|---|---|---|---|
| *PLA2R1 (133bp)* | GGAAGGTGGAGATTA<u>CG</u>G | 7 | G<u>CG</u>AATTTACAA<u>CG</u>AACAA<u>C</u> | 8 |
| *PLA2R1* (150bp) | GGGGTAAGGAAGGTGGAGAT | 53 | AATAAACAC<u>CGCG</u>AATTTACAAC | 9 |
| *PLA2R1* (161bp) | | | ACCTAAATTCTAATAAACAC<u>CG</u>C | 10 |
| *PLA2R1* (160bp) | | | CCTAAATTCTAATAAACAC<u>CG</u>C | 11 |

Optional werden anstelle des GSTP1 (120 bp) Forward-Primers (SEQ ID No. 5) die Forward-Primer gemäß SEQ ID No. 91, 93, 95 oder 97 und/oder anstelle des Reverse-Primers (SEG ID NO. 6) die Reverse-Primer gemäß SEQ ID No. 92, 94, 96 oder 98 eingesetzt:

| *Target* | Forward (5'→3') | SEQ ID | Reverse (5'→3') | SEQ ID |
|---|---|---|---|---|
| *GSTP1 (114bp)* | CGTAGCGGTTTTAGGGAATTT | 91 | TCCCCAACGAAACCTAAAAA | 92 |
| *GSTP1 (116bp)* | ATCGTAGCGGTTTTAGGGAA | 93 | TCCCCAACGAAACCTAAAAA | 94 |
| *GSTP1 (129bp)* | TGTAAGTTTCGGGATCGTAGC | 95 | TCCCCAACGAAACCTAAAAA | 96 |
| *GSTP1 (132bp)* | GTGTGTAAGTTTCGGGATCG | 97 | TCCCCAACGAAACCTAAAAA | 98 |

Weiter, optional wird anstelle des GSTP1 (120 bp) Forward-Primers (SEQ ID No. 5) der Forward-Primer gemäß SEQ ID No. 12 und/oder anstelle des Reverse-Primers (SEG ID NO. 6) der Reverse-Primer gemäß SEQ ID No. 13 eingesetzt:

| *Target* | Forward (5'→3') | SEQ ID | Reverse (5'→3') | SEQ ID |
|---|---|---|---|---|
| *GSTP1* (171 bp) | GTTCGGTTAATATGGTGAA | 12 | ACCCAAACTAAAATACAATAAC | 13 |

Dieses Primerpaar ist insbesondere bevorzugt, wenn anschließend in der dPCR die GSTP1-Sonden mit 4 CpG-Stellen (bevorzugt SEQ ID No 45 und 46 oder jeweils komplementäre Sequenzen) und/oder die GSTP1-Sonden mit 5 CpG-Stellen (bevorzugt SEQ ID No. 47 und 48 oder jeweils komplementäre Sequenzen) eingesetzt werden.

Um die Aussagekraft des erfindungsgemäßen Verfahrens oder dessen Anwendungsbereich (weitere Tumoren) weiter zu erhöhen, werden in einer bevorzugten Ausgestaltung zusätzlich methylierte und nicht-methylierte Sequenzen des/der Gene AOX-1 (Aldehyde oxidase 1, HGNC Acc HGNC 553, Ensembl: ENSG00000138356) und/oder SERPINE-1 (Serpin peptidase inhibitor, HGNC Acc HGNC 8583, Ensembl: ENSG00000106366) und/oder Thrombomodulin (HGNC THBD, HGNC:11784, Ensembl:ENSG00000178726) und/oder Septin-9 (SEPT9, HGNC:HGNC:7323 Ensembl:ENSG00000184640) prä-amplifiziert und in Schritt c) die Methylierung dieser Gene quantifiziert.

Bevorzugt sind die Primer zur Präamplifikation dieser Zielgene wie folgt ausgewählt:

| Target | Forward (5'→3') | SEQ ID | Reverse (5'→3') | SEQ ID |
|---|---|---|---|---|
| AOX1 (180 bp) | TGGGTTGGATTTTAGGTTTTAG | 14 | CTCACCTTACGACCGTTC | 15 |
| SERPINE1 (123 bp) | AGAGCGTTGTTAAGAAGA | 16 | CTCCTACCTAAAATTCTCAAAA | 17 |

Optional wird anstelle des AOX1 (180 bp) Forward-Primers (SEQ ID No. 14) der Forward-Primer gemäß SEQ ID No. 18 und/oder anstelle des Reverse-Primers (SEG ID NO. 15) der Reverse-Primer gemäß SEQ ID No. 19 eingesetzt:

| Target | Forward (5'→3') | SEQ ID | Reverse (5'→3') | SEQ ID |
|---|---|---|---|---|
| AOX1 (138 bp) | GTTGGATTTTAGGTTTTAGTAAG | 18 | GCCCGATCCATTATAATATC | 19 |

Dieses Primerpaar ist insbesondere bevorzugt, wenn anschließend in der dPCR die AOX1-Sonden mit 4 CpG-Stellen (bevorzugt SEQ ID No 39 und 40 oder jeweils komplementäre Sequenzen) und/oder die AOX1-Sonden mit 5 CpG-Stellen (bevorzugt SEQ ID No. 41 und 42 oder jeweils komplementäre Sequenzen) eingesetzt werden.

Für die Quantifizierung per dPCR werden in Schritt c) bevorzugt Sonden ausgewählt aus den folgenden Sequenzen eingesetzt:

| Target | methyliert (5'→3') | SEQ ID | nicht-methyliert (5'→3') | SEQ ID |
|---|---|---|---|---|
| *PLA2R1* (3 CpG) | CCCAACTACTCCGCGACGCAA | 20 | AACCCAACTACTCCACAACACAAA | 21 |
| *PLA2R1* (4 CpG) | CAACTACTCCGCGACGCAAACG | 22 | AACCCAACTACTCCACAACACAAACA | 23 |
| *RASSF1A* (3 CpG) | CGCCCAACGAATACCAACTCCCG | 24 | CACCCAACAAATACCAACTCCCACAA | 25 |
| *RASSF1A* (4 CpG) | CGCCCAACGAATACCAACTCCCGCG | 54 | CACCCAACAAATACCAACTCCCACAACTC | 55 |
| *GSTP1* (3 CpG) | CGCAACGAAATATACGCAAC | 56 | CACAACAAAATATACACAAC | 57 |
| *GSTP1* (4 CpG) | ACGAACTAACGCGCCGAAAC | 58 | ACAAACTAACACACCAAAAC | 59 |
| *GSTP1 (3 CpG)* | CGATCTCGACGACTCACTACAACC | 45 | CAATCTCAACAACTCACTACAACCTC | 46 |
| *GSTP1 (4 CpG)* | CGCGATCTCGACGACTCACTACAA | 47 | CACAATCTCAACAACTCACTACAACCT | 48 |

Diese sind komplementär für den coding strand.
Alternativ kann auch jeweils der (komplementäre) template strand separat oder gemeinsam mit dem coding strand in einem Ansatz quantifiziert werden. Dafür sind folgende (komplementäre) Sonden geeignet und bevorzugt:

| Target | methyliert (5'→3') | SEQ ID | nicht-methyliert (5'→3') | SEQ ID |
|---|---|---|---|---|
| *PLA2R1* (3 CpG) | TTGCGTCGCGGAGTAGTTGGG | 60 | TTTGTGTTGTGGAGTAGTTGGGTT | 26 |
| *PLA2R1* (4 CpG) | CGTTTGCGTCGCGGAGTAGTTG | 27 | TGTTTGTGTTGTGGAGTAGTTGGGTT | 28 |
| *RASSF1A* (3 CpG) | CGGGAGTTGGTATTCGTTGGGCG | 29 | TTGTGGGAGTTGGTATTTGTTGGGTG | 30 |
| *RASSF1A* (4 CpG) | CGCGGGAGTTGGTATTCGTTGGGCG | 31 | GAGTTGTGGGAGTTGGTATTTGTTGGGTG | 32 |
| *GSTP1* (3 CpG) | GTTGCGTATATTTCGTTGCG | 33 | GTTGTGTATATTTTGTTGTG | 34 |
| *GSTP1* (4 CpG) | GTTTCGGCGCGTTAGTTCGT | 35 | GTTTTGGTGTGTTAGTTTGT | 36 |
| *GSTP1 (3 CpG)* | GGTTGTAGTGAGTCGTCGAGATCG | 49 | GAGGTTGTAGTGAGTCGTCGAGATCG | 50 |
| *GSTP1 (4 CpG)* | TTGTAGTGAGTCGTCGAGATCGCG | 51 | AGGTTGTAGTGAGTCGTCGAGATCGCG | 52 |

Sofern zusätzlich methylierte und nicht-methylierte Sequenzen des/der Gene AOX-1 und/oder SERPINE-1 prä-amplifiziert werden, werden in Schritt c) zur Quantifizierung der Methylierung dieser Gene besonders bevorzugt Sonden ausgewählt aus den folgenden Sequenzen und komplementäre Sequenzen eingesetzt:

| Target | methyliert (5'→3') | SEQ ID | nicht-methyliert (5'→3') | SEQ ID |
|---|---|---|---|---|
| *AOX1* (3 CpG) | ACTCGAACGCCCGATCCATTATAA | 37 | ACAACTCAAACACCCAATCCATTATAA | 38 |
| *AOX1* (4 CpG) | CGCTAATTCGAAAACCCGAAACGA | 39 | CACTAATTCAAAAACCCAAAACAA | 40 |
| *AOX1* (5 CpG) | CGCGCTAATTCGAAAACCCGAAACGA | 41 | CACACTAATTCAAAAACCCAAAACAA | 42 |
| *SERPINE1* (4 CpG) | CGATTAACGATTCGTCCTACTCTAACG | 43 | CAATTAACAATTCATCCTACTCTAACA | 44 |

Alternativ oder ergänzend lassen sich in Schritt b) folgende weitere Primer einsetzen:

| *Target* | Forward (5'→3') | SEQ ID | Reverse(5'→3') | SEQ ID |
|---|---|---|---|---|
| *RASSF1A (124bp)* | GCGTTTGTTAGCGTTTAAAG | 61 | AACCGAATACGACCCTTC | 62 |
| *AOX1 (138bp)* | GTTGGATTTTAGGTTTTAGTAAG | 63 | GCCCGATCCATTATAATATC | 64 |
| *AOX1 (135bp)* | GGATTTTAGGTTTTAGTAAGTTTC | 65 | GCCCGATCCATTATAATATCCG | 66 |
| *AOX1 (134p)* | GATTTTAGGTTTTAGTAAGTTTCG | 67 | | |
| AOX1 (171p) | TTTTAATTAAGGTTTTTTTCGTCG | 99 | CCCGATCCATTATAATATCCG | 100 |
| *SERPINE1 (119bp)* | CGTTGTTAAGAAGATTTATAC | 68 | TAAACCCGAAATAAAAAATTAAA | 69 |
| *TM (125bp)* | GGTCGATTCGTATGTTAGA | 70 | AACCGTACCGAAACAAAA | 71 |
| *TM (144bp)* | GTTTGGGTTGGGACGGATA | 72 | AAAAACCAAAACCCCAAACA | 73 |
| *TM (166bp)* | GTTTGGGGTTTTGGTTTTTG | 74 | GCAATCCGTCGCAAATCTAA | 75 |
| *TM (165bp)* | | | CAATCCGTCGCAAATCTAAC | 76 |
| TM (160bp) | TTTGTGTTTTTTTGTTTCGGTAC | 101 | CACCCGACTACGACTCTACG | 102 |
| TM (159bp) | | | ACCCGACTACGACTCTACGA | 103 |
| RASSF1A (86bp) | TTTAGTTTGGATTTTGGGGGA | 104 | CTAACTTTAAACGCTAACAAA | 105 |
| RASSF1A (82bp) | GTTTGGATTTTGGGGGAGC | 106 | ACTTTAAACGCTAACAAACG | 107 |
| RASSF1A (82bp) | TTTGGATTTTGGGGGAGCG | 108 | CGCTAACTTTAAACGCTAAC | 109 |
| RASSF1A (86bp) | TTTAGTTTGGATTTTGGGGGAG | 110 | CGCTAACTTTAAACGCTAACAAA | 111 |
| TM (125bp) | GGTCGATTCGTATGTTAGA | 126 | AACCGTACCGAAACAAAA | 127 |
| TM (83bp) | TAGCGGTAAGAAGTGTTTG | 128 | | |
| TM (70bp) | TACGGTTTTGTCGTAGTG | 129 | CCCAAACATATTACCCAAAC | 130 |
| TM (113bp) | GGAGAGGTTGTCGTTATC | 131 | CCCAAACATATTACCCAAAC | 132 |
| TM (115bp) | | | ACCCCAAACATATTACCC | 133 |
| TM (99bp) | GTCGAGTACGATTGTTTC | 134 | ACGCACTATCATTAAATAACC | 135 |
| TM (97bp) | CGGTGGTTGTCGATGTTA | 136 | CCGCAACCGAATAACAAC | 137 |
| TM (125bp) | TTGCGGGGTTATTTAATG | 138 | CAACCGAATAACAACTACA | 139 |
| Septin-9 (72bp) | GCGATTCGTTGTTTATTAG | 140 | ATCCGAAATAATCCCATC | 141 |
| Septin-9 (169bp) | CGGTTAGTTTTGTATTGTAG | 142 | | |
| Septin-9 (94bp) | CGGGGTTGTTTTGTTTAAG | 143 | CCAACACCGACAATCAAA | 144 |

Die AOX1-Primerpaare (SEQ ID No 63 und 64 bzw. 65 und 66 bzw. 65 und 67 bzw. 99 und 100) sind insbesondere bevorzugt, wenn anschließend in der dPCR die AOX1-Sonden mit 4 CpG-Stellen (bevorzugt SEQ ID No 77 und 78 bzw. 79 oder jeweils komplementäre Sequenzen) bzw. die AOX1-Sonden mit 5 CpG-Stellen (bevorzugt SEQ ID No 112 und 113 bzw. 112 und 114 oder jeweils komplementäre Sequenzen) eingesetzt werden.

Die Thrombomodulin (TM)-Primerpaare (SEQ ID No 70 und 71 bzw. 72 und 73 bzw. 101 und 102 bzw. 101 und 103) sind insbesondere bevorzugt, wenn anschließend in der dPCR die TM-Sonden mit 3 CpG-Stellen (bevorzugt SEQ ID No 80 und 81 oder jeweils komplementäre Sequenzen) und die TM-Primerpaare (SEQ ID No 74 und 75 bzw. 74 und 76), wenn die TM-Sonden mit 4 CpG-Stellen (bevorzugt SEQ ID No. 82 und 83 oder jeweils komplementäre Sequenzen) eingesetzt werden.

Die Thrombomodulin (TM)-Primerpaare (SEQ ID No 126 und 127 bzw. 127 und 128) sind insbesondere bevorzugt, wenn anschließend in der dPCR die TM-Sonden mit 3 CpG-Stellen (bevorzugt SEQ ID No 145 und 146 oder jeweils komplementäre Sequenzen) und die TM-Primerpaare (SEQ ID No 129 und 130 bzw. 131 und 132), wenn die TM-Sonden mit 5 CpG-Stellen (bevorzugt SEQ ID No. 147 und 148 oder jeweils komplementäre Sequenzen) eingesetzt werden. Die Thrombomodulin (TM)-Primerpaare (SEQ ID No 131 und 133) sind insbesondere bevorzugt, wenn anschließend in der dPCR die TM-Sonden mit 5 CpG-Stellen (bevorzugt SEQ ID No 149 und 150 oder jeweils komplementäre Sequenzen), die TM-Primerpaare (SEQ ID No 134 und 135), wenn die TM-Sonden mit 3 CpG-Stellen (bevorzugt SEQ ID No. 151 und 152 oder jeweils komplementäre Sequenzen), die TM-Primerpaare (SEQ ID No 136 und 137), wenn die TM-Sonden mit 5 CpG-Stellen (bevorzugt SEQ ID No. 153 und 154 oder jeweils komplementäre Sequenzen), die TM-Primerpaare (SEQ ID No 138 und 139), wenn die TM-Sonden mit 4 CpG-Stellen (bevorzugt SEQ ID No. 155 und 156 bzw. 157 und 158 oder jeweils komplementäre Sequenzen) eingesetzt werden.

Die Septin-9-Primerpaare (SEQ ID No 140 und 141) sind insbesondere bevorzugt, wenn anschließend in der dPCR die TM-Sonden mit 4 CpG-Stellen (bevorzugt SEQ ID No 159 und 160 oder jeweils komplementäre Sequenzen), die Septin-9-Primerpaare (SEQ ID No 141 und 142), wenn die TM-Sonden mit 4 CpG-Stellen (bevorzugt SEQ ID No. 161 und 162 oder jeweils komplementäre Sequenzen) und die Septin-9-Primerpaare (SEQ ID No 143 und 144), wenn die TM-Sonden mit 2 CpG-Stellen (bevorzugt SEQ ID No. 163 und 164 oder jeweils komplementäre Sequenzen) eingesetzt werden.

Die RASSF1A-Primerpaare (SEQ ID No 104 und 105 bzw.106 und 107 bzw. 108 und 109 bzw. 110 und 111) sind insbesondere bevorzugt, wenn anschließend in der dPCR die RASSF1A-Sonden mit 4 CpG-Stellen (bevorzugt SEQ ID No 115 und 116 bzw. 115 und 117 bzw. 115 und 116 oder jeweils komplementäre Sequenzen) eingesetzt werden.

Für die Quantifizierung per dPCR werden in Schritt c) bevorzugt Sonden ausgewählt aus den folgenden Sequenzen eingesetzt:

| Target | methyliert (5'→3') | SEQ ID | nicht-methyliert (5'→3') | SEQ ID |
|---|---|---|---|---|
| *AOX1* (4 CpG) | CGCTAATTCGAAAACCCGAAACGA | 77 | CACTAATTCAAAAACCCAAAACAA | 78 |
| *AOX1* (4 CpG) | | | CACTAATTCAAAAACCCAAAACAAAAA | 79 |
| AOX1 (5 CpG) | CGCGCTAATTCGAAAACCCGAAACGA | 112 | CACACTAAT T CAAAAACCCAAAACAA | 113 |
| AOX1 (5 CpG) | | | CACACTAATTCAAAAACCCAAAACAAAAA | 114 |
| RASSF1A (4 CpG) | CGCGAACCGAACGAAACCAC | 115 | CACAAACCAAACAAAACCAC | 116 |
| RASSF1A (4 CpG) | | | CACAAACCAAACAAAACCACAAA | 117 |
| RASSF1A (4 CpG) | | | AAACACAAACCAAACAAAACCACAAA | 118 |
| TM (3 CpG) | ACGCCGATAACGACAACCTCT | 80 | AAAAAGCAGATAAAGACAACCTCT | 81 |
| TM (4 CpG) | CCGACTACGACTCTACGAATACGAA | 82 | CAGACTAAGACTCTAAGAATAAGAAAAAC | 83 |
| TM (3 CpG) | ACGCCGATAACGACAACCTCT | 145 | AAGCAGATAAAGACAACCTCT | 146 |
| TM (5 CpG) | CGCCGCGTACAAACGCCGAA | 147 | AGCAGAGTACAAAAGCAGAA | 148 |
| TM (5 CpG) | AACGCGCCGCGTACAAACGC | 149 | AAAGAGCAGAGTACAAAAGC | 150 |
| TM (3 CpG) | CGCAATCCGTCGCAAATCTAACT | 151 | AGCAATCAGTAGCAAATCTAACT | 152 |
| TM (5 CpG) | AACGCCGACGACCAACGCCG | 153 | AAAGCAGAAGACCAAAGCAG | 154 |
| TM (4 CpG) | AAAACGCCGACGACCAACGC | 155 | AAAAAGCAGAAGACCAAAGC | 156 |
| TM (4 CpG) | AAAACGCCGACGACCAACGCC | 157 | AAAAAGCAGAAGACCAAAGCC | 158 |
| Septin-9 (4 CpG) | CGTTAACCGCGAAATCCGACATAAT | 159 | AGTTAACAGAGAAATCAGACATAAT | 160 |
| Septin-9 (4 CpG) | CGTTAACCGCGAAATCCGACATAATAA | 161 | AGTTAACAGAGAAATCAGACATAATAA | 162 |
| Septin-9 (2 CpG) | AAACGCACGCACTCACAAACT | 163 | AAAAGCAAGCACTCACAAACT | 164 |

Diese sind komplementär für den coding strand.
Alternativ kann auch jeweils der (komplementäre) template strand separat oder gemeinsam mit dem coding strand in einem Ansatz quantifiziert werden. Dafür sind folgende (komplementäre) Sonden geeignet und bevorzugt:

| Target | methyliert (5'→3') | SEQ ID | nicht-methyliert (5'→3') | SEQ ID |
|---|---|---|---|---|
| *AOX1* (4 CpG) | TCGTTTCGGGTTTTCGAATTAGCG | 84 | TTGTTTTGGGTTTTTGAATTAGTG | 85 |
| *AOX1* (4 CpG) | | | TTTTTGTTTTGGGTTTTTGAATTAGTG | 86 |
| AOX1 (5 CpG) | TCGTTTCGGGTTTTCGAATTAGCGCG | 119 | TTGTTTTGGGTTTTTGAATTAGTGTG | 120 |
| AOX1 (5 CpG) | | | TTTTTGTTTTGGGTTTTTGAATTAGTGTG | 121 |
| RASSF1A (4 CpG) | GTGGTTTCGTTCGGTTCGCG | 122 | GTGGTTTTGTTTGGTTTGTG | 123 |
| RASSF1A (4 CpG) | | | TTTGTGGTTTTGTTTGGTTTGTG | 124 |
| RASSF1A (4 CpG) | | | TTTGTGGTTTTGTTTGGTTTGTGTTT | 125 |
| TM (3 CpG) | AGAGGTTGTCGTTATCGGCGT | 87 | AGAGGTTGTTGTTATTGGTGT | 88 |
| TM (4 CpG) | TTCGTATTCGTAGAGTCGTAGTCGG | 89 | TTTGTATTTGTAGAGTTGTAGTTGG | 90 |
| TM (3 CpG) | AGAGGTTGTCGTTATCGGCGT | 165 | AGAGGTTGTCTTTATCTGCTT | 166 |
| TM (5 CpG) | TTCGGCGTTTGTACGCGGCG | 167 | TTCTGCTTTTGTACTCTGCT | 168 |
| TM (5 CpG) | GCGTTTGTACGCGGCGCGTT | 169 | GCTTTTGTACTCTGCTCTTT | 170 |
| TM (3 CpG) | AGTTAGATTTGCGACGGATTGCG | 171 | AGTTAGATTTGCTACTGATTGCT | 172 |
| TM (5 CpG) | CGGCGTTGGTCGTCGGCGTT | 173 | CTGCTTTGGTCTTCTGCTTT | 174 |
| TM (4 CpG) | GCGTTGGTCGTCGGCGTTTT | 175 | GCTTTGGTCTTCTGCTTTTT | 176 |
| TM (4 CpG) | GGCGTTGGTCGTCGGCGTTTT | 177 | GGCTTTGGTCTTCTGCTTTTT | 178 |
| Septin-9 (4 CpG) | ATTATGTCGGATTTCGCGGTTAACG | 179 | ATTATGTCTGATTTCTCTGTTAACT | 180 |
| Septin-9 (4 CpG) | TTATTATGTCGGATTTCGCGGTTAAC G | 181 | TTATTATGTCTGATTTCTCTGTTAACT | 182 |
| Septin-9 (2 CpG) | AGTTTGTGAGTGCGTGCGTTT | 183 | AGTTTGTGAGTGCTGCTTTT | 184 |

Die Sonden sind besonders bevorzugt, wie in den Beispielen 5'-FAM (methylierte DNA) bzw. 5'-HEX (nicht methylierte DNA) markiert und am 3'-Ende mit dem Quencher BHQ-1 markiert.

Die Aufgabe wird weiter gelöst durch die Verwendung eines Kit zur Diagnose einer Tumorerkrankung in einer isolierten Probe mit dem erfindungsgemäßen Verfahren.

Das Kit enthält:

i) Primer zur Prä-Amplifikation von methylierten und nicht-methylierten DNA-Sequenzen der Gene PLA2R1, RASSF1A und GSTP1 mittels PCR, wobei die Primer jeweils so ausgewählt werden, dass Forward- und Reverse-Primer zusammen bis zu sieben, bevorzugt zwei bis sechs, bevorzugt eins bis vier, besonders bevorzugt ein bis drei, weiter bevorzugt drei bis vier, weiter bevorzugt vier bis fünf 5'-CG-3' Dinukleotide aufweisen,

ii) Sonden zur Quantifizierung der methylierten und nicht-methylierten DNA-Sequenzen der Gene PLA2R1, RASSF1A und GSTP1, bevorzugt wie oben beschrieben jeweils mit einem Fluoreszenz-Marker und einem Quencher.

Die für die digitale PCR eingesetzten Primer sind entweder identisch zu denen der Prä-Amplifikation. Alternativ und bevorzugt enthält der Kit zusätzlich:
iii) Primer für die digitale PCR zur Amplifikation von methylierten und nicht-methylierten DNA-Sequenzen der Gene PLA2R1, RASSF1A und GSTP1.

Diese zusätzlichen Primer werden bevorzugt als intrinsische Primer eingesetzt, wenn in der Prä-Amplifikation extrinsische Primer (als nested Primer) eingesetzt wurden.

Bevorzugt enthält das Kit weitere Bestandteile ausgewählt aus:

iv) Reaktionspuffer für die Bias basierte PCR Amplifikation, bevorzugt mit einer Magnesiumchloridkonzentration von 0,5 bis 15,0 mmol/l, bevorzugt 1,5 bis 8 mmol/l, weiter bevorzugt bis 3,5 mmol/l Endkonzentration, besonders bevorzugt 2,5 bis 3,5 mmol/l, oder alternativ einen Standard PCR Puffer und eine konzentrierte Magnesiumlösung,

v) Reaktionspuffer für die dPCR

vi) Desoxyribonukleotid-Mix

vii) eine DNA-Polymerase, wie z. B. HotStarTaq Plus,

viii) Kontroll-DNA, bevorzugt eine nicht-methylierte DNA-Kontrolle und eine methylierte DNA-Kontrolle,

ix) Gebrauchsanweisung und ggf. Computersoftware zur Auswertung.

Bevorzugt sind die Primer und Sonden sowie die Kontroll-DNA für das Kit wie weiter oben für das Verfahren beschrieben ausgewählt. Dies gilt insbesondere für die bevorzugten Zielgene PLAR1, RASSF1A und GSTP1 aber auch die optionalen zusätzlichen Zielgene AOX1, SERPINE1, Thrombomodulin (TM) und/oder Septin-9.

Als Kontroll-DNA für das erfindungsgemäße Verfahren oder das Kit werden bevorzugt aus primären Zellen oder Zelllinien isolierte DNA verwendet.

Vorzugsweise wird als nicht-methylierte Kontrolle (Negativkontrolle) DNA aus Zellen oder Zelllinien verwendet, in denen die Zielsequenzen nicht methyliert sind. Besonders bevorzugt wird als nicht-methylierte DNA-Kontrolle DNA aus Epithelzellen des dem Tumor entsprechenden gesunden Gewebes verwendet. So wird vorzugweise DNA aus humanen Prostata Epithelzellen (PrEC) oder humanen Mamma-Epithelzellen (HMEC) als nicht-methylierte Kontrolle für Diagnose von Prostata bzw. Mammakarzinomen verwendet. Als negative Kontrolle für den Nachweis von Brustkrebs wird alternativ DNA aus MCF10A-Zelllinien verwendet.

Als methylierte Kontrolle (Positivkontrolle) zum Nachweis von Prostatakarzinomen wird für jedes Zielgen bevorzugt DNA aus einer Zelllinie gewählt, in der die Zielsequenz homogen methyliert vorliegt. Für PLA2R1 wird bevorzugt DNA aus der U937-Leukämiezelllinie und/oder der LNCaP-Zelllinie verwendet; für RASSF1A bevorzugt DNA aus der U937-Leukämie- und/oder PC-3-Zelllinie und für GSTP1 bevorzugt DNA aus der U937-Leukämiezelllinie, der LNCaP- und/oder DU-145-Zelllinie. Für SERPINE1 und Thrombomodulin werden als positive Kontrollen bevorzugt DNA aus der DU-145-Zellinie und/oder für AOX1 bevorzugt DNA aus der U937-Leukämiezelllinie und/oder PC-3-Zellinie verwendet. Für Septin-9 werden als positive Kontrollen bevorzugt DNA aus den LNCaP, PC-3 und DU-145-Zellinien verwendet.

Für den Nachweis von Brustkrebs werden bevorzugt als positive Kontrollen DNA von MCF-7, Cal-51, UACC-812, BT-474, MDA-MB-453 und/oder MDA-MB231 Zelllinien verwendet.

Alternativ oder bevorzugt ergänzend wird als positive Kontrolle für heterogen methylierte Epi-Allele DNA verwendet, in denen zwei von drei bzw. drei von vier 5'-CG-3' Dinukleotiden methyliert vorliegen. Als Kontrollen für die Identifikation und Quantifizierung von homogen und heterogen methylierten Api-Allele werden besonders bevorzugt DNA-Proben der BPH-1 Zelllinie mitgeführt, in denen Epi-Allele nachgewiesen und als Vergleich bei der Differentialdiagnose von BPH, Prostatitis und Prostatakarzinom genutzt werden. Insbesondere für die Gene PLA2R1, RASSF1A und/oder GSTP1 wird hier bevorzugt DNA aus der BHP1-Zellinie verwendet. Bei der Untersuchung der benignen Prostatazelllinie BPH-1 im Vergleich zu normalen Epithelialzellen der Prostata (PrEC) konnten die Erfinder heterogen methylierte DNA-Fragmente, besonders in den Zielgenen PLA2R1 und RASSF1A nachweisen (Fig. 18 und 19). Bei Verwendung von Sonden mit mindestens 3 CpG-Stellen in ihren Sequenzen konnten die heterogen methylierten Epi-Allele von den homogen methylierten Epi-Allelen differenziert und quantifiziert werden (Fig. 20-29). Dabei zeigte sich, dass homogen methylierte Epi-Allele gegenüber heterogen methylierten Epi-Allelen spezifisch für Tumor-DNA sind und deren Bestimmung Grundlage für eine stringente und damit sichere Differentialdiagnose von benignen und malignen Erkrankungen, insbesondere der Prostata darstellt. Aus diesem Grund werden Sonden, die mindestens drei 5'-CG-3' bzw. 5'-CA-3' Dinukleotide in ihrer Sequenz enthalten, in der dPCR nach Prä-Amplifikation eingesetzt. Wenn in Einzelfällen mit Hilfe dieser Sonden eine Differenzierung z. B. von benignen Hyperplasien wie der BPH und malignen Erkrankungen wie dem Prostatakarzinom nicht möglich ist, werden Sonden mit vier, fünf oder mehr 5'-CG-3'-Stellen oder wenn auf Grund der Gensequenzen der Zielgene oder dem Design der Sonden dies nicht möglich ist, werden mehrere Sonden mit jeweils drei 5'-CG-3' bzw. 5'-CA-3' Dinukleotide für ein und das selbe Zielgen (target of interest) in der dPCR (Schritt c) des erfindungsgemäßen Verfahrens oder dem Kit verwendet. Auf diese Weise ist eine stringentere Unterscheidung zwischen benignen und malignen Erkrankungen möglich, indem das Vorliegen eines malignem Tumors nur den Proben zugeordnet wird, bei denen zunächst jeweils erhöhte Spiegel an homogen methylierten Epi-Allelen nachgewiesen und quantifiziert werden (d. h. vier, fünf oder sechs 5'-CG-3' sind gleichzeitig methyliert). Stellt sich diese Vorgabe für den Nachweis von Tumor-DNA als zu stringent heraus, so dass die diagnostische Sensitivität zu gering ist, werden bevorzugt heterogen methylierte Epi-Allele mit einer entsprechend hohen Anzahl von methylierten CpG-Stellen (d. h. mindestens jeweils zwei von drei 5'-CG-3' Dinukleotiden methyliert, also gleichzeitig insgesamt vier von sechs untersuchten 5'-CG-3' Dinukleotiden methyliert) quantifiziert und in die Datenauswertung einbezogen. Wie die Ergebnisse der Erfinder bereits demonstrierten, können heterogen methylierte Epi-Allele bei Verwendung der genannten Sondensequenzen quantifiziert werden (Figuren 20-29).

Gegenstand der Erfindung ist die Verwendung des Kits zur Durchführung des erfindungsgemäßen Verfahrens.

Der Begriff Tumordiagnostik im Sinne der Erfindung schließt insbesondere das Früherkennungsscreening (Vorsorge), die Prognose, die Verlaufsdiagnostik, Therapiekontrolle und den Nachweis von MRD mit ein.

Der Nachweis frei-zirkulierender Tumor-DNA mit Hilfe der BBPA-dPCR kann prinzipiell zur Diagnostik eines jeden soliden Tumors eingesetzt werden, insbesondere wenn in Körperflüssigkeiten oder Abstrichen ("Liquid Biopsien") entsprechende Zielgensequenzen (target of interest) vorkommen.

Das erfindungsgemäße Verfahren und das Kit eignen sich insbesondere zur Diagnostik von malignen Tumoren, wie Prostata-, Mamma-, Nierenzell- und ableitende Harnwegs- und Harnblasen-, Pankreas-, Hoden-, Mundhöhlen- und Rachen-, Speiseröhren-, Kehlkopf-, Schilddrüsen-, Magen-, Ösophagus-, Darm- (insbesondere Kolon- und Rektumkarzinom), Lungen-, Eierstock-, Gebärmutterhals- und Gebärmutterkörper-Karzinomen und Gallenblasen-Karzinomen, malignes Melanom der Haut, Astrozytom, Glioblastom, Neuroblastom, aber auch zur Diagnostik von Non-Hodgkin- und Hodgkin-Lymphomen, Lymphomen der Haut, des ZNS, des GI-Traktes, Magenlymphome und intestinalen Lymphome und Leukämien.

Eine bevorzugte Einsatzmöglichkeit des Verfahrens und Kits besteht in der allgemeinen Früherkennung (eigenständiges Screening bzw. Vorsorge) maligner Erkrankungen (wozu die oben genannten Tumorentitäten zählen), insbesondere aber in Kombination mit der PSA-Bestimmung und der Indikation für eine Gewebebiopsie bei erhöhten PSA-Werten und der Diagnostik eines Prostatakarzinoms oder in Kombination mit der Mammografie und suspekten Befunden bei der Diagnostik eines Mammakarzinoms oder in Kombination mit dem Vorliegen einer Genmutation mit erhöhtem familiären Risiko für das Mamma- und Ovarialkarzinom und der Entscheidung für eine prophylaktische Mastektomie und/oder Ovariektomie.

Dabei wird in einer Ausführungsform der Erfindung ein Screening bzw. Vorsorge anhand von gepoolten Proben, z. B. von Serum- oder Plasmaproben, durchgeführt, indem jeweils DNA-Probenmaterial von z. B. 10 und 100 Probanden/Patienten in einem Pool zusammengefasst und zunächst der 100-fache Pool untersucht wird. Lassen sich in einem Pool aus 100 Probanden/Patientenproben Tumor-DNA bestimmten, werden die parallel herstellten 10-fachen Poolproben der entsprechenden Probanden/Patientenproben analysiert. Ist in einem oder mehreren dieser 10-fachen Poolproben Tumor-DNA nachweisbar, werden die Einzelproben analysiert. Lassen sich in den 100-fachen Poolproben keine Tumor-DNA bestimmten, entfallen die Untersuchungen der 10-fachen Poolproben und der Einzelproben. Auf diese Weise können eine große Anzahl von Proben auf das Vorliegen von Tumor-DNA gescreent werden, da auf Grund des stringenten Verstärkungseffektes des BBPA-dPCR-Verfahrens einzelne Tumor-DNA-Moleküle detektiert werden, unabhängig wie hoch die Hintergrunds-DNA-Konzentration ist.

Wurde eine Tumorerkrankung diagnostiziert, kann durch die Bestimmung von Tumor-DNA mittels BBPA-dPCR nach Operation, Chemo- oder radiologischer Therapie der Verlauf der Erkrankung kontrolliert und das Vorliegen einer minimalen Resterkrankung (minimal residual disease, MRD) diagnostiziert bzw. ausgeschlossen werden. Lassen sich keine Tumor-DNA nach erfolgter Therapie nachweisen, ist von einem guten Ansprechen auf die Therapie auszugehen und eine MRD kann ausgeschlossen werden. Sind weiterhin Tumor-DNA nachweisbar, kann das Anlass für eine Therapieoptimierung sein. Lassen sich Tumor-DNA im Verlauf nachweisen, nachdem das zuvor nicht der Fall war, ist von einem Rezidiv auszugehen, das Anlass für eine erneute optimierte Therapie ist.

Vorteilhaft eignen sich das erfindungsgemäße Verfahren und das Kit besonders zur Differentialdiagnose von benignen Erkrankungen und malignen Tumorerkrankungen. Ganz besonders eignet sich die Erfindung zur Differentialdiagnose benigner Prostatahyperplasie(n), Prostatitis und Prostatakarzinomen, insbesondere wenn nach bisherigen Stand der Technik auf Grund von erhöhten PSA-Werten eine Prostatagewebebiopsie indiziert ist. Durch die erfindungsgemäße Bestimmung der *PLA2R1-* und *RASSF1A-* und *GSTP1*-Methylierungen (sowie ggf. *AOX1-, SERPINE1-,* Thrombomodulin- und/oder Septin-9-Methylierungen) mit Hilfe der BBPA-dPCR kann festgestellt werden, inwieweit fcT-DNA in Serum-, Plasma-, Urin- und/oder Seminalflüssigkeit nachweisbar sind. Basierend auf dem ermittelten Methylierungsgrad kann zwischen einer benignen Prostatahyperplasie, Prostatitis und Prostatakarzinomen unterschieden werden, so dass in vielen Fällen eine unnötige Prostatagewebebiopsie und eine Operation vermieden werden kann. Lassen sich z. B. in einer Serum-, Plasma- oder Urinprobe mit Hilfe der BBPA-dPCR keine Tumor-DNA finden, kann eine weitere PSA-Bestimmung (z. B. nach 3 oder 6 Monaten) abgewartet werden. Sind dagegen Tumor-DNA in den Proben nachweisbar, muss die Indikation für eine Gewebebiopsie verstärkt gestellt werden.

Vorteilhaft eignen sich das erfindungsgemäße Verfahren und das Kit auch zur Differentialdiagnose von Mamma-Karzinomen, insbesondere wenn auf Grund von suspekten Mammografie-Befunden nach bisherigem Stand der Technik eine Gewebebiopsie indiziert ist. Durch die erfindungsgemäße Bestimmung der *PLA2R1-* und *RASSF1A-* und *GSTP1-*Methylierungen (sowie ggf. *AOX1-, SERPINE1-,* Thrombomodulin- und/oder Septin-9-Methylierungen) mit Hilfe der BBPA-dPCR kann festgestellt werden, inwieweit fcT-DNA in Serum-, Plasma-, Urin- und/oder Brustsekreten nachweisbar sind. Basierend auf dem ermittelten Methylierungsgrad kann zwischen einer benignen Mikrokalzifizierung oder Zyste und einem Mammakarzinom unterschieden werden, so dass in vielen Fällen bei falsch-positiven Befunden des Mammografie-Screenings eine Gewebebiopsie und Operation vermieden werden kann. Etwa zwei Drittel aller Frauen, bei denen im Alter von 40 Jahren mit einem jährlichen Mammographie-Screening beginnen, treten in den ersten zehn Jahren falsch-positive Befunde auf. Bei 7 Prozent kommt es zu einer unnötigen Biopsie [20]. Andererseits können falsch-negative Befunde im Mammografie-Screening durch das erfindungsgemäße Verfahren und entsprechenden Kits vermindert werden.

Das erfindungsgemäße Verfahren und das Kit eignen sich auch zur Differentialdiagnose von Ovarialkarzinomen, insbesondere wenn auf Grund von suspekten Sonografie-Befunden die Frage nach einer benignen Veränderung wie Zysten oder einem Karzinom der Ovarien besteht und nach bisherigem Stand der Technik weitere invasive diagnostische Verfahren eingesetzt werden sollen.

Des Weiteren sind das erfindungsgemäße Verfahren und das Kit bei Vorliegen einer pathologischen Genmutation mit einem erhöhten familiären Risiko für Mamma- und Ovarialkarzinomen, wie z.B im BRCA1 und BRCA2-Gen, als weitere Entscheidungshilfe für eine prophylaktische Mastektomie und/oder Ovariektomie geeignet, insbesondere wenn die Familienplanung der betroffenen Patienten noch nicht beendet ist.

Das erfindungsgemäße Verfahren eröffnet neue Möglichkeiten in der Diagnostik von Tumorerkrankungen, insbesondere in der Frühdiagnostik, da hierdurch einzelne cf-Tumor-DNA-Kopien vor dem großen Hintergrund normaler Wildtyp-DNA in Blut, Urin oder anderen biologischen Proben entsprechend spezifisch vervielfältig werden, bevor die Quantifizierung in der dPCR folgt.

Wenngleich auf Grund der eingeführten Bias das ursprüngliche Verhältnis zwischen methylierten DNA-Fragmenten als Zeichen einer malignen Entartung und der normalen nicht-methylierten Wildtyp-DNA sich verändert, können die mit Hilfe der BBPA-dPCR ermittelten Daten für eine Prognose-Einschätzung (Tumor-Last), Ansprechen auf eine Therapie (Abfall oder Konstanz der fcT-DNA), Resterkrankung *(minimal residual disease)* und der Nachsorge von Tumorpatienten (Rezidiv durch Wiederanstieg von fcT-DNA) eingesetzt werden. Grundlage dafür ist, dass im erfindungsgemäßen Verfahren der Grad die Bias durch Wahl von Annealingtemperatur, Magnesiumkonzentration im Reaktionspuffer und Zyklenanzahl variabel gestaltet werden kann und die Bias unter konstanten Bedingungen zwischen den einzelnen zu untersuchenden Proben gleich groß und damit eine relative Quantifizierung möglich ist.

Ein weiterer Vorteil von Bias-induzierenden Oligonukleotiden (BIP) gegenüber methylspezifischen Oligonukleotiden (MSP) besteht darin, dass die Anzahl normaler Wildtyp-DNA-Fragmente als interne Kontrolle verwendet werden kann. Auch ist durch den Einsatz eines Primerpaares, das die gleichzeitige Quantifizierung methylierter und nicht-methylierter DNA-Sequenzen ermöglicht, ausgeschlossen, dass unterschiedliche Anteile von Zielgen-Fragmenten *(target of interest)* und internen Kontrollgenfragmenten wie ALU-Sequenzen zu falschen Ergebnissen führen.

Gegenstand der Erfindung ist auch die Verwendung der Primer gemäß den Sequenzen 1 bis 19, 53, 61 bis 76, 91 bis 111 sowie 126 bis 144 und die Nukleinsäuresonden gemäß den Sequenzen 20 bis 52, 54 bis 60, 77 bis 90, 112 bis 125 und 145 bis 184 zur Diagnose einer Tumorerkrankung in einer isolierten Probe im erfindingungsgemäßen Verfahren.

Anhand der nachfolgenden Abbildungen und Beispiele wird die Erfindung näher erläutert ohne diese zu beschränken.

Die Abbildungen zeigen:

Fig. 1: Skizze zur bevorzugten Durchführung der erfindungsgemäßen BBPA-dPCR

**Fig. 2:** Kopienanzahl methylierter (methyl) und nicht-methylierter (unmethyl) *RASSF1A*-Sequenzen in Abhängigkeit von der Annealingtemperatur bei 30 Zyklen und 2,5 mmol/l $MgCl_2$-Konzentration in der BBPA in Serumpoolproben gesunder Probandinnen (BA) oder Patientinnen mit Mamma-Karzinom (Mamma-CA, siehe auch Tabelle 2).

**Fig. 3:** Kopienanzahl methylierter (methyl) und nicht-methylierter (unmethyl) *RASSF1A*-Sequenzen in Abhängigkeit von der Zyklenanzahl (0 [d. h. ohne BBPA], 8, 12 und 16) bei 60°C und 2,5 mmol/l $MgCl_2$-Konzentration in der BBPA in Serumpoolproben gesunder Probandinnen (BA) oder Patientinnen mit Mamma-Karzinom (Mamma-CA, siehe auch Tabelle 3).

**Fig. 4:** Kopienanzahl methylierter (methyl) und nicht-methylierter (unmethyl) ***RASSF1A-***Sequenzen in Abhängigkeit von der Zyklenanzahl (0 [d. h. ohne BBPA], 8, 12, 16 [siehe Fig. 3 zuvor] und 40) bei 60°C und 2,5 mmol/l $MgCl_2$-Konzentration in der BBPA in Serumpoolproben gesunder Probandinnen (BA) oder Patientinnen mit Mamma-Karzinom (Mamma-CA, siehe auch Tabelle 3).

**Fig. 5 (Vergleichsdaten):** Anzahl FAM-positiver (obere Abbildung) und HEX-positiver (untere Abbildung) Signale für methylierte und nicht-methylierte **RASSF1A-**Sequenzen in der dPCR ohne BBPA in Serumpoolproben gesunder Probandinnen (BA) und Patientinnen mit Mamma-Karzinom (Mamma-CA). NTC, Non-Template-Kontrolle (ohne DNA). Geschlossene Pfeile zeigen zwei FAM-positive Signale in der Serumpoolprobe von Patientinnen mit Mamma-CA, die offenen Pfeile markieren FAM-positive Signale, die in ihrer Fluoreszenzstärke sowohl in der Serumpoolprobe gesunder Probandinnen und Patientinnen mit Mamma-CA vorkommen. In der unteren Abbildungen sind die HEX-positiven Signale für nicht-methylierte RASSF1A-Sequenzen in den Serumpoolproben gesunder Probandinnen und Patientinnen mit Mamma-CA oberhalb der Grenzlinie zu sehen, die in den NTC-Proben wie auch FAM-positive Signale fehlen. Unterhalb der Grenzlinien sind die Signale der doppelt-negativen Tröpfchen dargestellt. Die Poolproben BA und Mamma-CA sind identisch zu denen in Fig. 2-4 untersuchten Proben.

**Fig. 6 (Vergleichsdaten):** MS-HRM-Analyse des ***RASSF1A***-Methylierungsgrades in Serumpoolproben gesunder Probandinnen (offener Pfeil) und Probandinnen mit Mamma-Karzinom (geschlossener Pfeil) durch Schmelzkurvenanalyse, in der keine methylierten Anteile festzustellen waren. Unterbrochene Linien: Standard-DNA-Proben mit nicht-methylierter (0 %) und methylierter (100 %)-DNA. Die Poolproben BA und Mamma-CA sind identisch zu denen in Fig. 2-4 untersuchten Proben.

**Fig. 7 (Vergleichsdaten):** Relative Häufigkeit (Y-Achse in %) methylierter ***RASSF1A-***Sequenzen in fc-DNA-Proben aus Serum von gesunden Probandinnen (K1-K10, links) und an Mamma-CA erkrankten Patientinnen (P1-P10, rechts) nach dPCR ohne BBPA. Neben den Medianwerten sind die Poisson-Variationsbereiche dargestellt. Die offenen Pfeile zeigen 4 von 10 Proben von Patientinnen mit Mamma-CA, die richtig als pathologisch identifiziert wurden.

**Fig. 8:** Relative Häufigkeit (Y-Achse in %) methylierter **RASSF1A**-Sequenzen in fc-DNA-Proben aus Serum von gesunden Probandinnen (K1-K10, links) und an Mamma-CA erkrankten Patientinnen (P1-P10, rechts) nach BBPA-dPCR (15 Zyklen bei 52°C und 2,5 mmol/l $MgCl_2$-Konzentration unverdünnt in der anschließenden dPCR). Die ausgefüllten Pfeile links zeigen die Werte für die Proben K1-K10 der gesunden Probandinnen und rechts von 2 Proben (P5 und P9), für die keine erhöhten *RASSF1A*-Methylierungen gefunden wurden. Die nicht-ausgefüllten Pfeile zeigen 8 von 10 Proben von Mamma-CA-Patientinnen, für die ein richtig positiver Wert gefunden wurde. Die Ergebnisse der BBPA-dPCR und MS-HRM-Analysen sind in Tabelle 7 zusammengefasst.

**Fig. 9 (Vergleichsdaten):** Methylierungsgrad von **RASSF1A**-Sequenzen in fc-DNA-Proben aus Serum von gesunden Probandinnen (K1-K10, Tabelle 7) und an Mamma-CA erkrankten Patientinnen (P1-P10, Tabelle 7) nach MS-HRM-Analyse. Die Pfeile in C und D zeigen die Proben K8 und K9, die in der MS-HRM schwach positive Methylierungsgrade aufwiesen, in der BBPA-dPCR aber eindeutig negative Ergebnisse lieferten (siehe Fig. 8). Die Probe P1, für die in der BBPA-dPCR ein deutlich erhöhter Methylierungsgrad für *RASSF1A* zugeordnet wurde (Fig. 8), weist in der MS-HRM ein scheinbar negatives Ergebnis auf (E und F). A, C und E: relative Signaländerungen in Beziehung zum 0 %-DNA-Standard; B, D und F: Schmelzkurvencharakteristik der MS-HRM-Amplifikate in Beziehung zu den 0 %- und 100 %-DNA-Standards. Die Ergebnisse der MS-HRM- und BBPA-dPCR-Analysen sind in Tabelle 7 zusammengefasst.

**Fig. 10:** Bestimmung des Methylierungsgrades des **PLA2R1-**Gens (Y-Achse in %) in fc-DNA-Proben aus Serum gesunder Probanden (K1-K10) und Prostatakarzinom-Patienten (P1-P10) mit Hilfe der BBPA-dPCR (35 Zyklen bei 59°C und 2,5 mmol/l $MgCl_2$-Konzentration und 1:$10^4$-Verdünnung mit anschließender dPCR). In den Proben K10 und P3, für die keine Werte angezeigt sind, betrug der Methylierungsgrad jeweils 0 %.

**Fig. 11:** Bestimmung des Methylierungsgrades des **RASSF1A**-Gens (Y-Achse in %) in fc-DNA-Proben aus Serum gesunder Probanden (K1-K10) und PCa-Patienten (P1-P10) mit Hilfe der BBPA-dPCR (50 Zyklen bei 59°C und 2,5 mmol/l $MgCl_2$-Konzentration und 1:$10^4$-Verdünnung mit anschließender dPCR). In den Proben K2-K5, K8, K9 und P8, für die keine Werte angezeigt sind, betrug der Methylierungsgrad jeweils 0 %.

**Fig. 12:** Bestimmung des Methylierungsgrades des ***GSTP1***-Gens (Y-Achse in %) in fc-DNA-Proben aus Serum gesunder Probanden (K11-K20) und Prostatakarzinom-Patienten (P11-P19) mit Hilfe der BBPA-dPCR-Analyse (35 Zyklen bei 57°C und 2,5 mmol/l $MgCl_2$-Konzentration und 1:25 x $10^3$-Verdünnung mit anschließender dPCR bei 51,9°C). In den Proben K12, K12, K16 und K19, für die keine Werte angezeigt sind, betrug der Methylierungsgrad jeweils 0 %.

**Fig. 13:** 2-D-Darstellung der FAM-positiven Signale (methylierte DNA-Sequenzen, Y-Achse) und HEX-positiven Signale (nicht-methylierte DNA-Sequenzen, X-Achse) des ***GSTP1***-Gens in fc-DNA-Proben aus Serum gesunder Probanden (K1-K7, K9 und K10, in A) und von Prostatakarzinom-Patienten (P1 in B, P3 in C und P6 in D, siehe auch Tabelle 8) mit Hilfe der BBPA-dPCR (35 Zyklen bei 57°C und 2,5 mmol/l $MgCl_2$-Konzentration und 1:25 x $10^3$-Verdünnung in anschließender dPCR bei 51,9°C). Die Pfeile zeigen die positiven Tröpfchen, die in den Proben der gesunden Probanden (siehe in A) nicht vorkamen.

**Fig. 14:** ROC-Analyse der mittels BBPA-dPCR bestimmten Methylierungsgrade von *PLA2R1-, RASSF1A-* und GSTP1-Genen allein und in Kombination bei der Untersuchung von fc-DNA-Proben aus Serum gesunder Probanden und Prostatakarzinom-Patienten (die einzelnen Ergebnisse sind in Tabelle 8 zusammengefasst).

**Fig. 15:** Bestimmung des Methylierungsgrades des ***PLA2R1***-Gens (Y-Achse in %) in fc-DNA-Proben aus Serum von Patienten mit PSA-Werten zwischen 3,5-15,0 ng/ml (Ausnahme Probe M2 mit einem PSA-Wert von 3115 ng/ml) mit Hilfe der BBPA-dPCR (15 Zyklen bei 59°C und 2,5 mmol/l $MgCl_2$-Konzentration in der BBPA und unverdünnter Einsatz in der dPCR bei 58,8°C). Neben den Medianwerten sind die Poisson-Variationsbereiche gezeigt. In der Probe M15, für die kein Wert angezeigt ist, betrug der Methylierungsgrad 0 %.

**Fig. 16:** Bestimmung des Methylierungsgrades des ***GSTP1***-Gens (Y-Achse in %) in fc-DNA-Proben aus Serum von Patienten mit PSA-Werten zwischen 3,5-15,0 ng/ml (Ausnahme Probe M2 mit einem PSA-Wert von 3115 ng/ml) mit Hilfe der BBPA-dPCR (15 Zyklen bei 57°C und 2,5 mmol/l $MgCl_2$-Konzentration in der BBPA und unverdünnter Einsatz in der dPCR bei 51,9°C). Neben den Medianwerten sind die Poisson-Variationsbereiche gezeigt. In der Probe M10, für die kein Wert angezeigt ist, betrug der Methylierungsgrad 0 %.

**Fig. 17:** Relative Häufigkeit methylierter ***PLA2R1***-Sequenzen (Y-Achse in %) in fc-DNA-Proben aus Serum von gesunden Probanden (K11-K18) und an Nierenzellkarzinomen erkrankten Patienten (N1-N10) nach BBPA-dPCR (15 Zyklen bei 59°C und 2,5 mmol/l $MgCl_2$-Konzentration und unverdünnt in dPCR bei 58,8°C). Neben den Medianwerten sind die Poisson-Variationsbereiche gezeigt. In den Proben K11, K13-K18, N5 und N9, für die keine Werte angezeigt sind, betrug der Methylierungsgrad 0 %.

**Fig. 18 (Vergleichsdaten):** FAM-Signale (oben) für methylierte und HEX-Signale (unten) für nicht-methylierte ***PLA2R1***-Sequenzen in DNA-Proben aus PrEC, BPH-1, LNCaP, PC-3, DU-145 und U937-Zelllinien und Non-Template-Kontrolle (keine DNA vorgelegt, NTC). Die Bisulfit-umgewandelte DNA wurde ohne BBPA in 40 Zyklen bei 58,8°C in der dPCR quantifizert. Der schmale Pfeil zeigt die durch *mismatch* nachweisbaren heterogen methylierten Epi-Allele in den BPH-1-Zellen, die auch in LNCaP-, PC-3- und DU-145-Zellen nachweisbar waren. Im Vergleich dazu die homogen methylierten Epi-Allele in den Tumorzelllinien LNCaP, PC-3, DU-145 und U937-Zelllinien (dicker Pfeil).

**Fig. 19 (Vergleichsdaten):** FAM-Signale (oben) für methylierte und HEX-Signale (unten) für nicht-methylierte ***RASSF1A***-Sequenzen in DNA-Proben aus PrEC, BPH-1, LNCaP, PC-3, DU-145-Zelllinien, NTC (Non-Template-Kontrolle) und gDNA (genomische DNA-Kontrolle ohne Bisulfit-Umwandlung). Die Bisulfit-umgewandelte DNA wurde ohne BBPA in 40 Zyklen bei 51,9°C in der dPCR quantifiziert. Der schmale Pfeil zeigt 1 Kopie einer homogen methylierten RASSF1A-Sequenz in der PrEC und der dicke Pfeil die zahlreichen Kopien homogen methylierter RASSF1A-Sequenzen in den BPH-1-, LNCaP-, PC-3- und DU-145-Zelllinien. Die homogen methylierten Epi-Allele können von den heterogen methylierten Epi-Allelen, die sich durch *mismatch* nachweisen lassen, insbesondere durch entsprechendes Einstellen der Grenzlinien (*thresholds*), quantifiziert werden (siehe nachfolgende Fig. 20).

**Fig. 20:** Kopienanzahl methylierter und unmethlyierter ***RASSF1A***-Sequenzen (obere Abbildung, Y-Achse = Anzahl Ereignisse) und relative Häufigkeit (Y-Achse in %) homogen methylierter RASSF1A-Sequenzen (untere Abbildung) in PrEC, BPH-1, LNCaP, PC-3 und DU-145 Zellen. Obere Abbildung: Anzahl positiver Signale für homogen methylierte und nicht-methylierte Kopien in PrEC: 1/ 106; BPH-1: 47/ 89; LNCaP: 1397/ 12; PC-3: 1855/ 9, DU-145: 825/ 175, NTC (Non-Template-Kontrolle): 0/ 0 und gDNA (genomische DNA): 0/ 1. Untere Abbildung: Neben den Medianwerten sind die Poisson-Variationsbereiche gezeigt. Die relative homogene *RASSF1A*-Epi-Allel-Methylierung betrug in PrEC 0,9%, BPH-1: 35,0 %, LNCaP: 99,2 %, PC-3: 99,6 %, DU-145: 83,0 %, NTC: 0 % und gDNA: 0 %.

**Fig. 21:** Häufigkeit FAM-positiver (methylierter, obere Abbildung) und HEX-positiver (nicht-methylierter, untere Abbildung) ***SERPINE1***-Sequenzen in PrEC, BPH-1, LNCaP (LN), PC-3, DU-145 (DU)-Zellen und 0 %-, 100 %-DNA-Standards (Qiagen GmbH) und in NTC (Non-Template-Kontrolle). Der schmale Pfeil zeigt die Hauptfraktion heterogen methylierter Epi-Allele (3 von 4 CpG-Stellen methyliert) in der DU-145-Zelllinie im Vergleich zu den homogen methylierten DNA-Sequenzen im 100 %-DNA-Standard (dicker Pfeil). In PrEC und BPH-1 zeigten sich keine 2-, 3- und 4-fach-methylierten Epi-Allele im Gegensatz zu den malignen LNCaP, PC-3 und DU-145-Zellen, die entsprechend quantifizierbar waren (siehe Fig. 22).

**Fig. 22:** Oben: Signalhäufigkeiten (Y-Achse = Anzahl Ereignisse) für methylierte und nicht-methylierte ***SERPINE1***-Sequenzen in PrEC (0 methyliert/ 74 nicht-methyliert), BPH-1 (0/ 270), LNCaP (404/ 123), PC-3 (382/ 1241), DU-145 (1295/ 1), 0 %-DNA-Standard (0/ 947), 100 %-DNA-Standard (827/ 6) und Non-Template-Kontrolle (NTC: 0/ 1). Die Pfeile zeigen die nicht nachweisbaren Kopien methylierter ***SERPINE1***-Sequenzen in PrEC und der BPH-1-Zelllinie. Unten: relative Häufigkeit (Y-Achse in %) aller, d. h. sowohl homogen als auch heterogen methylierter Epi-Allele des *SERPINE1-Gens* in PrEC (0 %), BPH-1 (0 %), LNCaP (77 %), PC-3 (22,6 %), DU-145 (99,93 %), 0 %-DNA-Standard (0 %), 100 %-DNA-Standard (99,3 %) und NTC: 0 %. Die Pfeile zeigen die 0 % methylierter SERPINE1-Sequenzen in PrEC und der BPH-1-Zelllinie.

**Fig. 23:** Häufigkeit methylierter (obere Abbildung) und nicht-methylierter (untere Abbildung) ***GSTP1-***Sequenzen in PrEC, BPH-1, LNCaP (LN), PC-3, DU-145 (DU)-Zellen und 0 %-, 100 %-DNA-Standards und NTC (Non-Template-Kontrolle). Die schmalen Pfeile zeigen heterogen methylierte Epi-Allele (1 von 3 CpG-Stellen methyliert) in PrEC und BPH-1 im Gegensatz zu den überwiegend homogenen 3-fach methylierten Epi-Allelen in LNCaP-Zellen (dicke Pfeil) und 2-fach heterogen methylierten Allelen in PC-3-Zellen (2. senkrechter dicker Pfeil).

**Fig. 24 Oben:** Signalhäufigkeiten (Y-Achse = Anzahl Ereignisse) für methylierte und nicht-methylierte ***GSTP1***-Sequenzen in PrEC (3 methyliert/ 138 nicht-methyliert), BPH-1 (8/ 532), LNCaP (1777/ 22), PC-3 (987/ 2460), DU-145 (490/ 1947), 0 %-DNA-Standard (1/ 279), 100 %-DNA-Standard (200/ 37) und Non-Template-Kontrolle (NTC: 0/ 0). Unten: relative Häufigkeit (Y-Achse in %) methylierter GSTP1-Sequenzen in PrEC (2,1 %), BPH-1 (1,4 %), LNCaP (98,84 %), PC-3 (27,5 %) DU-145 (19,2 %), 0 %-DNA-Standard (0,4 %), 100 %-DNA-Standard (84,5 %) und NTC (0 %).

**Fig. 25:** 2-D-Darstellung der FAM- und HEX-positiven Signale bei der Bestimmung der ***GSTP1***-Methylierung (siehe auch Fig. 23). In der oberen Abbildung sind deutlich zwei Populationen von Tröpfchen mit homogen (obere Fraktion) und heterogen (untere Fraktion, 2 von 3 CpG-Stellen methyliert) methylierten Epi-Allelen sichtbar (dicke Pfeile). Untere Abbildung: Werden nur diese zwei Populationen von Tröpfchen mit homogen (obere Fraktion) und heterogen (untere Fraktion) mit 2 von 3 methylierten CpG-Stellen methylierten Epi-Allele quantifiziert (siehe Trennlinien in oberer und unterer Abbildung), zeigen sich in den PrEC und BPH-1-Zellen keinerlei methylierte GSTP1-Sequenzen.

**Fig. 26:** Oben: Signalhäufigkeiten (Y-Achse = Anzahl Ereignisse). Daraus ergaben sich folgende positive FAM- und HEX-Signale für methylierte und nicht-methylierte ***GSTP1-***Sequenzen in PrEC (0/ 141), BPH-1 (0/ 534), LNCaP (1488/ 22), PC-3 (682/ 2515), DU145 (312/ 1982), 0 %- (1/ 295), 100 %-DNA-Standard (155/ 46) und NTC (0/ 0). Unten: Relative Häufigkeiten (Y-Achse in %) der methylierten GSTP1-Sequenzen in PrEC (0 %), BPH-1 (0 %), LNCaP (98,6 %), PC-3 (20,2 %), DU-145 (12,9 %), 0 % (0,3 %), 100 %-DNA-Standard (77 %) und NTC (0 %). Im Vergleich dazu betrug die relative Häufigkeit 2,1% für PrEC und 1,4% für BPH-1-Zellen bei der entsprechenden Grenzeinstellung (threshold) in Fig. 23 und 24. Die 100 %-Standard-DNA ist für *GSTP1* nur zu 77 % vollständig methyliert, wie bereits in Fig. 25 ersichtlich war (untere Abbildung mit zwei dicken Pfeilen rechts markiert).

**Fig. 27:** 2-D-Darstellung der FAM- und HEX-positiven Signale bei der Bestimmung der ***PLA2R1***-Methylierung in Serumproben gesunder Probanden (K1-K20) und Patienten mit Prostatakarzinomen (P1-P40) nach 15 Zyklen in der BBPA bei 59°C und 2,5 mM $MgCl_2$-Konzentration und anschließend in der dPCR. Die Grenzen (*thresholds*) für die FAM- und HEX-positiven Signale wurden so eingestellt, dass alle drei mit Pfeilen markierten Fraktionen der FAM-positiven homogen als auch heterogen methylierten PLA2R1-Epi-Allelen in die Berechnungen (Tab. 12) einbezogen wurden.

**Fig. 28:** 2-D-Darstellung der FAM- und HEX-positiven Signale bei der Bestimmung der ***PLA2R1***-Methylierung in Serumproben gesunder Probanden (K1-K20) und Patienten mit Prostatakarzinomen (P1-P40) nach 15 Zyklen in der BBPA bei 59°C und 2,5 mM $MgCl_2$-Konzentration und anschließend in der dPCR. Die Grenzen (*thresholds*) für die FAM- und HEX-positiven Signale wurden so eingestellt, dass nur die mit dem Pfeil markierte Fraktion des FAM-positiven homogen methylierten PLA2R1-Epi-Allels in die Berechnungen (Tab. 12) einbezogen wurde.

**Fig. 29:** 2-D-Darstellung der FAM- und HEX-positiven Signale bei der Bestimmung der ***PLA2R1***-Methylierung in Serumproben gesunder Probanden (K1-K20) und Patienten mit Prostatakarzinomen (P1-P40) nach 15 Zyklen in der BBPA bei 59°C und 2,5 mM $MgCl_2$-Konzentration und anschließend in der dPCR. Die Grenzen (*thresholds*) für die FAM- und HEX-positiven Signale wurden so eingestellt, dass nur zwei mit Pfeilen markierten Fraktionen der FAM-positiven homogen als auch heterogen (2 von 3 CpG-Stellen) methylierten PLA2R1-Epi-Allele in die Berechnungen (Tab. 12) einbezogen wurden.

**Fig. 30:** Anzahl FAM-positiver Signale für methylierte ***PLA2R1-***Sequenzen ohne BBPA (Vergleichsdaten) und nach BBPA mit 50 Zyklen bei 63,0°C und 2,5 mmol/l $MgCl_2$-Konzentration in normalen Epithelzellen der Prostata (PrEC), benigner Prostatahyperplasie-Zelllinie (BPH-1) und malignen Prostatazelllinien LNCaP, PC-3 und DU-145. Offene Pfeile zeigen positive Signale in DNA-Proben aus PrEC und BPH-1 ohne BBPA im Vergleich nach BBPA; ausgefüllte Pfeile zeigen positive Signale in DNA-Proben aus LNCaP, PC-3 und DU-145 vor und nach BBPA; der Stern markiert die Grenze (*threshold*) zwischen negativen und positiven FAM-Signalen.

**Fig. 31:** Kopienanzahl (Y-Achse) methylierter (methyl) und nicht-methylierter (unmethyl) ***PLA2R1***-Sequenzen ohne BBPA (Vergleichsdaten) und nach BBPA mit 15, 30, 40 und 50 Zyklen bei 63,0°C und 2,5 mmol/l $MgCl_2$-Konzentration in normalen Epithelzellen der Prostata (PrEC), benigner Prostatahyperplasie-Zelllinie (BPH-1) und malignen Prostatazelllinien LNCaP, PC-3 und DU-145. Nach BBPA wurden 1 $\mu$l der 25 $\mu$l-PCR-Ansätze in die dPCR gegeben. Um die Kopienanzahl mit denen ohne BBPA vergleichen zu können, wurden diese mit 25 multipliziert.

**Fig. 32:** Anzahl FAM-positiver (Y-Achse, für methyliert) und HEX-positive (X-Achse, für nicht-methyliert) Signale für ***PLA2R1***-Sequenzen nach BBPA mit 50 Zyklen bei 63,0°C und 2,5 mmol/l $MgCl_2$-Konzentration in normalen Epithelzellen der Prostata (PrEC), benigner Prostatahyperplasie-Zelllinie (BPH-1) und maligenen Prostatazelllinien LNCaP, PC-3 und DU-145. Die Pfeile zeigen die Punktwolken für die PrEC, BPH-1, LNCaP, PC-3 und DU-145-Zelllinien und der Non-Template-Kontrolle (ohne DNA, NTC). Die NTC sind durch den unteren Pfeil ohne Bezeichnung markiert und die einzelnen Werte sind in Tab. 20 zusammengefasst.

**Fig. 33:** Anzahl FAM-positiver (Y-Achse, für methylierte) und HEX-positive (X-Achse, für nicht-methylierte) Signale für ***PLA2R1***-Sequenzen nach BBPA mit 50 Zyklen bei 63,0°C in normalen Epithelzellen der Prostata (PrEC), benigner Prostatahyperplasie-Zelllinie (BPH-1) und maligenen Prostatazelllinien LNCaP, PC-3 und DU-145. Die Pfeile zeigen die Punktwolken für die PrEC, BPH-1, LNCaP, PC-3 und DU-145-Zelllinien. Im Vergleich zur Fig. 29 ist die Non-Template-Kontrolle nicht dargestellt, wodurch deutlich wird, dass alle Proben keine doppelt-negativen Tröpfchen in der dPCR mehr enthielten. Die einzelnen Werte sind in Tab. 20 zusammengefasst.

**Fig. 34:** Kopienanzahl methylierter (ausgefüllte Kreise) und nicht-methylierter (offene Kreise) PLA2R1-DNA-Fragmente nach 16 Zyklen Prä-Amplifikation bei steigenden Annealingtemperaturen (50-63°C) in Abhängigkeit von der $MgCl_2$-Konzentration (1,5 mmol/l - 8,0 mmol/l) mit dem Primerpaar 168bp (SEQ ID: 1 und 2) und anschließender dPCR.

**Fig. 35:** Kopienanzahl methylierter (ausgefüllte Kreise) und nicht-methylierter (offene Kreise) PLA2R1-DNA-Fragmente nach 16 Zyklen Prä-Amplifikation bei steigenden Annealingtemperaturen (50-63°C) in Abhängigkeit von

$MgCl_2$-Konzentration (1,5 mmol/l - 8,0 mmol/l) mit dem Primerpaar 161bp (SEQ ID: 53 und 10) und anschließender dPCR.

**Fig. 36:** Kopienanzahl methylierter (ausgefüllte Kreise) und nicht-methylierter (offene Kreise) PLA2R1-DNA-Fragmente nach 16 Zyklen Prä-Amplifikation bei steigenden Annealingtemperaturen (50-63°C) in Abhängigkeit von der $MgCl_2$-Konzentration (1,5 mmol/l - 8,0 mmol/l) mit dem Primerpaar 150bp (SEQ ID: 53 und 9) und anschließender dPCR.

**Fig. 37:** Kopienanzahl methylierter (ausgefüllte Kreise) und nicht-methylierter (offene Kreise) PLA2R1-DNA-Fragmente nach 16 Zyklen Prä-Amplifikation bei steigenden Annealingtemperaturen (50-63°C) in Abhängigkeit von der $MgCl_2$-Konzentrationen (1,5 mmol/l - 8,0 mmol/l) mit dem Primerpaar 133bp (SEQ ID: 7 und 8) und anschließender dPCR.

**Fig. 38:** FAM-Signale (methylierte PLA2R1-DNA-Fragmente [obere Abbildung]) und HEX-Signale (nicht-methylierte PLA2R1-DNA-Fragmente [untere Abbildung] nach 15 (links vom Trennstrich) und 50 Zyklen Prä-Amplifikation (rechts vom Trennstrich) in Proben mit 3000 (Bahn 1), 20 (Bahn 2), 10 (Bahn 3), 5 (Bahn 4) und keiner Kopie methylierter PLA2R1-DNA-Fragmente (Bahn 5) und jeweils einem Anteil von 70.000 Kopien nicht-methylierter PLA2R1-DNA-Fragmente mit dem Primerpaar 133 bp (SEQ ID: 7 und 8) bei 6,0 mM $MgCl_2$-Konzentration und einer Annealingtemperatur von 63°C. NTC: Non-Template-Negativkontrolle. Die Rohdaten sind in Tab. 28 und 30 aufgeführt.

**Fig. 39:** Anzahl der positiven Tröpfchen nach 15 (links vom Trennstrich) und 50 Zyklen Prä-Amplifikation (rechts vom Trennstrich) in Proben mit 3000 (Bahn 1), 20 (Bahn 2), 10 (Bahn 3), 5 (Bahn 4) und keiner Kopie methylierter PLA2R1-DNA-Fragmente (Bahn 5) und jeweils einem Anteil von 70.000 Kopien nicht-methylierter PLA2R1-DNA-Fragmente mit dem Primerpaar 133 bp (SEQ ID: 7 und 8) bei 6,0 mmol/l $MgCl_2$-Konzentration und einer Annealingtemperatur von 63°C. NTC: Non-Template-Negativkontrolle. Die Rohdaten sind in Tab. 28 und 30 aufgeführt.

**Fig. 40:** FAM-Signale (methylierte PLA2R1-DNA-Fragmente [obere Abbildung]) und HEX-Signale (nicht-methylierte PLA2R1-DNA-Fragmente [untere Abbildung] nach 15 (links vom Trennstrich) und 50 Zyklen Prä-Amplifikation (rechts vom Trennstrich) in Proben mit 3000 (Bahn 1), 20 (Bahn 2), 10 (Bahn 3), 5 (Bahn 4) und keiner Kopie methylierter PLA2R1-DNA-Fragmente (Bahn 5) und jeweils einem Anteil von 175.000 Kopien nicht-nmethylierter PLA2R1-DNA-Fragmente mit dem Primerpaar 133 bp (SEQ ID: 7 und 8) bei 6,0 mmol/l $MgCl_2$-Konzentration und einer Annealingtemperatur von 63°C. NTC: Non-Template-Negativkontrolle. Die Rohdaten sind in Tab. 28 und 30 aufgeführt.

**Fig. 41:** Anzahl der positiven Tröpfchen nach 15 (links vom Trennstrich) und 50 Zyklen Prä-Amplifikation (rechts vom Trennstrich) in Proben mit 3000 (Bahn 1), 20 (Bahn 2), 10 (Bahn 3), 5 (Bahn 4) und keiner Kopie methylierter PLA2R1-DNA-Fragmente (Bahn 5) und jeweils einem Anteil von 175.000 Kopien nicht-methylierter PLA2R1-DNA-Fragmente mit dem Primerpaar 133 bp (SEQ ID: 7 und 8) bei 6,0 mmol/l $MgCl_2$-Konzentration und einer Annealingtemperatur von 63°C. NTC: Non-Template-Negativkontrolle. Die Rohdaten sind in Tab. 28 und 30 aufgeführt.

**Fig. 42:** FAM-Signale (methylierte PLA2R1-DNA-Fragmente [obere Abbildung]) und HEX-Signale (nicht-methylierte PLA2R1-DNA-Fragmente [untere Abbildung] nach 15 (links vom Trennstrich) und 50 Zyklen Prä-Amplifikation (rechts vom Trennstrich) in Proben mit 3000 (Bahn 1), 20 (Bahn 2), 10 (Bahn 3), 5 (Bahn 4) und keiner Kopie methylierter PLA2R1-DNA-Fragmente (Bahn 5) und jeweils einem Anteil von 350.000 Kopien nicht-methylierter PLA2R1-DNA-Fragmente mit dem Primerpaar 133 bp (SEQ ID: 7 und 8) bei 6,0 mmol/l $MgCl_2$-Konzentration und einer Annealingtemperatur von 63°C. NTC: Non-Template-Negativkontrolle. Die Rohdaten sind in Tab. 28 und 30 aufgeführt.

**Fig. 43:** Anzahl der positiven Tröpfchen nach 15 (links vom Trennstrich) und 50 Zyklen Prä-Amplifikation (rechts vom Trennstrich) in Proben mit 3000 (Bahn 1), 20 (Bahn 2), 10 (Bahn 3), 5 (Bahn 4) und keiner Kopie methylierter PLA2R1-DNA-Fragmente (Bahn 5) und jeweils einem Anteil von 350.000 Kopien nicht-methylierter PLA2R1-DNA-Fragmente mit dem Primerpaar 133 bp (SEQ ID: 7 und 8) bei 6,0 mmol/l $MgCl_2$-Konzentration und einer Annealingtemperatur von 63°C. NTC: Non-Template-Negativkontrolle. Die Rohdaten sind in Tab. 28 und 30 aufgeführt.

**Fig. 44:** FAM-Signale (methylierte PLA2R1-DNA-Fragmente [obere Abbildung]) und HEX-Signale (nicht-methylierte PLA2R1-DNA-Fragmente [untere Abbildung] nach 15 (links vom Trennstrich) und 50 Zyklen Prä-Amplifikation (rechts vom Trennstrich) in Proben mit 3000 (Bahn 1), 20 (Bahn 2), 10 (Bahn 3), 5 (Bahn 4) und keiner Kopie methylierter PLA2R1-DNA-Fragmente (Bahn 5) und jeweils einem Anteil von 700.000 Kopien nicht-methylierter PLA2R1-DNA-Fragmente mit dem Primerpaar 133 bp (SEQ ID: 7 und 8) bei 6,0 mM $MgCl_2$-Konzentration und einer Annealingtemperatur von 63°C. NTC: Non-Template-Negativkontrolle. Die Rohdaten sind in Tab. 28 und 30 aufgeführt.

**Fig. 45:** Anzahl der positiven Tröpfchen nach 15 (links vom Trennstrich) und 50 Zyklen Prä-Amplifikation (rechts vom Trennstrich) in Proben mit 3000 (Bahn 1), 20 (Bahn 2), 10 (Bahn 3), 5 (Bahn 4) und keiner Kopie methylierter PLA2R1-DNA-Fragmente (Bahn 5) und jeweils einem Anteil von 700.000 Kopien nicht-methylierter PLA2R1-DNA-Fragmente mit dem Primerpaar 133 bp (SEQ ID: 7 und 8) bei 6,0 mmol/l $MgCl_2$-Konzentration und einer Annealingtemperatur von 63°C. NTC: Non-Template-Negativkontrolle. Die Rohdaten sind in Tab. 28 und 30 aufgeführt.

**Fig. 46:** FAM-Signale (methylierte PLA2R1-DNA-Fragmente, obere Abbildung) und HEX-Signale (nicht-methylierte PLA2R1-DNA-Fragmente, untere Abbildung) aus normalen PrEC (Position 1), benigne BPH-Zellen (Position 2), malignen LNCaP (Position 3), PC-3 (Position 4) und DU-145-Zellen (Position 5) nach 50 Zyklen Prä-Amplifikation bei 2,5 mM $MgCl_2$-Konzentration bei steigenenden Annealingtemperaturen (50-63°C) mit dem Primerpaar 168bp (SEQ ID: 1 und 2) und anschließender dPCR. Die Pfeile zeigen den Verlauf der FAM- und HEX-Signale in Abhängigkeit der Annealingtemperaturen für die PC-3-Zellen.

**Fig. 47:** FAM-Signale (methylierte PLA2R1-DNA-Fragmente, obere Abbildung) und HEX-Signale (nicht-methylierte PLA2R1-DNA-Fragmente, untere Abbildung) aus normalen PrEC (Position 1), benigne BPH-Zellen (Position 2), malignen LNCaP (Position 3), PC-3 (Position 4) und DU-145-Zellen (Position 5) nach 50 Zyklen Prä-Amplifikation bei 2,5 mmol/l $MgCl_2$-Konzentration bei steigenenden Annealingtemperaturen (50-63°C) mit dem Primerpaar 161bp (SEQ ID: 53 und 10) und anschließender dPCR. Die Pfeile zeigen den Verlauf der FAM- und HEX-Signale in Abhängigkeit der Annealingtemperaturen für die PC-3-Zellen.

**Fig. 48:** FAM-Signale (methylierte PLA2R1-DNA-Fragmente, obere Abbildung) und HEX-Signale (nicht-methylierte PLA2R1-DNA-Fragmente, untere Abbildung) aus normalen PrEC (Position 1), benigne BPH-Zellen (Position 2), malignen LNCaP (Position 3), PC-3 (Position 4) und DU-145-Zellen (Position 5) nach 50 Zyklen Prä-Amplifikation bei 2,5 mmol/l $MgCl_2$-Konzentration bei steigenenden Annealingtemperaturen (50-63°C) mit dem Primerpaar 150bp (SEQ ID: 53 und 9) und anschließender dPCR. Die Pfeile zeigen den Verlauf der FAM- und HEX-Signale in Abhängigkeit der Annealingtemperaturen für die PC-3-Zellen.

**Fig. 49:** FAM-Signale (methylierte PLA2R1-DNA-Fragmente, obere Abbildung) und HEX-Signale (nicht-methylierte PLA2R1-DNA-Fragmente, untere Abbildung) aus normalen PrEC (Position 1), benigne BPH-Zellen (Position 2), malignen LNCaP (Position 3), PC-3 (Position 4) und DU-145-Zellen (Position 5) nach 50 Zyklen Prä-Amplifikation bei 2,5 mmol/l $MgCl_2$-Konzentration bei steigenenden Annealingtemperaturen (50-63°C) mit dem Primerpaar 133bp (SEQ ID: 7 und 8) und anschließender dPCR. Die Pfeile zeigen den Verlauf der FAM-Signale bis 52,6°C für die PC-3-Zellen und oberhalb von 52,6°C für PrEC und BPH-1.

**Fig. 50:** FAM-Signale (methylierte GSTP1-DNA-Fragmente, obere Abbildung) und HEX-Signale (nicht-methylierte GSTP1-DNA-Fragmente, untere Abbildung) aus normalen PrEC (Position 1), benigne BPH-Zellen (Position 2), malignen LNCaP (Position 3), PC-3 (Position 4) und DU-145-Zellen (Position 5) nach 50 Zyklen Prä-Amplifikation bei 2,5 mmol/l $MgCl_2$-Konzentrationen bei steigenenden Annealingtemperaturen (50-63°C) mit dem Primerpaar 120bp (SEQ ID: 5 und 6) und anschließender dPCR. Die Pfeile zeigen den Verlauf der FAM-Signale bis 60,8°C für die PC-3-Zellen und oberhalb von 60,8°C für PrEC und BPH-1.

Fig. 51: FAM-Signale (methylierte GSTP1-DNA-Fragmente) in Abhängigkeit von der Zyklenanzahl (20x, 30x, 40x und 50x) und der $MgCl_2$-Konzentration im Reaktionspuffer (1,5; 2,5; 4,5 und 6,0 mmol/l) in der Prä-Amplifikation mit dem Primerpaar 120 bp (SEQ ID: 5 und 6) bei einer Annealingtemperatur von 50,7°C. Bahn 1: 0% methylierte Standard-DNA, Bahn 2: 50% methylierte Standard-DNA und Bahn 3: Non-Template-Negativkontrolle. Die dünnen Pfeile zeigen die Streusignale bei 0% methylierter Standard-DNA und Non-Template-Negativkontrollen und die dicken Pfeile die steigende Signalintensitäten der 50% methylierten Standard-DNA mit steigender Zyklenanzahl (unten dargestellt).

**Fig. 52:** FAM-Signale (methylierte GSTP1-DNA-Fragmente) in Abhängigkeit von der Zyklenanzahl (20x, 30x, 40x und 50x) und der $MgCl_2$-Konzentration im Reaktionspuffer (1,5; 2,5; 4,5 und 6,0 mmol/l) in der Prä-Amplifikation mit dem Primerpaar 116 bp (SEQ ID: 84 und 85) bei einer Annealingtemperatur von 53,8°C. Bahn 1: 0% methylierte Standard-DNA, Bahn 2: 50% methylierte Standard-DNA und Bahn 3: Non-Template-Negativkontrolle. Die Pfeile zeigen die Intensitäten der FAM-Signale der 50% methylierten Standard-DNA in Abhängigkeit von der Zyklenanzahl in den Proben mit 1,5 und 6,0 mmol/l $MgCl_2$-Konzentrationen.

**Fig. 53:** FAM-Signale (methylierte GSTP1-DNA-Fragmente, obere Abbildung) und HEX-Signale (nicht-methylierte GSTP1-DNA-Fragmente, untere Abbildung) in Serumproben gesunder Probandinnen (GF1-GF20, links getrennt durch 1. Trennungslinie) und Serumproben von Patientinnen mit Mammakarzinom (MF1-MF20, rechts von der 1. Tennungslinie) und Non-Template-Kontrolle (NTC, ohne DNA bzw. genomische DNA ohne Bisulfitumwandlung, jeweils rechts der 2. Trennungslinie) nach 15 Zyklen in der BBPA mit dem GSTP1-116bp-Primern (SEQ ID: 93 und 94) und 1,5 mmol/l $MgCl_2$-Konzentration bei 53,8°C und anschließender dPCR. Die Pfeile in der oberen Abbildung zeigen die zusätzlichen Patientenproben, die bei einer $MgCl_2$-Konzentration von 4,5 mM (Fig. 54) positive Ergebnisse für die GSTP1-Methylierung lieferten und die Pfeile in der unteren Abbildung die schwachen HEX-Signale, die bei einer $MgCl_2$-Konzentration von 1,5 mM erhalten wurden.

**Fig. 54:** FAM-Signale (methylierte GSTP1-DNA-Fragmente, obere Abbildung) und HEX-Signale (nicht-methylierte GSTP1-DNA-Fragmente, untere Abbildung) in Serumproben gesunder Probandinnen (GF1-GF20, links getrennt durch 1. Trennungslinie) und Serumproben von Patientinnen mit Mammakarzinom (MF1-MF20, rechts von der 1. Tennungslinie) und Non-Template-Kontrolle (NTC, ohne DNA bzw. genomische DNA ohne Bisulfitumwandlung, jeweils rechts der 2. Trennungslinie) nach 15 Zyklen in der BBPA mit dem GSTP1-116bp-Primern (SEQ ID: 93 und 94) und 4,5 mmol/l $MgCl_2$-Konzentration bei 53,8°C und anschließender dPCR. Die Pfeile in der oberen Abbildung zeigen die zusätzlichen Patientenproben, die bei einer $MgCl_2$-Konzentration von 4,5 mM im Vergleich zu 1,5 mM (Fig. 53) positive Ergebnisse für die GSTP1-Methylierung lieferten und die Pfeile in der unteren Abbildung die starken HEX-Signale, die bei einer $MgCl_2$-Konzentration von 4,5 mM erhalten wurden.

**Fig. 55:** FAM-Signale (methylierte GSTP1-DNA-Fragmente, obere Abbildung) und HEX-Signale (nicht-methylierte GSTP1-DNA-Fragmente, untere Abbildung) in Serumproben gesunder Probanden (GM1-GM20, links getrennt durch 1. Trennlinie), Non-Template-Kontrolle (NTC, ohne DNA in der ddPCR, rechts von der 1. Trennlinie), Serumproben von Patienten mit Prostatakarzinom (PCa; PPCa1-PPCa20, rechts von der 2. Tennlinie) und Non-Template-Kontrollen (NTC, ohne DNA bzw. genomische DNA ohne Bisulfitumwandlung, rechts der 3. Trennlinie) nach 15 Zyklen in der BBPA mit dem GSTP1-120bp-Primern (SEQ ID: 5 und 5) und 2,5 mmol/l $MgCl_2$-Konzentration bei 50,7°C und anschließender dPCR. Die Pfeile zeigen die Serumproben der Patienten mit PCa an, in denen sich die FAM-Signale signifikant von denen in den Serumproben der gesunden Probanden unterscheiden lassen (Tab. 13).

**Fig. 56:** FAM-Signale (methylierte GSTP1-DNA-Fragmente, obere Abbildung) und HEX-Signale (nicht-methylierte GSTP1-DNA-Fragmente, untere Abbildung) in Serumproben gesunder Probanden (GM1-GM20, links getrennt durch 1. Trennlinie), Non-Template-Kontrolle (NTC, ohne DNA in der ddPCR, rechts von der 1. Trennlinie), Serumproben von Patienten mit Prostatakarzinom (PPCa1-PPCa20, rechts von der 2. Tennlinie) und Non-Template-Kontrollen (NTC, ohne DNA bzw. genomische DNA ohne Bisulfitumwandlung, rechts der 3. Trennlinie) nach 15 Zyklen in der BBPA mit dem GSTP1-116bp-Primern (SEQ ID: 93 und 94) und 4,5 mmol/l $MgCl_2$-Konzentration bei 53,8°C und anschließender dPCR. Die Pfeile zeigen die Serumproben der Patienten mit PCa an, in denen sich die FAM-Signale signifikant von denen in den Serumproben der gesunden Probanden unterscheiden lassen (Tab. 14).

**Fig. 57:** FAM-Signale (methylierte SERPINE1-DNA-Fragmente, obere Abbildung) und HEX-Signale (nicht-methylierte *SERPINE1*-DNA-Fragmente, untere Abbildung) in Serumproben gesunder Probanden (GM1-GM20, links getrennt durch 1. Trennlinie), Serumproben von Patienten mit Prostatakarzinom (PPCa1-PPCa20, rechts von der 1. Tennlinie) und Non-Template-Kontrollen (NTC, ohne DNA bzw. genomische DNA ohne Bisulfitumwandlung, rechts der 2. Trennlinie) nach 15 Zyklen in der BBPA mit dem *SERPINE1*-123bp-Primern (SEQ ID: 16 und 17) und 3,5 mM $MgCl_2$-Konzentration bei 52,0°C und anschließender dPCR. Die Pfeile zeigen die Serumproben der Patienten mit PCa an, in denen sich die FAM-Signale signifikant von denen in den Serumproben der gesunden Probanden unterscheiden lassen (Tab. 15).

**Fig. 58:** FAM-Signale (methylierte SERPINE1-DNA-Fragmente, obere Abbildung) und HEX-Signale (nicht-methylierte *SERPINE1*-DNA-Fragmente, untere Abbildung) in Serumproben gesunder Probanden (GM1-GM20, links getrennt durch 1. Trennlinie), Serumproben von Patienten mit Prostatakarzinom (PPCa1-PPCa20, rechts von der 1. Tennlinie) und Non-Template-Kontrollen (NTC, ohne DNA bzw. genomische DNA ohne Bisulfitumwandlung, rechts der 2. Trennlinie) nach 50 Zyklen in der BBPA mit dem *SERPINE1*-123bp-Primern (SEQ ID: 16 und 17) und 3,5 mmol/l $MgCl_2$-Konzentration bei 52,0°C und anschließender dPCR. Die Pfeile zeigen die Serumproben der Patienten mit PCa an, in denen sich die FAM-Signale signifikant von denen in den Serumproben der gesunden Probanden unterscheiden lassen (Tab. 16).

**Fig. 59:** FAM-Signale (methylierte AOX1-DNA-Fragmente, obere Abbildung) und HEX-Signale (nicht-methylierte AOX1-DNA-Fragmente, untere Abbildung) in Serumproben gesunder Probanden (GM1-GM20, links getrennt durch 1. Trennlinie), Serumproben von Patienten mit Prostatakarzinom (PPCa1-PPCa20, rechts von der 1. Tennlinie) und Non-Template-Kontrollen (NTC, ohne DNA bzw. genomische DNA ohne Bisulfitumwandlung, rechts der 2. Trennlinie) nach 15 Zyklen in der BBPA mit dem AOX1-138bp-Primern (SEQ ID: 18 und 19) und 2,5 mmol/l $MgCl_2$-Konzentration bei 50,0°C und anschließender dPCR. Die Pfeile zeigen die FAM-Signale in Serumproben von 3 gesunden Probanden und einem PCa-Patienten an, die nach 30 Zyklen in der BBPA deutlich in ihrer Intensität abfielen (Fig. 60 und Tab. 17 und 18).

**Fig. 60:** FAM-Signale (methylierte AOX1-DNA-Fragmente, obere Abbildung) und HEX-Signale (nicht-methylierte AOX1-DNA-Fragmente, untere Abbildung) in Serumproben gesunder Probanden (GM1-GM20, links von der 1. Trennlinie dargestellt), Serumproben von Patienten mit Prostatakarzinom (PPCa1-PPCa20, rechts von der 1. Tennlinie) und Non-Template-Kontrollen (NTC, ohne DNA bzw. genomische DNA ohne Bisulfitumwandlung, rechts der 2. Trennlinie) nach 30 Zyklen in der BBPA mit dem AOX1-138bp-Primern (SEQ ID: 18 und 19) und 2,5 mmol/l $MgCl_2$-Konzentration bei 50,0°C und anschließender dPCR. Die Pfeile zeigen die FAM-Signale in Serumproben von gesunden Probanden und einem PCa-Patienten an, die im Vergleich zu 15 Zyklen (Fig. 59) nach 30 Zyklen in der BBPA deutlich in ihrer Intensität abfielen. Der Pfeil rechts zeigt den Befund des Patienten PPCa14 an, bei dem nach 15 Zyklen in der BBPA noch deutlich erhöhte Werte sichtbar waren und nach 30 Zyklen im Gegensatz z.B. der Patienten PPCa12 und 17 deutlich vermindert und von denen der gesunden Probanden GM12 und 16 nicht mehr zu unterscheiden waren. Wird der Grenzwert *(threshold)* so eingestellt, dass diese Proben keine FAM-positiven Signale aufweisen, zeigen 7 Serumproben von Patienten mit PCa deutlich erhöhte FAM-Signalwerte und unterscheiden sich signifikant von denen gesunder Probanden (Tab. 17 und 18).

**Fig. 61:** Box-Blot für die PSA-Konzentrationswerte (ng/ml), die im Serum von 20 Patienten mit benigner Prostatahyperplasie (BPH, links Nr. 1) und 20 Patienten mit PCa (rechts Nr. 2) ermittelt wurden und sich nicht signifikant unterschieden ($p<0,552$).

**Allgemeine Vorschriften:**

**Isolierung und Bisulfit-Unwandlung frei-zirkulierender DNA (fc-DNA):** Als Erstes wird die frei-zirkulierende DNA (fc-DNA) aus der zu untersuchenden Probe, wie z. B. aus 1-5 ml Serumproben mit Hilfe des QIAamp Circulating Nucleis Acid Kit von Qiagen GmbH (Hilden, BRD) entsprechend der Testkitbeschreibung isoliert und in jew. 25 μl eluiert.

**Bisulfit-Umwandlung der fc-DNA:** Die Bisulfit-Umwandlung von jew. 20 μl der fc-DNA erfolgte mit Hilfe des EpiTect Fast Bisulfit Conversion Kit von Qiagen GmbH entsprechend der Testkitbeschreibung. Nach Eluation erhält man jew. 15 μl Bisulfit-behandelte fc-DNA-Lösung.

**Bias-basierte Prä-Amplifikation (BBPA) der Proben:**

Nach der Bisulfit-Umwandlung wurden die DNA-Konzentrationen der DNA-Proben mit Hilfe des Quantus™ Fluorometers (Promega) bestimmt. Lag die DNA-Konzentration unterhalb oder oberhalb von 1 ng/μl, wurden 2 bzw. 1 μl der Bisulfit-behandelten DNA in die BBPA eingesetzt.

*Mastermix für die BBPA:*

| Reagenz | Vol. [μl] | Endkonzentration. |
|---|---|---|
| PCR-Buffer [15 mM MgCl] | 2,50 | 1,5 mmol/l |
| dNTPs [2,5 mM] | 2,00 | 200 μmol/l |
| *forward* Primer | 1,00 | 400 nmol/l |
| *reverse* Primer | 1,00 | 400 nmol/l |
| HotStarTaq Plus [5 U/μl] | 0,125 | 0,625 U |
| $MgCl_2$ [25 mM] | 0-13,50, bevorzugt 1,00-2,00 | 0 bis +13,5, bevorzugt +1-2 mmol/l |
| RNase freies Aqua dest | 2,875-17,375, bevorzugt 13,50-15,50 | |
| Träger-DNA | 1,00-2,00 | |
| Endvolumen mM = mmol/l | 25,00 | |

*Primer für die BBPA:*

| **Target** | **Forward** (5‘->3‘) | **SEQ ID** | **Reverse** (5‘->3‘) | **SEQ ID** | **Annealing-temperatur BBPA** |
|---|---|---|---|---|---|
| *PLA2R1* (168bp) | GGGGTAAGGAAGGTGGAGAT | 1 | ACAAACCACCTAAATTCTAATAAACAC | 2 | 63,0°C und 2,5 mM $MgCl_2$ |
| *RASSF1A* (117bp) | GTTTGTTAGCGTTTAAAGTTAG | 3 | AATACGACCCTTCCCAAC | 4 | 52,0°C und 2,5 mM $MgCl_2$ |
| *GSTP1* (120bp) | GTGAAGCGGGTGTGTAAGTTT | 5 | TAAACAAACAACAAAAAAAAAAC | 6 | 50,7°C und 2,5 mM $MgCl_2$ |
| *GSTP1* (116bp) | ATCGTAGCGGTTTTAGGGAA | 93 | TCCCCAACGAAACCTAAAAA | 94 | 53,8°C und 4,5 mM $MgCl_2$ |
| *PLA2R1* (150bp) | GGGGTAAGGAAGGTGGAGAT | 53 | AATAAACACCGCGAATTTACAAC | 9 | 59,5°C und 2,5 mM $MgCl_2$ |
| *PLA2R1* (161bp) | | | ACCTAAATTCTAATAAACACCGC | 10 | 62,5°C und 2,5 mM $MgCl_2$ |
| *PLA2R1* (160bp) | | | CCTAAATTCTAATAAACACCGC | 11 | 62,5°C und 2,5 mM $MgCl_2$ |
| PLA2R1 (133bp) | GGAAGGTGGAGATTACGG | 7 | GCGAATTTACAACGAACAAC | 8 | 50,0°C und 1,5 mM $MgCl_2$ oder 63,0°C und 6,0 mM $MgCl_2$ |
| *AOX1* (180bp) | TGGGTTGGATTTTAGGTTTTAG | 14 | CTCACCTTACGACCGTTC | 15 | 52,6°C und 2,5 mM $MgCl_2$ |
| *SERPINE1* (123bp) | AGAGCGTTGTTAAGAAGA | 16 | CTCCTACCTAAAATTCTCAAAA | 17 | 52,0°C und 3,5 mM $MgCl_2$ |
| *AOX1* (138bp) | GTTGGATTTTAGGTTTTAGTAAG | 18 | GCCCGATCCATTATAATATC | 19 | 50,0°C und 2,5 mM $MgCl_2$ |

| ***Target*** | Forward (5'->3') | SEQ ID | Reverse(5'->3') | SEQ ID |
|---|---|---|---|---|
| *RASSF1A (124bp)* | GCGTTTGTTAGCGTTTAAAG | 61 | AACCGAATACGACCCTTC | 62 |
| *GSTP1 (114bp)* | CGTAGCGGTTTTAGGGAATTT | 91 | TCCCCAACGAAACCTAAAAA | 92 |
| *GSTP1 (129bp)* | TGTAAGTTTCGGGATCGTAGC | 95 | TCCCCAACGAAACCTAAAAA | 96 |
| *GSTP1 (132bp)* | GTGTGTAAGTTTCGGGATCG | 97 | TCCCCAACGAAACCTAAAAA | 98 |
| *AOX1 (138bp)* | GTTGGATTTTAGGTTTTAGTAAG | 63 | GCCCGATCCATTATAATATC | 64 |
| *AOX1 (135bp)* | GGATTTTAGGTTTTAGTAAGTTTC | 65 | GCCCGATCCATTATAATATCCG | 66 |
| *AOX1 (134p)* | GATTTTAGGTTTTAGTAAGTTTCG | 67 | | |
| *SERPINE1 (119bp)* | CGTTGTTAAGAAGATTTATAC | 68 | TAAACCCGAAATAAAAAATTAAA | 69 |
| *TM (125bp)* | GGTCGATTCGTATGTTAGA | 70 | 5`-AACCGTACCGAAACAAAA | 71 |
| *TM (144bp)* | GTTTGGGTTGGGACGGATA | 72 | 5`-AAAAACCAAAACCCCAAACA | 73 |
| *TM (166bp)* | GTTTGGGGTTTTGGTTTTTG | 74 | 5`-GCAATCCGTCGCAAATCTAA | 75 |
| *TM (165bp)* | | | 5`-CAATCCGTCGCAAATCTAAC | 76 |

Nachdem unter Einsatz methylspezifischer Primer nach Prä-Amplifikation und dPCR sowohl in Serumproben von Tumorpatienten als auch in fc-DNA-Proben aus Serum von gesunden Probanden methylierte DNA-Fragmenten nachgewiesen wurden, was bei Verwendung eines weniger methylspezifischen Primerpaares nicht der Fall war, wurden die methylspezifischen Primer empirisch so verändert, dass neben methylierten auch nicht-methylierte DNA-Fragmente amplifiziert wurden und dabei möglichst eine Bias zugunsten von methylierten DNA-Sequenzen während der Amplifikation auftrat. Dabei wurden die oben genannten Primer erhalten. So wurden für das *RASSF1A-Gen* ein Oligonukleotid-Paar generiert (117 bp-Amplifikatgröße), dass über einen großen Temperaturbereich sowohl methylierte als auch nicht-methylierte DNA-Fragmente amplifiziert und dabei eine deutlich Bias zugunsten methylierter DNA-Fragmente aufwies. Ähnliches erfolgte für die Zielgene *PLA2R1* (Fig. 34-37), *GSTP1* (Tab. 15), *SERPINE1, AOX1* und *Thrombomodulin,* ohne dass ausschließlich nur methylierte Fragmente amplifiziert werden.

Die oben genannten Primer wurden in den nachfolgenden Beispielen sowohl für die BBPA als auch die dPCR eingesetzt. Selbstverständlich können für die dPCR auch andere Primer (nested-PCR-Primer) eingesetzt werden, die mit den durch die BBPA präamplifizierten Sequenzabschnitten hybridisieren *(Annealing).* Die in der dPCR eingesetzten Primer müssen die DNA-Sequenz einschließen, für die die verwendeten Sonden spezifisch sind. Da in der dPCR ein Bias ausgeschlossen ist, können die Primer hier nach herkömmlichen Regeln ausgewählt werden oder es werden die BBPA-spezifischen Primer ebenfalls in der dPCR eingesetzt.

*Temperatur und Zeitschema für die BBPA:*

| **PCR** | **Temperatur** | **Zeit** |
|---|---|---|
| Denaturierung: | 95°C | 5 Minuten |
| PCR-Zyklus:<br>5x-50x | 94°C (Denaturierung)<br>Primer-spezifischeTemperaturen (Annealing: 40-72°C, bevorzugt-50-72°C)<br>72°C (Elongation) | 10 Sekunden<br>30 Sekunden<br>30 Sekunden |
| | 4°C | Halten |

*Mastermix für ddPCR:*

| **Bestandteil** | **Ansatz-Vol [µl]** | **Endkonzentration** |
|---|---|---|
| 2x ddPCR Supermix (BioRad) | 10,0 | |
| 20x Primer / Sonden-Mix | 1,0 | 900 mmol/l / 250 nmol/l |
| DNA | 2,0 | |
| RNase-freies Aqua dest. | 7,0 | |
| Endvolumen | 20,0 | |

*Sonden: für die dPCR*

| Target | methyliert (5'→3') | SEQ ID | nicht-methyliert (5'→3') | SEQ ID | Hybridisierg. temp. |
|---|---|---|---|---|---|
| *PLA2R1* (3 CpG) | CCCAACTACTCCGCGACGCAA | 20 | AACCCAACTACTCCACAACACAAA | 21 | 58,8°C |
| *PLA2R1* (4 CpG) | CAACTACTCCGCGACGCAAACG | 22 | AACCCAACTACTCCACAACACAAACA | 23 | 51,9°C |
| *RASSF1A* (3 CpG) | CGCCCAACGAATACCAACTCCCG | 24 | CACCCAACAAATACCAACTCCCACAA- | 25 | 51,9°C |
| *RASSF1A* (4 CpG) | CGCCCAACGAATACCAACTCCCGCG | 54 | CACCCAACAAATACCAACTCCCACAACTC- | 55 | 51,9°C |
| *GSTP1* (3 CpG) | CGCAACGAAATATACGCAAC | 56 | CACAACAAAATATACACAAC | 57 | 50,7°C |
| *GSTP1* (4 CpG) | ACGAACTAACGCGCCGAAAC | 58 | ACAAACTAACACACCAAAAC | 59 | 51,9°C |
| *AOX1* (3 CpG) | ACTCGAACGCCCGATCCATTATAA | 37 | ACAACTCAAACACCCAATCCATTATAA | 38 | 50,7°C |
| *AOX1* (4 CpG) | CGCTAATTCGAAAACCCGAAACGA | 39 | CACTAATTCAAAAACCCAAAACAA | 40 | 53,8°C |
| *AOX1* (5 CpG) | CGCGCTAATTCGAAAACCCGAAACGA | 41 | CACACTAATTCAAAAACCCAAAACAA | 42 | 51,9°C |
| *SERPINE1* (4 CpG) | CGATTAACGATTCGTCCTACTCTAACG | 43 | CAATTAACAATTCATCCTACTCTAACA | 44 | 58,8°C |
| Target | methyliert (5'→3') | | SEQ ID | nicht-methyliert (5'→3') | SEQ ID |
| AOX1 (4 CpG) | CGCTAATTCGAAAACCCGAAACGA | | 77 | CACTAATTCAAAAACCCAAAACAA | 78 |
| AOX1 (4 CpG) | | | | CACTAATTCAAAAACCCAAAACAAAA | 79 |

(fortgesetzt)

| Target | methyliert (5'→3') | SEQ ID | nicht-methyliert (5'→3') | SEQ ID |
|---|---|---|---|---|
| TM (3 CpG) | ACGCCGATAACGACAACCTCT | 80 | AAAAAGCAGA-TAAAGACAAC-CTC | 81 |
| TM (4 CpG) | CCGACTACGACTCTACGAATACGAA | 82 | CAGACTAA-GACTCTAA-GAATAA-GAAAAAC | 83 |

Alle Sonden sind in den Beispielen 5'- FAM (methylierte DNA) bzw. HEX (nicht methylierte DNA) markiert und am 3'-Ende mit dem Quencher BHQ-1 markiert.

*Herstellung der Tröpfchen (Droplet-Generator [BioRad]):*

20 µl Mastermix mit DNA-Probe
+ 70 µl Öl in entsprechende Cartridges (BioRad);
Generierung von etwa 20.000 Öl/Emulsionströpfchen,
davon 35 µl in die nachfolgende dPCR

*Temperatur und Zeitschema für die dPCR mittels T100-PCR-Gerät (BioRad):*

| PCR | Temperatur | Zeit |
|---|---|---|
| Denaturierung: | 95°C | 10 Minuten |
| PCR-Zyklus:<br>40 x | 94°C (Denaturierung)<br>Primer- und Sonden-spezifische Temperatur (Annealing und Elongation, 50-72°C) | 30 Sekunden<br>1 Minute |
| | 98°C (Stabilisierung der Tröpfchen) | 10 Minuten |
| | 20°C | halten |

*Messung der Fluoreszenzen in den Töpfchen:*

Die Messung der Tröpfchenfluoreszenz erfolgte mittels QY100 (BioRad) entsprechend der Herstellerbeschreibung.

**Genauswahl**

Anhand von Untersuchungen an Prostatazellen (normale PrEC und BPH-1 und maligne LNCaP, PC-3 und DU-145-Zelllinien), Brustkrebszellen (normale HMEC und maligne Cal-51, BT-474, MCF-7 und MDA-MB-453-Zelllinien), Leukämiezelllinien (U937 und Jurkat), Hepatomzelllinie (HepG2) und normale Endothelzellen (HUVEC und HCAEC) wurden die Gene PLA2R1, RASSF1A und GSTP1 ergänzt durch die Gene SERPINE1 (PAI-1) und AOX1 ausgewählt. Ein weiterer Genkandidat ist das Thrombomodulin (TM.). Bei den Genen uPA, Del-1 und Jam-C zeigten sich nur vereinzelt erhöhte Methylierungswerte in den Prostatatumorzelllinien. Das Gen PLA2G5 wies bereits in den normalen PrEC erhöhte Methylierungswerte auf und ist deshalb für den Einsatz als Biomarker zur Diagnose von Tumorerkranklungen ungeeignet.

**Tabelle 1:** Übersicht über die untersuchten Gen-Methylierungen (%) in Prostata- (normale PrEC und BPH-1 und maligne LNCaP, PC-3 und DU-145-Zellinien), Brustkrebs- (normale HMEC und maligne Cal-51, BT-474, MCF-7 und MDA-MB-453-Zelllinien), Leukämie- (U937 und Jurkat), Hepatomzelllinie (HepG2) und normale Endothelzellen (HUVEC und HCAEC)

| Zelltyp | *PLA2R1* | *PLA2G5* | *PAI-1* | *uPA* | *Del-1* | *Jam-C* | *RASSF1A* | *AOX1* | *GSTP1* | TM |
|---|---|---|---|---|---|---|---|---|---|---|
| **PrEC** | 2,2 | 25 | 0 | 0 | 5 | 0 | 2 | 0 | 0,7 | 0 |
| **BPH-1** | 6 | n.d. | 0 | n.d. | n.d. | n.d. | 7 | 8 | 0,6 | n.d. |
| **LNCaP** | 99 | 70 | 30 | 65 | 30 | 0 | 94 | 46 | 99 | 17 |
| **PC-3** | 43 | 40 | 16 | 0 | 0 | 0 | 96 | 69 | 26 | 28 |
| **DU145** | 24 | 100 | 64 | 0 | 0 | 23 | 55 | 64 | 18 | 64 |
| **U937** | 100 | 100 | 9 | n.d. | 100 | n.d. | 5 | n.d. | n.d. | n.d. |
| **Jurkat** | 100 | 65 | 1 | n.d. | 10 | n.d. | 80 | n.d. | n.d. | n.d. |
| **HepG2** | 42 | 19 | 1 | n.d. | 7 | n.d. | 70 | n.d. | n.d. | n.d. |
| **HUVEC** | 1 | 12 | n.d. | n.d. | 0 | n.d. | 4 | n.d. | n.d. | n.d. |
| **HCAEC** | 2 | 0 | 1 | n.d. | 0 | n.d. | 3 | n.d. | n.d. | n.d. |

(fortgesetzt)

| Zelltyp | *PLA2R1* | *PLA2G5* | *PAI-1* | *uPA* | *Del-1* | *Jam-C* | *RASSF1A* | *AOX1* | *GSTP1* | TM |
|---|---|---|---|---|---|---|---|---|---|---|
| **HMEC** | 1 | 5 | 0 | 0 | 0 | 0 | 3 | n.d. | n.d. | 0 |
| **Cal-51** | 5 | 11 | 40 | 0 | 95 | 20 | 100 | n.d. | n.d. | 70 |
| **BT-474** | 8 | 85 | 48 | 47 | 0 | 100 | 100 | n.d. | n.d. | 55 |
| **MCF-7** | 9 | 59 | 75 | 75 | 0 | 100 | 95 | n.d. | n.d. | 45 |
| **MDA-MB-453** | 71 | 35 | 65 | 100 | 65 | 100 | 70 | n.d. | n.d. | 0 |
| n.d. = nicht bestimmt. | | | | | | | | | | |

**Ausführungsbeispiel 1: Ergebnisse der BBPA-dPCR anhand der Methylierung von *RASSF1* in Serumpoolproben von Patientinnen mit Mammakarzinom im Vergleich zu gesunden Probandinnen**

Bei Verwendung des Bias-induzierenden RASSF1-(117 bp)-Primerpaares (SEQ ID No. 3 und 4) stellte sich überraschend heraus, dass mit steigender Zyklenanzahl und ohne zusätzlicher Verdünnung der prä-amplifizierten Amplifikate die Schere zwischen den ermittelten Methylierungsgraden des RASSF1A-Gens zwischen gesunden Probandinnen und Patientinnen mit Mammakarzinom sich zunehmend vergrößerte, soweit dass die Werte der gesunden Probandinnen sich in Richtung 0 % und die der Mammakarzinom-Patientinnen in Richtung 100 % sich verschoben. So zeigte sich, dass bei Nutzung des obigen Primerpaares bei einer Annealingtemperatur von 66,1°C bis 60,0°C in den Proben von Mammakarzinompatienten die Kopienzahl methylierter Sequenzen im Vergleich zu nicht-methylierten stärker anstiegen (Fig. 2). Unerwartet war jedoch, dass die in den fc-DNA gesunder Probandinnen vorkommenden methylierten DNA-Kopien, wenn sie auch in niedriger Menge vorliegen, gegenüber nicht-methylierten DNA-Kopien nicht bevorzugt vervielfältigt wurden. Ganz im Gegenteil wurden in den gesunden Proben die nicht-methylierten DNA-Kopien gegenüber den methylierten Kopien verstärkt vervielfältigt (Tabelle 2). Ohne BBPA betrug der Methylierungsgrad (fraktionale Häufigkeit) 0,6 % (Poisson-Wahrscheinlichkeitsbereich: 0-1,2%) bei gesunden Probandinnen und 1,5 % (Poisson-Wahrscheinlichkeitsbereich: 0,8-2,1%) bei Patientinnen mit Mammakarzinom (Tab. 2).

**Tabelle 2:** Kopienanzahl methylierter (Met) und nicht-methylierter (Unm) RASSF1A-Sequenzen in Abhängigkeit von der Annealingtemperatur (60,0-69,3°C) in der BBPA bei 30 Zyklen in Serumpoolproben gesunder Probandinnen (BA) oder Patientinnen mit Mamma-Karzinom (PatP). Nach BBPA wurden 1 µl der 25 µl-PCR-Ansätze in die dPCR eingesetzt, weshalb die Werte nach der BBPA zum Vergleich mit 25 multipliziert wurden. "ohne" in der Spalte Temp./ °C bedeutet ohne vorherige BBPA.

| Probe | Target | Kopien /20µLWell | 1:25 | Positive | Negative | Ch1+Ch2+ | Ch1+Ch2- | Ch1-Ch2+ | Ch1-Ch2- | Akzeptierte Tröpfchen | Fraktionale Häufigkeit | Poisson Fraktionale Häufigkeit Max | Poisson Fraktionale Häufigkeit Min | Temp./ °C |
|---|---|---|---|---|---|---|---|---|---|---|---|---|---|---|
| BA | RASSF1 Met | 9 | 9 | 4 | 10353 | 0 | 4 | 669 | 9684 | 10357 | 0,6 | 1,2 | 0 | ohne |
| BA | RASSF1 Unm | 1580 | 1580 | 669 | 9688 | | | | | | | | | ohne |
| PatP | RASSF1 Met | 40 | 40 | 19 | 11016 | 1 | 18 | 1202 | 9814 | 11035 | 1,5 | 2,1 | 0,8 | ohne |
| PatP | RASSF1 Unm | 2720 | 2720 | 1203 | 9832 | | | | | | | | | ohne |
| BA 30x | RASSF1 Met | 2 | 50 | 1 | 11872 | 1 | 0 | 65 | 11807 | 11873 | 1,5 | 5 | 0 | 69,3 |
| BA 30x | RASSF1 Unm | 132 | 3300 | 66 | 11807 | | | | | | | | | 69,3 |
| PatP 30x | RASSF1 Met | 11,2 | 280 | 6 | 12562 | 0 | 6 | 73 | 12489 | 12568 | 8 | 14 | 2 | 69,3 |
| PatP 30x | RASSF1 Unm | 138 | 3450 | 73 | 12495 | | | | | | | | | 69,3 |
| BA 30x | RASSF1 Met | 18 | 450 | 8 | 10515 | 4 | 4 | 447 | 10068 | 10523 | 1,7 | 2,9 | 0,5 | 68,0 |
| BA 30x | RASSF1 Unm | 1030 | 25750 | 451 | 10072 | | | | | | | | | 68,0 |
| PatP 30x | RASSF1 Met | 32 | 800 | 19 | 13584 | 0 | 19 | 351 | 13233 | 13603 | 5,1 | 7,3 | 2,8 | 68,0 |
| PatP 30x | RASSF1 Unm | 616 | 15400 | 351 | 13252 | | | | | | | | | 68,0 |
| BA 30x | RASSF1 Met | 162 | 4050 | 71 | 10332 | 35 | 36 | 3692 | 6640 | 10403 | 1,52 | 1,87 | 1,17 | 66,1 |

(fortgesetzt)

| Probe | Target | Kopien /20µLWell | 1:25 | Positive | Negative | Ch1+Ch2+ | Ch1+Ch2- | Ch1-Ch2+ | Ch1-Ch2- | Akzeptierte Tröpfchen | Fraktionale Häufigkeit | Poisson Fraktionale Häufigkeit Max | Poisson Fraktionale Häufigkeit Min | Temp./ °C |
|---|---|---|---|---|---|---|---|---|---|---|---|---|---|---|
| BA 30x | RASSF1 Unm | 10440 | 261000 | 3727 | 6676 | | | | | | | | | 66,1 |
| PatP 30x | RASSF1 Met | 17660 | 441500 | 6583 | 5888 | 2325 | 4258 | 2801 | 3087 | 12471 | 58,6 | 59,5 | 57,7 | 66,1 |
| PatP 30x | RAUSSF1 Unm | 12460 | 311500 | 5126 | 7345 | | | | | | | | | 66,1 |
| BA 30x | RASSF1 Met | 0 | 0 | 0 | 10851 | 0 | 0 | 6036 | 4815 | 10851 | 0 | 0 | 0 | 60,0 |
| BA 30x | RASSF1 Unm | 19120 | 478000 | 6036 | 4815 | | | | | | | | | 60,0 |
| PatP 30x | RASSF1 Met | 74000 | 1850000 | 12234 | 550 | 1678 | 10556 | 12 | 538 | 12784 | 95,69 | 95,91 | 95,46 | 60,0 |
| PatP 30x | RASSF1 Unm | 3340 | 83500 | 1690 | 11094 | | | | | | | | | 60,0 |

**Tabelle 3:** Kopienanzahl methylierter (methyl) und nicht-methylierter (unmethyl) *RASSF1A*-Sequenzen in Abhängigkeit von der Zyklenanzahl (0x [d.h. ohne BBPA], 8x, 12x, 16x und 40x) in der BBPA bei 60,0°C in Serumpoolproben gesunder Probandinnen (BA) oder Patientinnen mit Mamma-Karzinom (PatP). Nach BBPA wurden 1 µl der 25 µl-PCR-Ansätze in die dPCR eingesetzt, weshalb die Werte nach der BBPA zum Vergleich mit 25 multipliziert wurden.

| Probe | Kopien/ 20gL Well | 1:25 | Positive | Negative | Ch1+ Ch2+ | Ch1+ Ch2- | Ch1- Ch2+ | Ch1- Ch2- | Akzeptierte Tröp-fchen | Fraktionale Häufigkeit | Poisson Fraktionale Häufigkeit Min | Poisson Frakt-ionale Häufigkeit Max |
|---|---|---|---|---|---|---|---|---|---|---|---|---|
| BA (0x) methyl. | 12 | 12 | 5 | 9728 | 1 | 4 | 567 | 9161 | 9733 | 0,8 | 0,1 | 1,6 |
| BA (0x) unmeth. | 1420 | 1420 | 568 | 9165 | | | | | | | | |
| PatP(0x) methyl. | 40 | 40 | 19 | 11241 | 4 | 15 | 907 | 10334 | 11260 | 2 | 1.1 | 2.8 |
| PatP(0x) unmeth. | 1980 | 1980 | 911 | 10349 | | | | | | | | |
| BA 8x methyl. | 2,2 | 55 | 1 | 11053 | 1 | 0 | 20 | 11033 | 11054 | 5 | 0 | 15 |
| BA 8x unmeth. | 44 | 1100 | 21 | 11033 | | | | | | | | |
| PatP 8x methyl. | 22 | 550 | 8 | 8730 | 2 | 6 | 36 | 8694 | 8738 | 17 | 6 | 29 |
| PatP 8x unmeth. | 102 | 2550 | 38 | 8700 | | | | | | | | |
| BA 12x methyl. | 7 | 175 | 3 | 10206 | 3 | 0 | 43 | 10163 | 10209 | 6 | 0 | 13 |
| BA 12x unmeth. | 106 | 2650 | 46 | 10163 | | | | | | | | |
| PatP 12x methyl. | 150 | 3750 | 63 | 9832 | 0 | 63 | 58 | 9774 | 9895 | 52 | 43 | 61 |
| PatP 12x unmeth. | 138 | 3450 | 58 | 9837 | | | | | | | | |
| BA 6x methyl. | 8,4 | 210 | 4 | 11236 | 2 | 2 | 1014 | 10222 | 11240 | 0,37 | 0 | 0,76 |

(fortgesetzt)

| Probe | Kopien/ 20gL Well | 1:25 | Positive | Negative | Ch1+ Ch2+ | Ch1+ Ch2- | Ch1- Ch2+ | Ch1- Ch2- | Akzeptierte Tröp-fchen | Fraktionale Häufigkeit | Poisson Fraktionale Häufigkeit Min | Poisson Frakt-ionale Häufigkeit Max |
|---|---|---|---|---|---|---|---|---|---|---|---|---|
| BA 16x unmeth. | 2220 | 55500 | 1016 | 10224 | | | | | | | | |
| PatP 16x methyl. | 1544 | 38600 | 755 | 11125 | 8 | 747 | 144 | 10981 | 11880 | 83,6 | 81,2 | 86 |
| PatP 16x unmeth. | 302 | 7550 | 152 | 11728 | | | | | | | | |
| BA 40x methyl. | 30 | 750 | 16 | 12592 | 6 | 10 | 4345 | 8247 | 12608 | 0,3 | 0,15 | 0,45 |
| BA 40 unmeth. | 9960 | 249000 | 4351 | 8257 | | | | | | | | |
| PatP 40x methyl. | 46580 | 1164500 | 9298 | 1490 | 1665 | 7633 | 198 | 1292 | 10788 | 91,26 | 90,85 | 91,67 |
| PatP 40x unmeth. | 4460 | 111500 | 1863 | 8925 | | | | | | | | |

Dieses ungewöhnliche Verhalten wurde auch deutlich, wenn die Annealingtemperatur mit 60,0°C konstant gehalten und die Vervielfältigung in Abhängigkeit von der Zyklenanzahl analysiert wurde. Auch hier stiegt die methylierte gegenüber der nicht-methylierten Fraktion in den fc-DNA-Proben Tumorerkrankter bereits nach 16 Zyklen und weiter nach 40 Zyklen stark an (Fig. 3 und 4). Im Gegensatz dazu kam es zu einem deutlich starken Anstieg der nicht-methylierten DNA-Fraktion bei gesunden Probandinnen, während der Anteil der methylierten DNA nur geringfügig anstieg (Fig. 3 und 4, Tabelle 3). Ohne BBPA betrug der Methylierungsgrad (fraktionale Häufigkeit) 0,8% (Poisson-Wahrscheinlichkeitsbereich: 0,1-1,6%) bei gesunden Probandinnen und 2,0% (Poisson-Wahrscheinlichkeitsbereich: 1,1-2,8%) bei Patientinnen mit Mammakarzinom (Tab. 3).

Wie dieses Phänomen zu erklären ist, kann noch nicht vollständig beantwortet werden. Es erklärt zunächst die Beobachtung, dass mit steigender PCR-Zyklusanzahl der ermittelte fraktionale Methylierungsgrad des RASSF1A-Gens (Verhältnis zwischen methylierten und nicht-methylierten DNA-Fragmenten in Beziehung zur Gesamtmenge der methylierten und nicht-methylierten DNA-Fragmenten) in der fc-DNA-Probe Gesunder sich in Richtung 0 % verschiebt, während der Methylierungsgrad in der fc-DNA-Probe Mammakarzinomerkrankter in Richtung 100 % ansteigt.

Auf diese Weise kann anhand der Methylierungsbestimmung z. B. des RASSF1A-Gens eine stringente Unterscheidung zwischen Gesund und Krank erreicht werden, was mit Hilfe der dPCR allein oder der MS-HRM nicht möglich ist. So ließen sich in den gleichen Serumpoolproben mit der alleinigen dPCR fraktionale Methylierungsgrade des RASSF1AGens von 0,5 % (Poisson-Wahrscheinlichkeitsbereich: 0,05-0,99 %) bei gesunden Probandinnen und 1,00 % (Poisson-Wahrscheinlichkeitsbereich: 0-2,00 %) bei Patientinnen mit Mammakarzinom finden (Fig. 5), d.h. sie waren nicht statistisch signifikant unterscheidbar.

Auch zeigten sich bei den gleichen Poolproben keine signifikanten Unterschiede in den Schmelzkurveneigenschaften durch die MS-HRM-Analyse zwischen gesunden Probandinnen und Patientinnen mit Mammakarzinom (Fig. 6).

**Ausführungsbeispiel 2: Ergebnisse der BBPA-dPCR anhand der Methylierung von *RASSF1* in Serumpoolproben von Prostatakarzinompatienten im Vergleich zu gesunden Probanden**

Das zunächst an Mammakarzinom-Patientinnen beobachtete Phänomen, dass bei einer selektiven Annealingtemperatur und steigender Zyklenanzahl, d. h. steigenden prä-amplifizierten Kopienzahlen die Werte für die RASSF1A-Methylierung zwischen Gesund und Krank zunehmend auseinanderweichen, zeigte sich auch an Serumpoolproben von Patienten mit Prostatakarzinom im Vergleich zu gesunden Probanden (Tabellen 4 und 5). So ließ sich hier ebenfalls ein starker Unterschied zwischen gesunden Probanden und Prostatakarzinompatienten finden, wenn die Prä-Amplifiaktion mit 59°C im Vergleich zu 52°C durchgeführt wurde und nach 50 Zyklen unterschiedliche Mengen der prä-amplifizierten DNA-Kopien (z.B. 1:$10^7$ verdünnt im Vergleich zu Verdünnung von 1:$10^5$, d.h. 100-fach weniger verdünnt), in der dPCR analysiert wurden (Tabelle 4). Das zeigte sich auch bei konstanter Annealingtemperatur von 59°C und der Durchführung von 35 oder 50 Zyklen in der Prä-Amplifikation (Tabelle 5).

**Tabelle 4:** Relative Häufigkeit methylierter **RASSF1A**-Sequenzen in fc-DNA-Proben aus einem Serum-Pool von gesunden Probanden (BA) und an Prostatakarzinom erkrankten Patienten (Pat) nach BBPA (50 Zyklen bei 52°C bzw. 59°C) in Abhängigkeit der in der dPCR eingesetzten DNA-Menge (Verdünnungen von 1:$10^7$, 1:$10^6$ und 1:$10^5$).

| BBPA | BA | Pat |
|---|---|---|
| 50 Zyklen bei 52°C und Verdünnung von 1:$10^7$ | 2,52 % | 9,52 % |
| 50 Zyklen bei 59°C und Verdünnung von 1:$10^7$ | 0,91 % | 81,9 % |
| 50 Zyklen bei 52°C und Verdünnung von 1:$10^6$ | 0,153 % | 9,73 % |
| 50 Zyklen bei 59°C und Verdünnung von 1:$10^6$ | 0,067 % | 96,26 % |
| 50 Zyklen bei 52°C und Verdünnung von 1:$10^5$ | 0 % | 11,6 % |
| 50 Zyklen bei 59°C und Verdünnung von 1:$10^5$ | 0,006 % | 99,09 % |

**Tabelle 5:** Relative Häufigkeit methylierter ***RASSF1A***-Sequenzen in fc-DNA-Proben aus einem Serum-Pool von gesunden Probanden (BA) und an Prostatakarzinom erkrankten Patienten (Pat) nach BBPA (35 bzw. 50 Zyklen bei 59°C) in Abhängigkeit der in der dPCR eingesetzten DNA-Menge (Verdünnungen von 1:$10^7$ bis $10^3$).

| BBPA | BA | Pat |
|---|---|---|
| 35 Zyklen bei 59°C und Verdünnung von 1:$10^7$ | 4,7 % | 65,7 % |
| 50 Zyklen bei 59°C und Verdünnung von 1:$10^7$ | 0,42 % | 79,8 % |
| 35 Zyklen bei 59°C und Verdünnung von 1:$10^6$ | 2,7 % | 65,6 % |

(fortgesetzt)

| BBPA | BA | Pat |
|---|---|---|
| 50 Zyklen bei 59°C und Verdünnung von 1:$10^6$ | 0,011 % | 92,0 % |
| 35 Zyklen bei 59°C und Verdünnung von 1:$10^5$ | 1,1 % | 75,2 % |
| 50 Zyklen bei 59°C und Verdünnung von 1:$10^5$ | 0,006 % | 96,5 % |
| 35 Zyklen bei 59°C und Verdünnung von 1:$10^4$ | 0,01 % | 70,2 % |
| 50 Zyklen bei 59°C und Verdünnung von 1:$10^4$ | 0 % | 98,5 % |
| 35 Zyklen bei 59°C und Verdünnung von 1:$10^3$ | 0 % | 75,5 % |
| 50 Zyklen bei 59°C und Verdünnung von 1:$10^3$ | 0,004 % | 96,3 % |

**Tabelle 6:** Absolute Kopienanzahl methylierter (methyl) und nicht-methylierter (unmethyl) ***RASSF1A***-Sequenzen in fc-DNA-Proben aus einem Serum-Pool von gesunden Probanden (BA) und an Prostatakarzinom erkrankten Patienten (PatP) nach BBPA (50 Zyklen bei 59°C, unverdünnt [unv]). Die nach entsprechenden Verdünnungen (1:$10^3$, 1:$10^4$, 1:$10^5$, 1:$10^6$ und 1:$10^7$) resultierenden und in der dPCR eingesetzten Anzahl an DNA-Kopien sind aufgezeigt. Daraus ließen sich die KPKs entsprechend der Formel: KPK (Kopien pro Kompartiment) = Kopien/Kompatimentanzahl für die Untersuchungen, die in Tabelle 4 und 5 dargestellt sind und bei denen eine durchschnittliche Tröpfchenanzahl von 12000 vorlag, für die Proben der gesunden Probanden (BA KPK) und an Prostatakarzinomen erkrankten Patienten (PatP KPK) berechnen.

| **Proben** | **unv** | **1:10’** | **1:$10^6$** | **1:$10^5$** | **1:$10^4$** | **1:$10^3$** |
|---|---|---|---|---|---|---|
| BA methyl | 2,4 x $10^8$ | 24 | 240 | 2400 | 24000 | 240000 |
| BA unmethyl | 6,7 x $10^{10}$ | 6700 | 67000 | 670000 | 6,7 x $10^6$ | 6,7 x $10^7$ |
| PatP methyl | 1,828 x $10^{11}$ | 18280 | 182800 | 1,828 x $10^6$ | 1,828 x $10^6$ | 1,828 x $10^7$ |
| PatP unmethyl | 4,98 x $10^{10}$ | 4980 | 49800 | 49800 | 498000 | 4,98 x $10^6$ |
| BA Summe DNA-Kopien | | 6724 | 67240 | 672400 | 6,72 x $10^6$ | 6,72 x $10^7$ |
| **BA KPK** | | **0,56** | **5,6** | **56,0** | **560** | **5603** |
| PatP Summe DNA-Kopien | | 23260 | 232600 | 2,33 x $10^6$ | 2,33 x $10^7$ | 2,33 x $10^8$ |
| **PatP KPK** | | **1,9** | **19,4** | **193,8** | **1938** | **19383** |

Für die dPCR wird nach dem Stand der Technik ein KPK-Wert (engl. CPD - copies per droplets) von maximal 8 angegeben, damit die Poisson-Verteilung und die damit verbundene Statistik gelten [9]. Die hier vorgestellten Daten zeigen jedoch, dass bei einem KPK-Wert >8 die Unterscheidung zwischen Gesund und Krank sich noch stringenter verhält. Ein KPK von 8 bedeutet, dass maximal 80.000 DNA-Kopien in einer dPCR mit 10.000 Kompartimente (z. B. Öl-Emulsionströpfchen oder sogenannte Nano-Kammern auf einem festen Träger) eingesetzt werden. Nach 35 bzw. 50 Amplifikationszyklen kommt es theoretisch zu einer 3,43 x $10^{10}$ bzw. 1,12 x $10^{15}$-fachen Vervielfältigung der Ausgangs-DNA-Kopien, wird eine Effizienz der PCR von 100 % zugrunde gelegt. In dem dargestellten Beispiel resultierten nach 50 Zyklen 1,828 x $10^{11}$ methylierte und 4,98 x $10^{10}$ nicht-methylierte RASSF1A-DNA-Fragmentkopien in den Proben von Patienten mit Prostatakarzinom und 2,4 x $10^8$ methylierte und 6,7 x $10^{10}$ nicht-methylierte RASSF1A-DNA-Fragmentkopien in den Proben der gesunden Probanden (Tabelle 6). Wird z.B. der Ansatz mit der stärksten Trennung (Stringenz) selektiert, d. h. 50 Zyklen bei 59°C und einer Verdünnung von 1:$10^4$, bei der für Gesund (BA) eine 0 %-ige und Krank (PatP) eine 98,5 %-ige RASSF1A-Methylierung resultierte, betrugen die KPK-Werte für die BA-Probe 560 und PatP-Probe 1938 (Tabelle 6).

Auf Grundlage dieser hohen Stringenz gestattet die BBA-dPCR im Prinzip eine Ja/Nein-Antwort auf die Frage, ob eine Tumor-spezifische DNA in der zu untersuchenden Probe (*Liquid Biopsy*) und damit eine Tumorerkrankung vorliegt. Mit Hilfe der variablen Stellgröße in Form der Zyklenanzahl, ohne dass dabei unspezifische Signale mit amplifiziert und dadurch verstärkt werden, ist der Nachweis nur eines einzelnen Tumor-DNA-Moleküls spezifisch vor einem großen Hintergrund normaler DNA in einer zu untersuchenden Probe möglich. Solch ein sensitiver und vor allem spezifischer Nachweis ist mit alleiniger dPCR nicht möglich.

**Ausführungsbeispiel 3: Ergebnisse der BBPA-dPCR anhand der Methylierung von RASSF1 in Einzelserumproben von Mammakarzinompatientinnen - Vergleich mit dPCR allein und MS-HRM**

In jeweils 10 Serumproben von gesunden Probandinnen ohne Tumorerkrankung und 10 Patientinnen mit Mamma-Karzinomen zeigte sich in der *RASSF1A*-Methylierungsanalyse, dass mit der alleinigen dPCR nur 4 Patientinnen (P2, P3, P8 und P19) als richtig positiv identifiziert wurden (Fig. 7, Tabelle 7), während anhand der *RASSF1A*-Methylierungsanalyse durch die erfindungsgemäße BBPA-dPCR 8 der 10 Patientinnen mit Mamma-CA (P1-P4, P6-P8 und P10) richtig zugeordnet wurden (Fig. 8, Tabelle 7).

In einer parallel durchgeführten MS-HRM-Analyse an den identischen fc-DNA-Proben zeigte sich in einer Probe der Gruppe der gesunden Probandinnen eine deutlich erhöhte *RASSF1A*-Methylierung (Fig 9C und 9D, Proben-ID K9, Tabelle 7) und in einer weiteren Probe eine gering erhöhte *RASSF1A*-Methylierung (Fig. 9C, Proben-ID K8, Tabelle 7). Im Gegensatz dazu ließ sich in einer Probe (P1) aus der Gruppe der Mammakarzinom-Patientinnen kein positiver RASSF1-Befund mit Hilfe der MS-HRM-Analyse finden (Fig. 9F), obwohl sich in der BBPA-dPCR-Analyse eine deutlich erhöhte *RASSF1A*-Methylierung in dieser Probe zeigte (Fig. 8 und Tabelle 7).

**Tabelle 7:** Übersicht über die Methylierungsgrade von ***RASSF1A*** im Serum von gesunden Probandinnen (K1-K10) und Patentinnen mit Mamma-Karzinom (P1-P10), die mit Hilfe der dPCR allein, der MS-HRM- und der BBPA-dPCR-Technik erzielt wurden (dick geschrieben: Methylierungswerte >9 % als *cut-off*-Wert im Fall der dPCR allein und >0,460 % im Fall der BBPA-dPCR-Analyse; eine semi-quantitative Auswertung erfolgte in der MS-HRM-Analyse)

| ID | RASSF1A in der dPCR allein in % (Vergleichsdaten) | RASSF1A mittels MS-HRM -Analyse (Vergleichsdaten) | RASSF1 A mittels BBP A-dPCR-Analyse in % |
|---|---|---|---|
| K1 | 2,5 | - | 0,38 |
| K2 | 6 | - | 0,46 |
| K3 | 3,2 | - | 0,058 |
| K4 | 1,4 | - | 0,004 |
| K5 | 3,7 | - | 0,103 |
| K6 | 4,7 | - | 0,017 |
| K7 | 2,1 | - | 0 |
| K8 | 3 | **(+)** | 0,009 |
| K9 | 2,7 | **+** | 0,005 |
| K10 | 9 | - | 0,010 |
| P1 | 8 | - | **5,37** |
| P2 | **21** | **++** | **26,9** |
| P3 | **25** | **+++** | **29,8** |
| P4 | 9 | **++** | **5,62** |
| P5 | 2,2 | - | 0,006 |
| P6 | 1,4 | **++** | **1,33** |
| P7 | 7 | **+** | **4,4** |
| P8 | **19** | **+++** | **26,9** |
| P9 | 0 | - | 0,04 |
| P10 | **16** | **+** | **17,4** |

Im Ergebnis wurden bei der MS-HRM-Analyse 2 von 10 Proben der Gruppe der gesunden Probandinnen als falsch pathologisch eingestuft, was bei der BBPA-dPCR nicht der Fall war (Tabelle 7). Bei der MS-HRM allein betrug damit die diagnostische Sensitivität (d. h. Kranke richtig diagnostiziert) 70 % und die diagnostische Spezifität (d. h. Gesunde richtig diagnostiziert) 80 %. Bei der dPCR allein betrug die diagnostische Sensitivität 40 % und die diagnostische Spezifität 100 %, wenn ein Grenzwert für die *RASSF1A*-Methylierung von < 9,1 % für Gesund zugrunde gelegt wurde.

Bei der BBPA-dPCR betrug die diagnostische Sensitivität 80 % und die diagnostische Spezifität 100 %, wenn ein Grenzwert für die *RASSF1A*-Methylierung von < 0,460 % für Gesund zugrunde gelegt wurde. Für einen frühzeitigen Nachweis einer Tumorerkrankung (Screening bzw. Vorsorge) sind eine diagnostische Sensitivität und Spezifität nahe 100 % erforderlich, um falsch positive (Gesunde irrtümlicherweise als Krank diagnostiziert) und falsch negative Werte (Kranke als solche nicht erkannt) auszuschließen.

Bei der BBPA-dPCR kann wie nachfolgend in weiteren Beispielen gezeigt wird, die diagnostische Sensitivität durch Kombination mehrerer Kandidatengene in der Methylierungsuntersuchung erhöht werden. Auch kann durch Einsatz von 5 ml anstelle von 1 ml Serum, wie es hier der Fall war, die diagnostische Sensititvität erhöht werden. Weiterhin kann durch einen multiplexen Einsatz von Primern in der BBPA die diagnostische Sensititvität erhöht werden, in dem auf diese Weise die gesamte isolierte DNA für die Analytik der einzelnen Genkandidaten in der anschließenden dPCR zur Verfügung steht.

Auch kann - wie in dem Ausführungsbeispiel 12 gezeigt wird - die diagnostische Sensitivität des Verfahrens erhöht werden, indem mehrere Primerpaare für ein und dasselbe Gen in der Prä-Amplifikation eingesetzt werden, die die Sequenzen einschließen, die durch die Sonden in der dPCR detektiert werden.

**Ausführungsbeispiel 4: Ergebnisse der BBPA-dPCR anhand der *PLA2R1-, RASSF1A-, GSTP1-* und *AOX1*-Methylierungen in Serum von Prostatakarzinompatienten**

In einer weiteren Untersuchung an einer Gruppe von 19 Patienten mit Prostata-Karzinom (PCa) und einer Vergleichsgruppe von 20 Probanden ohne Tumorerkrankungen zeigte sich, dass mit Hilfe der BBPA-dPCR die Bestimmung der *PLA2R1-* (unter Einsatz des Primerpaares SEQ ID No. 1 und 2, Sondenpaares SEQ ID No. 20 und 21), *RASSF1A*-(Primerpaar SEQ ID No. 3 und 4, Sondenpaar SEQ ID No. 24 und 25), *GSTP1-* (Primerpaar SEQ ID No. 5 und 6, Sondenpaar SEQ ID No. 56 und 57) und *AOX1-* (Primerpaar SEQ ID No. 14 und 15, Sondenpaar SEQ ID No. 37 und 38) Methylierungen in 18 der 19 Patienten zu erhöhten Werten für mindestens eines der untersuchten Gene im Vergleich zu den gesunden Probanden nachweisbar war (Fig. 10-12, Tabelle 8).

Bei diesen Untersuchungen zeigte sich in der 2-D-Auswertung eine distinkte Population von Tröpfchen, die doppelt markiert (FAM- und HEX-positiv) waren und die sich deutlich von denen der nicht-methylierten fc-DNA (nur HEX-positiv) unterschieden, wenn die BBPA mit einer entsprechend hohen Zyklenanzahl durchgeführt und die resultierenden Amplifikate weniger verdünnt in der dPCR analysiert wurden (Fig. 13). Für die kombinierten BBPA-dPCR-basierten Untersuchungen der PLA2R1-, RASSF1A-, GSTP-1 und AOX1-Zielgene ergab sich eine diagnostische Sensitivität von 95 % und eine diagnostische Spezifität von 100 %. Die ROC-Analyse zeigten für die *PLA2R1*-Methylierung ein AUC-Wert von 0,718, für *RASSF1A* ein Wert von 0,692 und *GSTP1* ein Wert von 0,976. Wurden die 3 Biomarker kombiniert, betrug der AUC-Wert 0,982 für die Unterscheidung zwischen Patienten mit PCa im Vergleich zu Gesunden anhand der Serumuntersuchungen (Fig. 14).

**Tabelle 8:** Übersicht über die Bestimmungen der Methylierungsgrade der ***PLA2R1-, RASSF1A-, GSTP1-*** und **AOX1-Gene** mit Hilfe der BBPA-dPCR-Analysen in % in fc-DNA-Proben aus Serum von gesunden Probanden (K1-20) und Patienten mit PCa (P1-19) und der PSA-Konzentrationen (ng/ml); (fett gedruckt: Werte > 2,0 % im Fall des *PLA2R1*-; > 0,1 % im Fall des *RASSF1A*-; > 2,1 % im Fall des *GSTP1-* und > 1,0 % des AOX1-Gens).

| ID | PSA/ ng/ml | PLA2R1 in % | RASSF1A in % | GSTP1 in % | AOX1 in % |
|---|---|---|---|---|---|
| K1 | | 0,080 | 0,076 | 0,007 | 0 |
| K2 | | 0 | 0 | 0,003 | 0 |
| K3 | | 0,120 | 0 | 0,0026 | 0,006 |
| K4 | | 0 | 0 | 0,021 | 0 |
| K5 | | 0,130 | 0,0027 | 0,007 | 0,018 |
| K6 | | 0,090 | 0,006 | 0,0029 | 0,039 |
| K7 | | 0,070 | 0 | 0,018 | 0,138 |
| K8 | | 0,150 | 0 | 2,05 | 1,1 |
| K9 | | 0,027 | 0 | 0,0029 | 0 |
| K10 | | 0 | 0,002 | 0,0026 | 0 |
| K11 | | 0 | 0 | 0 | 0,017 |
| K12 | | 1,970 | 0,003 | 0 | 0 |
| K13 | | 1,250 | 0,003 | 0,009 | 0 |
| K14 | | 0,008 | 0 | 0,0014 | 0 |
| K15 | | 0 | 0 | 0,004 | 0,034 |

(fortgesetzt)

| ID | PSA/ ng/ml | PLA2R1 in % | RASSF1A in % | GSTP1 in % | AOX1 in % |
|---|---|---|---|---|---|
| K16 | | 0,011 | 0 | 0 | 0,02 |
| K17 | | 0 | 0 | 0,0025 | 0 |
| K18 | | 0,270 | 0 | 0,007 | 0 |
| K19 | | 0,910 | 0 | 0 | 0 |
| K20 | | 0,074 | 0 | 0,009 | 0 |
| P1 | 52,49 | **31,400** | **100** | **43,9** | 0 |
| P2 | 37,48 | 0,047 | **57,3** | **5,58** | 0 |
| P3 | 73,66 | 0 | **53,6** | **19,2** | 0 |
| P4 | 86,31 | **2,130** | **47,9** | **19,7** | **55,7** |
| P5 | 33,63 | 0,015 | **40,5** | 0,28 | 0 |
| P6 | 75,53 | **12,800** | **0,8** | **84,5** | **93,3** |
| P7 | 79,68 | 0,360 | **45,5** | **2,82** | 0,018 |
| P8 | 23,84 | 0,089 | 0 | 0,129 | 0,05 |
| P9 | 28,51 | 0,0022 | **46,9** | 0,118 | 0 |
| P10 | 37,98 | 0,0024 | 0 | **5,11** | 0,22 |
| P11 | 26,78 | **5,560** | 0 | 0,166 | 0,14 |
| P12 | 20,74 | **3,690** | 0 | 0,048 | 0 |
| P13 | 24,96 | **2,360** | 0 | 0,131 | 0 |
| P14 | 18,65 | 0,290 | 0 | **6,83** | **66,2** |
| P15 | 77,99 | 0,087 | 0 | **70,2** | **99,8** |
| P16 | 25,63 | **4,450** | **52,1** | 0,011 | 0 |
| P17 | 38,26 | **3,770** | 0 | **60,2** | **90,01** |
| P18 | 59,93 | **20,30** | 0 | **56,5** | **91,95** |
| P19 | 54,74 | 0 | **29,4** | **5,83** | **46,1** |

Neben den hohen AUC-Werten wies die BBPA-dPCR geringe Intra- und Interassay-Variationskoeffizienten (VK) von 3-10 % aus, die deutlich unterhalb der mittels MS-HRM-Analysen erzielten VK lagen, die insbesondere im Methylierungsbereich von 1-3 % Werte von 50-100 % erreichten. Darüber hinaus wurden mit der BBPA-dPCR eine analytische Sensitivität von < 0,003 % erzielt, obwohl für diese Untersuchungen nur 1 ml Serum zur Verfügung standen. Auch standen nur Serumproben und keine Plasmaproben hierfür zur Verfügung. Letztere werden bevorzugt für die Untersuchung von fc-DNA genutzt, da auf Grund der eintretenden Gerinnung bei Serumproben mit einer starken Freisetzung von DNA aus Blutleukozyten und deshalb mit einer hohen Hintergrundskonzentration nicht-methylierter DNA zu rechnen ist. Die Ergebnisse zeigten, dass bei Nutzung der BBPA-dPCR selbst bei einem hohen Hintergrund von normaler DNA sehr sensitiv fc-Tumor-DNA nachgewiesen werden kann. Ferner wurden die Serumproben prä-analytisch nicht gesondert behandelt, wozu ein schnelles Abzentrifugieren des Serums und anschließendes Einfrieren bis zur fc-DNA-Isolierung zählt. Im Vergleich dazu betrug die Sensitivität der MS-HRM-Technik in eigenen Untersuchungen maximal 1 %. Ein Grund für den negativen Befund (bei der BBPA-dPCR-Untersuchung) in der Patientenprobe P8 (Tab. 8) könnte in einer zu geringen Serummenge (1 ml) begründet sein, so dass möglicherweise keine methylierte DNA-Kopie in der untersuchten Probe vorlag.

Aus diesem Grund wurden als nächstes Serumproben mit PSA-Werten zwischen 3,5-15 ng/ml analysiert, von denen jeweils 3-5 ml zur Verfügung standen. Bei dem genannten PSA-Konzentrationsbereich handelt es sich um den kritischen Bereich für die Indikation einer Prostatagewebebiopsie. In 17 Serumproben wurden die *PLA2R1*- und *GSTP1*-Methylierungen unter Einsatz der BBPA-dPCR bestimmt. Dabei konnten signifikante Unterschiede hinsichtlich der Methylierungen in den Proben nachgewiesen werden (Fig. 15 und 16, Tabelle 9).

**Tabelle 9:** Übersicht über die Bestimmung der Methylierungsgrade von *PLA2R1* und *GSTP1* und der Gesamt-PSA-Konzentrationen (Referenzbereich: <4 ng/ml) und des Quotienten freies/ Gesamt-PSA (Referenzbereich < 20 %); dick gedruckt: Werte oberhalb des *cut-off*-Wertes > 0,1 % für die Methylierungen der PLA2R1- und GSTP1-Gene.

| ID | PSA in ng/ml | fPSA/Ge samt-PSA in % | PLA2R1 in % | GSTP1 in % | Befund |
|---|---|---|---|---|---|
| M1 | 6,88 | 11,48 | **4,45** | 0,02 | Prostatabiopsie mit 1/16 Zylindern rechts positiv |
| M2 | 3114,7 | nicht bestimmt | 0,003 | **4,64** | metastasierendes Prostatakarzinom |
| M3 | 6,51 | 24,6 | 0,0021 | **2,69** | |
| M4 | 13,87 | 12,11 | **0,65** | 0,04 | Prostatabiopsie positiv |
| M5 | 6,19 | 7,11 | **4,25** | **0,36** | ? |
| M6 | 3,52 | 52,27 | 0,019 | 0,009 | |
| M7 | 3,48 | 24,1 | 0,017 | **1,14** | |
| M8 | 4,36 | 19,5 | 0,085 | 0,016 | |
| M9 | 4,86 | 10,7 | **1,66** | **0,35** | ? |
| M10 | 6,24 | 8,49 | 0,005 | 0 | |
| M11 | 8,64 | 9,84 | 0,008 | 0,077 | |
| M12 | 7,49 | 4,27 | **1,24** | 0,0039 | Prostatabiopsie mit 1/12 Zylindern positiv |
| M13 | 8,9 | 30,79 | 0,011 | 0,023 | |
| M14 | 8,32 | 22,5 | **1,41** | 0,005 | Prostatabiopsie durchgeführt, noch kein Befund |
| M15 | 4,26 | 16,9 | 0 | 0,0025 | |
| M16 | 14,88 | 7,6 | **0,73** | **0,192** | mäßig vergrößert Prostata mit partiell obstruktiven Seitenlappen |
| M17 | 10,17 | 5,9 | **0,94** | 0,048 | Metastasierendes Kolon-Ca, 14/23 LM, pM1a (hepatisch) |

Wird ein *cut-off*-Wert von < 0,1 % für die Methylierung beider Biomarker zu Grunde gelegt, konnte in 7 Proben (M1, M4, M5, M9, M12, M16 und M17, Tabelle 9) die Indikation für eine Biopsie verstärkt werden, der auf Grund des Quotienten freies/Gesamt PSA < 20 % bestand. In 4 Proben mit einem Quotienten freies/Gesamt-PSA < 20 % ließen sich keine erhöhten Werte für die untersuchten Biomarker finden, so dass hier eine Gewebebiopsie nicht indiziert wäre (M8, M10, M11 und M15, Tabelle 9). Im Gegensatz dazu wurden in 3 Proben (M3, M7 und M14) trotz des freien PSA-Anteils von > 20 % erhöhte Werte für die *PLA2R1*- und *GSTP1*-Methylierungen gefunden. Bei den Patientenproben M1, M2, M4 und M12 wurden bereits Prostatakarzinome nachgewiesen. In einer weiteren Probe (M14) erfolgte bereits eine Prostatabiopsie (Tabelle 9).

**Ausführungsbeispiel 5: Kombination BBPA-ddPCR für *RASSF1A*-Methylierung mit MS-HRM für PLA2R1-Methylierungen in Serum von Prostatakarzinompatienten**

In weiteren 27 Patientenproben wurde die *RASSF1A*-Methylierung mit Hilfe der BBPA-dPCR analysiert. Für die *PLA2R1*-Methylierungsbestimmung mittels BBPA-dPCR stand nicht mehr genügend isolierte DNA-Probe zur Verfügung, so dass die RASSF1A-Ergebnisse mit den PLA2R1-Ergebnissen kombiniert wurden, die in der MS-HRM-Analyse untersucht worden waren.
Die Ergebnisse zeigten deutlich erhöhte Werte für die *RASSF1A*-Methylierung in den Proben I1, I2, I18, I19, I21 und I24. Bemerkenswert ist, dass in den Proben I19, I21 und I24 bereits ein PCa nachgewiesen wurde (Tabelle 10). In der Probe I12 war keine erhöhte *RASSF1A*-Methylierung feststellbar, obwohl der Patient an einem PCa mit PSA-positiven Metastasen erkrankt war. Hier zeigte sich jedoch, dass in dieser Probe eine deutlich methylierte Subfraktion *des*

*PLA2R1*-Gens vorlag (Tabelle 10). Ein positiver *PLA2R1*-Methylierungsbefund ohne gleichzeitig erhöhte *RASSF1A*-Methylierung ließ sich bei einem weiteren Patienten mit PCa finden (Probe I17, Tabelle 10). Bei dem Patienten, von dem die Probe I2 stammte, wurde ebenfalls eine Prostatabiopsie durchgeführt. Bei den Proben I1, I11 und I18 ist bisher nicht bekannt, inwieweit die Patienten an einem Prostatakarzinom erkrankt sind. Diese Frage kann erst in Zukunft beantwortet werden.

**Tabelle 10:** Übersicht über die Bestimmung der Methylierungsgrade von ***RASSF1A*** mittels BBPA-dPCR und ***PLA2R1*** mittels MS-HRM und der Gesamt-PSA-Konzentrationen (Referenzbereich <4 ng/ml) und des Quotienten freies/ Gesamt-PSA (Referenzbereich < 20 %); fett gedruckt: Werte oberhalb des *cut-off*-Wertes > 0,1 % für die *RASSF1A*-Methylierung, eine semi-quantitative Auswertung erfolgte in der MS-HRM-Analyse des PLA2R1-Gens; PCa, Prostatakarzinom.

| **ID** | **PSA in ng/ml** | **fPSA/Gesamt-PSA in %** | **RASSF1A in % mittels BBPA-dPCR** | **PLA2R1 mittels MS-HRM** | **Klinische Befund** |
|---|---|---|---|---|---|
| I1 | 3,25 | 18,2 | **6,7** | **+** | ? |
| I2 | 9,33 | 7,8 | **36,0** | **++** | Prostatabiopsie wurde durchgeführt, Befund noch offen |
| I3 | 4,14 | 12,3 | 0,147 | - | |
| I4 | 3,88 | 26,8 | 0,166 | - | P-Biopsie 2011 und 2014 jeweils negativ |
| I5 | 3,41 | 32,8 | 0 | - | Prostatitis |
| I6 | 3,47 | 17,0 | 0,017 | - | |
| I7 | 7,77 | 21,5 | 0,046 | - | |
| I8 | 4,82 | 30,3 | 0,5 | - | |
| I9 | 3,41 | 23,2 | 0,202 | - | |
| I10 | 4,78 | 16,9 | 0,6 | - | |
| I11 | 4,79 | 23,8 | 0,011 | **++** | ? |
| 112 | 3,30 | 27,6 | 0,023 | **+++** | Prostata-CA mit PSA-positiven Metastasen |
| I13 | 5,03 | 27,6 | 0,24 | - | |
| I14 | 6,67 | 8,9 | 0,28 | - | |
| I15 | 5,03 | 18,1 | 0,49 | - | |
| I16 | 8,28 | 10,6 | 0,71 | - | |
| I17 | 5,71 | 18,6 | 0,36 | **++** | Prostata-CA, Bestrahlung 7/2013 |
| I18 | 5,73 | 14,1 | **19,9** | **+** | ? |
| I19 | 7,44 | 2,2 | **21,1** | **++** | Prostata-CA in Prostatabiopsie (3 von 14 Zylindern positiv) |
| I20 | 3,17 | 44,5 | 0,025 | - | |
| 121 | 3,45 | 8,4 | **36,3** | - | PSA, fPSA u. BBPA-dPCR aus Blut vom September 2013; Juli 2015 histologisch gesichertes Prostata-CA mit Knochemmetastase |
| I22 | 3,85 | 19,0 | 0,3 | - | hier erfolgten vierteljährliche PSA-Kontrollen |
| I23 | 5,63 | 8,9 | 1,1 | - | |
| I24 | 2,54 | 1,2 | **34,5** | + | Prostata-CA in Prostatabiopsie (4 von 12 Zylindern positiv) |

**Ausführungsbeispiel 6: BBPA-dPCR für *RASSF1A*- und *GSTP1*-Methylierung in Serum von Nierenzellkarzinompatienten**

In einer weiteren Untersuchung wurden 10 Serumproben von Patienten mit einem Nierenzellkarzinom im Vergleich zu 8 gesunden Probanden analysiert.

Die Ergebnisse zeigten ebenfalls erhöhte *PLA2R1*-Methylierungen (unter Einsatz des Primerpaares SEQ ID No. 1 und 2 und des Sondenpaares SEQ ID No. 20 und 21) in den Proben N1-N4, N6-N8 und N10, während die Methylierung in den fc-DNA-Proben gesunder Probanden weniger als 0,005 % betrug (Fig. 17). Gemeinsam mit der Bestimmung der *RASSF1A*- (unter Einsatz des Primerpaares SEQ ID No. 3 und 4 und des Sondenpaares SEQ ID No. 24 und 25) und *GSTP1*- (Primerpaar SEQ ID No. 5 und 6, Sondenpaar SEQ ID No. 56 und 57) Methylierungen konnten alle Serumproben als pathologisch erhöht identifiziert werden (Tabelle 11).

**Tabelle 11:** Übersicht über die Bestimmung der Methylierungsgrade von ***PLA2R1, RASSF1A*** und ***GSTP1*** im Serumproben von Patienten mit Nierenzellkarzinom (NZK1-10; fett gedruckte Werte sind erhöhte Methylierungswerte [> 0,1%])

| **ID** | PLA2R1in % | RASSF1A in % | GSTP1 in % |
|---|---|---|---|
| NZK1 | **0,60** | 0 | **9,5** |
| NZK2 | **0,19** | **10,9** | 0 |
| NZK3 | **1,56** | **3,52** | **30,0** |
| NZK4 | **0,21** | 0 | **12,6** |
| NZK5 | 0 | **2,6** | 0 |
| NZK6 | **1,05** | **13,8** | **5,4** |
| NZK7 | 0,097 | **14,5** | 0 |
| NZK8 | 0,082 | **10,7** | 0 |
| NZK9 | 0 | **16,6** | 0 |
| NZK10 | **6,6** | 0 | 0 |

**Ausführungsbeispiel 7: BBPA-ddPCR für *PLA2R1-, RASSF1A-* und GSTP1-Methylierung zur Unterscheidung einer benignen Prostatahyperplasie von einem Prostatakarzinom in Serum von Patienten**

Nachdem mit Hilfe der neuen BBPA-dPCR-Methode Prostatakarzinome, Mammakarzinome und Nierenzellkarzinome eindeutig Patienten zugeordnet werden konnten, stellt sich die Frage, inwieweit bei erhöhten PSA-Werten eine benigne Prostatahyperplasie (BPH) von einem PCa mit Hilfe der BBPA-dPCR sicher unterschieden werden kann, um unnötige Prostatagewebebiopsien zu vermeiden. Dazu wurden die malignen Prostatazelllinien LNCaP, PC-3 und DU-145-Zellen und die benigne Prostatahyperplasie-Zelllinie BPH-1 untersucht.

Dabei zeigte sich, dass in der benignen Prostatazelllinie BPH-1 im Vergleich zu normalen Epithelialzellen der Prostata (PrEC) bereits heterogen methylierte DNA-Fragmente, besonders in den Zielgenen *PLA2R1* und *RASSF1A* auftraten (Fig. 18 und 19). Bei Verwendung von Sonden mit mindestens 3 CpG-Stellen in ihren Sequenzen ließen sich diese heterogen methylierten Epi-Allele von den homogen methylierten Epi-Allelen unterscheiden und quantifizieren (Fig. 20). Homogen methylierte Epi-Allele, d.h. 3 von 3 CpG-Stellen sind methyliert, scheinen bei der Unterscheidung zwischen BPH und PCa spezifisch für cf-Tumor-DNA zu sein und können Grundlage für eine sichere Differentialdiagnose von benignen und malignen Erkrankungen der Prostata darstellen.

Im Fall des SERPINE1-Methylierung (unter Einsatz des Primerpaares SEQ ID No. 16 und 17 und des Sondenpaares SEQ ID No. 43 und 44) zeigten sich in den DNA-Proben aus PrEC und BPH-1 keine homogen noch heterogen methylierte Epi-Allele, so dass sich dieses Gen ebenfalls als Kandidatengen für die Differentialdiagnostik von BPH und Prostatakarzinomen anbietet (Fig. 21 und 22).

Ebenso zeigte sich für das GSTP1-Gen, dass sowohl in PrEC als auch in BPH-1-Zellen im Gegensatz zu den malignen Prostatazelllinien LNCaP, PC-3 und DU-145 keine homogen als auch heterogen methylierte (2 von 3 CpG-Stellen methyliert) Epi-Allele nachgewiesen werden konnten (Fig. 23 und 24). Interessanterweise kamen in der malignen PC-3-Zelllinie hauptsächlich heterogen methylierte (2 von 3 CpG-Stellen methyliert) Epi-Allele vor, die sich deutlich von denen der PrEC und BPH-1 unterschieden (Fig. 25 und 26). Hier lagen nur heterogen methylierte (1 von 3 CpG-Stellen methyliert) Epi-Allele in geringer Konzentration vor, die durch Erhöhung der einzustellenden Grenzlinie gut von denen der maligenen Zelllinien abzutrennen waren (Fig. 25 und 26).

**Ausführungsbeispiel 8: Differenzierung zwischen Gesund, benignen (gutartigen) und malignen (bösartigen) Prostata-Erkrankungen unter Einbeziehung und Quantifizierung von *PLA2R1*-Epi-Allelen**

Eine verbesserte Unterscheidung zwischen gesunden Probanden und Patienten mit Prostatakarzinomen zeigte sich auch bei der Bestimmung und Quantifizierung von PLA2R1-Epi-Allelen. So ließen sich, wurden 3 Epi-Allel-Fraktionen in die Bestimmung einbezogen (siehe Fig. 27), nur 4 der 40 PCa-Patienten (P1, P5, P21 und P30; *cut-off* der fraktionellen Methylierungshäufigkeit <3,25%) klar von den gesunden Probanden unterscheiden (Tab. 12). Wurden nur die homogen methylierten PLA2R1-Epi-Allele, d.h. 3 von 3 CpG-Stellen waren methyliert, bestimmt und quantifiziert (siehe Fig. 28), ließen sich 5 der 40 PCa-Patienten identifizieren (P1, P12, P17, P21 und P30; *cut-off*-Wert der fraktionellen Methylierungshäufigkeit <0,011%; Tab. 12). Interessant hierbei ist, dass bei Einbeziehung und Quantifizierung von nur homogen methylierten PLA2R1-Epi-Allelen P12 und P17 als PCa-erkrankte Patienten, dafür aber nicht P5 als PCa-Patient identifiziert wurden, was bei Einbeziehung von drei PLA2R1-Epi-Allelen zuvor aber der Fall war.

Im Gegensatz dazu wurden sowohl P1, P21 und P30 als auch P5, P12 und P17 und darüber hinaus P15, P33, P34 und P38 als PCa-Patienten erkannt, wenn zwei der PLA2R1-Epi-Allele (homogen und heterogen methylierte Epi-Allel mit 2 von 3 methylierten CpG-Stellen) quantifiziert wurden, d.h. 10 der 40 PCa-Patienten wurden identifiziert mit einer diagnostischen Spezifität von 100% (*cut-off*-Wert der fraktionellen Methylierungshäufigkeit <0,123%; Tab. 12). Da für diese Untersuchungen nur 200 μl Serum zur Verfügung standen, kann die hier - bei Einbeziehung von zwei PLA2R1-Epi-Allelen - erreichte diagnostische Sensitivität von 25% bei Einsatz einer größeren Menge von Serumvolumen und/oder bei Kombination mit anderen Gensequenzen weiter erhöht werden.

**Tabelle 12:** Übersicht über die Bestimmung der Methylierungsgrade von ***PLA2R1*** in Serumproben von gesunden Probanden (K1-20) und Patienten mit Prostatakarzinom (P1-40) bei Einbeziehung von 1, 2 oder 3 PLA2R1-Epi-Allelen (Fig. 27-29). Die Kopienanzahl/ 20 µl Ratio (Verhältnis methyliert zu nicht-methyliert), FM (fraktionale Methylierung, d.h. Anteil methylierter im Verhältnis zu nicht-methylierten und methylierten Fragmenten) und GR (eingestellte Grenzen, *thresholds*) sind aufgeführt. Fett gedruckte Werte sind gegenüber den *cut-off*-Werten erhöhte Methylierungswerte. M: methylierte, U: nicht-methylierte DNA-Fragmente und NTC: Negativkontrollen ohne DNA-Template.

| | | Einbeziehung von 3 Epi-Allelen | | | | Einbeziehung von 1 Epi-Allel | | | | Einbeziehung von 2 Epi-Allelen | | | |
|---|---|---|---|---|---|---|---|---|---|---|---|---|---|
| **ID** | **Target** | **Kopien/ 20µl** | **Ratio** | **FM** | **GR** | **Kopien/ 20µl** | **Ratio** | **FM** | **GR** | **Kopien/ 20µl** | **Ratio** | **FR** | **GR** |
| K1 | M | 904 | 0.0203 | 1.99 | 6669 | 0 | 0 | 0 | 12799 | 40 | 0.00091 | 0.091 | 10415 |
| | U | 44600 | | | 3200 | 44600 | | | 3200 | 44600 | | | 3212 |
| K2 | M | 816 | 0.0108 | 1.07 | 6669 | 0 | 0 | 0 | 12799 | 0 | 0 | 0 | 10415 |
| | U | 75600 | | | 3200 | 75600 | | | 3200 | 75400 | | | 3212 |
| K3 | M | 966 | 0.0105 | 1.04 | 6669 | 0 | 0 | 0 | 12799 | 0 | 0 | 0 | 10415 |
| | U | 92000 | | | 3200 | 92000 | | | 3200 | 91800 | | | 3212 |
| K4 | M | 2900 | 0.0315 | 3.05 | 6669 | 0 | 0 | 0 | 12799 | 24 | 0.00026 | 0.026 | 10415 |
| | U | 91800 | | | 3200 | 91800 | | | 3200 | 91600 | | | 3212 |
| K5 | M | 1300 | 0.013 | 1.29 | 6669 | 0 | 0 | 0 | 12799 | 0 | 0 | 0 | 10415 |
| | U | 99600 | | | 3200 | 99600 | | | 3200 | 99400 | | | 3212 |
| K6 | M | 200 | 0.0026 | 0.26 | 6669 | 0 | 0 | 0 | 12799 | 0 | 0 | 0 | 10415 |
| | U | 77600 | | | 3200 | 77600 | | | 3200 | 77600 | | | 3212 |
| K7 | M | 266 | 0.0031 | 0.31 | 6669 | 0 | 0 | 0 | 12799 | 0 | 0 | 0 | 10415 |
| | U | 84800 | | | 3200 | 84800 | | | 3200 | 84600 | | | 3212 |
| K8 | M | 712 | 0.008 | 0.79 | 6669 | 0 | 0 | 0 | 12799 | 0 | 0 | 0 | 10415 |
| | U | 89600 | | | 3200 | 89600 | | | 3200 | 89400 | | | 3212 |
| K9 | M | 2060 | 0.0299 | 2.91 | 6669 | 2.2 | 3,00E-05 | 0.003 | 12799 | 18 | 0.00026 | 0.026 | 10415 |
| | U | 68600 | | | 3200 | 68600 | | | 3200 | 68400 | | | 3212 |
| K10 | M | 0 | 0 | 0 | 6669 | 0 | 0 | 0 | 12799 | 0 | 0 | 0 | 10415 |
| | U | 74000 | | | 3200 | 74000 | | | 3200 | 73800 | | | 3212 |
| K11 | M | 3.4 | 5,00E-05 | 0.005 | 6669 | 0 | 0 | 0 | 12799 | 0 | 0 | 0 | 10415 |

(fortgesetzt)

| | | Einbeziehung von 3 Epi-Allelen | | | | Einbeziehung von 1 Epi-Allel | | | | Einbeziehung von 2 Epi-Allelen | | | |
|---|---|---|---|---|---|---|---|---|---|---|---|---|---|
| ID | Target | Kopien/ 20µl | Ratio | FM | GR | Kopien/ 20µl | Ratio | FM | GR | Kopien/ 20µl | Ratio | FR | GR |
| | U | 72000 | | | 3200 | 72000 | | | 3200 | 71800 | | | 3212 |
| K12 | M | 1110 | 0.0124 | 1.22 | 6669 | 0 | 0 | 0 | 12799 | 4.6 | 5,00E-05 | 0.005 | 10415 |
| | U | 90000 | | | 3200 | 90000 | | | 3200 | 89600 | | | 3212 |
| K13 | M | 20 | 0.00025 | 0.025 | 6669 | 0 | 0 | 0 | 12799 | 0 | 0 | 0 | 10415 |
| | U | 77800 | | | 3200 | 77800 | | | 3200 | 77600 | | | 3212 |
| K14 | M | 814 | 0.012 | 1.19 | 6669 | 0 | 0 | 0 | 12799 | 1.8 | 2.6E-05 | 0.0026 | 10415 |
| | U | 68000 | | | 3200 | 68000 | | | 3200 | 67800 | | | 3212 |
| K15 | M | 786 | 0.0112 | 1.11 | 6669 | 0 | 0 | 0 | 12799 | 0 | 0 | 0 | 10415 |
| | U | 70200 | | | 3200 | 70200 | | | 3200 | 70000 | | | 3212 |
| K16 | M | 10 | 0.00014 | 0.014 | 6669 | 0 | 0 | 0 | 12799 | 0 | 0 | 0 | 10415 |
| | U | 77000 | | | 3200 | 77000 | | | 3200 | 76600 | | | 3212 |
| K17 | M | 218 | 0.0023 | 0.23 | 6669 | 0 | 0 | 0 | 12799 | 0 | 0 | 0 | 10415 |
| | U | 94600 | | | 3200 | 94600 | | | 3200 | 94200 | | | 3212 |
| K18 | M | 162 | 0.0022 | 0.21 | 6669 | 0 | 0 | 0 | 12799 | 0 | 0 | 0 | 10415 |
| | U | 75400 | | | 3200 | 75400 | | | 3200 | 75400 | | | 3212 |
| K19 | M | 48 | 0.0006 | 0.06 | 6669 | 0 | 0 | 0 | 12799 | 0 | 0 | 0 | 10415 |
| | U | 79600 | | | 3200 | 79600 | | | 3200 | 79400 | | | 3212 |
| K20 | M | 2160 | 0.0336 | 3.25 | 6669 | 7.2 | 0.00011 | 0.011 | 12799 | 78 | 0.00123 | 0.123 | 10415 |
| | U | 64000 | | | 3200 | 64000 | | | 3200 | 64000 | | | 3212 |
| P1 | M | 940 | 0.09 | **8.2** | 6669 | 288 | 0.0275 | **2.67** | 12799 | 590 | 0.056 | **5.3** | 10415 |
| | U | 10480 | | | 3200 | 10480 | | | 3200 | 10460 | | | 3212 |
| P2 | M | 0 | 0 | 0 | 6669 | 0 | 0 | 0 | 12799 | 0 | 0 | 0 | 10415 |
| | U | 51800 | | | 3200 | 51800 | | | 3200 | 51800 | | | 3212 |

(fortgesetzt)

| | | Einbeziehung von 3 Epi-Allelen | | | | Einbeziehung von 1 Epi-Allel | | | | Einbeziehung von 2 Epi-Allelen | | | |
|---|---|---|---|---|---|---|---|---|---|---|---|---|---|
| ID | Target | Kopien/ 20μl | Ratio | FM | GR | Kopien/ 20μl | Ratio | FM | GR | Kopien/ 20μl | Ratio | FR | GR |
| P3 | M | 0 | 0 | 0 | 6669 | 0 | 0 | 0 | 12799 | 0 | 0 | 0 | 10415 |
| | U | 27940 | | | 3200 | 27940 | | | 3200 | 27940 | | | 3212 |
| P4 | M | 564 | 0.0134 | 1.32 | 6669 | 0 | 0 | 0 | 12799 | 52 | 0.0012 | 0.12 | 10415 |
| | U | 42200 | | | 3200 | 42200 | | | 3200 | 42200 | | | 3212 |
| P5 | M | 1520 | 0.061 | **5.76** | 6669 | 2.4 | 9,00E-05 | 0.009 | 12799 | 394 | 0.0158 | 1.55 | 10415 |
| | U | 24980 | | | 3200 | 24980 | | | 3200 | 24960 | | | 3212 |
| P6 | M | 0 | 0 | 0 | 6669 | 0 | 0 | 0 | 12799 | 0 | 0 | 0 | 10415 |
| | U | 7000 | | | 3200 | 7000 | | | 3200 | 7000 | | | 3212 |
| P7 | M | 0 | 0 | 0 | 6669 | 0 | 0 | 0 | 12799 | 0 | 0 | 0 | 10415 |
| | U | 21000 | | | 3200 | 21000 | | | 3200 | 20980 | | | 3212 |
| P8 | M | 2360 | 0.0301 | 2.92 | 6669 | 0 | 0 | 0 | 12799 | 30 | 0.00038 | 0.038 | 10415 |
| | U | 78600 | | | 3200 | 78600 | | | 3200 | 78400 | | | 3212 |
| P9 | M | 1360 | 0.0159 | 1.57 | 6669 | 7.8 | 9,00E-05 | 0.009 | 12799 | 50 | 0.00058 | 0.058 | 10415 |
| | U | 85400 | | | 3200 | 85400 | | | 3200 | 84800 | | | 3212 |
| P10 | M | 0 | 0 | 0 | 6669 | 0 | 0 | 0 | 12799 | 0 | 0 | 0 | 10415 |
| | U | 5200 | | | 3200 | 5200 | | | 3200 | 5200 | | | 3212 |
| P11 | M | 790 | 0.0133 | 1.31 | 6669 | 3.8 | 6,00E-05 | 0.006 | 12799 | 30 | 0.00051 | 0.051 | 10415 |
| | U | 59400 | | | 3200 | 59400 | | | 3200 | 59200 | | | 3212 |
| P12 | M | 1920 | 0.0267 | 2.6 | 6669 | 20 | 0.00029 | **0.029** | 12799 | 166 | 0.0023 | **0.23** | 10415 |
| | U | 71800 | | | 3200 | 71800 | | | 3200 | 71600 | | | 3212 |
| P13 | M | 710 | 0.0126 | 1.24 | 6669 | 0 | 0 | 0 | 12799 | 64 | 0.00112 | 0.112 | 10415 |
| | U | 56400 | | | 3200 | 56400 | | | 3200 | 56400 | | | 3212 |
| P14 | M | 4.2 | 0.00018 | 0.018 | 6669 | 0 | 0 | 0 | 12799 | 4.2 | 0.00018 | 0.018 | 10415 |

(fortgesetzt)

| | | Einbeziehung von 3 Epi-Allelen | | | | Einbeziehung von 1 Epi-Allel | | | | Einbeziehung von 2 Epi-Allelen | | | |
|---|---|---|---|---|---|---|---|---|---|---|---|---|---|
| **ID** | **Target** | **Kopien/ 20µl** | **Ratio** | **FM** | **GR** | **Kopien/ 20µl** | **Ratio** | **FM** | **GR** | **Kopien/ 20µl** | **Ratio** | **FR** | **GR** |
| | U | 23480 | | | 3200 | 23480 | | | 3200 | 23460 | | | 3212 |
| P15 | M | 634 | 0.0334 | 3.24 | 6669 | 0 | 0 | 0 | 12799 | 250 | 0.0132 | **1.3** | 10415 |
| | U | 18960 | | | 3200 | 18960 | | | 3200 | 18920 | | | 3212 |
| P16 | M | 4.8 | 8,00E-05 | 0.008 | 6669 | 0 | 0 | 0 | 12799 | 0 | 0 | 0 | 10415 |
| | U | 60600 | | | 3200 | 60600 | | | 3200 | 60400 | | | 3212 |
| P17 | M | 762 | 0.0283 | 2.75 | 6669 | 176 | 0.0066 | **0.65** | 12799 | 432 | 0.0161 | **1.58** | 10415 |
| | U | 26900 | | | 3200 | 26900 | | | 3200 | 26860 | | | 3212 |
| P18 | M | 0 | 0 | 0 | 6669 | 0 | 0 | 0 | 12799 | 0 | 0 | 0 | 10415 |
| | U | 22080 | | | 3200 | 22080 | | | 3200 | 22060 | | | 3212 |
| P19 | M | 0 | 0 | 0 | 6669 | 0 | 0 | 0 | 12799 | 0 | 0 | 0 | 10415 |
| | U | 43580 | | | 3200 | 43580 | | | 3200 | 43460 | | | 3212 |
| P20 | M | 34 | 0.00031 | 0.031 | 6669 | 0 | 0 | 0 | 12799 | 0 | 0 | 0 | 10415 |
| | U | 107200 | | | 3200 | 107200 | | | 3200 | 106400 | | | 3212 |
| P21 | M | 1580 | 0.069 | **6.42** | 6669 | 202 | 0.0088 | **0.87** | 12799 | 700 | 0.0305 | **2.96** | 10415 |
| | U | 23000 | | | 3200 | 23000 | | | 3200 | 23000 | | | 3212 |
| P22 | M | 0 | 0 | 0 | 6669 | 0 | 0 | 0 | 12799 | 0 | 0 | 0 | 10415 |
| | U | 9060 | | | 3200 | 9060 | | | 3200 | 9060 | | | 3212 |
| P23 | M | 0 | 0 | 0 | 6669 | 0 | 0 | 0 | 12799 | 0 | 0 | 0 | 10415 |
| | U | 5420 | | | 3200 | 5420 | | | 3200 | 5420 | | | 3212 |
| P24 | M | 0 | 0 | 0 | 6669 | 0 | 0 | 0 | 12799 | 0 | 0 | 0 | 10415 |
| | U | 992 | | | 3200 | 992 | | | 3200 | 992 | | | 3212 |
| P25 | M | 0 | 0 | 0 | 6669 | 0 | 0 | 0 | 12799 | 0 | 0 | 0 | 10415 |
| | U | 342 | | | 3200 | 342 | | | 3200 | 342 | | | 3212 |

(fortgesetzt)

| | | Einbeziehung von 3 Epi-Allelen | | | | Einbeziehung von 1 Epi-Allel | | | | Einbeziehung von 2 Epi-Allelen | | | |
|---|---|---|---|---|---|---|---|---|---|---|---|---|---|
| ID | Target | Kopien/ 20µl | Ratio | FM | GR | Kopien/ 20µl | Ratio | FM | GR | Kopien/ 20µl | Ratio | FR | GR |
| P26 | M | 1.8 | 0.00012 | 0.012 | 6669 | 0 | 0 | 0 | 12799 | 1.8 | 0.00012 | 0.012 | 10415 |
| | U | 15740 | | | 3200 | 15740 | | | 3200 | 15720 | | | 3212 |
| P27 | M | 0 | 0 | 0 | 6669 | 0 | 0 | 0 | 12799 | 0 | 0 | 0 | 10415 |
| | U | 500 | | | 3200 | 500 | | | 3200 | 500 | | | 3212 |
| P28 | M | 890 | 0.0108 | 1.06 | 6669 | 0 | 0 | 0 | 12799 | 4.2 | 5,00E-05 | 0.005 | 10415 |
| | U | 82800 | | | 3200 | 82800 | | | 3200 | 82400 | | | 3212 |
| P29 | M | 680 | 0.0114 | 1.13 | 6669 | 0 | 0 | 0 | 12799 | 26 | 0.00044 | 0.044 | 10415 |
| | U | 59400 | | | 3200 | 59400 | | | 3200 | 59400 | | | 3212 |
| P30 | M | 2360 | 0.0585 | **5.53** | 6669 | 64 | 0.0016 | **0.16** | 12799 | 454 | 0.0112 | **1.11** | 10415 |
| | U | 40440 | | | 3200 | 40440 | | | 3200 | 40400 | | | 3212 |
| P31 | M | 244 | 0.0032 | 0.32 | 6669 | 0 | 0 | 0 | 12799 | 0 | 0 | 0 | 10415 |
| | U | 77200 | | | 3200 | 77200 | | | 3200 | 77000 | | | 3212 |
| P32 | M | 3.4 | 0.00019 | 0.019 | 6669 | 0 | 0 | 0 | 12799 | 3.4 | 0.00019 | 0.019 | 10415 |
| | U | 18480 | | | 3200 | 18480 | | | 3200 | 18460 | | | 3212 |
| P33 | M | 454 | 0.0244 | 2.38 | 6669 | 0 | 0 | 0 | 12799 | 152 | 0.0081 | **0.81** | 10415 |
| | U | 18660 | | | 3200 | 18660 | | | 3200 | 18640 | | | 3212 |
| P34 | M | 654 | 0.0178 | 1.74 | 6669 | 0 | 0 | 0 | 12799 | 46 | 0.0013 | **0.13** | 10415 |
| | U | 36840 | | | 3200 | 36840 | | | 3200 | 36820 | | | 3212 |
| P35 | M | 0 | 0 | 0 | 6669 | 0 | 0 | 0 | 12799 | 0 | 0 | 0 | 10415 |
| | U | 46000 | | | 3200 | 46000 | | | 3200 | 46000 | | | 3212 |
| P36 | M | 0 | 0 | 0 | 6669 | 0 | 0 | 0 | 12799 | 0 | 0 | 0 | 10415 |
| | U | 28920 | | | 3200 | 28920 | | | 3200 | 28900 | | | 3212 |
| P37 | M | 0 | 0 | 0 | 6669 | 0 | 0 | 0 | 12799 | 0 | 0 | 0 | 10415 |

(fortgesetzt)

| | | Einbeziehung von 3 Epi-Allelen | | | | Einbeziehung von 1 Epi-Allel | | | | Einbeziehung von 2 Epi-Allelen | | | |
|---|---|---|---|---|---|---|---|---|---|---|---|---|---|
| **ID** | **Target** | **Kopien/ 20µl** | **Ratio** | **FM** | **GR** | **Kopien/ 20µl** | **Ratio** | **FM** | **GR** | **Kopien/ 20µl** | **Ratio** | **FR** | **GR** |
| | U | 82 | | | 3200 | 82 | | | 3200 | 72 | | | 3212 |
| P38 | M | 676 | 0.0279 | 2.71 | 6669 | 0 | 0 | 0 | 12799 | 148 | 0.0061 | **0.61** | 10415 |
| | U | 24240 | | | 3200 | 24240 | | | 3200 | 24200 | | | 3212 |
| P39 | M | 0 | 0 | 0 | 6669 | 0 | 0 | 0 | 12799 | 0 | 0 | 0 | 10415 |
| | U | 73000 | | | 3200 | 73000 | | | 3200 | 72800 | | | 3212 |
| P40 | M | 62 | 0.0026 | 0.26 | 6669 | 0 | 0 | 0 | 12799 | 14 | 0.00062 | 0.062 | 10415 |
| | U | 23440 | | | 3200 | 23440 | | | 3200 | 23420 | | | 3212 |
| NTC | M | 0 | 0 | 0 | 6669 | 0 | 0 | 0 | 12799 | 0 | 0 | 0 | 10415 |
| | U | | | | 3200 | 0 | | | 3200 | 0 | | | 3212 |
| NTC | M | 0 | 0 | 0 | 6669 | 0 | 0 | 0 | 12799 | 0 | 0 | 0 | 10415 |
| | U | | | | 3200 | 0 | | | 3200 | 0 | | | 3212 |
| NTC | M | 0 | 0 | 0 | 6669 | 0 | 0 | 0 | 12799 | 0 | 0 | 0 | 10415 |
| | U | | | | 3200 | 0 | | | 3200 | 0 | | | 3212 |

Da aus Zelllinien isolierte DNA in ausreichend Menge vorliegt, was in Liquid Biopsien wie Serum, Plasma oder Seminalflüssigkeit oftmals nicht der Fall ist, stellte sich die Frage, wie sich die fraktionale PLA2R1-Methylierungshäufigkeit in normalen PrEC und BPH-1-Zellen, in denen vereinzelt homogen als auch heterogen methylierte Epi-Allel-Kopien auftraten, nach der BBPA-dPCR im Vergleich zur dPCR allein verhält. Im Vergleich zur dPCR allein (Fig. 30, links) zeigte sich auch hier nach Prä-Amplifikation mit 50 Zyklen bei 63°C, dass der resultierende PLA2R1-Methylierungsgrad bei normalen PrEC und nicht-malignen BPH-1-Zellen sich in Richtung 0 % veränderte und die Methylierung der malignen Prostatazelllinien sich deutlich von der BPH-1-Zelllinie abgrenzen ließ (Fig. 30, rechts). Grund dafür ist erneut das Phänomen, dass in den PrEC und BPH-1-Zellen, trotz dass eine bestimmte Anzahl methylierter PLA2R1-Fragmente, wenn auch in geringer Menge, in den Proben vorkam, mit steigender Anzahl der Zyklen in der BBPA die nicht-methylierten Sequenzen verstärkt vervielfältigt wurden, ohne dass die methylierten Fragmente gleichermaßen mit vervielfältigt wurden (Fig. 31). Umgekehrt zeigte sich in den DNA-Proben der LNCaP und DU-145-Zellen eine deutlich bevorzugte Amplifikation der methylierten gegenüber nicht-methylierten Sequenzen. In den PC-3-Zellen erfolgte eine in etwa vergleichbare effiziente Vervielfältigung sowohl der methylierten als auch der nicht-methylierten PLA2R1-Sequenzen (Fig. 31).

Die 2-D-Darstellung der FAM- und HEX-positiven Signale nach 50-Zyklen in der BBPA belegte, dass die BPH-1-Zellpopulation sich deutlich von den PC-3, LNCaP und DU-145-Zellen absetzen ließ (Fig. 32). Ohne Darstellung der NTC, d. h. der doppelt-negativen Tröpfchen zeigte sich auch, dass nach 50 Zyklen bei allen Zellproben keine doppelt-negativen Tröpfchen mehr vorkamen, was bedeutet, dass die dPCR stark mit präamplifizierten DNA-Kopien überladen war und die Poisson-Verteilung und -Statistik nicht mehr zutraf und trotzdem oder gerade deshalb die maligen Prostatazelllinien sich so gut von den BPH-1 abtrennen ließen (Fig. 33). In der alleinigen dPCR ergab sich für die PrEC und BPH-1 noch ein fraktionaler Methylierungsgrad von 2,1 % (1,1-3,1 %) bzw. 8,3 % (7,0-9,5 %). In der BBPA-dPCR betrug der Methylierungsgrad für beide Zelllinien 0 %, während der Methylierungsgrad für die malignen Prostatazelllinien LNCaP, PC-3 und DU-145 mit den Daten der alleinigen dPCR gut übereinstimmten (Tabelle 20).

**Ausführungsbeispiel 9: Bestimmung der Bias zugunsten methylierter bzw. nicht-methylierter PLA2R1-Gensequenzen in Abhängigkeit vom Primer-Design, Annealingtemperatur, $MgCl_2$-Konzentration und Zyklenanzahl in der BBPA**

An Hand von Untersuchungen des PLA2R1-Gens und unterschiedlicher Primerpaare konnte mit Hilfe der dPCR gezeigt werden, wie die PCR-Bias für methylierte gegenüber nicht-methylierter DNA-Sequenzen in Abhängigkeit der $MgCl_2$-Konzentrationen und Annealingtemperaturen sich veränderte. Auf Grund dieser Ergebnisse können für jedes zu untersuchende Gen neue Testsysteme entwickelt werden, bei denen einzelne Tumor-DNA-Fragmente auch vor einem großen Hintergrund von Wild-Typ-DNA hoch sensitiv und spezifisch detektiert werden sollen. Durch entsprechende Einstellungen von $MgCl_2$-Konzentration, Annealingtemperaturen, Zyklenanzahl und Konstruktion der Primer können die Amplifikationsraten methylierter und nicht-methylierter Sequenzen so eingestellt werden, dass eine optimale Bias resultiert, je nachdem, ob methylierte oder nicht-methylierte Sequenzen als Target zur Identifikation von Tumor-spezifischer DNA dienen sollen. Im Fall von nicht-methylierten Sequenzen als Zieltarget werden bevorzugt Primerpaare ohne CpG-Stellen verwendet. Ist das auf Grund der Gensequenz nicht möglich, sind Primer so zu konstruieren, dass sie so wenig wie möglich CpG-Sequenzen enthalten und diese eher in Richtung des 5'-Endes der Primer angeordnet sind. Des Weiteren sind hier die $MgCl_2$-Konzentrationen und Annealingtemperaturen so einzustellen, dass bevorzugt nicht-methylierte gegenüber methylierten Sequenzen vervielfältigt werden.

So zeigte sich bei Verwendung von Proben mit 50%-igem Anteil von jeweils methylierten und nicht-methylierten DNA-Kopien und dem Primerpaar ohne CpG-Stellen (Primerpaar 168 bp) bei einer $MgCl_2$-Konzentration von 1,5 mM über den gesamten Temperaturbereich von 50-63°C eine Bias zugunsten nicht-methylierter DNA-Sequenzen (Fig. 34). Bei einer $MgCl_2$-Konzentration von 2,5-4,5 mM war bei diesem Primerpaar keine wesentliche Bias zu erkennen. Bei einer $MgCl_2$-Konzentration von 6,0 mM und besonders bei 8,0 mM zeigte sich bei diesem Primerpaar jedoch eine deutliche Bias zugunsten methylierter DNA-Sequenzen in den Temperaturbereichen 58,2-60,8°C bzw. 55,1-63,0°C (Fig. 34).

Bei Verwendung von Primerpaaren mit 1 CpG-Stelle (PL-161 bp) bzw. 2 CpG-Stellen (PL-150 bp) zeigte sich gegenüber den Primern ohne CpG-Stelle (PL-168 bp) eine Bias zugunsten methylierter Sequenzen, deren Höhe über die $MgCl_2$-Konzentration und Annealingtemperatur entsprechend eingestellt werden kann (Fig. 35 und 36).

Enthielten die Primer 4 CpG-Stellen, wobei ein Cytosinrest einer CpG-Sequenz direkt am 3'-Ende eines Primers angeordnet war, zeigte sich eine am stärksten ausgeprägte Bias, wobei die Amplifikationseffizienz in Abhängigkeit von der gewählten $MgCl_2$-Konzentration bei steigenden Annealingtemperaturen deutlich abnahm (Fig. 37). So fiel bei einer 1,5 mM $MgCl_2$-Konzentration die Amplifikationseffizienz für methylierte Sequenzen oberhalb einer Annealingtemperatur von 52,6°C kontinuierlich ab (Fig. 37).

Eine Bias zugunsten methylierter DNA-Sequenzen in Abhängigkeit von der Konstruktion der Primer, Annealingtemperatur und $MgCl_2$-Konzentration zeigten sich auch für die RASSF1A- und GSTP1-Primer (Tab. 21 und 22).

**Ausführungsbeispiel 10: Analytische Sensitivität und Spezifität der BBPA-ddPCR in Abhängigkeit vom Design der Primer, Annealingtemperatur, MgCh-Konzentration und Zyklenanzahl der BBPA**

Um festzustellen, unter welchen Bedingungen die größte analytische Sensitivität, insbesondere vor einem großen Hintergrund von Wildtyp-DNA erhalten werden kann, ohne dass falsch-positive Signale verstärkt auftreten - eine Grundvoraussetzung für eine hohe analytische und diagnostische Spezifität des Testverfahrens - wurden Testproben generiert, die einen geringen Anteil methylierter DNA-Fragmente (DNA aus U937-Leukämiezellen) gegenüber einem steigenden Anteil normaler Wild-Typ-DNA enthielten. Dazu wurden genomische DNA aus Blutleukozyten gesunder Probanden in einer PCR amplifziert. Auf diese Weise konnten Proben generiert werden, die eine ausreichend große Menge an nicht-methylierten, normalen Wild-Typ-DNA enthielten und denen keine oder nur geringe Mengen methylierter DNA zugesetzt wurden, d.h. 5, 10, 20 oder 3000 Kopien methylierte DNA-Fragmente. Als Hintergrund dienten 70.000, 175.000, 350.000 und 700.000 Kopien nicht-methylierter DNA. Daraus ergaben sich z.B. in Proben mit 700.000 nicht-methylierter DNA-Fragmente ein Anteil von 0%, 0,0007%, 0,0014%, 0,0028% und 0,43% methylierter PLA2R1-DNA-Fragmente.

In einer anschließenden dPCR, d.h. ohne vorherige Prä-Amplifikation, zeigten sich bei Verwendung der Primerpaare ohne CpG-Stellen (PLA2R1 168 bp) und mit 4 CpG-Stellen (PLA2R1 133 bp), dass mit beiden Primerpaaren in den Proben mit 70.000 nicht-methylierten Kopien die methylierten Kopien gut nachzuweisen waren (Tab. 23 und 24). Bei weiterem Anstieg der nicht-methylierten Kopien, d.h. in den Proben mit 175.000, 350.000 und 700.000 nicht-methylierten Kopien zeigte sich bei Verwendung des Primerpaares ohne CpG-Stellen ein zunehmend verminderter Nachweis der 3000 methylierten DNA-Fragmente (von 2780 bei 70.000 nicht-methylierten Kopien auf 1968, 560 bzw. 114 methylierter Kopien bei steigenden nicht-methylierten Kopien von 175.000, 350.000 und 700.000 nicht-methylierten Kopien, Tab. 23). Ein ähnlicher Abfall stellte sich auch bei Verwendung dieses Primerpaares in den Proben mit 5, 10 und 20 Kopien methylierter DNA ein (Tab. 23). Im Gegensatz dazu konnten mit dem Primerpaar mit 4 CpG-Stellen (PLA2R1-133 bp) die 3000 Kopien methylierter DNA auch in den Proben mit 175.000, 350.000 und 700.000 nicht-methylierten Kopien vollständig nachgewiesen werden (Tab. 24). Die geringen Konzentrationen methylierten DNA-Fragmente ließen sich jedoch auch hier bei steigendem Hintergrund nicht-methylierter DNA nicht sicher detektieren (Tab. 24).

Im Folgenden wurde deshalb der Frage nachgegangen, unter welchen Bedingungen eine verbesserte Sensitivität für den Nachweis geringer Konzentrationen von Tumor-DNA vor einem großen Hintergrund an Wild-Typ-DNA erreicht werden kann, ohne dabei verstärkt falsch positive Signale zu generieren. Letzteres bedeutet, dass die erhaltenen Werte in den Proben ohne Kopien methylierter DNA sich von den Proben mit nur 5 Kopien methylierter DNA deutlich unterscheiden.

Bei Verwendung der Primerpaare mit einer (PLA2R1 161 bp) oder zwei CpG-Stellen (PLA2R1 150 bp) im Vergleich zu dem Primerpaar ohne CpG-Stelle (PLA2R1 168 bp) war eine erhöhte analytische Sensitivität nach einer Prä-Amplifikation durch 15 Zyklen bei einer Annealingtemperatur von 63°C und einer 2,5 mM $MgCl_2$-Konzentration nachweisbar (Tab. 25 und 26).

Eine erhöhte Sensitivität und vor allem aber eine deutlich verbesserte analytische Spezifität zeigte sich auch hier überraschend mit dem Primerpaar mit 4 CpG-Stellen (PLA2R1 133bp), wenn dabei die $MgCl_2$-Konzentration und Annealingtemperatur beachtet wurden. Während bei einer $MgCl_2$-Konzentration von 6,0 mM bei einer Annealingtemperatur von 50°C nur die 3000 methylierten Kopien, nicht aber die 5, 10 und 20 Kopien methylierter DNA nachzuweisen waren, wurde eine Unterscheidung der Proben mit dem geringen Anteil methylierter DNA möglich, wenn bei gleicher Annealingtemperatur von 50°C die $MgCl_2$-Konzentration auf 1,5 mM reduziert wurde (Tab. 27 und 29). Wurde im Vergleich dazu die Annealingtemperatur auf 63°C erhöht bei gleichzeitiger $MgCl_2$-Konzentration von 1,5 mM, ließen sich die Proben ohne und mit 5 Kopien methylierter DNA vor einem Hintergrund von 700.000 nicht-methylierten DNA-Kopien sowohl nach 15x als auch nach 50x Zyklen nicht voneinander unterscheiden (Tab. 28 und 30). Das änderte sich, wenn bei einer Annealingtemperatur von 63°C die $MgCl_2$-Konzentration auf 6,0 mM erhöht wurde. Hier zeigte sich nach 15 Zyklen unter Verwendung des Primerpaares mit 4 CpG-Stellen (PLA2R1 133 bp) bei allen Proben eine hohe analytische Sensitivität und Spezifität. Wurde die Zyklenzahl von 15 auf 50 erhöht, d.h. die KPK (Kopien pro Kompartiment) lag weit oberhalb der nach dem Stand der Technik empfohlenen KPK von maximal 8 [9], zeigten sich die besten Ergebnisse hinsichtlich analytischer Sensitivität und Spezifität, die jeweils 100% betrugen. Waren die Signale zwischen den Proben ohne methylierte Kopien mit denen mit 5 Kopien methylierter DNA bereits nach 15 Zyklen deutlich und signifikant zu unterscheiden, zeigten sich nach 50 Zyklen in allen Proben ohne Kopien methylierter DNA (mit Ausnahme der Probe mit 350.000 Kopien nicht-methylierter DNA (hier war 1 Tröpfchen der insgesamt 16.235 Tröpfchen falsch positiv für FAM-Signale), keine positiven Signale, während in allen anderen Proben mit methylierten DNA-Fragmenten deutlich erhöhte FAM-Signale nachweisbar waren (Tab. 28 und 30, Fig. 38-45).

Die Grundlage für die erreichten hohen analytischen Sensitivitäten und Spezifitäten könnte unter den gewählten Reaktionsbedingungen in einer besonderen Konkurrenzreaktion der Primer mit 4 CpG-Stellen, wobei ein Cytosinrest sich direkt am 3'-Ende befindet (PLA2R1 133 bp) um die methylierten und nicht-methylierten DNA-Fragmenten begründet sein. So zeigte sich in der 2-D-Grafik, dass nach 50 Zyklen Prä-Amplifikation die 70.000 nicht-methylierten DNA-Kopien nur zu einem positiven HEX-Signal führten, wenn absolut keine Kopie methylierter DNA in der Ausgangsproben vorlag (Fig. 38 und 39). Bereits 5 Kopien methylierter DNA verhinderten, dass nach 50 Zyklen Prä-Amplifikation 70.000 Kopien nicht-methylierter DNA zu einem Anstieg der HEX-Signale führte. Wurde die Kopienanzahl nicht-methylierter DNA auf 175.000 erhöht, reichten 10, nicht aber 5 Kopien methylierter DNA mehr aus, die Generierung positiver HEX-Signale vollständig zu verhindern (Fig. 40 und 41). Wurden die nicht-methylierten DNA-Kopien weiter auf 350.000 und 700.000 erhöht, reichten schließlich nur die 3000 Kopien methylierter DNA aus, die Generierung von positiven HEX-Signalen zu unterbinden (Fig. 42 und 44). Auf Grund dieser Konkurrenzreaktion scheinen unter diesen stringenten Reaktionsbedingungen falsch-positive Signale verhindert zu werden, was bei der PCR unter Verwendung methylspezifischer Primer (MSP) bisher nicht der Fall war [4, 16-19]. Werden die starken Signalunterschiede zwischen den Proben mit 5 bzw. keinen Kopien methylierter PLA2R1-DNA-Fragmente bei einem jeweiligen Hintergrund von 700.000 nicht-methylierten DNA-Kopien berücksichtigt (Fig. 44), kann davon ausgegangen werden, dass auch ein einzelnes methyliertes PLA2R1-Fragment mit dem Primerpaar mit 4 CpG-Stellen (PLA2R1-133 bp) bei 6,0 mM $MgCl_2$-Konzentration und 63,0°C sicher nachgewiesen werden kann. Daraus würde sich eine analytische Sensitivität von 1 zu 700.000 (Verhältnis von 1,4 x $10^{-6}$) bzw. einem Methylierungsgrad von 0,00014% ergeben.

**Tabelle 13:** FAM-Signale (methylierte ***GSTP1***-DNA-Fragmente) und HEX-Signale (nicht-methylierte ***GSTP1***-DNA-Fragmente) in Serumproben gesunder Probandinnen (GF1-GF20) und Serumproben von Patientinnen mit Mammakarzinom (MF1-MF20) und Non-Template-Kontrolle (NTC, ohne DNA bzw. genomische DNA ohne Bisulfitumwandlung) nach 15 Zyklen in der BBPA mit dem GSTP1-120bp-Primern (SEQ ID: 5 und 6) und 2,5 mM $MgCl_2$-Konzentration bei 50,7°C und anschließender dPCR. *Cut-off*-Wert: <0,07%; Rohdaten zur Fig. 55.

| Pos. | Probe | Kopien/ 20μL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| A01 | GM1 | 0 | 0 | 11659 | 0 | 0 | 9615 | 2044 | 11659 | 0 | 0 | 3979 |
| A01 | | 40960 | 9615 | 2044 | 0 | 0 | 9615 | 2044 | 11659 | | | 2133 |
| B01 | GM2 | 9.2 | 5 | 12808 | 3 | 2 | 7860 | 4948 | 12813 | 0.00041 | 0.041 | 3979 |
| B01 | | 22380 | 7863 | 4950 | 3 | 2 | 7860 | 4948 | 12813 | | | 2133 |
| C01 | GM3 | 9.6 | 5 | 12273 | 4 | 1 | 9038 | 3235 | 12278 | 0.00031 | 0.031 | 3979 |
| C01 | | 31380 | 9042 | 3236 | 4 | 1 | 9038 | 3235 | 12278 | | | 2133 |
| D01 | GM4 | 0 | 0 | 12271 | 0 | 0 | 10748 | 1523 | 12271 | 0 | 0 | 3979 |
| D01 | | 49000 | 10748 | 1523 | 0 | 0 | 10748 | 1523 | 12271 | | | 2133 |
| E01 | GM5 | 0 | 0 | 13518 | 0 | 0 | 12481 | 1037 | 13518 | 0 | 0 | 3979 |
| E01 | | 60400 | 12481 | 1037 | 0 | 0 | 12481 | 1037 | 13518 | | | 2133 |
| F01 | GM6 | 0 | 0 | 10542 | 0 | 0 | 9441 | 1101 | 10542 | 0 | 0 | 3979 |
| F01 | | 53200 | 9441 | 1101 | 0 | 0 | 9441 | 1101 | 10542 | | | 2133 |
| G01 | GM7 | 7.8 | 4 | 12116 | 2 | 2 | 8646 | 3470 | 12120 | 0.00026 | 0.026 | 3979 |
| G01 | | 29420 | 8648 | 3472 | 2 | 2 | 8646 | 3470 | 12120 | | | 2133 |
| H01 | GM8 | 3.8 | 2 | 12457 | 1 | 1 | 11087 | 1370 | 12459 | 7,00E-05 | 0.007 | 3979 |
| H01 | | 52000 | 11088 | 1371 | 1 | 1 | 11087 | 1370 | 12459 | | | 2133 |
| A02 | GM9 | 0 | 0 | 12452 | 0 | 0 | 10674 | 1778 | 12452 | 0 | 0 | 3979 |
| A02 | | 45800 | 10674 | 1778 | 0 | 0 | 10674 | 1778 | 12452 | | | 2133 |
| B02 | GM10 | 0 | 0 | 12588 | 0 | 0 | 11275 | 1313 | 12588 | 0 | 0 | 3979 |
| B02 | | 53200 | 11275 | 1313 | 0 | 0 | 11275 | 1313 | 12588 | | | 2133 |
| C02 | GM11 | 0 | 0 | 12385 | 0 | 0 | 11107 | 1278 | 12385 | 0 | 0 | 3979 |
| C02 | | 53400 | 11107 | 1278 | 0 | 0 | 11107 | 1278 | 12385 | | | 2133 |

(fortgesetzt)

| Pos. | Probe | Kopien/ 20µL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| D02 | GM12 | 5.6 | 3 | 12540 | 2 | 1 | 10747 | 1793 | 12543 | 0.00012 | 0.012 | 3979 |
| D02 | | 45800 | 10749 | 1794 | 2 | 1 | 10747 | 1793 | 12543 | | | 2133 |
| E02 | GM13 | 0 | 0 | 11600 | 0 | 0 | 10610 | 990 | 11600 | 0 | 0 | 3979 |
| E02 | | 58000 | 10610 | 990 | 0 | 0 | 10610 | 990 | 11600 | | | 2133 |
| F02 | GM14 | 19.2 | 10 | 12310 | 6 | 4 | 8428 | 3882 | 12320 | 0.0007 | 0.07 | 3979 |
| F02 | | 27140 | 8434 | 3886 | 6 | 4 | 8428 | 3882 | 12320 | | | 2133 |
| G02 | GM15 | 9.8 | 5 | 12025 | 4 | 1 | 9614 | 2411 | 12030 | 0.00026 | 0.026 | 3979 |
| G02 | | 37820 | 9618 | 2412 | 4 | 1 | 9614 | 2411 | 12030 | | | 2133 |
| H02 | GM16 | 1.6 | 1 | 13960 | 1 | 0 | 12996 | 964 | 13961 | 2.7E-05 | 0.0027 | 3979 |
| H02 | | 62800 | 12997 | 964 | 1 | 0 | 12996 | 964 | 13961 | | | 2133 |
| A03 | GM17 | 13.2 | 7 | 12464 | 5 | 2 | 9108 | 3356 | 12471 | 0.00043 | 0.043 | 3979 |
| A03 | | 30880 | 9113 | 3358 | 5 | 2 | 9108 | 3356 | 12471 | | | 2133 |
| B03 | GM18 | 3.8 | 2 | 12154 | 0 | 2 | 6479 | 5675 | 12156 | 0.00022 | 0.022 | 3979 |
| B03 | | 17920 | 6479 | 5677 | 0 | 2 | 6479 | 5675 | 12156 | | | 2133 |
| C03 | GM19 | 1.8 | 1 | 12487 | 0 | 1 | 7424 | 5063 | 12488 | 9,00E-05 | 0.009 | 3979 |
| C03 | | 21240 | 7424 | 5064 | 0 | 1 | 7424 | 5063 | 12488 | | | 2133 |
| D03 | GM20 | 0 | 0 | 14041 | 0 | 0 | 11809 | 2232 | 14041 | 0 | 0 | 3979 |
| D03 | | 43280 | 11809 | 2232 | 0 | 0 | 11809 | 2232 | 14041 | | | 2133 |
| E03 | ddNTC | 0 | 0 | 11889 | 0 | 0 | 2 | 11887 | 11889 | 0 | 0 | 3979 |
| E03 | | 4 | 2 | 11887 | 0 | 0 | 2 | 11887 | 11889 | | | 2133 |
| F03 | ddNTC | 0 | 0 | 13770 | 0 | 0 | 1 | 13769 | 13770 | 0 | 0 | 3979 |
| F03 | | 1.8 | 1 | 13769 | 0 | 0 | 1 | 13769 | 13770 | | | 2133 |
| G03 | ddNTC | 0 | 0 | 12580 | 0 | 0 | 11 | 12569 | 12580 | 0 | 0 | 3979 |
| G03 | | 20 | 11 | 12569 | 0 | 0 | 11 | 12569 | 12580 | | | 2133 |

(fortgesetzt)

| Pos. | Probe | Kopien/ 20µL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| H03 | ddNTC | 0 | 0 | 12067 | 0 | 0 | 4 | 12063 | 12067 | 0 | 0 | 3979 |
| H03 | | 7.8 | 4 | 12063 | 0 | 0 | 4 | 12063 | 12067 | | | 2133 |
| A04 | PPCa1 | 9.8 | 5 | 11906 | 3 | 2 | 8509 | 3397 | 11911 | 0.00033 | 0.033 | 3979 |
| A04 | | 29500 | 8512 | 3399 | 3 | 2 | 8509 | 3397 | 11911 | | | 2133 |
| B04 | PPCa2 | 2 | 1 | 12218 | 0 | 1 | 6498 | 5720 | 12219 | 0.00011 | 0.011 | 3979 |
| B04 | | 17860 | 6498 | 5721 | 0 | 1 | 6498 | 5720 | 12219 | | | 2133 |
| C04 | PPCa3 | 2580 | 1356 | 11727 | 389 | 967 | 2516 | 9211 | 13083 | 0.436 | **30.4** | 3979 |
| C04 | | 5900 | 2905 | 10178 | 389 | 967 | 2516 | 9211 | 13083 | | | 2133 |
| D04 | PPCa4 | 696 | 380 | 12663 | 110 | 270 | 7269 | 5394 | 13043 | 0.0354 | **3.42** | 3979 |
| D04 | | 19620 | 7379 | 5664 | 110 | 270 | 7269 | 5394 | 13043 | | | 2133 |
| E04 | PPCa5 | 2.6 | 1 | 8926 | 1 | 0 | 4 | 8922 | 8927 | 0 | 0 | 3979 |
| E04 | | 14 | 5 | 8922 | 1 | 0 | 4 | 8922 | 8927 | | | 2133 |
| F04 | PPCa6 | 0 | 0 | 13126 | 0 | 0 | 11605 | 1521 | 13126 | 0 | 0 | 3979 |
| F04 | | 50800 | 11605 | 1521 | 0 | 0 | 11605 | 1521 | 13126 | | | 2133 |
| G04 | PPCa7 | 76 | 40 | 12261 | 32 | 8 | 7991 | 4270 | 12301 | 0.0031 | **0.31** | 3979 |
| G04 | | 24860 | 8023 | 4278 | 32 | 8 | 7991 | 4270 | 12301 | | | 2133 |
| H04 | PPCa8 | 226 | 118 | 12178 | 96 | 22 | 7170 | 5008 | 12296 | 0.0108 | **1.07** | 3979 |
| H04 | | 21040 | 7266 | 5030 | 96 | 22 | 7170 | 5008 | 12296 | | | 2133 |
| A05 | PPCa9 | 11.4 | 6 | 12345 | 4 | 2 | 8044 | 4301 | 12351 | 0.00046 | 0.046 | 3979 |
| A05 | | 24800 | 8048 | 4303 | 4 | 2 | 8044 | 4301 | 12351 | | | 2133 |
| B05 | PPCa10 | 5 | 3 | 13879 | 0 | 3 | 7226 | 6653 | 13882 | 0.00029 | 0.029 | 3979 |
| B05 | | 17300 | 7226 | 6656 | 0 | 3 | 7226 | 6653 | 13882 | | | 2133 |
| C05 | PPCa1 1 | 0 | 0 | 13220 | 0 | 0 | 7999 | 5221 | 13220 | 0 | 0 | 3979 |
| C05 | | 21860 | 7999 | 5221 | 0 | 0 | 7999 | 5221 | 13220 | | | 2133 |

(fortgesetzt)

| Pos. | Probe | Kopien/ 20μL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| D05 | PPCa12 | 3.8 | 2 | 12680 | 1 | 1 | 9583 | 3097 | 12682 | 0.00011 | 0.011 | 3979 |
| D05 | | 33160 | 9584 | 3098 | 1 | 1 | 9583 | 3097 | 12682 | | | 2133 |
| E05 | PPCa13 | 26 | 16 | 14337 | 0 | 16 | 4798 | 9539 | 14353 | 0.0027 | **0.27** | 3979 |
| E05 | | 9580 | 4798 | 9555 | 0 | 16 | 4798 | 9539 | 14353 | | | 2133 |
| F05 | PPCa14 | 3.6 | 2 | 13179 | 0 | 2 | 7233 | 5946 | 13181 | 0.00019 | 0.019 | 3979 |
| F05 | | 18720 | 7233 | 5948 | 0 | 2 | 7233 | 5946 | 13181 | | | 2133 |
| G05 | PPCa15 | 210 | 100 | 11137 | 9 | 91 | 4348 | 6789 | 11237 | 0.0182 | **1.79** | 3979 |
| G05 | | 11540 | 4357 | 6880 | 9 | 91 | 4348 | 6789 | 11237 | | | 2133 |
| H05 | PPCa16 | 9 | 5 | 12963 | 3 | 2 | 8580 | 4383 | 12968 | 0.00036 | 0.036 | 3979 |
| H05 | | 25520 | 8583 | 4385 | 3 | 2 | 8580 | 4383 | 12968 | | | 2133 |
| A06 | PPCa17 | 316 | 202 | 14943 | 101 | 101 | 6978 | 7965 | 15145 | 0.0213 | **2.09** | 3979 |
| A06 | | 14820 | 7079 | 8066 | 101 | 101 | 6978 | 7965 | 15145 | | | 2133 |
| B06 | PPCa18 | 8.6 | 5 | 13773 | 3 | 2 | 5824 | 7949 | 13778 | 0.0007 | 0.07 | 3979 |
| B06 | | 12940 | 5827 | 7951 | 3 | 2 | 5824 | 7949 | 13778 | | | 2133 |
| C06 | PPCa19 | 7.4 | 4 | 12673 | 0 | 4 | 4727 | 7946 | 12677 | 0.0007 | 0.07 | 3979 |
| C06 | | 10980 | 4727 | 7950 | 0 | 4 | 4727 | 7946 | 12677 | | | 2133 |
| D06 | PPCa20 | 24 | 14 | 13839 | 0 | 14 | 4895 | 8944 | 13853 | 0.0023 | **0.23** | 3979 |
| D06 | | 10260 | 4895 | 8958 | 0 | 14 | 4895 | 8944 | 13853 | | | 2133 |
| E06 | gDNA 15 | 14.6 | 8 | 12975 | 0 | 8 | 879 | 12096 | 12983 | 0.009 | 0.9 | 3979 |
| E06 | | 1640 | 879 | 12104 | 0 | 8 | 879 | 12096 | 12983 | | | 2133 |
| F06 | NTC 15 | 0 | 0 | 13676 | 0 | 0 | 53 | 13623 | 13676 | 0 | 0 | 3979 |
| F06 | | 92 | 53 | 13623 | 0 | 0 | 53 | 13623 | 13676 | | | 2133 |

**Tabelle 14:** FAM-Signale (methylierte ***GSTP1***-DNA-Fragmente) und HEX-Signale (nicht-methylierte ***GSTP1***-DNA-Fragmente) in Serumproben gesunder Probandinnen (GF1-GF20) und Serumproben von Patientinnen mit Mammakarzinom (MF1-MF20) und Non-Template-Kontrolle (NTC, ohne DNA bzw. genomische DNA ohne Bisulfitumwandlung) nach 15 Zyklen in der BBPA mit dem GSTP1-116bp-Primern (SEQ ID: 93 und 94) und 4,5 mM $MgCl_2$-Konzentration bei 53,8°C und anschließender dPCR. *Cut-off*-Wert: < 0,023%; Rohdaten zur Fig. 56.

| Pos. | Probe | Kopien/ 20µL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| A01 | GM1 | 1.4 | 1 | 15767 | 0 | 1 | 7390 | 8377 | 15768 | 0.0001 | 0.01 | 2927 |
| A01 | | 14880 | 7390 | 8378 | 0 | 1 | 7390 | 8377 | 15768 | | | 1705 |
| B01 | GM2 | 0 | 0 | 14360 | 0 | 0 | 2251 | 12109 | 14360 | 0 | 0 | 2927 |
| B01 | | 4020 | 2251 | 12109 | 0 | 0 | 2251 | 12109 | 14360 | | | 1705 |
| C01 | GM3 | 0 | 0 | 14640 | 0 | 0 | 6362 | 8278 | 14640 | 0 | 0 | 2927 |
| C01 | | 13420 | 6362 | 8278 | 0 | 0 | 6362 | 8278 | 14640 | | | 1705 |
| D01 | GM4 | 0 | 0 | 14771 | 0 | 0 | 10267 | 4504 | 14771 | 0 | 0 | 2927 |
| D01 | | 27940 | 10267 | 4504 | 0 | 0 | 10267 | 4504 | 14771 | | | 1705 |
| E01 | GM5 | 0 | 0 | 14872 | 0 | 0 | 11131 | 3741 | 14872 | 0 | 0 | 2927 |
| E01 | | 32480 | 11131 | 3741 | 0 | 0 | 11131 | 3741 | 14872 | | | 1705 |
| F01 | GM6 | 1.8 | 1 | 13135 | 0 | 1 | 7488 | 5647 | 13136 | 9,00E-05 | 0.009 | 2927 |
| F01 | | 19860 | 7488 | 5648 | 0 | 1 | 7488 | 5647 | 13136 | | | 1705 |
| G01 | GM7 | 0 | 0 | 13798 | 0 | 0 | 3363 | 10435 | 13798 | 0 | 0 | 2927 |
| G01 | | 6580 | 3363 | 10435 | 0 | 0 | 3363 | 10435 | 13798 | | | 1705 |
| H01 | GM8 | 0 | 0 | 13949 | 0 | 0 | 4887 | 9062 | 13949 | 0 | 0 | 2927 |
| H01 | | 10140 | 4887 | 9062 | 0 | 0 | 4887 | 9062 | 13949 | | | 1705 |
| A02 | GM9 | 0 | 0 | 12667 | 0 | 0 | 6686 | 5981 | 12667 | 0 | 0 | 2927 |
| A02 | | 17660 | 6686 | 5981 | 0 | 0 | 6686 | 5981 | 12667 | | | 1705 |
| B02 | GM10 | 0 | 0 | 13982 | 0 | 0 | 10312 | 3670 | 13982 | 0 | 0 | 2927 |
| B02 | | 31480 | 10312 | 3670 | 0 | 0 | 10312 | 3670 | 13982 | | | 1705 |
| C02 | GM11 | 2 | 1 | 12293 | 1 | 0 | 3608 | 8685 | 12294 | 0.00023 | 0.023 | 2927 |
| C02 | | 8180 | 3609 | 8685 | 1 | 0 | 3608 | 8685 | 12294 | | | 1705 |

(fortgesetzt)

| Pos. | Probe | Kopien/ 20μL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| D02 | GM12 | 0 | 0 | 13369 | 0 | 0 | 3709 | 9660 | 13369 | 0 | 0 | 2927 |
| D02 | | 7640 | 3709 | 9660 | 0 | 0 | 3709 | 9660 | 13369 | | | 1705 |
| E02 | GM13 | 0 | 0 | 11019 | 0 | 0 | 7254 | 3765 | 11019 | 0 | 0 | 2927 |
| E02 | | 25260 | 7254 | 3765 | 0 | 0 | 7254 | 3765 | 11019 | | | 1705 |
| F02 | GM14 | 0 | 0 | 13031 | 0 | 0 | 4748 | 8283 | 13031 | 0 | 0 | 2927 |
| F02 | | 10660 | 4748 | 8283 | 0 | 0 | 4748 | 8283 | 13031 | | | 1705 |
| G02 | GM15 | 0 | 0 | 15122 | 0 | 0 | 4572 | 10550 | 15122 | 0 | 0 | 2927 |
| G02 | | 8480 | 4572 | 10550 | 0 | 0 | 4572 | 10550 | 15122 | | | 1705 |
| H02 | GM16 | 0 | 0 | 12342 | 0 | 0 | 5999 | 6343 | 12342 | 0 | 0 | 2927 |
| H02 | | 15660 | 5999 | 6343 | 0 | 0 | 5999 | 6343 | 12342 | | | 1705 |
| A03 | GM17 | 0 | 0 | 14605 | 0 | 0 | 2825 | 11780 | 14605 | 0 | 0 | 2927 |
| A03 | | 5060 | 2825 | 11780 | 0 | 0 | 2825 | 11780 | 14605 | | | 1705 |
| B03 | GM18 | 0 | 0 | 12935 | 0 | 0 | 3736 | 9199 | 12935 | 0 | 0 | 2927 |
| B03 | | 8020 | 3736 | 9199 | 0 | 0 | 3736 | 9199 | 12935 | | | 1705 |
| C03 | GM19 | 0 | 0 | 12230 | 0 | 0 | 4769 | 7461 | 12230 | 0 | 0 | 2927 |
| C03 | | 11620 | 4769 | 7461 | 0 | 0 | 4769 | 7461 | 12230 | | | 1705 |
| D03 | GM20 | 0 | 0 | 12400 | 0 | 0 | 7509 | 4891 | 12400 | 0 | 0 | 2927 |
| D03 | | 21880 | 7509 | 4891 | 0 | 0 | 7509 | 4891 | 12400 | | | 1705 |
| E03 | ddNTC | 0 | 0 | 13014 | 0 | 0 | 1 | 13013 | 13014 | 0 | 0 | 2927 |
| E03 | | 1.8 | 1 | 13013 | 0 | 0 | 1 | 13013 | 13014 | | | 1705 |
| F03 | ddNTC | 0 | 0 | 13618 | 0 | 0 | 2 | 13616 | 13618 | 0 | 0 | 2927 |
| F03 | | 3.4 | 2 | 13616 | 0 | 0 | 2 | 13616 | 13618 | | | 1705 |
| G03 | ddNTC | 0 | 0 | 12250 | 0 | 0 | 0 | 12250 | 12250 | 0 | 0 | 2927 |
| G03 | | 0 | 0 | 12250 | 0 | 0 | 0 | 12250 | 12250 | | | 1705 |

(fortgesetzt)

| Pos. | Probe | Kopien/ 20µL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| H03 | ddNTC | 0 | 0 | 12222 | 0 | 0 | 2 | 12220 | 12222 | 0 | 0 | 2927 |
| H03 | | 3.8 | 2 | 12220 | 0 | 0 | 2 | 12220 | 12222 | | | 1705 |
| A04 | PPCa1 | 166 | 87 | 12317 | 5 | 82 | 1552 | 10765 | 12404 | 0.052 | **5** | 2927 |
| A04 | | 3160 | 1557 | 10847 | 5 | 82 | 1552 | 10765 | 12404 | | | 1705 |
| B04 | PPCa2 | 716 | 372 | 12047 | 27 | 345 | 3074 | 8973 | 12419 | 0.106 | **9.6** | 2927 |
| B04 | | 6760 | 3101 | 9318 | 27 | 345 | 3074 | 8973 | 12419 | | | 1705 |
| C04 | PPCa3 | 69400 | 9844 | 546 | 7 | 9837 | 0 | 546 | 10390 | 4400 | **99.977** | 2927 |
| C04 | | 16 | 7 | 10383 | 7 | 9837 | 0 | 546 | 10390 | | | 1705 |
| D04 | PPCa4 | 5720 | 2688 | 9783 | 36 | 2652 | 602 | 9181 | 12471 | 4.62 | **82.2** | 2927 |
| D04 | | 1236 | 638 | 11833 | 36 | 2652 | 602 | 9181 | 12471 | | | 1705 |
| E04 | PPCa5 | 1.8 | 1 | 12478 | 0 | 1 | 1634 | 10844 | 12479 | 0.0006 | **0.06** | 2927 |
| E04 | | 3300 | 1634 | 10845 | 0 | 1 | 1634 | 10844 | 12479 | | | 1705 |
| F04 | PPCa6 | 0 | 0 | 11920 | 0 | 0 | 8589 | 3331 | 11920 | 0 | 0 | 2927 |
| F04 | | 30000 | 8589 | 3331 | 0 | 0 | 8589 | 3331 | 11920 | | | 1705 |
| G04 | PPCa7 | 26300 | 8374 | 4070 | 496 | 7878 | 994 | 3076 | 12444 | 8.76 | **89.8** | 2927 |
| G04 | | 3000 | 1490 | 10954 | 496 | 7878 | 994 | 3076 | 12444 | | | 1705 |
| H04 | PPCa8 | 33740 | 9434 | 2953 | 937 | 8497 | 761 | 2192 | 12387 | 9.7 | **90.68** | 2927 |
| H04 | | 3460 | 1698 | 10689 | 937 | 8497 | 761 | 2192 | 12387 | | | 1705 |
| A05 | PPCa9 | 0 | 0 | 12183 | 0 | 0 | 3952 | 8231 | 12183 | 0 | 0 | 2927 |
| A05 | | 9220 | 3952 | 8231 | 0 | 0 | 3952 | 8231 | 12183 | | | 1705 |
| B05 | PPCa10 | 0 | 0 | 12193 | 0 | 0 | 15 | 12178 | 12193 | 0 | 0 | 2927 |
| B05 | | 28 | 15 | 12178 | 0 | 0 | 15 | 12178 | 12193 | | | 1705 |
| C05 | PPCa11 | 1000 | 136 | 3161 | 32 | 104 | 977 | 2184 | 3297 | 0.115 | **10.3** | 2927 |
| C05 | | 8600 | 1009 | 2288 | 32 | 104 | 977 | 2184 | 3297 | | | 1705 |

(fortgesetzt)

| Pos. | Probe | Kopien/ 20µL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| D05 | PPCa12 | 9060 | 3807 | 8097 | 316 | 3491 | 2737 | 5360 | 11904 | 1.3 | **56.5** | 2927 |
| D05 | | 6980 | 3053 | 8851 | 316 | 3491 | 2737 | 5360 | 11904 | | | 1705 |
| E05 | PPCa13 | 0 | 0 | 13326 | 0 | 0 | 852 | 12474 | 13326 | 0 | 0 | 2927 |
| E05 | | 1560 | 852 | 12474 | 0 | 0 | 852 | 12474 | 13326 | | | 1705 |
| F05 | PPCa14 | 342 | 180 | 12273 | 21 | 159 | 2173 | 10100 | 12453 | 0.075 | 7 | 2927 |
| F05 | | 4560 | 2194 | 10259 | 21 | 159 | 2173 | 10100 | 12453 | | | 1705 |
| G05 | PPCa15 | 1364 | 733 | 12280 | 6 | 727 | 357 | 11923 | 13013 | 2.05 | **67.2** | 2927 |
| G05 | | 666 | 363 | 12650 | 6 | 727 | 357 | 11923 | 13013 | | | 1705 |
| H05 | PPCa16 | 508 | 265 | 12143 | 18 | 247 | 3706 | 8437 | 12408 | 0.06 | **5.7** | 2927 |
| H05 | | 8400 | 3724 | 8684 | 18 | 247 | 3706 | 8437 | 12408 | | | 1705 |
| A06 | PPCa17 | 8280 | 3811 | 9026 | 94 | 3717 | 853 | 8173 | 12837 | 4.6 | **82.1** | 2927 |
| A06 | | 1800 | 947 | 11890 | 94 | 3717 | 853 | 8173 | 12837 | | | 1705 |
| B06 | PPCa18 | 0 | 0 | 12031 | 0 | 0 | 2732 | 9299 | 12031 | 0 | 0 | 2927 |
| B06 | | 6060 | 2732 | 9299 | 0 | 0 | 2732 | 9299 | 12031 | | | 1705 |
| C06 | PPCa19 | 0 | 0 | 11204 | 0 | 0 | 2358 | 8846 | 11204 | 0 | 0 | 2927 |
| C06 | | 5560 | 2358 | 8846 | 0 | 0 | 2358 | 8846 | 11204 | | | 1705 |
| D06 | PPCa20 | 0 | 0 | 12688 | 0 | 0 | 1224 | 11464 | 12688 | 0 | 0 | 2927 |
| D06 | | 2380 | 1224 | 11464 | 0 | 0 | 1224 | 11464 | 12688 | | | 1705 |
| E06 | gDNA 15 | 0 | 0 | 12039 | 0 | 0 | 1 | 12038 | 12039 | 0 | 0 | 2927 |
| E06 | | 2 | 1 | 12038 | 0 | 0 | 1 | 12038 | 12039 | | | 1705 |
| G06 | NTC 15 | 0 | 0 | 13354 | 0 | 0 | 1 | 13353 | 13354 | 0 | 0 | 2927 |
| G06 | | 1.8 | 1 | 13353 | 0 | 0 | 1 | 13353 | 13354 | | | 1705 |

**Tabelle 15:** FAM-Signale (methylierte ***SERPINE1***-DNA-Fragmente) und HEX-Signale (nicht-methylierte ***SERPINE1***-DNA-Fragmente) in Serumproben gesunder Probandinnen (GF1-GF20) und Serumproben von Patientinnen mit Mammakarzinom (MF1-MF20) und Non-Template-Kontrolle (NTC, ohne DNA bzw. genomische DNA ohne Bisulfitumwandlung) nach 15 Zyklen in der BBPA mit dem *SERPINE1*-123bp-Primern (SEQ ID: 16 und 17) und 3,5 mM **$MgCl_2$-Konzentration** bei 52,0°C und anschließender dPCR. *Cut-off*-Wert: < 25,9%. Rohdaten zur Fig. 57.

| Pos. | Probe | Kopien/ 20µL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| A01 | GM1 | 20 | 12 | 14245 | 4 | 8 | 1132 | 13113 | 14257 | 0.01 | 1 | 3576 |
| A01 | | 1960 | 1136 | 13121 | 4 | 8 | 1132 | 13113 | 14257 | | | 3908 |
| B01 | GM2 | 2 | 1 | 12114 | 0 | 1 | 487 | 11627 | 12115 | 0.002 | 0.2 | 3576 |
| B01 | | 966 | 487 | 11628 | 0 | 1 | 487 | 11627 | 12115 | | | 3908 |
| C01 | GM3 | 24 | 15 | 14517 | 3 | 12 | 1088 | 13429 | 14532 | 0.013 | 1.3 | 3576 |
| C01 | | 1840 | 1091 | 13441 | 3 | 12 | 1088 | 13429 | 14532 | | | 3908 |
| D01 | GM4 | 210 | 102 | 11324 | 10 | 92 | 1724 | 9600 | 11426 | 0.054 | 5.2 | 3576 |
| D01 | | 3880 | 1734 | 9692 | 10 | 92 | 1724 | 9600 | 11426 | | | 3908 |
| E01 | GM5 | 18 | 11 | 14493 | 2 | 9 | 3160 | 11333 | 14504 | 0.0031 | 0.31 | 3576 |
| E01 | | 5780 | 3162 | 11342 | 2 | 9 | 3160 | 11333 | 14504 | | | 3908 |
| F01 | GM6 | 766 | 487 | 14709 | 36 | 451 | 1940 | 12769 | 15196 | 0.234 | 19 | 3576 |
| F01 | | 3280 | 1976 | 13220 | 36 | 451 | 1940 | 12769 | 15196 | | | 3908 |
| G01 | GM7 | 86 | 52 | 14280 | 0 | 52 | 455 | 13825 | 14332 | 0.113 | 10.1 | 3576 |
| G01 | | 760 | 455 | 13877 | 0 | 52 | 455 | 13825 | 14332 | | | 3908 |
| H01 | GM8 | 966 | 562 | 13412 | 51 | 511 | 1501 | 11911 | 13974 | 0.349 | 25.9 | 3576 |
| H01 | | 2780 | 1552 | 12422 | 51 | 511 | 1501 | 11911 | 13974 | | | 3908 |
| A02 | GM9 | 14 | 1 | 1724 | 0 | 1 | 0 | 1724 | 1725 | 0 | 0 | 3576 |
| A02 | | 0 | 0 | 1725 | 0 | 1 | 0 | 1724 | 1725 | | | 3908 |
| B02 | GM10 | 50 | 25 | 11855 | 4 | 21 | 2835 | 9020 | 11880 | 0.0077 | 0.77 | 3576 |
| B02 | | 6420 | 2839 | 9041 | 4 | 21 | 2835 | 9020 | 11880 | | | 3908 |
| C02 | GM11 | 5.2 | 3 | 13687 | 1 | 2 | 1253 | 12434 | 13690 | 0.0023 | 0.23 | 3576 |
| C02 | | 2260 | 1254 | 12436 | 1 | 2 | 1253 | 12434 | 13690 | | | 3908 |

(fortgesetzt)

| Pos. | Probe | Kopien/ 20µL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| D02 | GM12 | 552 | 305 | 12830 | 43 | 262 | 851 | 11979 | 13135 | 0.333 | 25 | 3576 |
| D02 | | 1660 | 894 | 12241 | 43 | 262 | 851 | 11979 | 13135 | | | 3908 |
| E02 | GM13 | 102 | 58 | 13391 | 4 | 54 | 2469 | 10922 | 13449 | 0.021 | 2.1 | 3576 |
| E02 | | 4780 | 2473 | 10976 | 4 | 54 | 2469 | 10922 | 13449 | | | 3908 |
| F02 | GM14 | 304 | 140 | 10770 | 3 | 137 | 718 | 10052 | 10910 | 0.189 | 15.9 | 3576 |
| F02 | | 1600 | 721 | 10189 | 3 | 137 | 718 | 10052 | 10910 | | | 3908 |
| G02 | GM15 | 10.4 | 6 | 13536 | 1 | 5 | 589 | 12947 | 13542 | 0.01 | 1 | 3576 |
| G02 | | 1048 | 590 | 12952 | 1 | 5 | 589 | 12947 | 13542 | | | 3908 |
| H02 | GM16 | 10.4 | 6 | 13557 | 2 | 4 | 1605 | 11952 | 13563 | 0.0035 | 0.35 | 3576 |
| H02 | | 2960 | 1607 | 11956 | 2 | 4 | 1605 | 11952 | 13563 | | | 3908 |
| A03 | GM17 | 3.8 | 2 | 12274 | 0 | 2 | 287 | 11987 | 12276 | 0.007 | 0.7 | 3576 |
| A03 | | 556 | 287 | 11989 | 0 | 2 | 287 | 11987 | 12276 | | | 3908 |
| B03 | GM18 | 0 | 0 | 12516 | 0 | 0 | 530 | 11986 | 12516 | 0 | 0 | 3576 |
| B03 | | 1018 | 530 | 11986 | 0 | 0 | 530 | 11986 | 12516 | | | 3908 |
| C03 | GM19 | 36 | 24 | 15274 | 4 | 20 | 1133 | 14141 | 15298 | 0.02 | 2 | 3576 |
| C03 | | 1820 | 1137 | 14161 | 4 | 20 | 1133 | 14141 | 15298 | | | 3908 |
| D03 | GM20 | 10.4 | 6 | 13578 | 2 | 4 | 3671 | 9907 | 13584 | 0.0014 | 0.14 | 3576 |
| D03 | | 7420 | 3673 | 9911 | 2 | 4 | 3671 | 9907 | 13584 | | | 3908 |
| A04 | PPCa1 | 48 | 24 | 11619 | 4 | 20 | 327 | 11292 | 11643 | 0.072 | 6.7 | 3576 |
| A04 | | 678 | 331 | 11312 | 4 | 20 | 327 | 11292 | 11643 | | | 3908 |
| B04 | PPCa2 | 8.4 | 5 | 14014 | 0 | 5 | 383 | 13631 | 14019 | 0.013 | 1.3 | 3576 |
| B04 | | 652 | 383 | 13636 | 0 | 5 | 383 | 13631 | 14019 | | | 3908 |
| C04 | PPCa3 | 6440 | 3173 | 10077 | 283 | 2890 | 1916 | 8161 | 13250 | 1.51 | **60.1** | 3576 |
| C04 | | 4280 | 2199 | 11051 | 283 | 2890 | 1916 | 8161 | 13250 | | | 3908 |

(fortgesetzt)

| Pos. | Probe | Kopien/ 20μL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| D04 | PPCa4 | 910 | 540 | 13698 | 0 | 540 | 158 | 13540 | 14238 | 3.5 | **77.6** | 3576 |
| D04 | | 262 | 158 | 14080 | 0 | 540 | 158 | 13540 | 14238 | | | 3908 |
| E04 | PPCa5 | 28 | 14 | 11907 | 0 | 14 | 257 | 11650 | 11921 | 0.054 | 5.1 | 3576 |
| E04 | | 512 | 257 | 11664 | 0 | 14 | 257 | 11650 | 11921 | | | 3908 |
| F04 | PPCa6 | 314 | 166 | 12345 | 15 | 151 | 2808 | 9537 | 12511 | 0.052 | 5 | 3576 |
| F04 | | 6020 | 2823 | 9688 | 15 | 151 | 2808 | 9537 | 12511 | | | 3908 |
| G04 | PPCa7 | 1560 | 859 | 12602 | 38 | 821 | 1295 | 11307 | 13461 | 0.63 | **38.7** | 3576 |
| G04 | | 2460 | 1333 | 12128 | 38 | 821 | 1295 | 11307 | 13461 | | | 3908 |
| H04 | PPCa8 | 2060 | 1070 | 11694 | 131 | 939 | 2340 | 9354 | 12764 | 0.407 | **28.9** | 3576 |
| H04 | | 5060 | 2471 | 10293 | 131 | 939 | 2340 | 9354 | 12764 | | | 3908 |
| A05 | PPCa9 | 44 | 25 | 13399 | 1 | 24 | 1095 | 12304 | 13424 | 0.022 | 2.1 | 3576 |
| A05 | | 2000 | 1096 | 12328 | 1 | 24 | 1095 | 12304 | 13424 | | | 3908 |
| B05 | PPCa10 | 9.8 | 5 | 12050 | 0 | 5 | 406 | 11644 | 12055 | 0.012 | 1.2 | 3576 |
| B05 | | 806 | 406 | 11649 | 0 | 5 | 406 | 11644 | 12055 | | | 3908 |
| C05 | PPCa11 | 4320 | 2523 | 12518 | 642 | 1881 | 3607 | 8911 | 15041 | 0.553 | **35.6** | 3576 |
| C05 | | 7820 | 4249 | 10792 | 642 | 1881 | 3607 | 8911 | 15041 | | | 3908 |
| D05 | PPCa12 | 758 | 509 | 15556 | 10 | 499 | 1073 | 14483 | 16065 | 0.461 | **31.6** | 3576 |
| D05 | | 1642 | 1083 | 14982 | 10 | 499 | 1073 | 14483 | 16065 | | | 3908 |
| E05 | PPCa13 | 256 | 155 | 14213 | 3 | 152 | 559 | 13654 | 14368 | 0.272 | 21.4 | 3576 |
| E05 | | 938 | 562 | 13806 | 3 | 152 | 559 | 13654 | 14368 | | | 3908 |
| F05 | PPCa14 | 624 | 394 | 14667 | 21 | 373 | 2408 | 12259 | 15061 | 0.151 | 13.1 | 3576 |
| F05 | | 4140 | 2429 | 12632 | 21 | 373 | 2408 | 12259 | 15061 | | | 3908 |
| G05 | PPCa15 | 50 | 29 | 13505 | 1 | 28 | 313 | 13192 | 13534 | 0.091 | 8.4 | 3576 |
| G05 | | 552 | 314 | 13220 | 1 | 28 | 313 | 13192 | 13534 | | | 3908 |

(fortgesetzt)

| Pos. | Probe | Kopien/ 20µL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| H05 | PPCa16 | 8.6 | 5 | 13566 | 0 | 5 | 1356 | 12210 | 13571 | 0.0035 | 0.35 | 3576 |
| H05 | | 2480 | 1356 | 12215 | 0 | 5 | 1356 | 12210 | 13571 | | | 3908 |
| A06 | PPCa17 | 68 | 39 | 13523 | 0 | 39 | 135 | 13388 | 13562 | 0.29 | 22 | 3576 |
| A06 | | 236 | 135 | 13427 | 0 | 39 | 135 | 13388 | 13562 | | | 3908 |
| B06 | PPCa18 | 0 | 0 | 12779 | 0 | 0 | 782 | 11997 | 12779 | 0 | 0 | 3576 |
| B06 | | 1480 | 782 | 11997 | 0 | 0 | 782 | 11997 | 12779 | | | 3908 |
| C06 | PPCa19 | 0 | 0 | 14452 | 0 | 0 | 350 | 14102 | 14452 | 0 | 0 | 3576 |
| C06 | | 576 | 350 | 14102 | 0 | 0 | 350 | 14102 | 14452 | | | 3908 |
| D06 | PPCa20 | 24 | 14 | 13202 | 0 | 14 | 226 | 12976 | 13216 | 0.061 | 5.8 | 3576 |
| D06 | | 406 | 226 | 12990 | 0 | 14 | 226 | 12976 | 13216 | | | 3908 |
| E03 | 15 gNTC | 3.4 | 2 | 13554 | 0 | 2 | 16 | 13538 | 13556 | 0.12 | 11 | 3576 |
| E03 | | 28 | 16 | 13540 | 0 | 2 | 16 | 13538 | 13556 | | | 3908 |
| F03 | 15 NTC | 8.8 | 6 | 16114 | 1 | 5 | 35 | 16079 | 16120 | 0.17 | 14 | 3576 |
| F03 | | 52 | 36 | 16084 | 1 | 5 | 35 | 16079 | 16120 | | | 3908 |

**Tabelle 16:** FAM-Signale (methylierte ***SERPINE1***-DNA-Fragmente) und HEX-Signale (nicht-methylierte ***SERPINE1***-DNA-Fragmente) in Serumproben gesunder Probandinnen (GF1-GF20) und Serumproben von Patientinnen mit Mammakarzinom (MF1-MF20) und Non-Template-Kontrolle (NTC, ohne DNA bzw. genomische DNA ohne Bisulfitumwandlung) nach 50 Zyklen in der BBPA mit dem *SERPINE1*-123bp-Primern (SEQ ID: 16 und 17) und 3,5 mM **$MgCl_2$-Konzentration** bei 52,0°C und anschließender dPCR. *Cut-off*-Wert: < 0,0012%, n.d.: nicht bestimmbar, da nicht-methylierte DNA-Fragmentanteil zu gering war. Rohdaten zur Fig. 58.

| Pos. | Probe | Kopien/20µL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| A07 | GM1 | 0 | 0 | 11322 | 0 | 0 | 11002 | 320 | 11322 | 0 | 0 | 3508 |
| A07 | | 84000 | 11002 | 320 | 0 | 0 | 11002 | 320 | 11322 | | | 3780 |
| B07 | GM2 | 0 | 0 | 11951 | 0 | 0 | 9517 | 2434 | 11951 | 0 | 0 | 3508 |
| B07 | | 37440 | 9517 | 2434 | 0 | 0 | 9517 | 2434 | 11951 | | | 3780 |
| C07 | GM3 | 0 | 0 | 14343 | 0 | 0 | 11625 | 2718 | 14343 | 0 | 0 | 3508 |
| C07 | | 39140 | 11625 | 2718 | 0 | 0 | 11625 | 2718 | 14343 | | | 3780 |
| D07 | GM4 | 0 | 0 | 10890 | 0 | 0 | 9128 | 1762 | 10890 | 0 | 0 | 3508 |
| D07 | | 42800 | 9128 | 1762 | 0 | 0 | 9128 | 1762 | 10890 | | | 3780 |
| E07 | GM5 | 0 | 0 | 14075 | 0 | 0 | 14057 | 18 | 14075 | 0 | 0 | 3508 |
| E07 | | 156000 | 14057 | 18 | 0 | 0 | 14057 | 18 | 14075 | | | 3780 |
| F07 | GM6 | 0 | 0 | 13893 | 0 | 0 | 13305 | 588 | 13893 | 0 | 0 | 3508 |
| F07 | | 74400 | 13305 | 588 | 0 | 0 | 13305 | 588 | 13893 | | | 3780 |
| G07 | GM7 | 0 | 0 | 16335 | 0 | 0 | 13 | 16322 | 16335 | | n.d. | 3508 |
| G07 | | 18 | 13 | 16322 | 0 | 0 | 13 | 16322 | 16335 | | | 3780 |
| H07 | GM8 | 0 | 0 | 12775 | 0 | 0 | 10756 | 2019 | 12775 | 0 | 0 | 3508 |
| H07 | | 43400 | 10756 | 2019 | 0 | 0 | 10756 | 2019 | 12775 | | | 3780 |
| A08 | GM9 | 0 | 0 | 13185 | 0 | 0 | 92 | 13093 | 13185 | | n.d. | 3508 |
| A08 | | 164 | 92 | 13093 | 0 | 0 | 92 | 13093 | 13185 | | | 3780 |
| B08 | GM10 | 0 | 0 | 14823 | 0 | 0 | 14755 | 68 | 14823 | 0 | 0 | 3508 |
| B08 | | 126600 | 14755 | 68 | 0 | 0 | 14755 | 68 | 14823 | | | 3780 |
| C08 | GM11 | 1.6 | 1 | 14258 | 1 | 0 | 14206 | 52 | 14259 | 1.2E-05 | 0.0012 | 3508 |
| C08 | | 132000 | 14207 | 52 | 1 | 0 | 14206 | 52 | 14259 | | | 3780 |

(fortgesetzt)

| Pos. | Probe | Kopien/20μL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| D08 | GM12 | 1.6 | 1 | 14402 | 1 | 0 | 110 | 14292 | 14403 | | n.d. | 3508 |
| D08 | | 182 | 111 | 14292 | 1 | 0 | 110 | 14292 | 14403 | | | 3780 |
| E08 | GM13 | 0 | 0 | 15072 | 0 | 0 | 15067 | 5 | 15072 | 0 | 0 | 3508 |
| E08 | | 188000 | 15067 | 5 | 0 | 0 | 15067 | 5 | 15072 | | | 3780 |
| F08 | GM14 | 0 | 0 | 12834 | 0 | 0 | 12596 | 238 | 12834 | 0 | 0 | 3508 |
| F08 | | 93800 | 12596 | 238 | 0 | 0 | 12596 | 238 | 12834 | | | 3780 |
| G08 | GM15 | 0 | 0 | 15407 | 0 | 0 | 14642 | 765 | 15407 | 0 | 0 | 3508 |
| G08 | | 70600 | 14642 | 765 | 0 | 0 | 14642 | 765 | 15407 | | | 3780 |
| H08 | GM16 | 0 | 0 | 14278 | 0 | 0 | 12857 | 1421 | 14278 | 0 | 0 | 3508 |
| H08 | | 54200 | 12857 | 1421 | 0 | 0 | 12857 | 1421 | 14278 | | | 3780 |
| A09 | GM17 | 0 | 0 | 12396 | 0 | 0 | 1546 | 10850 | 12396 | 0 | 0 | 3508 |
| A09 | | 3140 | 1546 | 10850 | 0 | 0 | 1546 | 10850 | 12396 | | | 3780 |
| B09 | GM18 | 0 | 0 | 16490 | 0 | 0 | 9336 | 7154 | 16490 | 0 | 0 | 3508 |
| B09 | | 19640 | 9336 | 7154 | 0 | 0 | 9336 | 7154 | 16490 | | | 3780 |
| C09 | GM19 | 0 | 0 | 16559 | 0 | 0 | 12271 | 4288 | 16559 | 0 | 0 | 3508 |
| C09 | | 31800 | 12271 | 4288 | 0 | 0 | 12271 | 4288 | 16559 | | | 3780 |
| D09 | GM20 | 0 | 0 | 15035 | 0 | 0 | 12902 | 2133 | 15035 | 0 | 0 | 3508 |
| D09 | | 45940 | 12902 | 2133 | 0 | 0 | 12902 | 2133 | 15035 | | | 3780 |
| A10 | PPCa1 | 0 | 0 | 13235 | 0 | 0 | 9609 | 3626 | 13235 | 0 | 0 | 3508 |
| A10 | | 30460 | 9609 | 3626 | 0 | 0 | 9609 | 3626 | 13235 | | | 3780 |
| B10 | PPCa2 | 1.6 | 1 | 13988 | 1 | 0 | 13495 | 493 | 13989 | 2.1 E-05 | **0.0021** | 3508 |
| B10 | | 78800 | 13496 | 493 | 1 | 0 | 13495 | 493 | 13989 | | | 3780 |
| C10 | PPCa3 | 20000000 | 14683 | 0 | 4147 | 10536 | 0 | 0 | 14683 | 2600 | **99.961** | 3508 |
| C10 | | 7800 | 4147 | 10536 | 4147 | 10536 | 0 | 0 | 14683 | | | 3780 |

(fortgesetzt)

| Pos. | Probe | Kopien/20μL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| D10 | PPCa4 | 20000000 | 15104 | 0 | 19 | 15085 | 0 | 0 | 15104 | 680000 | **100** | 3508 |
| D10 | | 30 | 19 | 15085 | 19 | 15085 | 0 | 0 | 15104 | | | 3780 |
| E10 | PPCa5 | 30 | 18 | 14361 | 18 | 0 | 14335 | 26 | 14379 | 0.0002 | **0.02** | 3508 |
| E10 | | 148600 | 14353 | 26 | 18 | 0 | 14335 | 26 | 14379 | | | 3780 |
| F10 | PPCa6 | 1.6 | 1 | 14999 | 1 | 0 | 14951 | 48 | 15000 | 1.2E-05 | 0.0012 | 3508 |
| F10 | | 135200 | 14952 | 48 | 1 | 0 | 14951 | 48 | 15000 | | | 3780 |
| G10 | PPCa7 | 135400 | 14833 | 47 | 11752 | 3081 | 1 | 46 | 14880 | 3.69 | **78.7** | 3508 |
| G10 | | 36700 | 11753 | 3127 | 11752 | 3081 | 1 | 46 | 14880 | | | 3780 |
| H10 | PPCa8 | 56980 | 12597 | 1227 | 12556 | 41 | 1115 | 112 | 13824 | 0.538 | **35** | 3508 |
| H10 | | 106000 | 13671 | 153 | 12556 | 41 | 1115 | 112 | 13824 | | | 3780 |
| A11 | PPCa9 | 0 | 0 | 16071 | 0 | 0 | 15879 | 192 | 16071 | 0 | 0 | 3508 |
| A11 | | 104200 | 15879 | 192 | 0 | 0 | 15879 | 192 | 16071 | | | 3780 |
| B11 | PPCa10 | 0 | 0 | 15132 | 0 | 0 | 14281 | 851 | 15132 | 0 | 0 | 3508 |
| B11 | | 67800 | 14281 | 851 | 0 | 0 | 14281 | 851 | 15132 | | | 3780 |
| C11 | PPCa11 | 766 | 376 | 11348 | 376 | 0 | 10945 | 403 | 11724 | 0.0097 | **0.96** | 3508 |
| C11 | | 79400 | 11321 | 403 | 376 | 0 | 10945 | 403 | 11724 | | | 3780 |
| D11 | PPCa12 | 384 | 219 | 13304 | 63 | 156 | 918 | 12386 | 13523 | 0.217 | **17.8** | 3508 |
| D11 | | 1780 | 981 | 12542 | 63 | 156 | 918 | 12386 | 13523 | | | 3780 |
| E11 | PPCa13 | 70 | 42 | 13912 | 41 | 1 | 12039 | 1873 | 13954 | 0.0015 | **0.15** | 3508 |
| E11 | | 47240 | 12080 | 1874 | 41 | 1 | 12039 | 1873 | 13954 | | | 3780 |
| F11 | PPCa14 | 3.4 | 2 | 14113 | 2 | 0 | 14049 | 64 | 14115 | 2.6E-05 | **0.0026** | 3508 |
| F11 | | 127000 | 14051 | 64 | 2 | 0 | 14049 | 64 | 14115 | | | 3780 |
| G11 | PPCa15 | 0 | 0 | 15448 | 0 | 0 | 15138 | 310 | 15448 | 0 | 0 | 3508 |
| G11 | | 92000 | 15138 | 310 | 0 | 0 | 15138 | 310 | 15448 | | | 3780 |

(fortgesetzt)

| Pos. | Probe | Kopien/20µL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| H11 | PPCa16 | 0 | 0 | 13649 | 0 | 0 | 13583 | 66 | 13649 | 0 | 0 | 3508 |
| H11 | | 125400 | 13583 | 66 | 0 | 0 | 13583 | 66 | 13649 | | | 3780 |
| E09 | PPCa17 | 18.8 | 13 | 16318 | 8 | 5 | 1868 | 14450 | 16331 | 0.0065 | **0.65** | 3508 |
| E09 | | 2880 | 1876 | 14455 | 8 | 5 | 1868 | 14450 | 16331 | | | 3780 |
| F09 | PPCa18 | 0 | 0 | 16149 | 0 | 0 | 15886 | 263 | 16149 | 0 | 0 | 3508 |
| F09 | | 96800 | 15886 | 263 | 0 | 0 | 15886 | 263 | 16149 | | | 3780 |
| G09 | PPCa19 | 0 | 0 | 14312 | 0 | 0 | 10027 | 4285 | 14312 | 0 | 0 | 3508 |
| G09 | | 28380 | 10027 | 4285 | 0 | 0 | 10027 | 4285 | 14312 | | | 3780 |
| H09 | PPCa20 | 0 | 0 | 15428 | 0 | 0 | 14086 | 1342 | 15428 | 0 | 0 | 3508 |
| H09 | | 57400 | 14086 | 1342 | 0 | 0 | 14086 | 1342 | 15428 | | | 3780 |
| G06 | 50 gDNA | 0 | 0 | 14576 | 0 | 0 | 3 | 14573 | 14576 | 0 | 0 | 3508 |
| G06 | | 4.8 | 3 | 14573 | 0 | 0 | 3 | 14573 | 14576 | | | 3780 |
| H06 | 50 NTC | 0 | 0 | 14603 | 0 | 0 | 3 | 14600 | 14603 | 0 | 0 | 3508 |
| H06 | | 4.8 | 3 | 14600 | 0 | 0 | 3 | 14600 | 14603 | | | 3780 |

**Tabelle 17:** FAM-Signale (methylierte ***AOX1***-DNA-Fragmente) und HEX-Signale (nicht-methylierte *AOX1*-DNA-Fragmente) in Serumproben gesunder Probandinnen (GF1-GF20) und Serumproben von Patientinnen mit Mammakarzinom (MF1-MF20) und Non-Template-Kontrolle (NTC, ohne DNA bzw. genomische DNA ohne Bisulfitumwandlung) nach 15 Zyklen in der BBPA mit dem AOX1-138bp-Primern (SEQ ID: 18 und 19) und 2,5 mM $MgCl_2$-Konzentration bei 50,0°C und anschließender dPCR. *Cut-off*-Wert: < 3,2%. Rohdaten zur Fig. 59.

| Pos. | Probe | Kopien/ 20μL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| A01 | GM1 | 0 | 0 | 12775 | 0 | 0 | 6165 | 6610 | 12775 | 0 | 0 | 2946 |
| A01 | | 15500 | 6165 | 6610 | 0 | 0 | 6165 | 6610 | 12775 | | | 4102 |
| B01 | GM2 | 0 | 0 | 12119 | 0 | 0 | 2097 | 10022 | 12119 | 0 | 0 | 2946 |
| B01 | | 4480 | 2097 | 10022 | 0 | 0 | 2097 | 10022 | 12119 | | | 4102 |
| C01 | GM3 | 0 | 0 | 12749 | 0 | 0 | 4875 | 7874 | 12749 | 0 | 0 | 2946 |
| C01 | | 11340 | 4875 | 7874 | 0 | 0 | 4875 | 7874 | 12749 | | | 4102 |
| D01 | GM4 | 0 | 0 | 13129 | 0 | 0 | 10076 | 3053 | 13129 | 0 | 0 | 2946 |
| D01 | | 34320 | 10076 | 3053 | 0 | 0 | 10076 | 3053 | 13129 | | | 4102 |
| E01 | GM5 | 0 | 0 | 13716 | 0 | 0 | 10633 | 3083 | 13716 | 0 | 0 | 2946 |
| E01 | | 35120 | 10633 | 3083 | 0 | 0 | 10633 | 3083 | 13716 | | | 4102 |
| F01 | GM6 | 0 | 0 | 14337 | 0 | 0 | 8638 | 5699 | 14337 | 0 | 0 | 2946 |
| F01 | | 21700 | 8638 | 5699 | 0 | 0 | 8638 | 5699 | 14337 | | | 4102 |
| G01 | GM7 | 1.6 | 1 | 15201 | 1 | 0 | 4846 | 10355 | 15202 | 0.00017 | 0.017 | 2946 |
| G01 | | 9040 | 4847 | 10355 | 1 | 0 | 4846 | 10355 | 15202 | | | 4102 |
| H01 | GM8 | 0 | 0 | 13459 | 0 | 0 | 4734 | 8725 | 13459 | 0 | 0 | 2946 |
| H01 | | 10200 | 4734 | 8725 | 0 | 0 | 4734 | 8725 | 13459 | | | 4102 |
| A02 | GM9 | 84 | 36 | 10028 | 12 | 24 | 6311 | 3717 | 10064 | 0.0036 | 0.36 | 2946 |
| A02 | | 23280 | 6323 | 3741 | 12 | 24 | 6311 | 3717 | 10064 | | | 4102 |
| B02 | GM10 | 0 | 0 | 10929 | 0 | 0 | 9009 | 1920 | 10929 | 0 | 0 | 2946 |
| B02 | | 40920 | 9009 | 1920 | 0 | 0 | 9009 | 1920 | 10929 | | | 4102 |
| C02 | GM11 | 1.6 | 1 | 14000 | 0 | 1 | 6620 | 7380 | 14001 | 0.00011 | 0.011 | 2946 |
| C02 | | 15060 | 6620 | 7381 | 0 | 1 | 6620 | 7380 | 14001 | | | 4102 |

(fortgesetzt)

| Pos. | Probe | Kopien/ 20μL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| D02 | GM12 | 282 | 136 | 11314 | 24 | 112 | 3455 | 7859 | 11450 | 0.033 | 3.2 | 2946 |
| D02 | | 8520 | 3479 | 7971 | 24 | 112 | 3455 | 7859 | 11450 | | | 4102 |
| E02 | GM13 | 0 | 0 | 12943 | 0 | 0 | 8381 | 4562 | 12943 | 0 | 0 | 2946 |
| E02 | | 24540 | 8381 | 4562 | 0 | 0 | 8381 | 4562 | 12943 | | | 4102 |
| F02 | GM14 | 8 | 4 | 11691 | 0 | 4 | 3261 | 8430 | 11695 | 0.001 | 0.1 | 2946 |
| F02 | | 7700 | 3261 | 8434 | 0 | 4 | 3261 | 8430 | 11695 | | | 4102 |
| G02 | GM15 | 0 | 0 | 12712 | 0 | 0 | 3386 | 9326 | 12712 | 0 | 0 | 2946 |
| G02 | | 7280 | 3386 | 9326 | 0 | 0 | 3386 | 9326 | 12712 | | | 4102 |
| H02 | GM16 | 274 | 123 | 10496 | 26 | 97 | 4435 | 6061 | 10619 | 0.0214 | 2.09 | 2946 |
| H02 | | 12820 | 4461 | 6158 | 26 | 97 | 4435 | 6061 | 10619 | | | 4102 |
| A03 | GM17 | 0 | 0 | 12345 | 0 | 0 | 2934 | 9411 | 12345 | 0 | 0 | 2946 |
| A03 | | 6380 | 2934 | 9411 | 0 | 0 | 2934 | 9411 | 12345 | | | 4102 |
| B03 | GM18 | 0 | 0 | 12222 | 0 | 0 | 3388 | 8834 | 12222 | 0 | 0 | 2946 |
| B03 | | 7640 | 3388 | 8834 | 0 | 0 | 3388 | 8834 | 12222 | | | 4102 |
| C03 | GM19 | 0 | 0 | 11851 | 0 | 0 | 5087 | 6764 | 11851 | 0 | 0 | 2946 |
| C03 | | 13200 | 5087 | 6764 | 0 | 0 | 5087 | 6764 | 11851 | | | 4102 |
| D03 | GM20 | 0 | 0 | 10021 | 0 | 0 | 97 | 9924 | 10021 | 0 | 0 | 2946 |
| D03 | | 228 | 97 | 9924 | 0 | 0 | 97 | 9924 | 10021 | | | 4102 |
| A04 | PPCa1 | 0 | 0 | 11521 | 0 | 0 | 921 | 10600 | 11521 | 0 | 0 | 2946 |
| A04 | | 1960 | 921 | 10600 | 0 | 0 | 921 | 10600 | 11521 | | | 4102 |
| B04 | PPCa2 | 34 | 18 | 12312 | 3 | 15 | 2476 | 9836 | 12330 | 0.0065 | 0.65 | 2946 |
| B04 | | 5280 | 2479 | 9851 | 3 | 15 | 2476 | 9836 | 12330 | | | 4102 |
| C04 | PPCa3 | 50080 | 10976 | 1483 | 114 | 10862 | 216 | 1267 | 12459 | 79 | **98.75** | 2946 |
| C04 | | 632 | 330 | 12129 | 114 | 10862 | 216 | 1267 | 12459 | | | 4102 |

(fortgesetzt)

| Pos. | Probe | Kopien/ 20μL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| D04 | PPCa4 | 670 | 341 | 11814 | 1 | 340 | 487 | 11327 | 12155 | 0.69 | **41** | 2946 |
| D04 | | 964 | 488 | 11667 | 1 | 340 | 487 | 11327 | 12155 | | | 4102 |
| E04 | PPCa5 | 0 | 0 | 11024 | 0 | 0 | 445 | 10579 | 11024 | 0 | 0 | 2946 |
| E04 | | 970 | 445 | 10579 | 0 | 0 | 445 | 10579 | 11024 | | | 4102 |
| F04 | PPCa6 | 188 | 96 | 11922 | 26 | 70 | 9738 | 2184 | 12018 | 0.0048 | 0.48 | 2946 |
| F04 | | 39380 | 9764 | 2254 | 26 | 70 | 9738 | 2184 | 12018 | | | 4102 |
| G04 | PPCa7 | 3760 | 1605 | 9243 | 38 | 1567 | 2960 | 6283 | 10848 | 0.495 | **33.1** | 2946 |
| G04 | | 7620 | 2998 | 7850 | 38 | 1567 | 2960 | 6283 | 10848 | | | 4102 |
| H04 | PPCa8 | 27180 | 8330 | 3833 | 640 | 7690 | 1347 | 2486 | 12163 | 6.47 | **86.6** | 2946 |
| H04 | | 4200 | 1987 | 10176 | 640 | 7690 | 1347 | 2486 | 12163 | | | 4102 |
| A05 | PPCa9 | 0 | 0 | 12275 | 0 | 0 | 4339 | 7936 | 12275 | 0 | 0 | 2946 |
| A05 | | 10260 | 4339 | 7936 | 0 | 0 | 4339 | 7936 | 12275 | | | 4102 |
| B05 | PPCa10 | 0 | 0 | 12250 | 0 | 0 | 3684 | 8566 | 12250 | 0 | 0 | 2946 |
| B05 | | 8420 | 3684 | 8566 | 0 | 0 | 3684 | 8566 | 12250 | | | 4102 |
| C05 | PPCa1 1 | 60800 | 13449 | 1094 | 894 | 12555 | 172 | 922 | 14543 | 34 | **97.14** | 2946 |
| C05 | | 1800 | 1066 | 13477 | 894 | 12555 | 172 | 922 | 14543 | | | 4102 |
| D05 | PPCa12 | 1750 | 1018 | 13185 | 41 | 977 | 2474 | 10711 | 14203 | 0.382 | **27.6** | 2946 |
| D05 | | 4580 | 2515 | 11688 | 41 | 977 | 2474 | 10711 | 14203 | | | 4102 |
| E05 | PPCa13 | 0 | 0 | 11674 | 0 | 0 | 710 | 10964 | 11674 | 0 | 0 | 2946 |
| E05 | | 1480 | 710 | 10964 | 0 | 0 | 710 | 10964 | 11674 | | | 4102 |
| F05 | PPCa14 | 714 | 403 | 13078 | 74 | 329 | 5960 | 7118 | 13481 | 0.051 | **4.87** | 2946 |
| F05 | | 13960 | 6034 | 7447 | 74 | 329 | 5960 | 7118 | 13481 | | | 4102 |
| G05 | PPCa15 | 0 | 0 | 13606 | 0 | 0 | 382 | 13224 | 13606 | 0 | 0 | 2946 |
| G05 | | 670 | 382 | 13224 | 0 | 0 | 382 | 13224 | 13606 | | | 4102 |

(fortgesetzt)

| Pos. | Probe | Kopien/ 20μL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| H05 | PPCa16 | 0 | 0 | 13111 | 0 | 0 | 4457 | 8654 | 13111 | 0 | 0 | 2946 |
| H05 | | 9780 | 4457 | 8654 | 0 | 0 | 4457 | 8654 | 13111 | | | 4102 |
| A06 | PPCa17 | 2440 | 1261 | 11550 | 34 | 1227 | 1063 | 10487 | 12811 | 1.16 | **53.7** | 2946 |
| A06 | | 2100 | 1097 | 11714 | 34 | 1227 | 1063 | 10487 | 12811 | | | 4102 |
| B06 | PPCa18 | 0 | 0 | 12831 | 0 | 0 | 3704 | 9127 | 12831 | 0 | 0 | 2946 |
| B06 | | 8020 | 3704 | 9127 | 0 | 0 | 3704 | 9127 | 12831 | | | 4102 |
| C06 | PPCa19 | 1.6 | 1 | 14781 | 0 | 1 | 4803 | 9978 | 14782 | 0.00017 | 0.017 | 2946 |
| C06 | | 9240 | 4803 | 9979 | 0 | 1 | 4803 | 9978 | 14782 | | | 4102 |
| D06 | PPCa20 | 0 | 0 | 14244 | 0 | 0 | 1678 | 12566 | 14244 | 0 | 0 | 2946 |
| D06 | | 2940 | 1678 | 12566 | 0 | 0 | 1678 | 12566 | 14244 | | | 4102 |
| E03 | 15 gDNA | 0 | 0 | 13787 | 0 | 0 | 63 | 13724 | 13787 | 0 | 0 | 2946 |
| E03 | | 108 | 63 | 13724 | 0 | 0 | 63 | 13724 | 13787 | | | 4102 |
| F03 | 15 NTC | 0 | 0 | 14654 | 0 | 0 | 84 | 14570 | 14654 | 0 | 0 | 2946 |
| F03 | | 136 | 84 | 14570 | 0 | 0 | 84 | 14570 | 14654 | | | 4102 |

**Tabelle 18:** FAM-Signale (methylierte ***AOX1**-DN*A-Fragmente) und HEX-Signale (nicht-methylierte ***AOX1***-DNA-Fragmente) in Serumproben gesunder Probandinnen (GF1-GF20) und Serumproben von Patientinnen mit Mammakarzinom (MF1-MF20) und Non-Template-Kontrolle (NTC, ohne DNA bzw. genomische DNA ohne Bisulfitumwandlung) nach 30 Zyklen in der BBPA mit dem AOX1-138bp-Primern (SEQ ID: 18 und 19) und 2,5 mM $MgCl_2$-Konzentration bei 50,0°C und anschließender dPCR. *Cut-off*-Wert: < 0,008%. Rohdaten zur Fig. 60.

| Pos. | Probe | Kopien/20µL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| A07 | GM1 | 0 | 0 | 13363 | 0 | 0 | 12829 | 534 | 13363 | 0 | 0 | 2522 |
| A07 | | 75800 | 12829 | 534 | 0 | 0 | 12829 | 534 | 13363 | | | 4026 |
| B07 | GM2 | 1.8 | 1 | 13210 | 0 | 1 | 7853 | 5357 | 13211 | 8,00E-05 | 0.008 | 2522 |
| B07 | | 21240 | 7853 | 5358 | 0 | 1 | 7853 | 5357 | 13211 | | | 4026 |
| C07 | GM3 | 0 | 0 | 13321 | 0 | 0 | 11436 | 1885 | 13321 | 0 | 0 | 2522 |
| C07 | | 46000 | 11436 | 1885 | 0 | 0 | 11436 | 1885 | 13321 | | | 4026 |
| D07 | GM4 | 0 | 0 | 13023 | 0 | 0 | 12677 | 346 | 13023 | 0 | 0 | 2522 |
| D07 | | 85400 | 12677 | 346 | 0 | 0 | 12677 | 346 | 13023 | | | 4026 |
| E07 | GM5 | 0 | 0 | 13143 | 0 | 0 | 12216 | 927 | 13143 | 0 | 0 | 2522 |
| E07 | | 62400 | 12216 | 927 | 0 | 0 | 12216 | 927 | 13143 | | | 4026 |
| F07 | GM6 | 0 | 0 | 12889 | 0 | 0 | 7603 | 5286 | 12889 | 0 | 0 | 2522 |
| F07 | | 20980 | 7603 | 5286 | 0 | 0 | 7603 | 5286 | 12889 | | | 4026 |
| G07 | GM7 | 0 | 0 | 12650 | 0 | 0 | 12048 | 602 | 12650 | 0 | 0 | 2522 |
| G07 | | 71600 | 12048 | 602 | 0 | 0 | 12048 | 602 | 12650 | | | 4026 |
| H07 | GM8 | 0 | 0 | 12586 | 0 | 0 | 12099 | 487 | 12586 | 0 | 0 | 2522 |
| H07 | | 76600 | 12099 | 487 | 0 | 0 | 12099 | 487 | 12586 | | | 4026 |
| A08 | GM9 | 0 | 0 | 12154 | 0 | 0 | 11019 | 1135 | 12154 | 0 | 0 | 2522 |
| A08 | | 55800 | 11019 | 1135 | 0 | 0 | 11019 | 1135 | 12154 | | | 4026 |
| B08 | GM10 | 0 | 0 | 13926 | 0 | 0 | 13704 | 222 | 13926 | 0 | 0 | 2522 |
| B08 | | 97400 | 13704 | 222 | 0 | 0 | 13704 | 222 | 13926 | | | 4026 |
| C08 | GM11 | 0 | 0 | 13333 | 0 | 0 | 8498 | 4835 | 13333 | 0 | 0 | 2522 |
| C08 | | 23860 | 8498 | 4835 | 0 | 0 | 8498 | 4835 | 13333 | | | 4026 |

(fortgesetzt)

| Pos. | Probe | Kopien/20μL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| D08 | GM12 | 1.6 | 1 | 14130 | 1 | 0 | 13577 | 553 | 14131 | 2.2E-05 | 0.0022 | 2522 |
| D08 | | 76200 | 13578 | 553 | 1 | 0 | 13577 | 553 | 14131 | | | 4026 |
| E08 | GM13 | 0 | 0 | 12767 | 0 | 0 | 12491 | 276 | 12767 | 0 | 0 | 2522 |
| E08 | | 90200 | 12491 | 276 | 0 | 0 | 12491 | 276 | 12767 | | | 4026 |
| F08 | GM14 | 0 | 0 | 12841 | 0 | 0 | 5483 | 7358 | 12841 | 0 | 0 | 2522 |
| F08 | | 13100 | 5483 | 7358 | 0 | 0 | 5483 | 7358 | 12841 | | | 4026 |
| G08 | GM15 | 0 | 0 | 13297 | 0 | 0 | 12877 | 420 | 13297 | 0 | 0 | 2522 |
| G08 | | 81200 | 12877 | 420 | 0 | 0 | 12877 | 420 | 13297 | | | 4026 |
| H08 | GM16 | 1.6 | 1 | 13873 | 0 | 1 | 13246 | 627 | 13874 | 2.3E-05 | 0.0023 | 2522 |
| H08 | | 72800 | 13246 | 628 | 0 | 1 | 13246 | 627 | 13874 | | | 4026 |
| A09 | GM17 | 0 | 0 | 13562 | 0 | 0 | 831 | 12731 | 13562 | 0 | 0 | 2522 |
| A09 | | 1480 | 831 | 12731 | 0 | 0 | 831 | 12731 | 13562 | | | 4026 |
| B09 | GM18 | 0 | 0 | 14121 | 0 | 0 | 13408 | 713 | 14121 | 0 | 0 | 2522 |
| B09 | | 70200 | 13408 | 713 | 0 | 0 | 13408 | 713 | 14121 | | | 4026 |
| C09 | GM19 | 0 | 0 | 14979 | 0 | 0 | 14395 | 584 | 14979 | 0 | 0 | 2522 |
| C09 | | 76400 | 14395 | 584 | 0 | 0 | 14395 | 584 | 14979 | | | 4026 |
| D09 | GM20 | 0 | 0 | 13243 | 0 | 0 | 18 | 13225 | 13243 | 0 | 0 | 2522 |
| D09 | | 32 | 18 | 13225 | 0 | 0 | 18 | 13225 | 13243 | | | 4026 |
| E09 | PPCa1 | 0 | 0 | 13470 | 0 | 0 | 4 | 13466 | 13470 | 0 | 0 | 2522 |
| E09 | | 7 | 4 | 13466 | 0 | 0 | 4 | 13466 | 13470 | | | 4026 |
| F09 | PPCa2 | 1.6 | 1 | 14957 | 0 | 1 | 10789 | 4168 | 14958 | 5,00E-05 | 0.005 | 2522 |
| F09 | | 30060 | 10789 | 4169 | 0 | 1 | 10789 | 4168 | 14958 | | | 4026 |
| G09 | PPCa3 | 20000000 | 14287 | 0 | 2 | 14285 | 0 | 0 | 14287 | 1000000 | **100** | 2522 |
| G09 | | 3.2 | 2 | 14285 | 2 | 14285 | 0 | 0 | 14287 | | | 4026 |

(fortgesetzt)

| Pos. | Probe | Kopien/20μL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| H09 | PPCa4 | 74200 | 13824 | 619 | 4240 | 9584 | 0 | 619 | 14443 | 9.06 | **90.06** | 2522 |
| H09 | | 8180 | 4240 | 10203 | 4240 | 9584 | 0 | 619 | 14443 | | | 4026 |
| E10 | PPCa5 | 0 | 0 | 13312 | 0 | 0 | 5568 | 7744 | 13312 | 0 | 0 | 2522 |
| E10 | | 12740 | 5568 | 7744 | 0 | 0 | 5568 | 7744 | 13312 | | | 4026 |
| F10 | PPCa6 | 1.6 | 1 | 15410 | 0 | 1 | 15331 | 79 | 15411 | 1.2E-05 | 0.0012 | 2522 |
| F10 | | 123800 | 15331 | 80 | 0 | 1 | 15331 | 79 | 15411 | | | 4026 |
| G10 | PPCa7 | 79200 | 14676 | 523 | 13689 | 987 | 124 | 399 | 15199 | 1.407 | **58.5** | 2522 |
| G10 | | 56400 | 13813 | 1386 | 13689 | 987 | 124 | 399 | 15199 | | | 4026 |
| H10 | PPCa8 | 145600 | 14096 | 29 | 183 | 13913 | 0 | 29 | 14125 | 470 | **99.79** | 2522 |
| H10 | | 306 | 183 | 13942 | 183 | 13913 | 0 | 29 | 14125 | | | 4026 |
| A11 | PPCa9 | 0 | 0 | 14180 | 0 | 0 | 12203 | 1977 | 14180 | 0 | 0 | 2522 |
| A11 | | 46360 | 12203 | 1977 | 0 | 0 | 12203 | 1977 | 14180 | | | 4026 |
| B11 | PPCa10 | 0 | 0 | 15288 | 0 | 0 | 13636 | 1652 | 15288 | 0 | 0 | 2522 |
| B11 | | 52400 | 13636 | 1652 | 0 | 0 | 13636 | 1652 | 15288 | | | 4026 |
| C11 | PPCa11 | 228000 | 15823 | 1 | 24 | 15799 | 0 | 1 | 15824 | 6400 | **99.984** | 2522 |
| C11 | | 36 | 24 | 15800 | 24 | 15799 | 0 | 1 | 15824 | | | 4026 |
| D11 | PPCa12 | 62280 | 15829 | 1207 | 11567 | 4262 | 95 | 1112 | 17036 | 2.29 | **69.6** | 2522 |
| D11 | | 27140 | 11662 | 5374 | 11567 | 4262 | 95 | 1112 | 17036 | | | 4026 |
| E11 | PPCa13 | 1.4 | 1 | 16189 | 0 | 1 | 1474 | 14715 | 16190 | 0.0006 | **0.06** | 2522 |
| E11 | | 2240 | 1474 | 14716 | 0 | 1 | 1474 | 14715 | 16190 | | | 4026 |
| F11 | PPCa14 | 0 | 0 | 16641 | 0 | 0 | 16278 | 363 | 16641 | 0 | 0 | 2522 |
| F11 | | 90000 | 16278 | 363 | 0 | 0 | 16278 | 363 | 16641 | | | 4026 |
| G11 | PPCa15 | 0 | 0 | 16513 | 0 | 0 | 12586 | 3927 | 16513 | 0 | 0 | 2522 |
| G11 | | 33800 | 12586 | 3927 | 0 | 0 | 12586 | 3927 | 16513 | | | 4026 |

(fortgesetzt)

| Pos. | Probe | Kopien/20µL | Positiv | Negativ | Ch1+ Ch2+ | Ch1+ Ch2- | Ch1- Ch2+ | Ch1- Ch2- | Tröpfchenanzahl | Ratio | Frakt. Häufigkeit | Grenzwert |
|---|---|---|---|---|---|---|---|---|---|---|---|---|
| H11 | PPCa16 | 0 | 0 | 14607 | 0 | 0 | 8223 | 6384 | 14607 | 0 | 0 | 2522 |
| H11 | | 19480 | 8223 | 6384 | 0 | 0 | 8223 | 6384 | 14607 | | | 4026 |
| A10 | PPCa17 | 11640 | 5373 | 8388 | 6 | 5367 | 1 | 8387 | 13761 | 1000 | **99.9** | 2522 |
| A10 | | 12 | 7 | 13754 | 6 | 5367 | 1 | 8387 | 13761 | | | 4026 |
| B10 | PPCa18 | 0 | 0 | 14246 | 0 | 0 | 11148 | 3098 | 14246 | 0 | 0 | 2522 |
| B10 | | 35900 | 11148 | 3098 | 0 | 0 | 11148 | 3098 | 14246 | | | 4026 |
| C10 | PPCa19 | 0 | 0 | 14141 | 0 | 0 | 13102 | 1039 | 14141 | 0 | 0 | 2522 |
| C10 | | 61400 | 13102 | 1039 | 0 | 0 | 13102 | 1039 | 14141 | | | 4026 |
| D10 | PPCa20 | 0 | 0 | 15013 | 0 | 0 | 13431 | 1582 | 15013 | 0 | 0 | 2522 |
| D10 | | 53000 | 13431 | 1582 | 0 | 0 | 13431 | 1582 | 15013 | | | 4026 |
| G06 | 30 gDNA | 0 | 0 | 15273 | 0 | 0 | 5 | 15268 | 15273 | 0 | 0 | 2522 |
| G06 | | 7.8 | 5 | 15268 | 0 | 0 | 5 | 15268 | 15273 | | | 4026 |
| H06 | 30 NTC | 0 | 0 | 14501 | 0 | 0 | 2 | 14499 | 14501 | 0 | 0 | 2522 |
| H06 | | 3.2 | 2 | 14499 | 0 | 0 | 2 | 14499 | 14501 | | | 4026 |

**Ausführungsbeispiel 11: Unterscheidung von benigner Prostatahyperplasie und Prostata-Karzinom**

Da für ein Screening und damit verbunden für eine Vorsorge von Tumorerkrankungen die sichere Unterscheidung einer benignen (nicht-bösartigen) von einer malignen Erkrankung Grundvoraussetzung ist, d.h. die analytische und diagnostische Spezifität muss nahezu 100% betragen, wurden unter Einsatz verschiedener PLA2R1-Primerpaare neben normalen PrEC-Zellen und malignen Prostatazelllinien (LNCaP, PC-3 und DU-145) die benigne Prostatahyperplasiezelllinie BPH-1 analysiert. Dabei zeigte sich nach 50 Zyklen Prä-Amplifikation bei Verwendung einer $MgCl_2$-Konzentration von 2,5 mM und dem Einsatz des PLA2R1-Primerpaares 168 bp, dass die Höhe der FAM- und HEX-Signale in Abhängigkeit der Annealingtemperatur konstant blieben, d.h. keine Bias auftrat (Fig. 46). Das entspricht den Ergebnissen, die bei der Untersuchung von 50%-iger Standard-DNA-Proben erhalten wurden (Fig. 34). Im Gegensatz dazu trat bei Verwendung des PLA2R1-Primerpaares 161 bp ab einer Temperatur von 58,2°C und bei Einsatz des PLA2R1-Primerpaares 150 bp ab einer Temperatur von 50,8°C eine deutliche Bias zugunsten methylierter Sequenzen ein, die durch steigende FAM-Signale für die PC-3-Zelllinie ersichtlich wurde (Fig. 47 und 48). Eine besonders starke Bias zeigte sich für das PLA2R1-Primerpaare 133 bp bereits bei einer Annealingtemperatur von 50,0°C (Fig. 49). Oberhalb einer Temperatur von 52,6°C stellten sich dabei jedoch zunehmend Artefakte (d.h. falsch-positive Signale) ein, indem für die normalen PrEC und BPH-1 Zelllinien erhöhte FAM-Signale gemessen wurden und die Werte nicht von denen maligner Prostatazelllinien zu unterscheiden waren (Fig. 49). Daraus läßt sich schließen, dass bei einer $MgCl_2$-Konzentration von 2,5 mM für den Einsatz dieses Primerpaares eine Annealingtemperatur von maximal 52,6°C gewählt werden darf. Eine Alternative dazu besteht darin, bei höheren Annealingtemperaturen, z.B. bei 63,0°C die $MgCl_2$-Konzentration auf 6,0 mM zu erhöhen, wodurch das Auftreten falsch-positiver Signale vermieden werden kann (siehe Tab. 28 und 30 und Fig. 38-45).

Ein ähnliches Phänomen, dass bei einer $MgCl_2$-Konzentration von 2,5 mM bei steigenden Annealingtemperaturen falsch-positive Signale verstärkt gebildet wurden, zeigte sich beim Primerpaar GSTP1-120bp (Fig. 50). Hier traten oberhalb einer Annealingtemperatur von 60,8°C ebenfalls Artefakte, d.h. falsch-positive Werte auf. Auch hier kann durch Erhöhung der $MgCl_2$-Konzentration das Auftreten von falsch-positiven Signalen bei steigender Annealingtemperatur vermieden und dadurch wesentlich höhere diagnostische Spezifitäten erreicht werden.

Die Beobachtung, dass mit steigenden Prä-Amplifikationszyklenzahlen die Signale in Proben Gesunder gegen 0% und in Proben von Tumorpatienten gegen 100% tendieren, wodurch eine sichere Unterscheidung zwischen Gesund und Krank möglich wird, zeigte sich auch am Beispiel der *GSTP1*-Methylierung an Proben mit 0% und 50%-igem Anteil methylierter Standard-DNA (Fig. 51 und 52). Während bei Verwendung des GSTP1-120 bp-Primerpaares unspezifische Signale der 0%-Standard-DNA-Probe bei 20 Zyklen auftraten, verschwanden diese nach 50 Zyklen vollständig (Fig. 51). Im Gegensatz dazu nahmen die spezifischen FAM-Signale in der 50%-Probe mit steigender Zyklenanzahl in ihrer Intensität zu, wenn $MgCl_2$-Konzentrationen von 1,5-2,5 mM eingesetzt wurden (Fig. 51). Dass dieses Phänomen stark vom Primerdesign und der $MgCl_2$-Konzentration abhängt, zeigte sich auch am Beispiel des GSTP1-116bp-Primerpaares. Hier nahm die FAM-Signalintensität nach mehr als 20 Zyklen ab, wenn die $MgCl_2$-Konzentration mehr als 2,5 mM betrug (Fig. 52). Aus diesem Grund ist bei diesem Primerpaar die Zyklenzahl in der Prä-Amplifikation auf 20 zu begrenzen, wenn eine $MgCl_2$-Konzentration zwischen 2,5-6,0 mM genutzt wird. Alternativ kann die $MgCl_2$-Konzentration auf 1,5 mM erniedrigt und die Zyklenanzahl entsprechend erhöht werden (Fig. 52).

Des Weiteren zeigte sich im Fall des GSTP1-116bp-Primers an Serumproben von Patientinnen mit Mammakarzinom, dass die diagnostische Sensitivität und Spezifität deutlich anstieg, wenn die $MgCl_2$-Konzentration anstelle von 1,5 mM auf eine Konzentration von 4,5 mM in der BBPA heraufgesetzt wurde (Fig. 53 und 54). Während bei einer 1,5 mM $MgCl_2$-Konzentration 4 von 20 Mammakarzinom-Patientinnen richtig identifiziert wurden, stieg diese Anzahl auf 6 bei Verwendung von 4,5 mM $MgCl_2$ an, obwohl bei der höherer $MgCl_2$-Konzentration auch der Anteil nicht-methylierter DNA-Fragmente anstieg. Letzteres kann unter diesen Bedingungen als ideale interne Kontrolle eingesetzt werden, was bei einer $MgCl_2$-Konzentration von 1,5 mM nicht der Fall war.

In einer weiteren Untersuchung an Serumproben von Patienten mit PCa zeigte sich eine verbesserte diagnostische Sensitivität und Spezifität, wenn unter den optimierten Bedingungen (4,5 mM $MgCl_2$ und 53,8°C Annealingtemperatur) das GSTP1-116bp-Primerpaar mit dem GSTP1-120bp-Primerpaar (2,5 mM $MgCl_2$ und 50,7°C Annealingtemperatur) verglichen wurde. So ließen sich mit dem GSTP1-116 bp-Primerpaar in der Gruppe von 20 PCa-Patienten 13 richtig als erkrankt identifizieren, ohne dass von den 20 gesunden Probanden einer als falsch krank identifiziert wurde, während mit dem GSTP1-120bp-Primerpaar 8 richtig als PCa-erkrankt identifiziert wurden (Fig. 55 und 56, Tab. 13 und 14). Dabei wurden in den Serumproben der Patienten PPCa13 und 20 positive Signale mit Hilfe des GSTP1-120bp-Primerpaares gefunden, die in den Untersuchungen mit dem GSTP1-116bp-Primerpaar negativ waren, so dass sich die diagnostische Sensitivität des Tests durch die Verwendung beider Primerpaare; separat in zwei verschiedenen BBPA-dPCR-Ansätzen oder gleichzeitig in einem Ansatz weiter erhöhen lässt.

Beim Einsatz der BBPA-dPCR-Technik für die Zielgene SERPINE1 und AOX1 stellte sich - wie bereits zu Beginn für das RASSF1A-117bp-Primerpaar beschrieben wurde - heraus, dass insbesondere im Fall des SERPINE1-Primerpaares 50 Zyklen, aber auch im Fall des AOX1-Primerpaares 30 Zyklen im Vergleich zu 15 Zyklen in der BBPA zu einer deutlich verbesserten Zuordnung der PCa-Patienten gegenüber den gesunden Probanden in der anschließenden dPCR führten. Dabei resultierten durch beide Zyklenzahlen keine redundanten, sondern sich ergänzende Ergebnisse (Fig. 57-60, Tab.15-18). Das bedeutet, dass durch den gleichzeitigen Einsatz unterschiedlicher Zyklenzahlen in der BBPA, z.B. 15 und 30 oder 15 und 50 Zyklen, die Sensitivität und Spezifität des Verfahrens ebenfalls weiter erhöht werden kann. So lässt sich z.B. nach 15 Zyklen der BBPA ein Teil des Probenmaterials entnehmen und die restlichen Probenmaterialien für weitere 35 Zyklen (insgesamt 50 Zyklen) in der BBPA prä-amplifizieren. Liegt genügend Untersuchungsmaterial vor, können die Patientenproben auch separat voneinander in der BBPA für 15 und 30 oder 15 und 50 Zyklen prä-amplifizert werden, um eine mögliche Kontamination der Proben untereinander zu minimieren.

Um zu prüfen, ob die neue BBPA-dPCR-Technik in der Lage ist, durch Bestimmung von methylierten DNA-Fragmenten der PLA2R1, RASSF1A, GSTP1 und PAI1-Gene zwischen Gesund, BPH und PCa differentialdiagnostisch zu unterscheiden und damit die Frage nach der Indikation für eine Gewebebiopsie zu unterstützen, wurden jeweils 20 Serumproben von gesunden Probanden, Patienten mit BPH und Patienten mit PCa analysiert. Dabei wurden Patientenproben für beide Gruppen analysiert, die sich in ihren PSA-Serumkonzentrationen nicht signifikant unterschieden, wobei die PSA-Werte in dem kritischen Bereich zwischen 2 und 15 ng/ml lagen (p<0,552; Fig. 61). Eine Differentialdiagnostik zwischen BPH und PCa war anhand des Quotienten freies PSA/ Gesamt-PSA (Referenzwert >20%) bei den Patienten ebenfalls nicht möglich (Tab. 19).

In 20 Serumproben gesunder Probanden, d.h. ohne BPH- oder PCa-Erkrankungen, fanden sich nur geringe Anteile methylierter DNA-Fragmente, deren Anteile anschließend als *cut-off*-Werte (PLA2R1<0,11%; RASSF1A<0,11%, GSTP1<0,05% und SERPINE1<0,13%) genutzt wurden. Basierend auf diesen Werten ließen sich keine erhöhten Werte bei 11 Patienten der Gruppe der 20 BPH-Patienten nachweisen. Diese 11 Patienten wiesen bis heute, d.h. 2 Jahre nach der Blutentnahme und durchgeführten Gewebebiopsie kein PCa auf (Tab. 19; Stand 15.12. 2016). Im Gegensatz dazu konnten in 13 Serumproben methylierte Tumor-DNA für mindestens eins der untersuchten 4 Gene nachgewiesen werden und alle diese Patienten waren tatsächlich an einem PCa erkrankt (Tab. 19).

Bei 9 der 20 Serumproben der BPH-Gruppe wurden ebenfalls methylierte Tumor-DNA für mindestens eins der untersuchten 4 Gene festgestellt. Bei vier dieser BPH-Patienten (Patienten BPH14, BPH15, BPH17 und BPH18 entwickelte sich nach der Blutentnahmer und Untersuchung in der Folgezeit ein PCa.

So ließen sich 10/2016 bei dem Patienten BPH14 ein PCa nachweisen, nachdem 2014 und 8/2016 in 2 Prostatabiopsien keine malignen Zellen gefunden wurden. In der BBPA-dPCR-Analyse zeigten sich bereits 10/2014 in der Serumprobe Tumor-DNA für RASSF1A und GSTP1 (Tab. 19).

Bei zwei weiteren BPH-Patienten (BPH15 und BPH17) zeigten sich 11/2014 nach TUR-P bzw. Biopsie jeweils ein PCa mit einem Malignitätsgrad IIa. In den Serumproben konnten 2 bzw. 4 Monate zuvor bereits Tumor-DNA für GSTP1 und SERPINE1 festgestellt werden. Bei dem BPH17-Patient betrug der Quotient freies PSA/ Gesamt-PSA 40,2% und war damit deutlich oberhalb des *cut-oft*-Wertes von 20%, trotzdem entwickelte der Patient ein PCa, was bereits 3 Monate zuvor durch deutlich erhöhte Anteile von methylierten GSTP1 und SERPINE1-DNA-Fragementen mit 9% (normal <0,05%) bzw. 31,7% (normal <0,13%) sicher angezeigt wurde (Tab. 19).

Bei dem BPH18-Patienten wurde auf Grund eines PSA-Anstieges 8/2014 eine Blutentnahme und Gewebebiopsie durchgeführt. In der Histologie zeigte sich in dem Einsendungsmaterial kein Anhalt für intraepitheliale Neoplasie/Dysplasie oder Malignität. Der QfPSA/PSA lag mit 22,5% oberhalb des *cut-off*-Wertes. Auf Grund einer Verdopplung des PSA auf 13,9 ng/ml innerhalb eines Jahres wurde 9/2015 eine weitere Biopsie durchgeführt mit einem erneuten stanzbioptischen Malginitätsausschluß. Im Juli 2016 wurde der Patient auf Grund eines akutem Harnverhaltes und einer Dauerkatheter-pflichtigen Prostatahyperplasie notfallmäßig behandelt und bei einer 9/2016 schließlich durchgeführten TUR-P zeigte sich ein PCa. Interessanterweise war in der Blutprobe von 8/2014, d.h. 2 Jahre zuvor, bereits ein deutlicher Anteil methylierter SERPINE1-DNA-Fragmente (11,4% bei einem *cut-off*-Wert von 0,13%) detektierbar (Tab. 19).

Bei dem BPH16-Patienten, bei dem 2014 ebenfalls eine Blutentnahme und Biopsie durchgeführt wurde, zeigte sich in der Histologie kein Anhalt für intraepitheliale Neoplasie/Dysplasie oder Malignität. Der QfPSA/PSA lag mit 12,9% allerdings unterhalb des *cut-off*-Wertes von 20%. Im März 2016 wurde erneut eine Biopsie durchgeführt, bei der in 1 Biopsiestanze kleinherdig hochgradige intraepitheliale Neoplasieherde (HGPIN) gefunden wurden und deshalb eine erneute Gewebebiopsie in 6 Monaten empfohlen wurde. Diese steht noch aus. Auch bei diesem Patienten wurden in der Serumprobe von 9/2014 bereits mit Hilfe der BBPA-dPCR-Technik erhöhte Werte für alle 4 untersuchten Gene bestimmt (Tab. 19).

Ebenfalls erhöhte Werte für alle 4 Gene zeigten sich in der Serumprobe von 10/2014 bei dem BPH12-Patienten, bei dem gleichzeitig eine TUR-P durchgeführt wurde und eine atypische adenomatöse Hyperplasie (AAH), aber kein Karzinom in dem eingeschickten Untersuchungsmaterial (Späne) nachweisbar war.

Bei dem BPH13-Patienten, bei dem in der Serumprobe von 10/2014 eine deutlich erhöhte Anzahl methylierter GSTP1-Genfragmente gefunden wurde, zeigten sich in einer ebenfalls 10/2014 durchgeführten Prostatabiopsie einzelne Drüsenproliferate unklarer Dignität. In den Nachuntersuchungen ließ sich keine Racemase-Überexpression und damit kein PCa nachweisen. Im Februar 2016 erfolgte bei diesem Patienten auf Grund einer Dauerkathederpflichtigen Prostatahyperplasie eine TUR-P. Auch hier zeigten sich einzelne Drüsenproliferate unklarer Dignität, deren Nachuntersuchungen aber bisher kein PCa identifizierten (Tab. 19).

Auch bei den BPH-19 und BPH-20-Patienten, bei denen im Serum erhöhte methylierte DNA-Fragmente für alle 4 Gene feststellbar waren, zeigten sich in den Histologieberichten suspekte Befunde nach TUR-P bzw. Prostatabiopsie, wenngleich bisher in beiden Fällen kein PCa eindeutig nachweisbar war (Tab. 19).

Zusammengefasst kann festgestellt werden, dass mit Hilfe der BBPA-dPCR-Technik bei allen 20 gesunden Probanden die erhaltenen Werte unterhalb der festgelegten *cut-off*-Werte lagen und damit in keinen der Fälle ein falschpositives Signal erhalten wurde (diagnostische Spezifität 100%). In der Gruppe der 20 Patienten mit einem PCa wurden 13 als richtig positiv identifiziert (diagnostische Sensitivität 65%). In der Gruppe der 20 Patienten mit einer BPH wurden bei 11 Patienten negative Befunde erhalten, d.h. diese Patienten als Gesund (ohne PCa) klassifizeirt. Bei diesen Patienten konnten nach mehr als 2 Jahren (Stand 15.12.2016) seit den erfolgten Blutuntersuchungen tatsächlich kein PCa festgestellt werden. Bei 9 der 20 untersuchten BPH-Patienten fanden sich positive Signale für mindestens ein untersuchtes Zielgen in der BBPA-dPCR. In 4 Fällen fand sich bei diesen Patienten im Zeitraum von 4 Monaten bis 2 Jahren nach den Blutuntersuchungen ein PCa. Bei den anderen 5 BPH-Patienten zeigten sich in der Folgezeit nach der Blutuntersuchung suspekte Befunde in der Histologie, ohne dass bisher maligne Veränderungen klar identifiert werden konnten (Tab. 19). Inwieweit sich auch bei diesen Patienten in der Folgezeit ein PCa entwickeln wird, lässt sich nur durch Untersuchungen in Zukunft beantworten.

Damit konnte gezeigt werden, dass mit Hilfe der neuen BBPA-dPCR-Technik eine wesentlich höhere diagnostische Spezifität und ein wesentlich höherer positiver prädiktiver Wert im Vergleich zu den PSA-Bestimmungen gefunden wurden, insbesondere im PSA-Konzentrationsbereich von 2-15 ng/ml, wo keine signifikanten Unterschiede zwischen den Gruppen BPH und PCa vorlagen (Fig. 61).

Wie wichtig die Etablierung neuer Biomarker und die Bereitstellung entsprechender kommerzieller Testkits für die Diagnostik von PCa ist, wurde bereits zu Beginn ausgeführt.

Das neu entwickelte Verfahren der BBPA-dPCR-Technik kann hier einen wesentlichen Beitrag liefern, um unnötige Biopsie zu vermindern oder zu einer rechtzeitigen Biopsie führen, wodurch einerseits unnötige Belastungen und Nebenwirkungen reduziert und andererseits auch die Kosten dieser Behandlungen deutlich vermindert werden können. Allein in Deutschland werden auf Grund der PSA-Bestimmungen jährlich zwischen 250.000 und 350.000 Biopsien durchgeführt, von denen bis zu 75% unnötig sind.

Auch die diagnostische Sensitivität des neuen Verfahrens ist im Vergleich zum PSA deutlich verbessert, insbesondere wenn berücksichtigt wird, dass für die hier beschriebenen Untersuchungen max. 1 ml Serum zur Verfügung standen, die zudem hinsichtlich einer speziellen Präanalytik wie Hemmung von DNasen nicht speziell vorbehandelt wurden. So ist für den Nachweis von Tumor-DNA in Patientenproben das Vorliegen mindestens 1 Tumor-DNA-Kopie Grundvoraussetzung, so dass die Wahrscheinlichkeit, dass so eine Kopie vorliegt, mit der Menge des zu untersuchenden Probenmaterials naturgemäß ansteigt, insbesondere wenn die Tumorerkrankung so früh wie möglich diagnostiziert werden soll, d.h in einer frühen Entwicklungsphase der Tumorerkrankung, um damit eine bereits stattgefundene Metastasierung zu vermeiden.

Darüber hinaus stand für die Untersuchung kein weiteres Probenmaterial zur Verfügung, um weitere Primerpaare wie das GSTP1-116bp-Primerpaar einzusetzen oder weitere Zielgene wie das AOX1, Thrombomodulin und/oder Septin-9 zu untersuchen, wodurch die diagnostische Sensitivität weiter gesteigert werden kann.

Insbesondere bei der Diagnostik von PCa, Nieren- und Blasenkarzinomen bieten sich neben der Untersuchung von Blutproben (Serum oder Plasma) auch Urinproben an, welches nichtinvasiv und in größeren Menge gewonnen und für diese Untersuchungen leicht zur Verfügung gestellt werden kann.

Eine hohe analytische und diagnostische Sensitivität bei nahezu 100%-iger Spezifität stellt sich insbesodnere für die Diagnostik von minimalen Resterkrankungen (MRD), z.B. nach einer chirurgischen Operation, wo sich die Frage nach der Notwendigkeit einer anschließenden Chemotherapie/Bestrahlung stellt und auch für die spätere Nachsorge der Patienten ist eine diagnostische Sensitivität und Spezifität von nahezu 100% unabdingbar, sollen so früh wie möglich Rezidive erkannt werden. So zeigte sich z.B. bei dem PCa1-Patienten in der Histologie nach operativer Entfernung des Tumors eine Infiltration des Resektionsrandes, so dass sich hier die Frage stellt, inwieweit z.B. in der Blutbahn oder über die Urinausscheidung fc-Tumor-DNA nachweisen lässt, die Grundlage für eine intensive Nachsorge z.B. durch einen frühzeitigen erneuten operativen Eingriff sein könnte.

**Tabelle 19** : Untersuchungsergebnisse zur Differenzierung zwischen gesunden Probanden, Patienten mit benigner Prostatahyperplasie (BPH) und Patienten mit Prostatakarzinom (PCa) mit PSA-Konzentrationen im Serum, Quotient zwischen freiem und Gesamt-PSA (QfPSA/PSA), Menge methylierter ***PLA2R1-, RASSF1A* (*RASS*)-, *GSTP1-*** und ***SERPINE-1 (PAI1)***-DNA-Fragmenten im Verhältnis zu nicht-methylierten Fragmenten und den klinischen Daten. Die *cut-off*-Werte sind in der ersten Zeile angegeben.

| | **Datum** | **Alter Jahre** | **PSA <4,0 ng/ml** | **QfPSA/ TPSA >20%** | **PLA2R1 <0,11%** | **RASS <0,4%** | **GSTP1 <0,05%** | **PAI1 <0,13%** | **Klinik** |
|---|---|---|---|---|---|---|---|---|---|
| GM1 | 5/2015 | 22 | | | 0,009 | 0 | 0 | 0,056 | |
| GM2 | 5/2015 | 28 | | | 0 | 0 | 0 | 0 | |
| GM3 | 5/2015 | 22 | | | 0 | 0,4 | 0 | 0,003 | |
| GM4 | 5/2015 | 32 | | | 0,023 | 0 | 0 | 0 | |
| GM5 | 5/2015 | 35 | | | 0 | 0 | 0 | 0 | |
| GM6 | 5/2015 | 23 | | | 0 | 0,007 | 0 | 0 | |
| GM7 | 5/2015 | 30 | | | 0 | 0 | 0 | 0 | |
| GM8 | 5/2015 | 28 | | | 0 | 0 | 0,007 | 0,018 | |
| GM9 | 5/2015 | 44 | | | 0,019 | 0 | 0,046 | 0,03 | |
| GM1O | 5/2015 | 50 | | | 0 | 0 | 0 | 0 | |
| GM11 | 5/2015 | 49 | | | 0 | 0 | 0 | 0,017 | |
| GM12 | 5/2015 | 63 | | | 0,0025 | 0,025 | 0 | 0 | |
| GM13 | 5/2015 | 21 | | | 0 | 0 | 0 | 0,008 | |
| GM14 | 5/2015 | 23 | | | 0,0026 | 0 | 0 | 0,0029 | |
| GM15 | 5/2015 | 23 | | | 0 | 0 | 0 | 0 | |
| GM16 | 5/2015 | 28 | | | 0 | 0,007 | 0,005 | 0,015 | |
| GM17 | 5/2015 | 62 | | | 0 | 0 | 0 | 0,0027 | |
| GM18 | 5/2015 | 29 | | | 0 | 0 | 0 | 0,044 | |
| GM19 | 5/2015 | 23 | | | 0 | 0 | 0 | 0,007 | |
| GM20 | 5/2015 | 46 | | | 0,104 | 0,012 | 0 | 0,13 | |
| BPH-1 | 9/2014 | 60 | **8,6** | | 0 | 0,029 | 0 | 0,06 | bis heute kein PCa |
| BPH-2 | 9/2014 | 73 | **6,27** | **18,50** | 0 | 0,05 | 0 | 0 | bis heute kein PCa |

(fortgesetzt)

| | Datum | Alter Jahre | PSA <4,0 ng/ml | QfPSA/ TPSA >20% | PLA2R1 <0,11% | RASS <0,4% | GSTP1 <0,05% | PAI1 <0,13% | Klinik |
|---|---|---|---|---|---|---|---|---|---|
| BPH-3 | 8/2014 | 75 | **7,92** | | 0,032 | 0,003 | 0 | 0 | bis heute kein PCa |
| BPH-4 | 8/2014 | 59 | **8,56** | | 0,052 | 0 | 0 | 0,009 | bis heute kein PCa |
| BPH-5 | 8/2014 | 83 | **5,2** | | 0,051 | 0 | 0,117 | 0,004 | bis heute kein PCa |
| BPH-6 | 8/2014 | 73 | **4,19** | **15,27** | 0,18 | 0 | 0,062 | 0,168 | bis heute kein PCa |
| BPH-7 | 8/2014 | 64 | **6,56** | **13,72** | 0,104 | 0,42 | 0,066 | 0 | bis heute kein PCa |
| BPH-8 | 8/2014 | 66 | **6,44** | **15,06** | 0 | 0,005 | 0,04 | 0,003 | bis heute kein PCa |
| BPH-9 | 7/2014 | 65 | **4,84** | | 0 | 0,017 | 0,008 | 1,88 | bis heute kein PCa |
| BPH-10 | 7/2014 | 58 | **13,2** | | 0 | 0,028 | 0,051 | 0,028 | bis heute kein PCa |
| BPH-11 | 7/2014 | 74 | 2 | | 0 | 0 | 0 | 0,17 | bis heute kein PCa |
| BPH-12 | 10/2014 | 75 | 0,33 | | **5,2** | **2,62** | **3,24** | **4,2** | 10/2014 TUR-P (12g) auf Grund obstruktiver Prostatahyperplasie; Histologie: Atypische Adematöse Hyperplasie (AAH) **[1]** |
| BPH-13 | 10/2014 | 71 | **14,23** | | 0 | 0,009 | **14,49** | 0,005 | Prostatabiopsie (Pb) 2007 und 2011 negativ, 10/2014 negativ **[2];** 2/2016 Pb vom Patienten abgelehnt, aber TUR-P auf Grund Dauerkatheder-pflichtiger Prostatahyperplasie: Histologie **[3]** |
| BPH-14 | 10/2014 | 67 | **6,39** | 23,63 | 0 | **0,83** | **1,41** | 0,012 | Pb 2010, 2011, 2014 und 8/2016 negativ **[4]; 10/2016 PCa-Nachweis** |
| BPH-15 | 9/2014 | 66 | **4,12** | | 0,12 | 0,061 | **3,39** | 0 | 11/2014 TUR-P bei **Prostatakarzinom,** pTla, pNx, L0, V0, Rx, Gleason-Score 3+3=6, Malignitätsgrad IIa mit symptomatischer Prostatahyperplasie |
| BPH-16 | 9/2014 | 47 | **6,53** | **12,86** | **1,45** | **0,75** | **7,42** | **1,99** | Pb 2012 und 2014 negativ, Pb 3/2016 Nachweis von HGPIN in einer Biopsiestanze **[5]; Rebiopsie in 6 Monaten empfohlen,** bisher noch nicht durchgeführt. |

(fortgesetzt)

| | Datum | Alter Jahre | PSA <4,0 ng/ml | QfPSA/ TPSA >20% | PLA2R1 <0,11% | RASS <0,4% | GSTP1 <0,05% | PAI1 <0,13% | Klinik |
|---|---|---|---|---|---|---|---|---|---|
| BPH-17 | 8/2014 | 66 | **4,75** | 40,21 | 0 | 0,12 | **9** | **31,7** | Pb 2011 und 7/2014 negativ, hier allerdings Empfehlung einer erneuten Pb auf Grund einer atypischen kleindrüsigen Proliferation in 3-6 Monaten bei PSA: 4,75 ng/ml; Q=40,2%; **11/2014: Nachweis eines PCa** (Gleason-Score 3+4=7; GIIa) in der Pb **[6]; 2/2015: OP des PCa** |
| BPH-18 | 8/2014 | 72 | **6,31** | 22,50 | 0,018 | 0,1 | **0** | **11,4** | Pb 2013 negativ, 8/2014 PSA-Anstieg auf 6,31 ng/ml, Q=22,5% und Pb **[7]; 9/2015** auf Grund PSA-Erhöhung auf 13,9 ng/ml erneute Pb **[8];** 7/2016 notfallmäßige Vorstellung bei akutem Harnverhalt; Dauerkatheterpflichtige Prostatahyperplasie; 9/2016: TUR-P mit **postoperative Tumorklassifikation** T1a; Rx, Gleason-Score 3+3=6 **[9].** |
| BPH-19 | 8/2014 | 75 | **9,31** | 21,27 | **1,27** | **2,26** | **0,21** | 0,019 | Pb 2006, 2008, 2012 und 2014 negativ; 3/2016 Therapie durch TUR-P (50 g) bei persistierender PSA-Erhöhung von 9,28 ng/ml; Histologie **[10];** |
| BPH-20 | 7/2014 | 73 | **4,75** | | **1,52** | **2,16** | 0 | **1,6** | Pb 2012 und 2013 negativ; 8/2014: Pb bei PSA von 4,75 ng/ml; Histologie **[11];** |
| PCa1 | | 59 | **6,35** | | **2,86** | 0 | 0 | 0,08 | 10/2014: OP des PCa; pT2c, cN0, cM0, L0, V0, Pn1, Rx (im linkseitigen Apex beschriebene **Infiltration des Resektionsrandes),** Gleason-Score 3+4=7, Malignitätsgrad IIb **[12]** |
| PCa2 | | 60 | **9,42** | | 0 | **0,19** | 0 | 0,14 | 10/2014 OP des PCa; pT2c, pN0 (0/10 LK), L0, V0, Pn1, R0 (lokal), Gleason-Score: 3 + 3 = 6, Malignitätsgrad IIa Pb 04/2014: 3/12 Zylindern positiv |
| PCa3 | | 72 | **8,4** | | 0 | **0,88** | 0 | 0 | 10/2014 OP des PCa; pT2c, pN0 (0/18 LK), cM0, L0, V0, Pn1, R0 (lokal), Gleason-Score: 4+3=7; tertiäres Differenzierungsmuster Gleason 5, Malignitätsgrad IIIb 8/2014 Pb: 1/12 Zyl. links positiv |
| PCa4 | | 60 | 3,53 | | 0 | **6,5** | **11,5** | **0,27** | 10/2014 OP des PCa; pT2c, pN0 (0/19 LK), cM0, L0, V0, Pn1, R0 (lokal), Gleason-Score 3+4=7, Malignitätsgrad IIa 8/2014 Pb: cT1c (8/12 Zyl. pos. bds.) |
| PCa5 | | 67 | **13,16** | | 0,012 | 0,28 | **1,2** | 0 | 9/2014 OP des PCa; pT2c, pN0 (0/28 LK), L0, V0, Pn0, R0 (lokal), Gleason-Score: 3+4=7, Malignitätsgrad IIa |

(fortgesetzt)

| | Datum | Alter Jahre | PSA <4,0 ng/ml | QfPSA/ TPSA >20% | PLA2R1 <0,11% | RASS <0,4% | GSTP1 <0,05% | PAI1 <0,13% | Klinik |
|---|---|---|---|---|---|---|---|---|---|
| PCa6 | | 62 | 3,6 | | 0 | **21,6** | **7,4** | 0,07 | 9/2014 OP des PCa; pT2c, pN0 (0/11 LK), cM0, L0, V0, Pn1, R0 (lokal) Gleason-Score: 3+4=7, Malignitätsgrad IIa; 5/2014: Pb: 1/12 Zylinder links positiv (5%) |
| PCa7 | | 68 | **4,25** | | **1,02** | **1,28** | 0 | 0,011 | 9/2014 OP des PCa; pT2c pN0 (0/20 LK) cM0 L0 V0 Pn1 R1 (dorsal beidseits), Gleason-Score: 3+4=7, tertiäres Differenzierungsmuster Gleason 5, Malignitätsgrad IIa, 7/2014 Pb: 2/12 Zyl. rechts positiv |
| PCa8 | | 60 | **6,45** | | 0,019 | 0,4 | **0,85** | 0,014 | 9/2014 MR-fusionierte perineale (6 Zylinder) und TRUS-gestützte transrektale (12 Zylinder) Pb: 1/18 Zylindern rechts positiv; Gleason-Score: 3+3=6 |
| PCa9 | | 44 | **8,73** | | **0,79** | 0,015 | **0,52** | 0,045 | 9/2014 OP des PCa; pT2c, pN0 (0/17 LK), cM0, L0, V0, Pn1, R0 (lokal), Gleason-Score: 3+4=7, Malignitätsgrad IIa 6/2014 Pb rechts 1/6 und links 3/6 Zylindern positiv |
| PCa10 | | 60 | **10,86** | | 0,11 | 0,037 | **1,36** | **0,42** | 9/2014 OP des PCa; pT2c, pN0 (0/11 LK), cM0, L0, V0, Pn1, R0 (lokal), Gleason-Score 3+4=7; Malignitätsgrad IIa |
| PCa11 | | 67 | **15,05** | | 0 | 0 | **0,51** | 0,077 | 8/2014 OP des PCa; pT2c, pN0 (0/16 LK), cM0, L0, V0, Pn0, R0 (lokal), Gleason-Score: 3+4=7; Malignitätsgrad IIa 12/2013 Pb: 3/20 Zylindern bds. Gleason 3+3=6 |
| PCa12 | | 70 | **14,19** | | 0 | **0,67** | 0 | 0,043 | 8/2014 OP des PCa; pT3b, pN0 (0/13 LK), cM0, L0, V0, Pn1, R0 (lokal), Gleason-Score 4+3=7, Malignitätsgrad IIb |
| PCa13 | | 73 | **7,2** | | **0,59** | **0,6** | 0 | 0,0026 | 8/2014 OP des PCa; pT2c, pN0 (0/10 LK), cM0, L0, V0, Pn1, R0 (lokal), Gleason-Score 3+4=7; Malignitätsgrad IIa |
| PCa14 | | 64 | 2,22 | | 0 | 0,06 | 0 | 0 | 10/2014 OP des PCa; pT2c, pN0 (0/14 LK), cM0, L0, V0, Pn1, R0 (lokal), Gleason-Score: 3+4=7, Malignitätsgrad IIa 06/2014: TUR-P Gleason 3+3 =6 |
| PCa15 | | 75 | **10,34** | | 0,005 | 0,004 | 0 | 0,017 | 9/2014 OP des PCa; pT2c, pN0 (0/16 LK), cM0, L0, V0, Pn1, R1 (multifokal dorsoperipher rechts), Gleason-Score 4+3=7, Malignitätsgrad IIb |

(fortgesetzt)

| | Datum | Alter Jahre | PSA <4,0 ng/ml | QfPSA/ TPSA >20% | PLA2R1 <0,11% | RASS <0,4% | GSTP1 <0,05% | PAI1 <0,13% | Klinik |
|---|---|---|---|---|---|---|---|---|---|
| PCa16 | | 69 | **5,72** | | 0,044 | 0,011 | 0,033 | 0,133 | 9/2014 OP des PCa; pT2c, pN0 (0/24 LK), cM0, L0, V0, Pn1, R0 (lokal), Gleason-Score 3+4=7, Malignitätsgrad IIb, 07/2014 Pb (5/7 Zylinder links positiv) |
| PCa17 | | 59 | **8,52** | | 0 | 0,054 | 0 | 0 | 9/2014 Pb mit 2/20 Zylinder pos., Gleason-Score 3+3=6; Malignitätsgrad IIa |
| PCa18 | | 71 | 0,12 | | 0 | 0,059 | 0,096 | 0,025 | 8/2014 OP des PCa; pT2a, pN0 (0/13 LK), cM0, L0, V0, Pn1, R0 (lokal), Gleason-Score: 4+3=7, drittes Differenzierungsmuster Gleason 5, Malignitätsgrad IIIa 02/2014 Pb: 1/6 Zylindern rechts positiv, Gleason-Score: 3+3=6 |
| PCa19 | | 79 | **4,48** | | 0 | 0 | 0 | 0,01 | 8/2014 OP des PCa; pT2c, pN0 (0/10 LK), cM0, L0, V0, Pn1, R0 (lokal), Gleason-Score: 3+4=7, Malignitätsgrad IIb 7/2014 Pb: 6/12 Zylindern pos. bds., Gleason 3+4=7 |

(fortgesetzt)

| | **Datum** | **Alter Jahre** | **PSA <4,0 ng/ml** | **QfPSA/ TPSA >20%** | **PLA2R1 <0,11%** | **RASS <0,4%** | **GSTP1 <0,05%** | **PAI1 <0,13%** | **Klinik** |
|---|---|---|---|---|---|---|---|---|---|
| PCa20 | | 60 | **10,88** | | 0,062 | 0 | 0 | 0,009 | 8/2014 OP des PCa; pT3a, pN0 (0/25 LK), cM0, L0, V0, Pn1, R0 (lokal), Gleason-Score: 3+4=7, Malignitätsgrad IIb |

**014:** TUR-P (12g) infolge obstruktiver Prostatahyperplasie; Histologie: Aufgrund einzelner Drüsenproliferate unklarer Dignität wurden immunhistocher ersuchungen (mit Antikörpern gegen CK5/14 und Racemase) durchgeführt. Dabei zeigten sich die Drüsen mit inkompletten Verlust basaler Zytoker liche Überexpression der Racemase, sodass es sich hier um eine atypische adenomatöse Hyperplasie (AAH) handelte, was ein invasives Karzinom am vo l bisher nicht belegen konnte.

**014:** TRUS-gestützte Sättigungsbiopsie (25 Biopsiezylinder) in Folge einer PSA-Erhöhung auf 14,23 ng/ml. 1. bis 13. Prostatastanzzylindermaterial mit e in kleineren Gruppen gelegenen hyperplastischen Drüsen sowie einer überwiegend diffusen Hyperplasie des fibromuskulären Stromas, herdförmi iven Veränderungen mit Atrophie und postatropher Hyperplasie prostatischer Drüsen sowie einer örtlich mäßig ausgeprägten Basalzellhyperplasie, im l äßiggradigen chronischen, fokal gering aktiven Prostatitis, wobei aufgrund einzelner Drüsenproliferate unklarer Dignität in Einsendung 2 und 7 noch in che Zusatzuntersuchungen durchgeführt wurden. In der Nachuntersuchung zeigte sich im Bereich der beschriebenen Drüsenproliferate überwiegend ein sion basaler Zytokeratine (mit Expression von CK5/14 ohne Nachweis einer Überexpression der Racemase, sodass es sich hier um atrophe Prostatadrüs egenden Material kein Anhalt für Malignität.

**016:** TUR-P (94 g) in Folge einer Dauerkatheter-pflichtigen Prostatahyperplasie bei weiteren PSA-Anstieg auf 20,5 ng/ml. Aufgrund einzelner Drüser r Dignität wurden noch immunhistochemische Untersuchungen (mit Antikörpern gegen CK5/14, Racemase, Calretinin, Panzytokeratin, CK7, Cadher CD34, 34ßE12, Androgen-Rezeptor, EMA, PSA und WT1) durchgeführt. Dabei ließ sich in dem beschriebenen auffälligen Drüsenproliferat eine Expr okeratin sowie z.T. von CK7 und EMA und nukleär von Androgen-Rezeptor nachweisen bei fehlender Expression von WT1, PSA, Racemase, Cadherin 1 3 sowie überwiegend fehlender Expression von CD34 und PAX8 sowie auch von Calretinin. Der Ki67-Proliferationsindex gering (ca. 1 %), so dass es sich inbeziehung der Immunhistologie und der in der PAS nachweisbaren PAS-positiven intraluminalen Einschlüsse bzw. Sekretionen um ein nephrogene . Im vorliegenden Material kein Anhalt für Malignität.

**016:** MR-fusionierte perineale (4 Biopsiezylinder) und TRUS-gestützte transrektale (12 Biopsiezylinder) Prostatabiopsie 1.-16. Prostatastanzzylinde ärer nodulärer Stromahyperplasie, mit Atrophie der Prostatadrüsen sowie auch hypertrophen Prostatadrüsen, fokal mit einer chronischen uncharakt dungsreaktion sowie fokal mit Plattenepithelmetaplasie in den größeren Ausführungsgängen sowie mit Nachweis einer Urothelmetaplasie in größeren Au . Im vorliegenden Einsendungsmaterial kein Anhalt für intraepitheliale Neoplasie/Dysplasie oder Malignität. Der PSA-Wert betrug 10,94 ng/ml. **10/201** lerdings der **PCa-Nachweis**

**16:** 1. -17. Prostatastanzzylinder mit benigner adenomatöser, teils fibromyomatöser Hyperplasie des Parenchyms, stellenweise mit deutlich ausgeprägte asie des Drüsenepithels bzw. Atrophie der Drüsen, stellenweise mit geringer Sekretretention bzw. Nachweis einzelner Corpora amylacea in den Drüse sendung 7 mit kleinherdig nachweisbaren **hochgradigen prostatischen intraepithelialen Neoplasieherden (HGPIN)** und stellenweise einer nicht me ßiggradig chronischen und floriden unspezifischen Prostatitis.

**2014:** 1 von 17 Prostatastanzzylindermaterial (rechts apikal peripher) mit kleinherdiger Infiltration durch ein überwiegend gering drüsig, kleinherdig, k endes Adenokarzinom der Prostata mit mittelgradiger Kernpleomorphie, etwa 5% der Stammzylinderfläche einnehmend, größte zusammenhängende Ka ,2 mm messend. Gleason-Score: 3 + 4 = 7 (davon Gleason 3 90%, Gleason 4 10 %). Malignitätsgrad IIa

**14:** MRT-fusionierte, ultraschallgestützte transperineale Prostatabiopsie (6 Biopsiezylinder) und TRUS-gestützte Prostatasbiopsie: 1.-18. Prostatastanz r Stromahyperplasie, Hyperplasie der Prostatadrüsen und z.T. atrophen Prostatadrüsen, mit mehreren Corpora amylacea in etwas dilatierten Drüsen. Im vo dungsmaterial kein Anhalt für intraepitheliale Neoplasie/Dysplasie oder Malignität.

**15:** MRT-fusionierte transperineale Prostatabiopsie (8 Zylinder) und transrektale Prostatabiopsie (12 Zylinder); MRT zeigte eine Pirads 4 Läsionen lin nsitionalzone und rechts im mittleren Prostatadrittel in der Transitionalzone; Die digital-rektale Untersuchung ergab keinen suspekten Tastbefund. amt 20 Zylinder entnommen und es ergab sich ein stanzbioptischer Malginitätsausschluss bei erneuter PSA-Messung von 14,2 ng/ml.

**16:** Histologisch zeigte sich ein kleindrüsig wachsendes Adenokarzinom mit mäßiggradiger Kernpleomorphie in einzelnen Spänen, wobei das Karzinor r als 5% der untersuchten Fläche einnahm. Therapieempfehlung: aktive Überwachung mit Kontrollbiopsie in 3-6 Monaten oder alternativ *Wachtful Wai*

**016:** Prostataresektionsspäne mit nodulären, muskulären und glandulären (adenomyomatösen) Prostatahyperplasie, fokal mit Basalzellhyperplasie und rten Drüsen mit Sekretretention und einer teils chronischen, teils resorptiven Entzündungsreaktion, vermehrt mit Nachweis von Corpora amylacea, fo stationen im Stroma, miterfasst Anteile der Pars prostatica urethrae mit weitgehend regelhaftem Urothel. Am vorliegenden Material kein Anhalt für intra sie/Dysplasie oder Malignität.

**2014:** MRT-fusionierte, ultraschall-gestützte transperineale (8 Zylinder) Prostatabiopsie und transrektale ultraschallgestützte Prostatabiopsie (12 Zylin statastanzzylinder mit knotiger Stromahyperplasie und Atrophie der Prostatadrüsen und z.T. hypertrophen Prostatadrüsen, mit einer fokalen chronisch ischen Entzündungsreaktion, in den Einsendungen 9 und 12 jeweils mit auffälligen kleindrüsigen Proliferaten unklarer Dignität (ehemals ASAP). Immunhis tersuchungen zeigten in den Einsendungen 9 und 12 kleindrüsige Proliferate mit unterbrochener CK5/14-positiven Basalzellschicht. Die Reaktion für egativ und somit kein Anhalt für ein Prostatakarzinom.

**2014:** Aufgrund des histopathologischen Befundes (Rx-Resektion) empfehlen wir die PSA-gestützte Nachsorge mit frühzeitiger Salvage-Strahlentherap , alternativ eine adjuvante RTx. Außerdem PSA-Kontrollen (PSA=0,12 ng/ml) zur Bestätigung der postoperativen PSA-Normalisierung.

**Ausführungsbeispiel 12: Untersuchung von Serumproben gesunder und an Mamma- und Ovarialkarzinomen erkrankter Patientinnen**

Die Untersuchungen haben gezeigt, dass vor allem unter Einsatz des GSTP1-116bp-Primerpaares sehr niedrige Methylierungsraten (≤ 0,05%) in allen Serumproben gesunder Probanden zu finden waren, während in den Serumproben von Probandinnen mit Mamma- und Ovarialkarzinomen deutlich davon zu unterscheidende erhöhte Werte gemessen wurden (Tab. 31). Während mit dem Primerpaar-GSTP1-116bp 18 von 23 Proben positiv waren, ließen sich in 5 von 23 Proben unter Verwendung des GSTP1-120bp-Primerpaares und in 12 von 23 Proben unter Verwendung des RASSF1A-117bp-Primerpaares positive Signale finden. Interessant dabei war, dass unter Verwendung des GSTP1-120bp-Primerpaares erneut in 2 Patientenproben positive Signale gemessen wurden, die unter Einsatz des GSTP1-116bp-Primerpaares negativ waren, so dass die Kombination beider Primerpaare, getrennt oder gleichzeitig in einem Ansatz, zu einer erhöhten diagnostischen Sensitivität führen kann. Eine diagnostische Sensitivität von 100% bei einer diagnostischen Spezifität von 100% wurde erreicht, wenn die Daten, die mit den GSTP1-Primern erhalten wurden, mit denen der mittels RASSF1A-Primern erhaltenen Daten kombiniert wurden (Tab. 31).

Interessant waren auch die Daten, die von einer Patientin mit primär ossär und hepatisch metastasiertem Mamma-CA vor (P8a) und nach 8-wöchiger Chemotherapie (P8b) stammten. Neben dem Abfall des CA-15-3-Wertes von 941,4 U/ml auf 133,0 U/ml war ein Abfall der Methylierungswerte für GSTP1-116bp von 0,9% auf 0% und RASSF1A-117bp von 18,9% auf 2% zu verzeichnen. Dieser Befund lässt auf eine gute Therapieantwort schließen. Die Methylierungswerte GSTP1 und RASSF1A lagen nach Beendigung der Behandlung bereits im Normbereich, während der CA-15-3-Wert noch erhöht war, was durch eine längere biologische Halbwertzeit begründet sein kann. Diese Ergebnisse verdeutlichen das Potential der BBPA-dPCR-Untersuchungen für die unmittelbare Therapie- und Verlaufskontrolle und dem Nachweis einer MRD, indem die Konzentration von fcT-DNA bei erfolgreicher Therapie wesentlich schneller abfallen als die herkömmlichen Tumormarker wie das CA 15-3. Damit kann dieses Verfahren auch für Therapieentscheidungen wie die Frage nach dem Erfordernis einer anschließenden unmittelbaren Chemo- und/oder Bestrahlungstherapie nach einer erfolgreichen Operation des Tumors unterstützend eingesetzt werden, je nachdem ob die initial erhöht gefundenen fcT-DNA-Konzentrationen in den Normbereich gefallen bzw. im günstigsten Fall nicht mehr nachweisbar sind, so dass eine MRD ausgeschlossen werden kann.

Die Probe (P24) stammte von einer 32-jährigen Patientin, bei der eine pathologische Mutation im *BRCA1*-Gen vorlag und die mit einem familiären Risiko für Mamma- und Ovarialkarzinom einhergeht. So erkrankte die Mutter der Patientin bereits mit 33 Jahren an einem Mamma-Karzinom, die Großmutter mit 56 Jahren. Bei der Patientin wurde auf eigenem Wunsch eine prophylaktische subkutane Mastektomie durchgeführt. Die histologischen Untersuchungen der Operationspräparate ergaben keinen Anhalt für intraepitheliale Neoplasie/Dysplasie oder Malignität und auch der Tumormarker CA 15-3 war normal. Überraschend zeigten sich jedoch bereits erhöhte Werte beim Einsatz des GSTP1-116bp-Primers in der BBPA-dPCR (Tab. 31). Daraus lässt sich schließen, dass mit Hilfe dieser Technik frühzeitig pathologische Veränderungen z.B. durch eine Blutuntersuchung (*Liquid Biopsy*) bei Patientinnen mit erhöhtem familiären Risiko für Mamma- und Ovarialkarzinom angezeigt werden können und dieses Verfahren bei der Entscheidung für eine prophylaktische Mastektomie bzw. Ovarektomie unterstützend eingesetzt werden kann, insbesondere wenn die Familienplanungen bei den betroffenen Patientinnen noch nicht abgeschlossen sind.

Die hier beschriebene neue BBPA-dPCR-Technik kann prinzipiell für alle potentiellen Zielgensequenzen eingesetzt werden, deren Methylierungsgrad sich zwischen Gesund und Krank unterscheidet. Nach Konstruktion von Primern und Sonden entsprechend den beschriebenen Prinzipien wird die Bias für methylierte und nicht-methylierte Zielsequenzen in Abhängigkeit von der Annealingtemperatur und gleichzeitig von der $MgCl_2$-Konzentration, z.B. für Septin-9 (SEPT9, HGNC:HGNC:7323 Ensembl:ENSG00000184640 , SEQ ID No. 140-144, 159-164 und 179-184), als auch die optimale Zyklenanzahl in der BBPA empirisch mit einer Probe mit bekanntem Methylierungsgrad bestimmt. Anhand der erhaltenen Daten werden die Bedingungen für jedes Primerpaar so festgelegt, dass die zu analysierende Zielsequenz (methyliert oder nicht-methyliert) maximal und die Kontrollzielsequenzen (nichtmethlyierte oder methylierte Wildtyp-Sequenzen) noch ausreichend mit vervielfältigt werden. Auf diese Weise können einerseits die ermittelten Werte für die Zielsequenzen mit denen der Wildtyp-DNA-Sequenzen als interne Kontrolle ins Verhältnis gesetzt und damit die Menge der in die BBPA-dPCR eingebrachten Bisulfit-umgewandelten DNA berücksichtigt werden. Andererseits lassen sich auf diese Weise hohe analytische Sensitivitäten ähnlich der MS-PCR-Technik (mit methylspezifischen [MS]-Primern) erreichen, ohne dass dabei verstärkt falsch-positive Signale auftreten, wie das bei der MS-PCR-Technik der Fall ist [4]. Im Vergleich zur MS-HRM-Technik (mit methylunabhängigen [MIP]-Primern), bei der im Gegesatz zur MS-PCR weniger falsch-positive Signale auftreten, weist die BBPA-dPCR-Technik eine wesentlich höhere analytische und damit diagnostische Sensitivität auf. Lassen sich für neue Zielsequenzen auf diese Weise mit der BBPA-dPCR ebenfalls diagnostische Spezifitäten von nahezu 100% erreichen, können diese Zielsequenzen zu dem beschriebenen Panel an Zielgenen (PLA2R1, RASSF1A, GSTP1, SERPINE1, AOX1, TM und/oder Septin-9) hinzugefügt werden, um die diagnostische Sensititvität zum Nachweis von malignen Erkrankungen wie dem Prostata-, Mamma-, Ovarial- und Nierenzellkarzinom, aber auch anderer solider Tumoren wie z.B. dem kolorektalem Karzinom weiter zu steigern.

**Tabelle 20:** Kopienanzahl methylierter (met) und nicht-methylierter (unm) ***PLA2R1***-Sequenzen ohne BBPA und nach BBPA mit 50 Zyklen bei 63,0°C und 2,5 mM $MgCl_2$-Konzentration in normalen Epithelzellen der Prostata (PrEC), benigner Prostatahyperplasie-Zelllinie (BPH-1) und maligenen Prostatazelllinien LNCaP, PC-3 und DU-145. Nach BBPA wurden 1 µl der 25 µl-PCR-Ansätze in die dPCR eingesetzt, weshalb die Werte nach BBPA zum Vergleich mit 25 multipliziert wurden. Die Bezeichnungen PrEC 0x, BPH-1 0x, LNCaP 0x, PC-3 0x und DU-145 0x in der ersten Spalte zeigen die Daten ohne BBPA und die Bezeichnungen PrEC 50x, BPH-1 50x, LNCaP 50x, PC-3 50x und DU-145 50x nach 50 Zyklen in der BBPA.

| Probe | Target | Kopien/ 20 µL Well | 1:25 | Positive | Negative | Ch1+Ch2+ | Ch1+Ch2- | Ch1-Ch2+ | Ch1-Ch2- | Akzeptierte Tröpfchen | Fraktionale Häufigkeit | Poisson Fraktionale Häufigkeit Min | Poisson Fraktionale Häufigkeit Max |
|---|---|---|---|---|---|---|---|---|---|---|---|---|---|
| PrEC Ox | PLA2R1 met | 34 | 34 | 18 | 12233 | 0 | 18 | 813 | 11420 | 12251 | 2,1 | 1,1 | 3,1 |
| PrEC Ox | PLA2R1 unm | 1620 | 1620 | 813 | 11438 | | | | | | | | |
| BPH-10x | PLA2R1 met | 270 | 270 | 157 | 13568 | 16 | 141 | 1632 | 11936 | 13725 | 8,3 | 7 | 9.5 |
| BPH-10x | PLA2R1 unm | 3000 | 3000 | 1648 | 12077 | | | | | | | | |
| LNCaP 0x | PLA2R1 met | 1660 | 1660 | 961 | 13172 | 9 | 952 | 116 | 13056 | 14133 | 88,8 | 86,9 | 90,7 |
| LNCaP 0x | PLA2R1 unm | 210 | 210 | 125 | 14008 | | | | | | | | |
| PC-30x | PLA2R1 met | 1178 | 1178 | 623 | 12131 | 38 | 585 | 872 | 11259 | 12754 | 40,4 | 37,9 | 42,8 |
| PC-30x | PLA2R1 unm | 1740 | 1740 | 910 | 11844 | | | | | | | | |
| DU-145 0x | PLA2R1 met | 3280 | 3280 | 1363 | 9117 | 0 | 1363 | 6 | 9111 | 10480 | 99,59 | 99,25 | 99,93 |
| DU-145 0x | PLA2R1 unm | 14 | 14 | 6 | 10474 | | | | | | | | |
| PrEC 50x | PLA2R1 met | 0 | 0 | 0 | 12402 | 0 | 0 | 12269 | 133 | 12402 | 0 | 0 | 0 |
| PrEC 50x | PLA2R1 unm | 106800 | 2670000 | 12269 | 133 | | | | | | | | |

(fortgesetzt)

| Probe | Target | Kopien/ 20 µL Well | 1:25 | Positive | Negative | Ch1+Ch2+ | Ch1+Ch2- | Ch1- Ch2+ | Ch1- Ch2- | Akzeptierte Tröpfchen | Fraktionale Häufigkeit | Poisson Fraktionale Häufigkeit Min | Poisson Fraktionale Häufigkeit Max |
|---|---|---|---|---|---|---|---|---|---|---|---|---|---|
| BPH-150x | PLA2R1 met | 0 | 0 | 0 | 11839 | 0 | 0 | 11690 | 149 | 11839 | 0 | 0 | 0 |
| BPH-1 50x | PLA2R1 unm | 103000 | 2575000 | 11690 | 149 | | | | | | | | |
| LNCaP 50x | PLA2R1 met | 115400 | 2885000 | 11674 | 87 | 140 | 11534 | 0 | 87 | 11761 | 99,757 | 99,715 | 99,798 |
| LNCaP 50x | PLA2R1 unm | 282 | 7050 | 140 | 11621 | | | | | | | | |
| PC-3 50x | PLA2R1 met | 72600 | 1815000 | 11867 | 566 | 11858 | 9 | 229 | 337 | 12433 | 46,3 | 45,3 | 47,3 |
| PC-3 50x | PLA2R1 unm | 84200 | 2105000 | 12087 | 346 | | | | | | | | |
| DU-145 50x | PLA2R1 met | 186000 | 4650000 | 13181 | 5 | 0 | 13181 | 0 | 5 | 13186 | 100 | 100 | 100 |
| DU-145 50x | PLA2R1 unm | 0 | 0 | 0 | 13186 | | | | | | | | |

**Tabelle 21:** Kopienanzahl methylierter (M) und nicht-methylierter (U) ***RASSF1A***-DNA-Fragmente nach 15 Zyklen Prä-Amplifikation bei steigenden Annealingtemperaturen (50-60°C) in Abhängigkeit der $MgCl_2$-Konzentrationen (1,5 mM-8,0 mM) mit dem RASSF1A-Primerpaar 117bp (SEQ ID: 3 und 4) und RASSF1A-Primerpaar 124bp (SEQ ID: 61 und 62) und anschließender dPCR.

| Tm in °C | R-117 | | | | | | | | | | | |
|---|---|---|---|---|---|---|---|---|---|---|---|---|
| | **M** | **U** | **M** | **U** | **M** | **U** | **M** | **U** | **M** | **U** | **M** | **U** |
| 60 | 44 | 0 | 1580 | 0 | 868 | 454 | 3800 | 402 | 2520 | 270 | 6060 | 300 |
| 59,2 | 130 | 3,2 | 1960 | 0 | 942 | 310 | 4460 | 5 | 3520 | 26 | 5340 | 38 |
| 58 | 664 | 104 | 3020 | 118 | 456 | 272 | 3360 | 92 | 2680 | 184 | 8080 | 134 |
| 56,1 | 2800 | 1,8 | 1016 | 46 | 476 | 224 | 1978 | 532 | 1352 | 788 | 4780 | 1920 |
| 53,8 | 1724 | 40 | 392 | 920 | 90 | 194 | 628 | 860 | 980 | 1258 | 1892 | 1960 |
| 51,9 | 1142 | 706 | 184 | 572 | 34 | 48 | 78 | 366 | 158 | 258 | 566 | 680 |
| 50,7 | 1082 | 776 | 70 | 344 | 9.6 | 28 | 19.4 | 64 | 52 | 78 | 56 | 280 |
| 50 | 1014 | 1336 | 74 | 154 | 2.6 | 9.2 | 10.4 | 44 | 22.8 | 30 | 76 | 126 |
| **$MgCl_2$** | **1,5 mM** | | **2,5 mM** | | **3,5 mM** | | **4,5 mM** | | **6,0 mM** | | **8,0 mM** | |
| Tm in °C | R-124 | | | | | | | | | | | |
| | **M** | **U** | **M** | **U** | **M** | **U** | **M** | **U** | **M** | **U** | **M** | **U** |
| 60 | 292 | 0 | 2240 | 168 | 4040 | 122 | 6600 | 0 | 9460 | 0 | 4640 | 0 |
| 59,2 | 1232 | 0 | 7580 | 120 | 5060 | 8.4 | 4840 | 0 | 11720 | 0 | 4760 | 0 |
| 58 | 2230 | 1,6 | 5380 | 8 | 5380 | 0 | 8240 | 0 | 8340 | 20 | 7600 | 9 |
| 56,1 | 4840 | 0 | 5920 | 244 | 5220 | 10,6 | 1466 | 0 | 5480 | 0 | 8100 | 0 |
| 53,8 | 5900 | 14 | 1470 | 514 | 10780 | 0 | 8580 | 720 | 5920 | 3 | 5560 | 474 |
| 51,9 | 5220 | 20 | 470 | 124 | 7500 | 48 | 7620 | 58 | 5460 | 330 | 4200 | 78 |
| 50,7 | 5180 | 30 | 202 | 140 | 4960 | 138 | 2430 | 234 | 3180 | 616 | 3840 | 144 |
| 50 | 3000 | 290 | 136 | 82 | 4720 | 298 | 2880 | 472 | 1906 | 372 | 3760 | 768 |
| **$MgCl_2$** | **1,5 mM** | | **2,5 mM** | | **3,5 mM** | | **4,5 mM** | | **6,0 mM** | | **8,0 mM** | |

**Tabelle 22:** Kopienanzahl methylierter (M) und nicht-methylierter (U) ***GSTP1***-DNA-Fragmente nach 15 Zyklen Prä-Amplifikation bei steigenden Annealingtemperaturen (50-60°C) in Abhängigkeit der $MgCl_2$-Konzentrationen (1,5 mM-8,0 mM) mit dem GSTP1-Primerpaar 114bp (SEQ ID: 91und 92), GSTP1-Primerpaar 116bp (SEQ ID: 93 und 94), GSTP1-Primerpaar 129bp (SEQ ID: 95 und 96) und GSTP1-Primerpaar 132bp (SEQ ID: 97 und 98) und anschließender dPCR.

| Tm in °C | GSTP-114bp (3 CpG-Stellen) | | | | | | | | | | | |
|---|---|---|---|---|---|---|---|---|---|---|---|---|
| | **M** | **U** | **M** | **U** | **M** | **U** | **M** | **U** | **M** | **U** | **M** | **U** |
| 60 | 764 | 1,8 | 9660 | 0 | 11180 | 26 | 17220 | 124 | 8080 | 12 | 17140 | 130 |
| 59,2 | 726 | 1,6 | 7820 | 14 | 17660 | 30 | 19560 | 44 | 6740 | 12 | 14980 | 36 |
| 58 | 2380 | 12,6 | 10440 | 7,2 | 12640 | 92 | 17640 | 64 | 16180 | 146 | 6480 | 56 |
| 56,1 | 6640 | 0 | 14820 | 30 | 21760 | 198 | 17660 | 624 | 19600 | 896 | 14060 | 1516 |
| 53,8 | 8520 | 13,6 | 15100 | 938 | 11440 | 1068 | 13940 | 2500 | 8680 | 2560 | 7960 | 1960 |
| 51,9 | 9200 | 200 | 12140 | 1580 | 7520 | 2560 | 5460 | 1860 | 2900 | 1780 | 960 | 666 |
| 50,7 | 8680 | 390 | 7140 | 3740 | 4140 | 2500 | 2700 | 1126 | 868 | 824 | 122 | 100 |
| 50 | 6780 | 678 | 6740 | 1680 | 2620 | 3160 | 2360 | 926 | 506 | 466 | 460 | 254 |
| **$MgCl_2$** | **1,5 mM** | | **2,5 mM** | | **3,5 mM** | | **4,5 mM** | | **6,0 mM** | | **8,0 mM** | |
| Tm in °C | GSTP-116bp (3 CpG-Stellen) | | | | | | | | | | | |
| | **M** | **U** | **M** | **U** | **M** | **U** | **M** | **U** | **M** | **U** | **M** | **U** |
| 60 | 284 | 24 | 5700 | 1,6 | 11260 | 2,8 | 16340 | 1,6 | 14960 | 252 | 7960 | 4,8 |
| 59,2 | 778 | 2,8 | 7760 | 5 | 13700 | 11,4 | 18920 | 14 | 17420 | 10,2 | 8160 | 11,4 |
| 58 | 1544 | 6,8 | 7560 | 5 | 22640 | 16,6 | 16740 | 4,4 | 17560 | 17,2 | 16220 | 6,2 |
| 56,1 | 4260 | 6 | 19880 | 12,8 | 25420 | 100 | 20640 | 390 | 17980 | 376 | 12640 | 134 |
| 53,8 | 9040 | 9,2 | 23020 | 108 | 24540 | 774 | 18480 | 1760 | 23280 | 1014 | 13560 | 582 |
| 51,9 | 10560 | 262 | 11520 | 776 | 16740 | 2400 | 15620 | 5280 | 15600 | 4560 | 8740 | 2100 |
| 50,7 | 5400 | 46 | 16120 | 1028 | 12960 | 4800 | 10520 | 5680 | 13500 | 4060 | 6200 | 2460 |
| 50 | 9200 | 86 | 14800 | 2300 | 14900 | 5100 | 14720 | 4800 | 8480 | 3660 | 4760 | 1800 |
| **$MgCl_2$** | **1,5 mM** | | **2,5 mM** | | **3,5 mM** | | **4,5 mM** | | **6,0 mM** | | **8,0 mM** | |
| Tm in °C | GSTP-129bp (4 CpG-Stellen) | | | | | | | | | | | |
| | **M** | **U** | **M** | **U** | **M** | **U** | **M** | **U** | **M** | **U** | | |
| 60 | 578 | 14 | 8940 | 16 | 15220 | 0 | 19040 | 5.4 | 15140 | 24 | | |

(fortgesetzt)

| Tm in °C | GSTP-129bp (4 CpG-Stellen) | | | | | | | | | |
|---|---|---|---|---|---|---|---|---|---|---|
| | **M** | **U** | **M** | **U** | **M** | **U** | **M** | **U** | **M** | **U** |
| 59,2 | 450 | 4.6 | 7420 | 22 | 15900 | 204 | 26660 | 46 | 18400 | 15.4 |
| 58 | 1462 | 9.4 | 10660 | 14 | 22880 | 10 | 35400 | 4.4 | 15620 | 34 |
| 56,1 | 5480 | 10.8 | 24640 | 6 | 21860 | 16 | 25800 | 44 | 24880 | 30 |
| 53,8 | 12020 | 11.4 | 17060 | 22 | 15820 | 170 | 27780 | 350 | 16460 | 216 |
| 51,9 | 8740 | 22 | 20160 | 254 | 20480 | 370 | 21740 | 1068 | 18740 | 234 |
| 50,7 | 8720 | 7.8 | 15960 | 208 | 19100 | 544 | 22980 | 1144 | 16060 | 474 |
| 50 | 9220 | 110 | 19480 | 312 | 19520 | 892 | 16900 | 962 | 10440 | 516 |
| **$MgCl_2$** | **1,5 mM** | | **2,5 mM** | | **3,5 mM** | | **6,0 mM** | | **8,0 mM** | |
| Tm in °C | GSTP-132bp (3 CpG-Stellen, eine CPG direkt am 3'-Ende) | | | | | | | | | |
| | **M** | **U** | **M** | **U** | **M** | **U** | **M** | **U** | **M** | **U** |
| 60 | 98 | 6 | 5420 | 1.6 | 8640 | 3 | 15740 | 380 | 11080 | 8.4 |
| 59,2 | 264 | 4.8 | 4860 | 10.2 | 13100 | 0 | 12120 | 0 | 9260 | 9.6 |
| 58 | 988 | 3.2 | 8520 | 282 | 19520 | 4.8 | 17740 | 14 | 19060 | 20 |
| 56,1 | 4160 | 24 | 14040 | 7.8 | 13580 | 14 | 22340 | 4.2 | 12020 | 40 |
| 53,8 | 4920 | 2.8 | 20460 | 94 | 19300 | 66 | 18540 | 174 | 11700 | 422 |
| 51,9 | 6820 | 16 | 11360 | 100 | 18440 | 360 | 13400 | 718 | 8260 | 1100 |
| 50,7 | 6580 | 18 | 11820 | 314 | 12920 | 808 | 14760 | 1002 | 6820 | 756 |
| 50 | 5020 | 82 | 10820 | 230 | 14660 | 1182 | 7380 | 334 | 5520 | 1000 |
| **$MgCl_2$** | **1,5 mM** | | **2,5 mM** | | **3,5 mM** | | **4,5 mM** | | **6,0 mM** | |

**Tabelle 23:** Kopienanzahl methylierter (M) und nicht-methylierte ***PLA2R1***-DNA-Fragmente (U) [Copies/20 µl Well), Anteil M/U (Ratio) und relativer Anteil M/M+U (Fractional Abundance) in Proben mit 3000 (Bahn 1), 20 (Bahn 2), 10 (Bahn 3), 5 (Bahn 4) und keiner Kopie methylierter PLA2R1-DNA-Fragmente (Bahn 5) und jeweils einem Anteil von 70.000 Kopien (70K/0-3000), 175.000 (170K/0-3000), 350.000 (350K/0-3000) und 700.000 (700K/0-3000) nicht-methylierter PLA2R1-DNA-Fragmente mit dem Primerpaar 168 bp (SEQ ID: 1 und 2) ohne Prä-Amplifikation. NTC: Non-Template-Negativkontrolle.

| Probe | Target | Kopien/ 20µLWell | Positive | Negative | Ch1+Ch2+ | Ch1+Ch2- | Ch1- Ch2+ | Ch1- Ch2- | **Akzeptierte Tröpfchen** | Verhältnis | **Fraktionale Häufigkeit** |
|---|---|---|---|---|---|---|---|---|---|---|---|
| 70K/0 | M | 0 | 0 | 14071 | 0 | 0 | 13369 | 702 | 14071 | 0 | 0 |
| | U | 70600 | 13369 | 702 | | | | | | | |
| 70K/5 | M | 5.4 | 3 | 13198 | 3 | 0 | 12584 | 614 | 13201 | 7,00E-05 | 0.007 |
| | U | 72200 | 12587 | 614 | | | | | | | |
| 70K/10 | M | 10 | 6 | 14086 | 4 | 2 | 13438 | 648 | 14092 | 0.00014 | 0.014 |
| | U | 72400 | 13442 | 650 | | | | | | | |
| 70K/20 | M | 28 | 13 | 11255 | 12 | 1 | 10718 | 537 | 11268 | 0.00038 | 0.038 |
| | U | 71600 | 10730 | 538 | | | | | | | |
| 70K/ 3000 | M | 2780 | 1640 | 13103 | 1479 | 161 | 12415 | 688 | 14743 | 0.0413 | 3.97 |
| | U | 67200 | 13894 | 849 | | | | | | | |
| 175 K/0 | M | 0 | 0 | 13938 | 0 | 0 | 13926 | 12 | 13938 | 0 | 0 |
| | U | 166000 | 13926 | 12 | | | | | | | |
| 175 K/5 | M | 3.8 | 2 | 12554 | 2 | 0 | 12548 | 6 | 12556 | 2.1 E-05 | 0.0021 |
| | U | 180000 | 12550 | 6 | | | | | | | |
| 175K/ 10 | M | 9.4 | 5 | 12592 | 5 | 0 | 12585 | 7 | 12597 | 5.3E-05 | 0.0053 |
| | U | 176000 | 12590 | 7 | | | | | | | |
| 175K/ 20 | M | 16 | 9 | 13287 | 9 | 0 | 13286 | 1 | 13296 | 7,00E-05 | 0.007 |
| | U | 224000 | 13295 | 1 | | | | | | | |
| 175K/ 3000 | M | 1968 | 891 | 10215 | 887 | 4 | 10210 | 5 | 11106 | 0.0117 | 1.16 |
| | U | 168000 | 11097 | 9 | | | | | | | |

(fortgesetzt)

| Probe | Target | Kopien/ 20μLWell | Positive | Negative | Ch1+Ch2+ | Ch1+Ch2- | Ch1- Ch2+ | Ch1- Ch2- | **Akzeptierte Tröpfchen** | Verhältnis | **Fraktionale Häufigkeit** |
|---|---|---|---|---|---|---|---|---|---|---|---|
| 350K/0 | M | 0 | 0 | 14661 | 0 | 0 | 14661 | 0 | 14661 | 0 | 0 |
| | U | 20000000 | 14661 | 0 | | | | | | | |
| 350K/5 | M | 2 | 1 | 12059 | 1 | 0 | 12059 | 0 | 12060 | 1,00E-07 | 1,00E-05 |
| | U | 20000000 | 12060 | 0 | | | | | | | |
| 350K/ 10 | M | 0 | 0 | 12984 | 0 | 0 | 12981 | 3 | 12984 | 0 | 0 |
| | U | 198000 | 12981 | 3 | | | | | | | |
| 350K/ 20 | M | 6.4 | 3 | 10969 | 3 | 0 | 10967 | 2 | 10972 | 3.2E-05 | 0.0032 |
| | U | 202000 | 10970 | 2 | | | | | | | |
| 350K/ 3000 | M | 560 | 283 | 11757 | 283 | 0 | 11756 | 1 | 12040 | 0.0025 | 0.25 |
| | U | 222000 | 12039 | 1 | | | | | | | |
| 700K/0 | M | 0 | 0 | 11327 | 0 | 0 | 11327 | 0 | 11327 | 0 | 0 |
| | U | 20000000 | 11327 | 0 | | | | | | | |
| 700K/5 | M | 0 | 0 | 10189 | 0 | 0 | 10189 | 0 | 10189 | 0 | 0 |
| | U | 20000000 | 10189 | 0 | | | | | | | |
| 700K/ 10 | M | 0 | 0 | 9889 | 0 | 0 | 9872 | 17 | 9889 | 0 | 0 |
| | U | 150000 | 9872 | 17 | | | | | | | |
| 700K/ 20 | M | 0 | 0 | 11500 | 0 | 0 | 11499 | 1 | 11500 | 0 | 0 |
| | U | 220000 | 11499 | 1 | | | | | | | |
| 700K/ 3000 | M | 114 | 50 | 10332 | 50 | 0 | 10328 | 4 | 10382 | 0.00061 | 0.061 |
| | U | 184000 | 10378 | 4 | | | | | | | |

(fortgesetzt)

| Probe | Target | Kopien/ 20µLWell | Positive | Negative | Ch1+Ch2+ | Ch1+Ch2- | Ch1- Ch2+ | Ch1- Ch2- | **Akzeptierte Tröpfchen** | Verhältnis | **Fraktionale Häufigkeit** |
|---|---|---|---|---|---|---|---|---|---|---|---|
| NTC | M | 0 | 0 | 12186 | 0 | 0 | 1 | 12185 | 12186 | 0 | 0 |
| | U | 2 | 1 | 12185 | | | | | | | |

**Tabelle 24:** Kopienanzahl methylierter (M) und nicht-methylierte ***PLA2R1***-DNA-Fragmente (U) [Copies/20 μl Well), Anteil M/U (Ratio) und relativer Anteil M/M+U (Fractional Abundance) in Proben mit 3000 (Bahn 1), 20 (Bahn 2), 10 (Bahn 3), 5 (Bahn 4) und keiner Kopie methylierter PLA2R1-DNA-Fragmente (Bahn 5) und jeweils einem Anteil von 70.000 Kopien (70K/0-3000), 175.000 (170K/0-3000), 350.000 (350K/0-3000) und 700.000 (700K/0-3000) nicht-methylierter PLA2R1-DNA-Fragmente mit dem Primerpaar 133 bp (SEQ ID: 7 und 8) ohne Prä-Amplifikation. NTC: Non-Template-Negativkontrolle.

| Probe | Targets | Kopien/ 20μLWell | Positive | Negative | Ch1+Ch2+ | Ch1+Ch2- | Ch1- Ch2+ | Ch1- Ch2- | **Akzeptierte Tröpfchen** | Verhältnis | **Fraktionale Häufigkeit** |
|---|---|---|---|---|---|---|---|---|---|---|---|
| 70K/0 | M | 0 | 0 | 15481 | 0 | 0 | 2 | 15479 | 15481 | 0 | 0 |
| | U | 3 | 2 | 15479 | | | | | | | |
| 70K/5 | M | 1.8 | 1 | 13718 | 0 | 1 | 6 | 13712 | 13719 | 0.17 | 14 |
| | U | 10.2 | 6 | 13713 | | | | | | | |
| 70K/10 | M | 13 | 8 | 14504 | 0 | 8 | 7 | 14497 | 14512 | 1.1 | 53 |
| | U | 11.4 | 7 | 14505 | | | | | | | |
| 70K/20 | M | 24 | 13 | 12954 | 0 | 13 | 8 | 12946 | 12967 | 1.6 | 62 |
| | U | 14 | 8 | 12959 | | | | | | | |
| 70K/ 3000 | M | 3320 | 1585 | 10455 | 0 | 1585 | 9 | 10446 | 12040 | 190 | 99.47 |
| | U | 18 | 9 | 12031 | | | | | | | |
| 175 K/0 | M | 2.6 | 2 | 17436 | 0 | 2 | 27 | 17409 | 17438 | 0.07 | 7 |
| | U | 36 | 27 | 17411 | | | | | | | |
| 175 K/5 | M | 1.4 | 1 | 17244 | 0 | 1 | 1 | 17243 | 17245 | 1 | 50 |
| | U | 1.4 | 1 | 17244 | | | | | | | |
| 175K/ 10 | M | 8 | 5 | 14708 | 0 | 5 | 26 | 14682 | 14713 | 0.19 | 16 |
| | U | 42 | 26 | 14687 | | | | | | | |
| 175K/ 20 | M | 22 | 12 | 12577 | 0 | 12 | 14 | 12563 | 12589 | 0.9 | 46 |
| | U | 26 | 14 | 12575 | | | | | | | |
| 175K/ 3000 | M | 3020 | 1749 | 12789 | 1 | 1748 | 21 | 12768 | 14538 | 85 | 98.83 |
| | U | 36 | 22 | 14516 | | | | | | | |

(fortgesetzt)

| Probe | Targets | Kopien/ 20µLWell | Positive | Negative | Ch1+Ch2+ | Ch1+Ch2- | Ch1- Ch2+ | Ch1- Ch2- | **Akzeptierte Tröpfchen** | Verhältnis | **Fraktionale Häufigkeit** |
|---|---|---|---|---|---|---|---|---|---|---|---|
| 350K/0 | M | 32 | 20 | 14348 | 0 | 20 | 19 | 14329 | 14368 | 1.1 | 51 |
| | U | 32 | 19 | 14349 | | | | | | | |
| 350K/5 | M | 142 | 71 | 11757 | 0 | 71 | 28 | 11729 | 11828 | 2.5 | 72 |
| | U | 56 | 28 | 11800 | | | | | | | |
| 350K/ 10 | M | 16 | 8 | 11558 | 0 | 8 | 38 | 11520 | 11566 | 0.21 | 17 |
| | U | 78 | 38 | 11528 | | | | | | | 18 |
| 350K/ 20 | M | 20 | 11 | 13597 | 0 | 11 | 50 | 13547 | 13608 | 0.22 | |
| | U | 86 | 50 | 13558 | | | | | | | |
| 350K/ 3000 | M | 3160 | 1553 | 10816 | 0 | 1553 | 40 | 10776 | 12369 | 41 | 97.6 |
| | U | 76 | 40 | 12329 | | | | | | | |
| 700K/0 | M | 0 | 0 | 12883 | 0 | 0 | 62 | 12821 | 12883 | 0 | 0 |
| | U | 114 | 62 | 12821 | | | | | | | |
| 700K/5 | M | 0 | 0 | 11231 | 0 | 0 | 66 | 11165 | 11231 | 0 | 0 |
| | U | 138 | 66 | 11165 | | | | | | | |
| 700K/ 10 | M | 6 | 3 | 11863 | 0 | 3 | 40 | 11823 | 11866 | 0.07 | 7 |
| | U | 80 | 40 | 11826 | | | | | | | |
| 700K/ 20 | M | 8 | 4 | 11745 | 0 | 4 | 75 | 11670 | 11749 | 0.05 | 5 |
| | U | 150 | 75 | 11674 | 0 | | | | | | |
| 700K/ 3000 | M | 3140 | 1360 | 9515 | 1 | 1359 | 84 | 9431 | 10875 | 17 | 94.5 |
| | U | 184 | 85 | 10790 | | | | | | | |

(fortgesetzt)

| Probe | Targets | Kopien/ 20µLWell | Positive | Negative | Ch1+Ch2+ | Ch1+Ch2- | Ch1- Ch2+ | Ch1- Ch2- | **Akzeptierte Tröpfchen** | Verhältnis | **Fraktionale Häufigkeit** |
|---|---|---|---|---|---|---|---|---|---|---|---|
| NTC | M | 0 | 0 | 16702 | 0 | 0 | 1 | 16701 | 16702 | 0 | 0 |
| | U | 1.4 | 1 | 16701 | 0 | 0 | 1 | 16701 | 16702 | | |

**Tabelle 25:** Kopienanzahl methylierter (M) und nicht-methylierte ***PLA2R1***-DNA-Fragmente (U) [Copies/20 μl Well), Anteil M/U (Ratio) und relativer Anteil M/M+U (Fractional Abundance) in Proben mit keiner, 5, 10, 20 und 3000 Kopien methylierter PLA2R1-DNA-Fragmente und jeweils einem Anteil von 70.000 Kopien (70K/0-3000), 175.000 (170K/0-3000), 350.000 (350K/0-3000) und 700.000 (700K/0-3000) nicht-methylierter PLA2R1-DNA-Fragmente mit dem Primerpaar 168 bp (SEQ ID: 1 und 2, angezeigt in rechter Spalte), Primerpaar 161 bp (SEQ ID: 53 und 10) und Primerpaar 150 bp (SEQ ID: 53 und 9) nach 15 Zyklen Prä-Amplifikation mit 2,5 mM $MgCl_2$-Konzentration und 63°C Annealingtemperatur und anschließender dPCR. NTC: Non-Template-Negativkontrolle.

| **Probe** | **Target** | Kopien/ 20μL Well | Positive | Negative | **Ch1+Ch2 +** | **Ch1+Ch2-** | **Ch1- Ch2+** | **Ch1- Ch2-** | **Akzeptierte Tröpfchen** | Verhältnis | **Fraktionale Häufigkeit** | **PLA2R 1** |
|---|---|---|---|---|---|---|---|---|---|---|---|---|
| 70K/0 | M | 0 | 0 | 13332 | 0 | 0 | 1333 1 | 1 | 13332 | 0 | 0 | 168bp |
| | U | 224000 | 13331 | 1 | | | | | | | | |
| 70K/5 | M | 0 | 0 | 10775 | 0 | 0 | 1077 5 | 0 | 10775 | 0 | 0 | |
| | U | 20000000 | 10775 | 0 | | | | | | | | |
| 70K/10 | M | 0 | 0 | 11431 | 0 | 0 | 1143 1 | 0 | 11431 | 0 | 0 | |
| | U | 20000000 | 11431 | 0 | | | | | | | | |
| 70K/20 | M | 1.8 | 1 | 13839 | 1 | 0 | 1382 4 | 15 | 13840 | 1.1 E-05 | 0.0011 | |
| | U | 160000 | 13825 | 15 | | | | | | | | |
| 70K/ 3000 | M | 5880 | 2845 | 10030 | 2845 | 0 | 1003 0 | 0 | 12875 | 0.0002 9 | 0.029 | |
| | U | 20000000 | 12875 | 0 | | | | | | | | |
| 175 K/0 | M | 0 | 0 | 12634 | 0 | 0 | 1263 2 | 2 | 12634 | 0 | 0 | |
| | U | 206000 | 12632 | 2 | | | | | | | | |
| 175 K/5 | M | 0 | 0 | 12656 | 0 | 0 | 1265 6 | 0 | 12656 | 0 | 0 | |
| | U | 20000000 | 12656 | 0 | | | | | | | | |
| 175K/ 10 | M | 0 | 0 | 12889 | 0 | 0 | 1288 8 | 1 | 12889 | 0 | 0 | |
| | U | 222000 | 12888 | 1 | | | | | | | | |
| 175K/ 20 | M | 0 | 0 | 12562 | 0 | 0 | 1256 2 | 0 | 12562 | 0 | 0 | |
| | U | 20000000 | 12562 | 0 | | | | | | | | |
| 175K/ 300 0 | M | 11.2 | 6 | 12622 | 6 | 0 | 1262 2 | 0 | 12628 | 6,00E-07 | 6,00E-05 | |

(fortgesetzt)

| Probe | Target | Kopien/ 20μL Well | Positive | Negative | Ch1+Ch2 + | Ch1+Ch2- | Ch1- Ch2+ | Ch1- Ch2- | Akzeptierte Tröpfchen | Verhältnis | Fraktionale Häufigkeit | PLA2R 1 |
|---|---|---|---|---|---|---|---|---|---|---|---|---|
| | U | 20000000 | 12628 | 0 | | | | | | | | |
| 350K/0 | M | 0 | 0 | 13868 | 0 | 0 | 1386 8 | 0 | 13868 | 0 | 0 | |
| | U | 20000000 | 13868 | 0 | | | | | | | | |
| 350K/5 | M | 0 | 0 | 13080 | 0 | 0 | 1307 7 | 3 | 13080 | 0 | 0 | |
| | U | 198000 | 13077 | 3 | | | | | | | | |
| 350K/ 10 | M | 0 | 0 | 14193 | 0 | 0 | 1418 8 | 5 | 14193 | 0 | 0 | |
| | U | 188000 | 14188 | 5 | | | | | | | | |
| 350K/ 20 | M | 0 | 0 | 14508 | 0 | 0 | 1450 3 | 5 | 14508 | 0 | 0 | |
| | U | 188000 | 14503 | 5 | | | | | | | | |
| 350K/ 300 0 | M | 0 | 0 | 13295 | 0 | 0 | 1329 5 | 0 | 13295 | 0 | 0 | |
| | U | 20000000 | 13295 | 0 | | | | | | | | |
| 700K/0 | M | 0 | 0 | 14732 | 0 | 0 | 1472 9 | 3 | 14732 | 0 | 0 | |
| | U | 200000 | 14729 | 3 | | | | | | | | |
| 700K/5 | M | 0 | 0 | 13799 | 0 | 0 | 1379 9 | 0 | 13799 | 0 | 0 | |
| | U | 20000000 | 13799 | 0 | | | | | | | | |
| 700K/ 10 | M | 0 | 0 | 13579 | 0 | 0 | 1357 9 | 0 | 13579 | 0 | 0 | |
| | U | 20000000 | 13579 | 0 | | | | | | | | |
| 700K/ 20 | M | 0 | 0 | 10322 | 0 | 0 | 1032 1 | 1 | 10322 | 0 | 0 | |
| | U | 218000 | 10321 | 1 | | | | | | | | |
| 700K/ 300 0 | M | 0 | 0 | 12072 | 0 | 0 | 1207 1 | 1 | 12072 | 0 | 0 | |

(fortgesetzt)

| Probe | Target | Kopien/ 20μL Well | Positive | Negative | Ch1+Ch2 + | Ch1+Ch2- | Ch1- Ch2+ | Ch1- Ch2- | Akzeptierte Tröpfchen | Verhältnis | Fraktionale Häufigkeit | PLA2R 1 |
|---|---|---|---|---|---|---|---|---|---|---|---|---|
| | U | 222000 | 12071 | 1 | | | | | | | | |
| 70K/0 | M | 252 | 144 | 13392 | 17 | 127 | 1400 | 1199 2 | 13536 | 0.097 | 8.8 | 161bp |
| | U | 2600 | 1417 | 12119 | | | | | | | | |
| 70K/5 | M | 264 | 142 | 12622 | 62 | 80 | 5878 | 6744 | 12764 | 0.0179 | 1.76 | |
| | U | 14740 | 5940 | 6824 | | | | | | | | |
| 70K/10 | M | 174 | 99 | 13355 | 36 | 63 | 4202 | 9153 | 13454 | 0.0195 | 1.91 | |
| | U | 8900 | 4238 | 9216 | | | | | | | | |
| 70K/20 | M | 1180 | 668 | 12992 | 262 | 406 | 7252 | 5740 | 13660 | 0.0628 | 5.91 | |
| | U | 18800 | 7514 | 6146 | | | | | | | | |
| 70K/ 3000 | M | 95000 | 14721 | 265 | 17 | 14704 | 0 | 265 | 14986 | 3600 | 99.972 | |
| | U | 26 | 17 | 14969 | | | | | | | | |
| 175 K/0 | M | 38 | 23 | 14359 | 5 | 18 | 1270 | 1308 9 | 14382 | 0.017 | 1.7 | |
| | U | 2180 | 1275 | 13107 | | | | | | | | |
| 175 K/5 | M | 344 | 187 | 12734 | 96 | 91 | 5027 | 7707 | 12921 | 0.0289 | 2.81 | |
| | U | 11880 | 5123 | 7798 | | | | | | | | |
| 175K/ 10 | M | 476 | 263 | 12886 | 196 | 67 | 9772 | 3114 | 13149 | 0.0142 | 1.4 | |
| | U | 33400 | 9968 | 3181 | | | | | | | | |
| 175K/ 20 | M | 1382 | 873 | 14429 | 535 | 338 | 8245 | 6184 | 15302 | 0.0689 | 6.44 | |
| | U | 20060 | 8780 | 6522 | | | | | | | | |
| 175K/ 300 0 | M | 132800 | 13824 | 49 | 743 | 13081 | 2 | 47 | 13873 | 102 | 99.03 | |
| | U | 1298 | 745 | 13128 | | | | | | | | |
| 350K/0 | M | 0 | 0 | 14731 | 0 | 0 | 2731 | 1200 0 | 14731 | 0 | 0 | |

(fortgesetzt)

| Probe | Target | Kopien/ 20μL Well | Positive | Negative | Ch1+Ch2 + | Ch1+Ch2- | Ch1- Ch2+ | Ch1- Ch2- | Akzeptierte Tröpfchen | Verhältnis | Fraktionale Häufigkeit | PLA2R 1 |
|---|---|---|---|---|---|---|---|---|---|---|---|---|
| | U | 4820 | 2731 | 12000 | | | | | | | | |
| 350K/5 | M | 48 | 32 | 15565 | 18 | 14 | 5530 | 1003 5 | 15597 | 0.0047 | 0.47 | |
| | U | 10340 | 5548 | 10049 | | | | | | | | |
| 350K/ 10 | M | 172 | 116 | 15774 | 75 | 41 | 7280 | 8494 | 15890 | 0.0118 | 1.17 | |
| | U | 14620 | 7355 | 8535 | | | | | | | | |
| 350K/ 20 | M | 994 | 578 | 13382 | 552 | 26 | 1319 3 | 189 | 13960 | 0.0101 | 1 | |
| | U | 98200 | 13745 | 215 | | | | | | | | |
| 350K/ 300 0 | M | 104800 | 15203 | 179 | 2323 | 12880 | 11 | 168 | 15382 | 27.1 | 96.44 | |
| | U | 3880 | 2334 | 13048 | | | | | | | | |
| 700K/0 | M | 50 | 28 | 13360 | 23 | 5 | 8016 | 5344 | 13388 | 0.0023 | 0.23 | |
| | U | 21580 | 8039 | 5349 | | | | | | | | |
| 700K/5 | M | 0 | 0 | 13344 | 0 | 0 | 14 | 1333 0 | 13344 | 0 | 0 | |
| | U | 24 | 14 | 13330 | | | | | | | | |
| 700K/ 10 | M | 312 | 155 | 11614 | 154 | 1 | 1076 4 | 850 | 11769 | 0.005 | 0.5 | |
| | U | 61800 | 10918 | 851 | | | | | | | | |
| 700K/ 20 | M | 680 | 417 | 14213 | 417 | 0 | 1378 3 | 430 | 14630 | 0.0082 | 0.81 | |
| | U | 83000 | 14200 | 430 | | | | | | | | |
| 700K/ 300 0 | M | 121600 | 13316 | 76 | 9932 | 3384 | 49 | 27 | 13392 | 3.78 | 79.1 | |
| | U | 32180 | 9981 | 3411 | | | | | | | | |
| 70K/0 | M | 1.8 | 1 | 13056 | 0 | 1 | 2201 | 1085 5 | 13057 | 0.0004 | 0.04 | 150bp |

(fortgesetzt)

| Probe | Target | Kopien/ 20μL Well | Positive | Negative | Ch1+Ch2 + | Ch1+Ch2- | Ch1- Ch2+ | Ch1- Ch2- | Akzeptierte Tröpfchen | Verhältnis | Fraktionale Häufigkeit | PLA2R 1 |
|---|---|---|---|---|---|---|---|---|---|---|---|---|
| | U | 4340 | 2201 | 10856 | | | | | | | | |
| 70K/5 | M | 1126 | 597 | 12181 | 84 | 513 | 2285 | 9896 | 12778 | 0.233 | 18.9 | |
| | U | 4820 | 2369 | 10409 | | | | | | | | |
| 70K/10 | M | 2214 | 1176 | 11914 | 238 | 938 | 2937 | 8977 | 13090 | 0.339 | 25.3 | |
| | U | 6540 | 3175 | 9915 | | | | | | | | |
| 70K/20 | M | 1954 | 917 | 10593 | 83 | 834 | 1273 | 9320 | 11510 | 0.66 | 39.8 | |
| | U | 2940 | 1356 | 10154 | | | | | | | | |
| 70K/ 3000 | M | 208000 | 13610 | 2 | 5 | 13605 | 0 | 2 | 13612 | 24000 | 100 | |
| | U | 8.6 | 5 | 13607 | | | | | | | | |
| 175 K/0 | M | 336 | 208 | 14436 | 78 | 130 | 7291 | 7145 | 14644 | 0.0204 | 2 | |
| | U | 16460 | 7369 | 7275 | | | | | | | | |
| 175K/5 | M | 26 | 15 | 13524 | 0 | 15 | 7 | 1351 7 | 13539 | 2.1 | 68 | |
| | U | 12.2 | 7 | 13532 | | | | | | | | |
| 175K/ 10 | M | 3140 | 1872 | 13111 | 740 | 1132 | 6118 | 6993 | 14983 | 0.218 | 17.9 | |
| | U | 14400 | 6858 | 8125 | | | | | | | | |
| 175K/ 20 | M | 4360 | 2416 | 11841 | 445 | 1971 | 3608 | 8233 | 14257 | 0.555 | 35.7 | |
| | U | 7860 | 4053 | 10204 | | | | | | | | |
| 175K/ 300 0 | M | 196000 | 12343 | 3 | 29 | 12314 | 0 | 3 | 12346 | 3500 | 99.972 | |
| | U | 56 | 29 | 12317 | | | | | | | | |
| 350K/0 | M | 13.8 | 8 | 13569 | 3 | 5 | 8570 | 4999 | 13577 | 0.0005 9 | 0.059 | |
| | U | 23480 | 8573 | 5004 | | | | | | | | |
| 350K/5 | M | 740 | 398 | 12472 | 221 | 177 | 7918 | 4554 | 12870 | 0.0314 | 3.04 | |

(fortgesetzt)

| Probe | Target | Kopien/ 20μL Well | Positive | Negative | Ch1+Ch2 + | Ch1+Ch2- | Ch1-Ch2+ | Ch1-Ch2- | Akzeptierte Tröpfchen | Verhältnis | Fraktionale Häufigkeit | PLA2R 1 |
|---|---|---|---|---|---|---|---|---|---|---|---|---|
| | U | 23540 | 8139 | 4731 | | | | | | | | |
| 350K/ 10 | M | 1278 | 578 | 10354 | 293 | 285 | 5884 | 4470 | 10932 | 0.065 | 6.13 | |
| | U | 19580 | 6177 | 4755 | | | | | | | | |
| 350K/ 20 | M | 4300 | 2065 | 10286 | 1093 | 972 | 6630 | 3656 | 12351 | 0.186 | 15.7 | |
| | U | 23100 | 7723 | 4628 | | | | | | | | |
| 350K/ 300 0 | M | 222000 | 12675 | 1 | 0 | 12675 | 0 | 1 | 12676 | 100 | 100 | |
| | U | 0 | 0 | 12676 | | | | | | | | |
| 700K/0 | M | 6.6 | 4 | 14339 | 3 | 1 | 1146 9 | 2870 | 14343 | 0.0001 7 | 0.017 | |
| | U | 37840 | 11472 | 2871 | | | | | | | | |
| 700K/5 | M | 458 | 281 | 14297 | 202 | 79 | 1233 8 | 1959 | 14578 | 0.0099 | 0.98 | |
| | U | 46300 | 12540 | 2038 | | | | | | | | |
| 700K/ 10 | M | 1396 | 801 | 13102 | 617 | 184 | 1128 5 | 1817 | 13903 | 0.0306 | 2.97 | |
| | U | 45620 | 11902 | 2001 | | | | | | | | |
| 700K/ 20 | M | 5300 | 2600 | 10289 | 1756 | 844 | 8252 | 2037 | 12889 | 0.15 | 13.1 | |
| | U | 35260 | 10008 | 2881 | | | | | | | | |
| 700K/ 3000 | M | 196000 | 12054 | 3 | 271 | 11783 | 0 | 3 | 12057 | 370 | 99.73 | |
| | U | 534 | 271 | 11786 | | | | | | | | |
| NTC | M | 0 | 0 | 11564 | 0 | 0 | 1 | 1156 3 | 11564 | 0 | 0 | |
| | U | 2 | 1 | 11563 | | | | | | | | |

**Tabelle 26:** Kopienanzahl methylierter (M) und nicht-methylierte ***PLA2R1***-DNA-Fragmente (U) [Copies/20 µl Well), Anteil M/U (Ratio) und relativer Anteil M/M+U (Fractional Abundance) in Proben mit keiner, 5, 10, 20 und 3000 Kopien methylierter PLA2R1-DNA-Fragmente und jeweils einem Anteil von 70.000 Kopien (70K/0-3000), 175.000 (170K/0-3000), 350.000 (350K/0-3000) und 700.000 (700K/0-3000) nicht-methylierter PLA2R1-DNA-Fragmente mit dem Primerpaar 168 bp (SEQ ID: 1 und 2, angezeigt in rechter Spalte), Primerpaar 161 bp (SEQ ID: 53 und 10) und Primerpaar 150 bp (SEQ ID: 53 und 9) nach 50 Zyklen Prä-Amplifikation mit 2,5 mM $MgCl_2$-Konzentration und 63°C Annealingtemperatur und anschließender dPCR. NTC: Non-Template-Negativkontrolle.

| | **Probe** | **Target** | Kopien/ 20µLWell | Positive | Negative | **Ch1+Ch2+** | **Ch1+Ch2-** | **Ch1- Ch2+** | **Ch1- Ch2-** | **Akzeptierte Tröpfchen** | Verhältnis | **Fraktionale Häufigkeit** | **PLA2R1** |
|---|---|---|---|---|---|---|---|---|---|---|---|---|---|
| | 70K/0 | M | 0 | 0 | 12316 | 0 | 0 | 12315 | 1 | 12316 | 0 | 0 | 168bp |
| | | U | 222000 | 12315 | 1 | | | | | | | | |
| | 70K/5 | M | 1.8 | 1 | 12779 | 1 | 0 | 12779 | 0 | 12780 | 9,00E-08 | 9,00E-06 | |
| | | U | 20000000 | 12780 | 0 | | | | | | | | |
| | 70K/10 | M | 0 | 0 | 13964 | 0 | 0 | 13964 | 0 | 13964 | 0 | 0 | |
| | | U | 20000000 | 13964 | 0 | | | | | | | | |
| | 70K/20 | M | 0 | 0 | 11379 | 0 | 0 | 11379 | 0 | 11379 | 0 | 0 | |
| | | U | 20000000 | 11379 | 0 | | | | | | | | |
| | 70K/3000 | M | 66400 | 10448 | 662 | 10448 | 0 | 661 | 1 | 11110 | 0.3 | 23.2 | |
| | | U | 220000 | 11109 | 1 | | | | | | | | |
| | 175K/0 | M | 0 | 0 | 12717 | 0 | 0 | 12711 | 6 | 12717 | 0 | 0 | |
| | | U | 180000 | 12711 | 6 | | | | | | | | |
| | 175K/5 | M | 0 | 0 | 13886 | 0 | 0 | 13883 | 3 | 13886 | 0 | 0 | |
| | | U | 198000 | 13883 | 3 | | | | | | | | |
| | 175K/10 | M | 0 | 0 | 13828 | 0 | 0 | 13827 | 1 | 13828 | 0 | 0 | |
| | | U | 224000 | 13827 | 1 | | | | | | | | |
| | 175K/20 | M | 0 | 0 | 13782 | 0 | 0 | 13781 | 1 | 13782 | 0 | 0 | |
| | | U | 224000 | 13781 | 1 | | | | | | | | |
| | 175K/ 3000 | M | 1010 | 560 | 12764 | 560 | 0 | 12764 | 0 | 13324 | 5.1 E-05 | 0.0051 | |
| | | U | 20000000 | 13324 | 0 | | | | | | | | |
| | 350K/0 | M | 2 | 1 | 11827 | 1 | 0 | 11827 | 0 | 11828 | 1,00E-07 | 1,00E-05 | |

(fortgesetzt)

| | Probe | Target | Kopien/ 20μLWell | Positive | Negative | Ch1+Ch2+ | Ch1+Ch2- | Ch1-Ch2+ | Ch1-Ch2- | Akzeptierte Tröpfchen | Verhältnis | Fraktionale Häufigkeit | PLA2R1 |
|---|---|---|---|---|---|---|---|---|---|---|---|---|---|
| | | U | 20000000 | 11828 | 0 | | | | | | | | |
| | 350K/5 | M | 3.8 | 2 | 12441 | 2 | 0 | 12441 | 0 | 12443 | 1.9E-07 | 1.9E-05 | |
| | | U | 20000000 | 12443 | 0 | | | | | | | | |
| | 350K/10 | M | 0 | 0 | 12247 | 0 | 0 | 12247 | 0 | 12247 | 0 | 0 | |
| | | U | 20000000 | 12247 | 0 | | | | | | | | |
| | 350K/20 | M | 0 | 0 | 12760 | 0 | 0 | 12759 | 1 | 12760 | 0 | 0 | |
| | | U | 222000 | 12759 | 1 | | | | | | | | |
| | 350K/ 3000 | M | 6.2 | 3 | 11393 | 3 | 0 | 11393 | 0 | 11396 | 3.1E-07 | 3.1E-05 | |
| | | U | 20000000 | 11396 | 0 | | | | | | | | |
| | 700K/0 | M | 10.6 | 6 | 13199 | 6 | 0 | 13199 | 0 | 13205 | 5,00E-07 | 5,00E-05 | |
| | | U | 20000000 | 13205 | 0 | | | | | | | | |
| | 700K/5 | M | 0 | 0 | 11011 | 0 | 0 | 11011 | 0 | 11011 | 0 | 0 | |
| | | U | 20000000 | 11011 | 0 | | | | | | | | |
| | 700K/10 | M | 0 | 0 | 10392 | 0 | 0 | 10392 | 0 | 10392 | 0 | 0 | |
| | | U | 20000000 | 10392 | 0 | | | | | | | | |
| | 700K/20 | M | 2.4 | 1 | 9789 | 1 | 0 | 9789 | 0 | 9790 | 1.2E-07 | 1.2E-05 | |
| | | U | 20000000 | 9790 | 0 | | | | | | | | |
| | 700K/ 3000 | M | 2 | 1 | 11840 | 1 | 0 | 11839 | 1 | 11841 | 9,00E-06 | 0.0009 | |
| | | U | 220000 | 11840 | 1 | | | | | | | | |
| | 70K/0 | M | 0 | 0 | 16276 | 0 | 0 | 1169 | 15107 | 16276 | 0 | 0 | 161bp |
| | | U | 1760 | 1169 | 15107 | | | | | | | | |
| | 70K/5 | M | 1.8 | 1 | 12665 | 1 | 0 | 12642 | 23 | 12666 | 1.3E-05 | 0.0013 | |
| | | U | 148400 | 12643 | 23 | | | | | | | | |

(fortgesetzt)

| Probe | Target | Kopien/ 20μLWell | Positive | Negative | Ch1+Ch2+ | Ch1+Ch2- | Ch1-Ch2+ | Ch1-Ch2- | Akzeptierte Tröpfchen | Verhältnis | Fraktionale Häufigkeit | PLA2R1 |
|---|---|---|---|---|---|---|---|---|---|---|---|---|
| 70K/10 | M | 1.4 | 1 | 16320 | 1 | 0 | 16191 | 129 | 16321 | 1.3E-05 | 0.0013 | |
| | U | 113800 | 16192 | 129 | | | | | | | | |
| 70K/20 | M | 542 | 315 | 13509 | 315 | 0 | 13509 | 0 | 13824 | 2.7E-05 | 0.0027 | |
| | U | 20000000 | 13824 | 0 | | | | | | | | |
| 70K/3000 | M | 20000000 | 13433 | 0 | 0 | 13433 | 0 | 0 | 13433 | | 100 | |
| | U | 0 | 0 | 13433 | | | | | | | | |
| 175K/0 | M | 4 | 2 | 12059 | 2 | 0 | 11282 | 777 | 12061 | 6,00E-05 | 0.006 | |
| | U | 64600 | 11284 | 777 | | | | | | | | |
| 175K/5 | M | 1.8 | 1 | 12715 | 1 | 0 | 12706 | 9 | 12716 | 1.1 E-05 | 0.0011 | |
| | U | 170000 | 12707 | 9 | | | | | | | | |
| 175K/10 | M | 0 | 0 | 12541 | 0 | 0 | 12537 | 4 | 12541 | 0 | 0 | |
| | U | 190000 | 12537 | 4 | | | | | | | | |
| 175K/20 | M | 17.6 | 10 | 13290 | 10 | 0 | 13288 | 2 | 13300 | 9,00E-05 | 0.009 | |
| | U | 208000 | 13298 | 2 | | | | | | | | |
| 175K/ 3000 | M | 226000 | 14492 | 1 | 362 | 14130 | 0 | 1 | 14493 | 380 | 99.74 | |
| | U | 596 | 362 | 14131 | | | | | | | | |
| 350K/0 | M | 0 | 0 | 14659 | 0 | 0 | 14574 | 85 | 14659 | 0 | 0 | |
| | U | 121200 | 14574 | 85 | | | | | | | | |
| 350K/5 | M | 0 | 0 | 15066 | 0 | 0 | 15055 | 11 | 15066 | 0 | 0 | |
| | U | 170000 | 15055 | 11 | | | | | | | | |
| 350K/10 | M | 1.8 | 1 | 13384 | 1 | 0 | 13383 | 1 | 13385 | 8,00E-06 | 0.0008 | |
| | U | 224000 | 13384 | 1 | | | | | | | | |
| 350K/20 | M | 100 | 59 | 13894 | 59 | 0 | 13892 | 2 | 13953 | 0.00048 | 0.048 | |
| | U | 208000 | 13951 | 2 | | | | | | | | |

(fortgesetzt)

| | Probe | Target | Kopien/ 20μLWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | PLA2R1 |
|---|---|---|---|---|---|---|---|---|---|---|---|---|---|
| | 350K/ 3000 | M | 206000 | 12365 | 2 | 1741 | 10624 | 0 | 2 | 12367 | 58 | 98.29 | |
| | | U | 3580 | 1741 | 10626 | | | | | | | | |
| | 700K/0 | M | 9.8 | 5 | 12126 | 5 | 0 | 12119 | 7 | 12131 | 6,00E-05 | 0.006 | |
| | | U | 176000 | 12124 | 7 | | | | | | | | |
| | 700K/5 | M | 0 | 0 | 13123 | 0 | 0 | 233 | 12890 | 13123 | 0 | 0 | |
| | | U | 422 | 233 | 12890 | | | | | | | | |
| | 700K/10 | M | 0 | 0 | 14715 | 0 | 0 | 14712 | 3 | 14715 | 0 | 0 | |
| | | U | 200000 | 14712 | 3 | | | | | | | | |
| | 700K/20 | M | 7 | 4 | 13428 | 4 | 0 | 13427 | 1 | 13432 | 3.1 E-05 | 0.0031 | |
| | | U | 224000 | 13431 | 1 | | | | | | | | |
| | 700K/ 3000 | M | 20000000 | 14850 | 0 | 14847 | 3 | 0 | 0 | 14850 | 100 | 99 | |
| | | U | 200000 | 14847 | 3 | | | | | | | | |
| | 70K/0 | M | 1.4 | 1 | 16313 | 1 | 0 | 16313 | 0 | 16314 | 7,00E-08 | 7,00E-06 | 150bp |
| | | U | 20000000 | 16314 | 0 | | | | | | | | |
| | 70K/5 | M | 136000 | 12892 | 40 | 12838 | 54 | 40 | 0 | 12932 | 1.05 | 51.3 | |
| | | U | 129000 | 12878 | 54 | | | | | | | | |
| | 70K/10 | M | 104600 | 13330 | 158 | 13209 | 121 | 149 | 9 | 13488 | 0.958 | 48.9 | |
| | | U | 109200 | 13358 | 130 | | | | | | | | |
| | 70K/20 | M | 172000 | 12971 | 9 | 1311 | 11660 | 0 | 9 | 12980 | 68 | 98.56 | |
| | | U | 2500 | 1311 | 11669 | | | | | | | | |
| | 70K/3000 | M | 20000000 | 13448 | 0 | 0 | 13448 | 0 | 0 | 13448 | | 100 | |
| | | U | 0 | 0 | 13448 | | | | | | | | |
| | 175K/0 | M | 4.4 | 3 | 15894 | 3 | 0 | 15893 | 1 | 15897 | 2,00E-05 | 0.002 | |

(fortgesetzt)

| | Probe | Target | Kopien/ 20μLWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | PLA2R1 |
|---|---|---|---|---|---|---|---|---|---|---|---|---|---|
| | | U | 228000 | 15896 | 1 | | | | | | | | |
| | 175K/5 | M | 19.2 | 11 | 13482 | 0 | 11 | 0 | 13482 | 13493 | 0 | 0 | |
| | | U | 0 | 0 | 13493 | | | | | | | | |
| | 175K/10 | M | 20000000 | 14399 | 0 | 14399 | 0 | 0 | 0 | 14399 | 1 | 50 | |
| | | U | 20000000 | 14399 | 0 | | | | | | | | |
| | 175K/20 | M | 20000000 | 14118 | 0 | 13754 | 364 | 0 | 0 | 14118 | 230 | 99.57 | |
| | | U | 86000 | 13754 | 364 | | | | | | | | |
| | 175K/ 3000 | M | 206000 | 12998 | 2 | 0 | 12998 | 0 | 2 | 13000 | | 100 | |
| | | U | 0 | 0 | 13000 | | | | | | | | |
| | 350K/0 | M | 0 | 0 | 13480 | 0 | 0 | 13479 | 1 | 13480 | 0 | 0 | |
| | | U | 224000 | 13479 | 1 | | | | | | | | |
| | 350K/5 | M | 892 | 536 | 13866 | 536 | 0 | 13866 | 0 | 14402 | 4.5E-05 | 0.0045 | |
| | | U | 20000000 | 14402 | 0 | | | | | | | | |
| | 350K/10 | M | 17580 | 8007 | 7211 | 8007 | 0 | 7199 | 12 | 15218 | 0.105 | 9.5 | |
| | | U | 168000 | 15206 | 12 | | | | | | | | |
| | 350K/20 | M | 184000 | 14938 | 6 | 14938 | 0 | 2 | 4 | 14944 | 0.95 | 48.7 | |
| | | U | 194000 | 14940 | 4 | | | | | | | | |
| | 350K/ 3000 | M | 20000000 | 14921 | 0 | 0 | 14921 | 0 | 0 | 14921 | | 100 | |
| | | U | 0 | 0 | 14921 | | | | | | | | |
| | 700K/0 | M | 6.8 | 4 | 13729 | 4 | 0 | 13729 | 0 | 13733 | 3.4E-07 | 3.4E-05 | |
| | | U | 20000000 | 13733 | 0 | | | | | | | | |
| | 700K/5 | M | 8.4 | 5 | 13937 | 5 | 0 | 13937 | 0 | 13942 | 4.2E-07 | 4.2E-05 | |
| | | U | 20000000 | 13942 | 0 | | | | | | | | |

(fortgesetzt)

| | Probe | Target | Kopien/ 20µLWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | PLA2R1 |
|---|---|---|---|---|---|---|---|---|---|---|---|---|---|
| | 700K/10 | M | 142 | 74 | 12298 | 74 | 0 | 12297 | 1 | 12372 | 0.00064 | 0.064 | |
| | | U | 222000 | 12371 | 1 | | | | | | | | |
| | 700K/20 | M | 54220 | 12087 | 1340 | 12087 | 0 | 1340 | 0 | 13427 | 0.0027 | 0.27 | |
| | | U | 20000000 | 13427 | 0 | | | | | | | | |
| | 1700K/ 3000 | M | 220000 | 11331 | 1 | 7 | 11324 | 0 | 1 | 11332 | 15000 | 99.993 | |
| | | U | 14 | 7 | 11325 | | | | | | | | |
| | NTC | M | 0 | 0 | 15130 | 0 | 0 | 0 | 15130 | 15130 | 0 | 0 | |
| | | U | 0 | 0 | 15130 | | | | | | | | |

**Tabelle 27:** Kopienanzahl methylierter (M) und nicht-methylierte ***PLA2R1***-DNA-Fragmente (U) [Copies/20 µl Well), Anteil M/U (Ratio) und relativer Anteil M/M+U (Fractional Abundance) in Proben mit keiner Kopie, 5, 10, 20 und 3000 Kopien methylierter PLA2R1-DNA-Fragmente und jeweils einem Anteil von 70.000 Kopien (70K/0-3000), 175.000 (170K/0-3000), 350.000 (350K/0-3000) und 700.000 (700K/0-3000) nicht-methylierter PLA2R1-DNA-Fragmente mit dem Primerpaar 133 bp (SEQ ID: 7 und 8) nach 15 Zyklen Prä-Amplifikation mit 1,5 mM bzw. 6,0 mM $MgCl_2$-Konzentration und 50°C Annealingtemperatur und anschließender dPCR. NTC: Non-Template-Negativkontrolle.

| **Probe** | **Target** | Kopien/20µL | Positive | Negative | **Ch1+Ch2+** | **Ch1+Ch2-** | **Ch1-Ch2+** | **Ch1-Ch2-** | **Akzeptierte Tröpfchen** | Verhältnis | **Fraktionale Häufigkeit** | **$MgCl_2$** |
|---|---|---|---|---|---|---|---|---|---|---|---|---|
| 70K/0 | M | 7.4 | 4 | 12620 | 1 | 3 | 8774 | 3846 | 12624 | 0.00027 | 0.027 | 1,5mM |
| | U | 27940 | 8775 | 3849 | | | | | | | | |
| 70K/5 | M | 1938 | 1081 | 12592 | 893 | 188 | 10688 | 1904 | 13673 | 0.0439 | 4.2 | |
| | U | 44180 | 11581 | 2092 | | | | | | | | |
| 70K/10 | M | 2642 | 1585 | 13335 | 1280 | 305 | 11016 | 2319 | 14920 | 0.0646 | 6.07 | |
| | U | 40900 | 12296 | 2624 | | | | | | | | |
| 70K/20 | M | 4980 | 2716 | 11547 | 1991 | 725 | 8731 | 2816 | 14263 | 0.152 | 13.17 | |
| | U | 32780 | 10722 | 3541 | | | | | | | | |
| 70K/3000 | M | 228000 | 16143 | 1 | 13 | 16130 | 0 | 1 | 16144 | 12.7 | 99.992 | |
| | U | 18 | 13 | 16131 | | | | | | | | |
| 175K/0 | M | 3 | 2 | 15991 | 0 | 2 | 15267 | 724 | 15993 | 4,00E-05 | 0.004 | |
| | U | 72800 | 15267 | 726 | | | | | | | | |
| 175K/5 | M | 1374 | 827 | 13748 | 820 | 7 | 13599 | 149 | 14575 | 0.0129 | 1.27 | |
| | U | 106800 | 14419 | 156 | | | | | | | | |
| 175K/10 | M | 2920 | 1518 | 11463 | 1504 | 14 | 11337 | 126 | 12981 | 0.0275 | 2.67 | |
| | U | 106600 | 12841 | 140 | | | | | | | | |
| 175K/20 | M | 4340 | 2534 | 12520 | 2443 | 91 | 12217 | 303 | 15054 | 0.0506 | 4.82 | |
| | U | 85800 | 14660 | 394 | | | | | | | | |
| 175K/3000 | M | 200000 | 15362 | 3 | 1274 | 14088 | 0 | 3 | 15365 | 99 | 99 | |
| | U | 2040 | 1274 | 14091 | | | | | | | | |
| 350K/0 | M | 394 | 249 | 14709 | 247 | 2 | 14670 | 39 | 14958 | 0.00285 | 0.284 | |
| | U | 138800 | 14917 | 41 | | | | | | | | |

(fortgesetzt)

| Probe | Target | Kopien/20µL | Positive | Negative | Ch1+Ch2+ | Ch1+Ch2- | Ch1-Ch2+ | Ch1-Ch2- | Akzeptierte Tröpfchen | Verhältnis | Fraktionale Häufigkeit | $MgCl_2$ |
|---|---|---|---|---|---|---|---|---|---|---|---|---|
| 350K/5 | M | 1980 | 1101 | 12543 | 1095 | 6 | 12514 | 29 | 13644 | 0.0141 | 1.39 | |
| | U | 140400 | 13609 | 35 | | | | | | | | |
| 350K/10 | M | 2660 | 1420 | 11905 | 1403 | 17 | 11765 | 140 | 13325 | 0.0254 | 2.47 | |
| | U | 104400 | 13168 | 157 | | | | | | | | |
| 350K/20 | M | 6660 | 3064 | 9355 | 3061 | 3 | 9340 | 15 | 12419 | 0.0433 | 4.15 | |
| | U | 154000 | 12401 | 18 | | | | | | | | |
| 350K/ 3000 | M | 20000000 | 13089 | 0 | 5406 | 7683 | 0 | 0 | 13089 | 1600 | 99.937 | |
| | U | 12540 | 5406 | 7683 | | | | | | | | |
| 700K/0 | M | 4.6 | 2 | 10144 | 2 | 0 | 10139 | 5 | 10146 | 2.6E-05 | 0.0026 | |
| | U | 180000 | 10141 | 5 | | | | | | | | |
| 700K/5 | M | 1206 | 512 | 9729 | 512 | 0 | 9721 | 8 | 10241 | 0.0072 | 0.71 | |
| | U | 168000 | 10233 | 8 | | | | | | | | |
| 700K/10 | M | 1914 | 954 | 11254 | 954 | 0 | 11247 | 7 | 12208 | 0.0109 | 1.08 | |
| | U | 176000 | 12201 | 7 | | | | | | | | |
| 700K/20 | M | 5820 | 3030 | 10810 | 3030 | 0 | 10805 | 5 | 13840 | 0.0312 | 3.02 | |
| | U | 186000 | 13835 | 5 | | | | | | | | |
| 700K/ 3000 | M | 148800 | 15035 | 27 | 10868 | 4167 | 0 | 27 | 15062 | 4.95 | 83.2 | |
| | U | 30080 | 10868 | 4194 | | | | | | | | |
| 70K/0 | M | 384 | 166 | 10081 | 166 | 0 | 10081 | 0 | 10247 | 1.9E-05 | 0.0019 | 6 mM |
| | U | 20000000 | 10247 | 0 | | | | | | | | |
| 70K/5 | M | 4.4 | 2 | 10676 | 2 | 0 | 10676 | 0 | 10678 | 2.2E-07 | 2.2E-05 | |
| | U | 20000000 | 10678 | 0 | | | | | | | | |
| 70K/10 | M | 11.2 | 6 | 12627 | 6 | 0 | 12594 | 33 | 12633 | 8,00E-05 | 0.008 | |

(fortgesetzt)

| Probe | Target | Kopien/20µL | Positive | Negative | Ch1+Ch2+ | Ch1+Ch2- | Ch1-Ch2+ | Ch1-Ch2- | Akzeptierte Tröpfchen | Verhältnis | Fraktionale Häufigkeit | $MgCl_2$ |
|---|---|---|---|---|---|---|---|---|---|---|---|---|
| | U | 140000 | 12600 | 33 | | | | | | | | |
| 70K/20 | M | 8 | 4 | 11819 | 4 | 0 | 11819 | 0 | 11823 | 4,00E-07 | 4,00E-05 | |
| | U | 20000000 | 11823 | 0 | | | | | | | | |
| 70K/3000 | M | 20000000 | 12141 | 0 | 12140 | 1 | 0 | 0 | 12141 | 90 | 98.9 | |
| | U | 222000 | 12140 | 1 | | | | | | | | |
| 175K/0 | M | 0 | 0 | 13218 | 0 | 0 | 13218 | 0 | 13218 | 0 | 0 | |
| | U | 20000000 | 13218 | 0 | | | | | | | | |
| 175K/5 | M | 0 | 0 | 11762 | 0 | 0 | 11762 | 0 | 11762 | 0 | 0 | |
| | U | 20000000 | 11762 | 0 | | | | | | | | |
| 175K/10 | M | 2 | 1 | 11472 | 1 | 0 | 11472 | 0 | 11473 | 1,00E-07 | 1,00E-05 | |
| | U | 20000000 | 11473 | 0 | | | | | | | | |
| 175K/20 | M | 6.2 | 3 | 11227 | 3 | 0 | 11213 | 14 | 11230 | 4,00E-05 | 0.004 | |
| | U | 158000 | 11216 | 14 | | | | | | | | |
| 175K/ 3000 | M | 180000 | 14518 | 7 | 14516 | 2 | 2 | 5 | 14525 | 1 | 50 | |
| | U | 180000 | 14518 | 7 | | | | | | | | |
| 350K/0 | M | 3.2 | 2 | 14917 | 2 | 0 | 14915 | 2 | 14919 | 1.5E-05 | 0.0015 | |
| | U | 210000 | 14917 | 2 | | | | | | | | |
| 350K/5 | M | 0 | 0 | 16205 | 0 | 0 | 16205 | 0 | 16205 | 0 | 0 | |
| | U | 20000000 | 16205 | 0 | | | | | | | | |
| 350K/10 | M | 0 | 0 | 15567 | 0 | 0 | 15567 | 0 | 15567 | 0 | 0 | |
| | U | 20000000 | 15567 | 0 | | | | | | | | |
| 350K/20 | M | 1.4 | 1 | 15904 | 1 | 0 | 15904 | 0 | 15905 | 7,00E-08 | 7,00E-06 | |
| | U | 20000000 | 15905 | 0 | | | | | | | | |

(fortgesetzt)

| Probe | Target | Kopien/20μL | Positive | Negative | Ch1+Ch2+ | Ch1+Ch2- | Ch1-Ch2+ | Ch1-Ch2- | Akzeptierte Tröpfchen | Verhältnis | Fraktionale Häufigkeit | $MgCl_2$ |
|---|---|---|---|---|---|---|---|---|---|---|---|---|
| 350K/3000 | M | 98400 | 13487 | 209 | 13487 | 0 | 209 | 0 | 13696 | 0.0049 | 0.49 | |
| | U | 20000000 | 13696 | 0 | | | | | | | | |
| 700K/0 | M | 0 | 0 | 12117 | 0 | 0 | 12117 | 0 | 12117 | 0 | 0 | |
| | U | 20000000 | 12117 | 0 | | | | | | | | |
| 700K/5 | M | 0 | 0 | 15213 | 0 | 0 | 15213 | 0 | 15213 | 0 | 0 | |
| | U | 20000000 | 15213 | 0 | | | | | | | | |
| 700K/10 | M | 3 | 2 | 15564 | 2 | 0 | 15563 | 1 | 15566 | 1.3E-05 | 0.0013 | |
| | U | 228000 | 15565 | 1 | | | | | | | | |
| 700K/20 | M | 18.2 | 11 | 14274 | 11 | 0 | 14272 | 2 | 14285 | 9,00E-05 | 0.009 | |
| | U | 208000 | 14283 | 2 | | | | | | | | |
| 700K/3000 | M | 16840 | 8103 | 7752 | 8103 | 0 | 7750 | 2 | 15855 | 0.08 | 7.4 | |
| | U | 212000 | 15853 | 2 | | | | | | | | |
| NTC | M | 0 | 0 | 16573 | 0 | 0 | 0 | 16573 | 16573 | 0 | 0 | |
| | U | 0 | 0 | 16573 | | | | | | | | |

**Tabelle 28:** Kopienanzahl methylierter (M) und nicht-methylierte ***PLA2R1***-DNA-Fragmente (U) [Copies/20 µl Well), Anteil M/U (Ratio) und relativer Anteil M/M+U (Fractional Abundance) in Proben mit keiner Kopie, 5, 10, 20 und 3000 Kopien methylierter PLA2R1-DNA-Fragmente und jeweils einem Anteil von 70.000 Kopien (70K/0-3000), 175.000 (170K/0-3000), 350.000 (350K/0-3000) und 700.000 (700K/0-3000) nicht-methylierter PLA2R1-DNA-Fragmente mit dem Primerpaar 133 bp (SEQ ID: 7 und 8) nach 15 Zyklen Prä-Amplifikation mit 1,5 mM bzw. 6,0 mM $MgCl_2$-Konzentration und 63°C Annealingtemperatur und anschließender dPCR. NTC: Non-Template-Negativkontrolle.

| Well | Probe | Target | Kopien/ 20µlWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | $MgCl_2$ |
|---|---|---|---|---|---|---|---|---|---|---|---|---|---|
| D03 | 70K/0 | M | 0 | 0 | 15574 | 0 | 0 | 64 | 15510 | 15574 | 0 | 0 | 1,5mM |
| | | U | 96 | 64 | 15510 | | | | | | | | |
| C03 | 70K/5 | M | 14 | 9 | 15186 | 0 | 9 | 54 | 15132 | 15195 | 0.17 | 14 | |
| | | U | 84 | 54 | 15141 | | | | | | | | |
| B03 | 70K/10 | M | 42 | 26 | 14512 | 0 | 26 | 64 | 14448 | 14538 | 0.41 | 29 | |
| | | U | 104 | 64 | 14474 | | | | | | | | |
| A03 | 70K/20 | M | 42 | 26 | 14816 | 0 | 26 | 55 | 14761 | 14842 | 0.47 | 32 | |
| | | U | 88 | 55 | 14787 | | | | | | | | |
| H02 | 70K/ 3000 | M | 3032 | 2179 | 15843 | 2 | 2177 | 91 | 15752 | 18022 | 25 | 96.1 | |
| | | U | 122 | 93 | 17929 | | | | | | | | |
| G02 | 175K/0 | M | 0 | 0 | 14398 | 0 | 0 | 188 | 14210 | 14398 | 0 | 0 | |
| | | U | 310 | 188 | 14210 | | | | | | | | |
| F02 | 175K/5 | M | 7.4 | 5 | 15993 | 0 | 5 | 223 | 15770 | 15998 | 0.022 | 2.2 | |
| | | U | 330 | 223 | 15775 | | | | | | | | |
| E02 | 175K/10 | M | 32 | 22 | 15991 | 0 | 22 | 260 | 15731 | 16013 | 0.084 | 7.7 | |
| | | U | 386 | 260 | 15753 | | | | | | | | |
| D02 | 175K/20 | M | 36 | 24 | 15674 | 0 | 24 | 237 | 15437 | 15698 | 0.101 | 9.1 | |
| | | U | 358 | 237 | 15461 | | | | | | | | |
| C02 | 175K/ 3000 | M | 6660 | 3889 | 11890 | 5 | 3884 | 143 | 11747 | 15779 | 30 | 96.8 | |
| | | U | 222 | 148 | 15631 | | | | | | | | |
| B02 | 350K/0 | M | 0 | 0 | 13714 | 0 | 0 | 294 | 13420 | 13714 | 0 | 0 | |

(fortgesetzt)

| Well | Probe | Target | Kopien/ 20μlWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | $MgCl_2$ |
|---|---|---|---|---|---|---|---|---|---|---|---|---|---|
| | | U | 510 | 294 | 13420 | | | | | | | | |
| A02 | 350K/5 | M | 1.4 | 1 | 15766 | 0 | 1 | 387 | 15379 | 15767 | 0.0026 | 0.25 | |
| | | U | 584 | 387 | 15380 | | | | | | | | |
| H01 | 350K/10 | M | 7 | 4 | 13299 | 0 | 4 | 373 | 12926 | 13303 | 0.011 | 1 | |
| | | U | 670 | 373 | 12930 | | | | | | | | |
| G01 | 350K/20 | M | 16.2 | 10 | 14489 | 0 | 10 | 493 | 13996 | 14499 | 0.02 | 2 | |
| | | U | 814 | 493 | 14006 | | | | | | | | |
| F01 | 350K/ 3000 | M | 4260 | 1456 | 7323 | 5 | 1451 | 286 | 7037 | 8779 | 5.4 | 84.3 | |
| | | U | 794 | 291 | 8488 | | | | | | | | |
| E01 | 700K/0 | M | 1.8 | 1 | 13385 | 0 | 1 | 616 | 12769 | 13386 | 0.0016 | 0.16 | |
| | | U | 1108 | 616 | 12770 | | | | | | | | |
| D01 | 700K/5 | M | 1.6 | 1 | 13958 | 0 | 1 | 909 | 13049 | 13959 | 0.0011 | 0.11 | |
| | | U | 1580 | 909 | 13050 | | | | | | | | |
| C01 | 700K/10 | M | 54 | 27 | 11833 | 0 | 27 | 765 | 11068 | 11860 | 0.034 | 3.3 | |
| | | U | 1560 | 765 | 11095 | | | | | | | | |
| B01 | 700K/20 | M | 2.2 | 1 | 10432 | 0 | 1 | 709 | 9723 | 10433 | 0.0014 | 0.14 | |
| | | U | 1660 | 709 | 9724 | | | | | | | | |
| A01 | 700K/ 3000 | M | 2780 | 1400 | 11205 | 7 | 1393 | 876 | 10329 | 12605 | 1.62 | 61.8 | |
| | | U | 1700 | 883 | 11722 | | | | | | | | |
| H05 | 70K/0 | M | 0 | 0 | 8874 | 0 | 0 | 42 | 8832 | 8874 | 0 | 0 | 6mM |
| | | U | 112 | 42 | 8832 | | | | | | | | |
| G05 | 70K/5 | M | 1990 | 1039 | 11769 | 3 | 1036 | 47 | 11722 | 12808 | 22 | 95.6 | |
| | | U | 92 | 50 | 12758 | | | | | | | | |

(fortgesetzt)

| Well | Probe | Target | Kopien/ 20µlWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | $MgCl_2$ |
|---|---|---|---|---|---|---|---|---|---|---|---|---|---|
| F05 | 70K/10 | M | 1866 | 1039 | 12595 | 0 | 1039 | 66 | 12529 | 13634 | 16.3 | 94.2 | |
| | | U | 114 | 66 | 13568 | | | | | | | | |
| E05 | 70K/20 | M | 6060 | 2669 | 9102 | 1 | 2668 | 54 | 9048 | 11771 | 55 | 98.21 | |
| | | U | 110 | 55 | 11716 | | | | | | | | |
| D05 | 70K/ 3000 | M | 224000 | 13388 | 1 | 3 | 13385 | 0 | 1 | 13389 | 42000 | 100 | |
| | | U | 5.2 | 3 | 13386 | | | | | | | | |
| C05 | 175K/0 | M | 5.6 | 3 | 12577 | 1 | 2 | 123 | 12454 | 12580 | 0.024 | 2.4 | |
| | | U | 234 | 124 | 12456 | | | | | | | | |
| B05 | 175K/5 | M | 770 | 432 | 12989 | 1 | 431 | 189 | 12800 | 13421 | 2.29 | 69.6 | |
| | | U | 336 | 190 | 13231 | | | | | | | | |
| A05 | 175K/10 | M | 3820 | 1801 | 10204 | 2 | 1799 | 134 | 10070 | 12005 | 14.3 | 93.4 | |
| | | U | 268 | 136 | 11869 | | | | | | | | |
| H04 | 175K/20 | M | 4980 | 2559 | 10837 | 2 | 2557 | 153 | 10684 | 13396 | 18.2 | 94.8 | |
| | | U | 274 | 155 | 13241 | | | | | | | | |
| G04 | 175K/ 3000 | M | 20000000 | 15139 | 0 | 0 | 15139 | 0 | 0 | 15139 | | 100 | |
| | | U | 0 | 0 | 15139 | | | | | | | | |
| F04 | 350K/0 | M | 9.4 | 6 | 14969 | 0 | 6 | 289 | 14680 | 14975 | 0.021 | 2 | |
| | | U | 458 | 289 | 14686 | | | | | | | | |
| E04 | 350K/5 | M | 976 | 588 | 13876 | 7 | 581 | 429 | 13447 | 14464 | 1.36 | 57.6 | |
| | | U | 720 | 436 | 14028 | | | | | | | | |
| D04 | 350K/10 | M | 1270 | 711 | 12815 | 2 | 709 | 344 | 12471 | 13526 | 2.08 | 67.6 | |
| | | U | 610 | 346 | 13180 | | | | | | | | |
| C04 | 350K/20 | M | 4700 | 2644 | 11951 | 15 | 2629 | 312 | 11639 | 14595 | 8.8 | 89.8 | |

(fortgesetzt)

| Well | Probe | Target | Kopien/ 20µlWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | $MgCl_2$ |
|---|---|---|---|---|---|---|---|---|---|---|---|---|---|
| | | U | 534 | 327 | 14268 | | | | | | | | |
| B04 | 350K/ 3000 | M | 20000000 | 15336 | 0 | 2 | 15334 | 0 | 0 | 15336 | | 100 | |
| | | U | 3 | 2 | 15334 | | | | | | | | |
| A04 | 700K/0 | M | 1.6 | 1 | 14278 | 0 | 1 | 538 | 13740 | 14279 | 0.0018 | 0.18 | |
| | | U | 904 | 538 | 13741 | | | | | | | | |
| H03 | 700K/5 | M | 584 | 337 | 13425 | 12 | 325 | 976 | 12449 | 13762 | 0.333 | 25 | |
| | | U | 1760 | 988 | 12774 | | | | | | | | |
| G03 | 700K/10 | M | 1536 | 1093 | 16213 | 22 | 1071 | 1076 | 15137 | 17306 | 1 | 49.9 | |
| | | U | 1542 | 1098 | 16208 | | | | | | | | |
| F03 | 700K/20 | M | 4760 | 2541 | 11344 | 28 | 2513 | 496 | 10848 | 13885 | 5.25 | 84 | |
| | | U | 906 | 524 | 13361 | | | | | | | | |
| E03 | 700K/ 3000 | M | 20000000 | 14373 | 0 | 0 | 14373 | 0 | 0 | 14373 | | 100 | |
| | | U | 0 | 0 | 14373 | | | | | | | | |
| H11 | NTC | M | 0 | 0 | 12407 | 0 | 0 | 0 | 12407 | 12407 | 0 | 0 | |
| | | U | 0 | 0 | 12407 | | | | | | | | |

**Tabelle 29:** Kopienanzahl methylierter (M) und nicht-methylierte ***PLA2R1***-DNA-Fragmente (U) [Copies/20 µl Well), Anteil M/U (Ratio) und relativer Anteil M/M+U (Fractional Abundance) in Proben mit keiner Kopie, 5, 10, 20 und 3000 Kopien methylierter PLA2R1-DNA-Fragmente und jeweils einem Anteil von 70.000 Kopien (70K/0-3000), 175.000 (170K/0-3000), 350.000 (350K/0-3000) und 700.000 (700K/0-3000) nicht-methylierter PLA2R1-DNA-Fragmente mit dem Primerpaar 133 bp (SEQ ID: 7 und 8) nach 50 Zyklen Prä-Amplifikation mit 1,5 mM bzw. 6,0 mM $MgCl_2$-Konzentration und 50°C Annealingtemperatur und anschließender dPCR. NTC: Non-Template-Negativkontrolle.

| Well | Probe | Target | Kopien/ 20µLWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | $MgCl_2$ |
|---|---|---|---|---|---|---|---|---|---|---|---|---|---|
| D08 | 70K/0 | M | 1.6 | 1 | 14934 | 1 | 0 | 14933 | 1 | 14935 | 7,00E-06 | 0.0007 | 1,5mM |
| | | U | 226000 | 14934 | 1 | | | | | | | | |
| C08 | 70K/5 | M | 2800 | 1611 | 12708 | 1611 | 0 | 12708 | 0 | 14319 | 0.00014 | 0.014 | |
| | | U | 20000000 | 14319 | 0 | | | | | | | | |
| B08 | 70K/10 | M | 101800 | 16700 | 223 | 16700 | 0 | 223 | 0 | 16923 | 0.0051 | 0.51 | |
| | | U | 20000000 | 16923 | 0 | | | | | | | | |
| A08 | 70K/20 | M | 176000 | 14400 | 8 | 14400 | 0 | 6 | 2 | 14408 | 0.84 | 45.8 | |
| | | U | 208000 | 14406 | 2 | | | | | | | | |
| H07 | 70K/ 3000 | M | 20000000 | 14647 | 0 | 0 | 14647 | 0 | 0 | 14647 | | 100 | |
| | | U | 0 | 0 | 14647 | | | | | | | | |
| G07 | 175K/0 | M | 0 | 0 | 15079 | 0 | 0 | 15079 | 0 | 15079 | 0 | 0 | |
| | | U | 20000000 | 15079 | 0 | | | | | | | | |
| F07 | 175K/5 | M | 1.4 | 1 | 15751 | 1 | 0 | 15751 | 0 | 15752 | 7,00E-08 | 7,00E-06 | |
| | | U | 20000000 | 15752 | 0 | | | | | | | | |
| E07 | 175K/10 | M | 1458 | 847 | 13258 | 847 | 0 | 13258 | 0 | 14105 | 7.3E-05 | 0.0073 | |
| | | U | 20000000 | 14105 | 0 | | | | | | | | |
| D07 | 175K/20 | M | 2192 | 1482 | 15175 | 1482 | 0 | 15174 | 1 | 16657 | 0.0096 | 0.95 | |
| | | U | 228000 | 16656 | 1 | | | | | | | | |
| C07 | 175K/ 3000 | M | 20000000 | 14743 | 0 | 4 | 14739 | 0 | 0 | 14743 | | 100 | |
| | | U | 6.4 | 4 | 14739 | | | | | | | | |
| B07 | 350K/0 | M | 0 | 0 | 15783 | 0 | 0 | 15779 | 4 | 15783 | 0 | 0 | |

(fortgesetzt)

| Well | Probe | Target | Kopien/ 20μLWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | $MgCl_2$ |
|---|---|---|---|---|---|---|---|---|---|---|---|---|---|
| | | U | 194000 | 15779 | 4 | | | | | | | | |
| A07 | 350K/5 | M | 430 | 318 | 17265 | 318 | 0 | 17239 | 26 | 17583 | 0.0028 | 0.279 | |
| | | U | 153400 | 17557 | 26 | | | | | | | | |
| H06 | 350K/10 | M | 286 | 119 | 9764 | 119 | 0 | 9763 | 1 | 9883 | 0.00132 | 0.132 | |
| | | U | 216000 | 9882 | 1 | | | | | | | | |
| G06 | 350K/20 | M | 33680 | 9971 | 3131 | 9971 | 0 | 3131 | 0 | 13102 | 0.0017 | 0.17 | |
| | | U | 20000000 | 13102 | 0 | | | | | | | | |
| F06 | 350K/ 3000 | M | 20000000 | 11525 | 0 | 367 | 11158 | 0 | 0 | 11525 | 26000 | 100 | |
| | | U | 762 | 367 | 11158 | | | | | | | | |
| E06 | 700K/0 | M | 0 | 0 | 13798 | 0 | 0 | 13798 | 0 | 13798 | 0 | 0 | |
| | | U | 20000000 | 13798 | 0 | | | | | | | | |
| D06 | 700K/5 | M | 22 | 11 | 12214 | 11 | 0 | 12212 | 2 | 12225 | 0.0001 | 0.01 | |
| | | U | 206000 | 12223 | 2 | | | | | | | | |
| C06 | 700K/10 | M | 5.8 | 3 | 12099 | 3 | 0 | 12098 | 1 | 12102 | 2.6E-05 | 0.0026 | |
| | | U | 222000 | 12101 | 1 | | | | | | | | |
| B06 | 700K/20 | M | 856 | 470 | 12694 | 470 | 0 | 12694 | 0 | 13164 | 4.3E-05 | 0.0043 | |
| | | U | 20000000 | 13164 | 0 | | | | | | | | |
| A06 | 700K/ 3000 | M | 20000000 | 11920 | 0 | 7202 | 4718 | 0 | 0 | 11920 | 920 | 99.89 | |
| | | U | 21800 | 7202 | 4718 | | | | | | | | |
| H10 | 70K/0 | M | 0 | 0 | 16220 | 0 | 0 | 16216 | 4 | 16220 | 0 | 0 | 6mM |
| | | U | 196000 | 16216 | 4 | | | | | | | | |
| G10 | 70K/5 | M | 0 | 0 | 15609 | 0 | 0 | 15609 | 0 | 15609 | 0 | 0 | |
| | | U | 20000000 | 15609 | 0 | | | | | | | | |

(fortgesetzt)

| Well | Probe | Target | Kopien/ 20µLWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | $MgCl_2$ |
|---|---|---|---|---|---|---|---|---|---|---|---|---|---|
| F10 | 70K/10 | M | 1.6 | 1 | 15284 | 1 | 0 | 15284 | 0 | 15285 | 8,00E-08 | 8,00E-06 | |
| | | U | 20000000 | 15285 | 0 | | | | | | | | |
| E10 | 70K/20 | M | 0 | 0 | 16842 | 0 | 0 | 16841 | 1 | 16842 | 0 | 0 | |
| | | U | 228000 | 16841 | 1 | | | | | | | | |
| D10 | 70K/ 3000 | M | 20000000 | 15099 | 0 | 15089 | 10 | 0 | 0 | 15099 | 120 | 99.15 | |
| | | U | 172000 | 15089 | 10 | | | | | | | | |
| C10 | 175K/0 | M | 1.6 | 1 | 14131 | 1 | 0 | 14127 | 4 | 14132 | 9,00E-06 | 0.0009 | |
| | | U | 192000 | 14128 | 4 | | | | | | | | |
| B10 | 175K/5 | M | 0 | 0 | 14404 | 0 | 0 | 14400 | 4 | 14404 | 0 | 0 | |
| | | U | 192000 | 14400 | 4 | | | | | | | | |
| A10 | 175K/10 | M | 0 | 0 | 14134 | 0 | 0 | 14129 | 5 | 14134 | 0 | 0 | |
| | | U | 186000 | 14129 | 5 | | | | | | | | |
| H09 | 175K/20 | M | 0 | 0 | 12685 | 0 | 0 | 12684 | 1 | 12685 | 0 | 0 | |
| | | U | 222000 | 12684 | 1 | | | | | | | | |
| G09 | 175K/ 3000 | M | 116000 | 13858 | 101 | 13847 | 11 | 79 | 22 | 13959 | 0.82 | 44.9 | |
| | | U | 142200 | 13926 | 33 | | | | | | | | |
| F09 | 350K/0 | M | 0 | 0 | 13733 | 0 | 0 | 13733 | 0 | 13733 | 0 | 0 | |
| | | U | 20000000 | 13733 | 0 | | | | | | | | |
| E09 | 350K/5 | M | 1.6 | 1 | 14852 | 1 | 0 | 14851 | 1 | 14853 | 7,00E-06 | 0.0007 | |
| | | U | 226000 | 14852 | 1 | | | | | | | | |
| D09 | 350K/10 | M | 3.2 | 2 | 14367 | 2 | 0 | 14367 | 0 | 14369 | 1.6E-07 | 1.6E-05 | |
| | | U | 20000000 | 14369 | 0 | | | | | | | | |
| C09 | 350K/20 | M | 0 | 0 | 13833 | 0 | 0 | 13833 | 0 | 13833 | 0 | 0 | |

(fortgesetzt)

| Well | Probe | Target | Kopien/ 20µLWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | $MgCl_2$ |
|---|---|---|---|---|---|---|---|---|---|---|---|---|---|
| | | U | 20000000 | 13833 | 0 | | | | | | | | |
| B09 | 350K/ 3000 | M | 137200 | 13956 | 41 | 13956 | 0 | 41 | 0 | 13997 | 0.0069 | 0.68 | |
| | | U | 20000000 | 13997 | 0 | | | | | | | | |
| A09 | 700K/0 | M | 1.6 | 1 | 13937 | 1 | 0 | 13937 | 0 | 13938 | 8,00E-08 | 8,00E-06 | |
| | | U | 20000000 | 13938 | 0 | | | | | | | | |
| H08 | 700K/5 | M | 1.4 | 1 | 16530 | 1 | 0 | 16528 | 2 | 16531 | 7,00E-06 | 0.0007 | |
| | | U | 212000 | 16529 | 2 | | | | | | | | |
| G08 | 700K/10 | M | 7.4 | 5 | 16058 | 5 | 0 | 16053 | 5 | 16063 | 3.9E-05 | 0.0039 | |
| | | U | 190000 | 16058 | 5 | | | | | | | | |
| F08 | 700K/20 | M | 0 | 0 | 16236 | 0 | 0 | 16221 | 15 | 16236 | 0 | 0 | |
| | | U | 164000 | 16221 | 15 | | | | | | | | |
| E08 | 700K/ 3000 | M | 7760 | 4538 | 11614 | 4538 | 0 | 11614 | 0 | 16152 | 0.00039 | 0.039 | |
| | | U | 20000000 | 16152 | 0 | | | | | | | | |
| G11 | NTC | M | 0 | 0 | 16573 | 0 | 0 | 0 | 16573 | 16573 | 0 | 0 | |
| | | U | 0 | 0 | 16573 | | | | | | | | |

**Tabelle** 30: Kopienanzahl methylierter (M) und nicht-methylierte *PLA2R1*-DNA-Fragmente (U) [Copies/20 µl Well), Anteil M/U (Ratio) und relativer Anteil M/M+U (Fractional Abundance) in Proben mit keiner Kopie, 5, 10, 20 und 3000 Kopien methylierter PLA2R1-DNA-Fragmente und jeweils einem Anteil von 70.000 Kopien (70K/0-3000), 175.000 (170K/0-3000), 350.000 (350K/0-3000) und 700.000 (700K/0-3000) nicht-methylierter PLA2R1-DNA-Fragmente mit dem Primerpaar 133 bp (SEQ ID: 7 und 8) nach 50 Zyklen Prä-Amplifikation mit 1,5 mM bzw. 6,0 mM $MgCl_2$-Konzentration und 63°C Annealingtemperatur und anschließender dPCR. NTC: Non-Template-Negativkontrolle.

| Well | Probe | Target | Kopien/ 20µLWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | $MgCl_2$ |
|---|---|---|---|---|---|---|---|---|---|---|---|---|---|
| D08 | 70K/0 | M | 0 | 0 | 12948 | 0 | 0 | 12947 | 1 | 12948 | 0 | 0 | 1,5mM |
| | | U | 222000 | 12947 | 1 | | | | | | | | |
| C08 | 70K/5 | M | 20000000 | 12682 | 0 | 2227 | 10455 | 0 | 0 | 12682 | 4400 | 99.977 | |
| | | U | 4540 | 2227 | 10455 | | | | | | | | |
| B08 | 70K/10 | M | 20000000 | 14665 | 0 | 5625 | 9040 | 0 | 0 | 14665 | 1800 | 99.943 | |
| | | U | 11380 | 5625 | 9040 | | | | | | | | |
| A08 | 70K/20 | M | 190000 | 12963 | 4 | 568 | 12395 | 0 | 4 | 12967 | 180 | 99.45 | |
| | | U | 1054 | 568 | 12399 | | | | | | | | |
| H07 | 70K/ 3000 | M | 20000000 | 14373 | 0 | 0 | 14373 | 0 | 0 | 14373 | | 100 | |
| | | U | 0 | 0 | 14373 | | | | | | | | |
| G07 | 175K/0 | M | 7.4 | 5 | 15986 | 5 | 0 | 15978 | 8 | 15991 | 4.1 E-05 | 0.0041 | |
| | | U | 178000 | 15983 | 8 | | | | | | | | |
| F07 | 175K/5 | M | 224000 | 13736 | 1 | 13735 | 1 | 1 | 0 | 13737 | 1 | 50 | |
| | | U | 224000 | 13736 | 1 | | | | | | | | |
| E07 | 175K/10 | M | 20000000 | 13236 | 0 | 3441 | 9795 | 0 | 0 | 13236 | 2800 | 99.965 | |
| | | U | 7080 | 3441 | 9795 | | | | | | | | |
| D07 | 175K/20 | M | 20000000 | 13433 | 0 | 4918 | 8515 | 0 | 0 | 13433 | 1900 | 99.946 | |
| | | U | 10720 | 4918 | 8515 | | | | | | | | |
| C07 | 175K/ 3000 | M | 226000 | 14414 | 1 | 1 | 14413 | 0 | 1 | 14415 | 140000 | 99.993 | |
| | | U | 1.6 | 1 | 14414 | | | | | | | | |
| B07 | 350K/0 | M | 0 | 0 | 14207 | 0 | 0 | 14207 | 0 | 14207 | 0 | 0 | |

(fortgesetzt)

| Well | Probe | Target | Kopien/ 20µLWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | $MgCl_2$ |
|---|---|---|---|---|---|---|---|---|---|---|---|---|---|
| | | U | 20000000 | 14207 | 0 | | | | | | | | |
| A07 | 350K/5 | M | 58600 | 12525 | 1133 | 12525 | 0 | 996 | 137 | 13658 | 0.541 | 35.1 | |
| | | U | 108200 | 13521 | 137 | | | | | | | | |
| H06 | 350K/10 | M | 20000000 | 13957 | 0 | 13957 | 0 | 0 | 0 | 13957 | 1 | 50 | |
| | | U | 20000000 | 13957 | 0 | | | | | | | | |
| G06 | 350K/20 | M | 20000000 | 13838 | 0 | 13838 | 0 | 0 | 0 | 13838 | 1 | 50 | |
| | | U | 20000000 | 13838 | 0 | | | | | | | | |
| F06 | 350K/ 3000 | M | 20000000 | 13946 | 0 | 0 | 13946 | 0 | 0 | 13946 | | 100 | |
| | | U | 0 | 0 | 13946 | | | | | | | | |
| E06 | 700K/0 | M | 0 | 0 | 13541 | 0 | 0 | 13538 | 3 | 13541 | 0 | 0 | |
| | | U | 198000 | 13538 | 3 | | | | | | | | |
| D06 | 700K/5 | M | 0 | 0 | 13368 | 0 | 0 | 13368 | 0 | 13368 | 0 | 0 | |
| | | U | 20000000 | 13368 | 0 | | | | | | | | |
| C06 | 700K/10 | M | 20000000 | 11725 | 0 | 11724 | 1 | 0 | 0 | 11725 | 90 | 98.9 | |
| | | U | 220000 | 11724 | 1 | | | | | | | | |
| B06 | 700K/20 | M | 224000 | 13323 | 1 | 13323 | 0 | 1 | 0 | 13324 | 0.011 | 1.1 | |
| | | U | 20000000 | 13324 | 0 | | | | | | | | |
| A06 | 700K/ 3000 | M | 224000 | 13197 | 1 | 0 | 13197 | 0 | 1 | 13198 | | 100 | |
| | | U | 0 | 0 | 13198 | | | | | | | | |
| H10 | 70K/0 | M | 0 | 0 | 13306 | 0 | 0 | 13306 | 0 | 13306 | 0 | 0 | 6mM |
| | | U | 20000000 | 13306 | 0 | | | | | | | | |
| G10 | 70K/5 | M | 20000000 | 16759 | 0 | 1 | 16758 | 0 | 0 | 16759 | 1000000 | 100 | |
| | | U | 1.4 | 1 | 16758 | | | | | | | | |

(fortgesetzt)

| Well | Probe | Target | Kopien/ 20µLWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | $MgCl_2$ |
|---|---|---|---|---|---|---|---|---|---|---|---|---|---|
| F10 | 70K/10 | M | 2260000 | 14386 | 1 | 0 | 14386 | 0 | 1 | 14387 | | 100 | |
| | | U | 0 | 0 | 14387 | | | | | | | | |
| E10 | 70K/20 | M | 228000 | 15578 | 1 | 2 | 15576 | 0 | 1 | 15579 | 80000 | 100 | |
| | | U | 3 | 2 | 15577 | | | | | | | | |
| D10 | 70K/ 3000 | M | 20000000 | 14384 | 0 | 11 | 14373 | 0 | 0 | 14384 | 1000000 | 100 | |
| | | U | 18 | 11 | 14373 | | | | | | | | |
| C10 | 175K/0 | M | 0 | 0 | 14344 | 0 | 0 | 14288 | 56 | 14344 | 0 | 0 | |
| | | U | 1304000 | 14288 | 56 | | | | | | | | |
| B10 | 175K/5 | M | 20000000 | 16380 | 0 | 10788 | 5592 | 0 | 0 | 16380 | 790 | 99.87 | |
| | | U | 25280 | 10788 | 5592 | | | | | | | | |
| A10 | 175K/10 | M | 20000000 | 14283 | 0 | 0 | 14283 | 0 | 0 | 14283 | | 100 | |
| | | U | 0 | 0 | 14283 | | | | | | | | |
| H09 | 175K/20 | M | 20000000 | 12692 | 0 | 0 | 12692 | 0 | 0 | 12692 | | 100 | |
| | | U | 0 | 0 | 12692 | | | | | | | | |
| G09 | 175K/ 3000 | M | 20000000 | 15891 | 0 | 2 | 15889 | 0 | 0 | 15891 | 1000000 | 100 | |
| | | U | 3 | 2 | 15889 | | | | | | | | |
| F09 | 350K/0 | M | 1.4 | 1 | 16234 | 1 | 0 | 16220 | 14 | 16235 | 9,00E-06 | 0.0009 | |
| | | U | 1660000 | 16221 | 14 | | | | | | | | |
| E09 | 350K/5 | M | 20000000 | 14935 | 0 | 11316 | 3619 | 0 | 0 | 14935 | 600 | 99.83 | |
| | | U | 33360 | 11316 | 3619 | | | | | | | | |
| D09 | 350K/10 | M | 20000000 | 15498 | 0 | 682 | 14816 | 0 | 0 | 15498 | 19000 | 100 | |
| | | U | 1058 | 682 | 14816 | | | | | | | | |
| C09 | 350K/20 | M | 20000000 | 11865 | 0 | 21 | 11844 | 0 | 0 | 11865 | 480000 | 100 | |

(fortgesetzt)

| Well | Probe | Target | Kopien/ 20μLWell | Positive | Negative | Ch1+Ch2+ | Ch1+Ch2- | Ch1- Ch2+ | Ch1- Ch2- | Akzeptierte Tröpfchen | Verhältnis | Fraktionale Häufigkeit | $MgCl_2$ |
|---|---|---|---|---|---|---|---|---|---|---|---|---|---|
| | | U | 42 | 21 | 11844 | | | | | | | | |
| B09 | 350K/ 3000 | M | 20000000 | 13551 | 0 | 0 | 13551 | 0 | 0 | 13551 | | 100 | |
| | | U | 0 | 0 | 13551 | | | | | | | | |
| A09 | 700K/0 | M | 0 | 0 | 14046 | 0 | 0 | 14046 | 0 | 14046 | 0 | 0 | |
| | | U | 20000000 | 14046 | 0 | | | | | | | | |
| H08 | 700K/5 | M | 20000000 | 13431 | 0 | 13427 | 4 | 0 | 0 | 13431 | 100 | 99.05 | |
| | | U | 192000 | 13427 | 4 | | | | | | | | |
| G08 | 700K/10 | M | 20000000 | 12296 | 0 | 1472 | 10824 | 0 | 0 | 12296 | 6700 | 99.985 | |
| | | U | 3000 | 1472 | 10824 | | | | | | | | |
| F08 | 700K/20 | M | 188000 | 14635 | 5 | 8 | 14627 | 0 | 5 | 14640 | 15000 | 100 | |
| | | U | 12.8 | 8 | 14632 | | | | | | | | |
| E08 | 700K/ 3000 | M | 20000000 | 14344 | 0 | 0 | 14344 | 0 | 0 | 14344 | | 100 | |
| | | U | 0 | 0 | 14344 | | | | | | | | |
| H11 | NTC | M | 0 | 0 | 12407 | 0 | 0 | 0 | 12407 | 12407 | 0 | 0 | |
| | | U | 0 | 0 | 12407 | | | | | | | | |

**Tabelle 31:** Bestimmung der ***GSTP1-*** und *RASSF1A*-Methylierung mit der BBPA-dPCR-Methode unter Verwendung der GSTP1-116bp- Primer (SEQ ID No: 93 und 94; 4,5 mM $MgCl_2$-Konzentration; 53,7°C Annealingtemperatur und 15x Zyklen in der BBPA), GSTP1-120bp-Primer (SEQ ID No: 5 und 6; 2,5 mM $MgCl_2$-Konzentration; 50,7°C Annealingtemperatur und 15x Zyklen in der BBPA) und RASSF1A-117bp-Primer (SEQ ID No: 3 und 4; 2,5 mM $MgCl_2$-Konzentration; 55,7°C Annealingtemperatur und 15x Zyklen in der BBPA) in Serumproben gesunder Frauen (K1-K25) und Patientinnen mit Mamma-CA (P1-P16) und Ovarial-CA (P17-P23), einer Serumprobe der Patientin P8a nach 8-wöchiger Chemotherapie (P8b) und einer Patientin (P24) mit pathogener BRCA1-Mutation. Pathologische Werte für die Methylierungen sind dick gedruckt. Die *cut-off*-Werte für die jeweiligen Gene sind in der ersten Zeile angegeben.

| **ID** | **Alter** | **CA15-3 bzw. CA125 in U/ml** | **GSTP1-116bp <0,05%** | **GSTP1-120bp <0,067%** | **RASSF1A-117bp <3,0%** | **Klinische Befunde** |
|---|---|---|---|---|---|---|
| K1 | 21 | | 0 | 0 | 1,1 | |
| K2 | 21 | | 0 | 0 | 1 | |
| K3 | 29 | | 0 | 0 | 0,94 | |
| K4 | 23 | | 0 | 0 | 0,9 | |
| K5 | 20 | | 0 | 0,0022 | 0,63 | |
| K6 | 18 | | 0 | 0 | 0,4 | |
| K7 | 26 | | 0 | 0 | 1,5 | |
| K8 | 20 | | 0 | 0 | 2,7 | |
| K9 | 31 | | 0,029 | 0 | 1,26 | |
| K10 | 45 | | 0 | 0 | 1,6 | |
| K11 | 48 | | 0 | 0 | 0,7 | |
| K12 | 34 | | 0 | 0 | 0,8 | |
| K13 | 16 | | 0 | 0 | 0,8 | |
| K14 | 23 | | 0 | 0 | 1,6 | |
| K15 | 20 | | 0 | 0 | 0,9 | |
| K16 | 43 | | 0,05 | 0 | 1,1 | |
| K17 | 41 | | 0 | 0,017 | 0 | |
| K18 | 22 | | 0 | 0 | 1 | |
| K19 | 71 | | 0 | 0,014 | 3 | |
| K20 | 36 | | 0 | 0,055 | 0,4 | |
| K21 | 25 | | 0 | 0 | 1,3 | |
| K22 | 34 | | 0 | 0,067 | 0,6 | |
| K23 | 46 | | 0,03 | 0 | 1,4 | |
| K24 | 19 | | 0 | 0 | 0,9 | |
| K25 | 49 | | 0 | 0 | 0 | |
| | | **CA15-3** | | | | |
| P1 | 57 | 28,5 | **0,19** | 0 | **3,4** | bifokales invasives Mamma-CA |
| P2 | 67 | 27,8 | **0,54** | 0 | 0,88 | Invasives Mamma-CA links |

(fortgesetzt)

| | | | | | | |
|---|---|---|---|---|---|---|
| | | **CA15-3** | | | | |
| P3 | 42 | 25,4 | **7,9** | 0 | **1** | lymphogen, ossär, pulmonal und hepatisch metastasierendes Mamma-CA rechts |
| P4 | 71 | 99,7 | **0,65** | 0 | **5,7** | Mamma-CA bei bekannter *BRCA1*-Mutation |
| P5 | 75 | 59,0 | **27,6** | 0 | 1,4 | sekundär ossär, lymphogen, pleural und pulmonal metastasiertes Mamma-CA |
| P6 | 76 | 73,0 | **1,9** | 0 | **7,6** | sekundär cutan, ossär und in den Magen metastasiertes invasiv-lobuläres Mamma-CA |
| P7 | 47 | 504,3 | **37,7** | **0,15** | **10,0** | sekundär ossär metastasiertes Mamma-CA mit Tumorprogression |
| P8a | 63 | 941,3 | **0,9** | 0 | **18,0** | primär ossär und hepatisch metastasiertes Mamma-CA |
| P9 | 58 | 120,5 | 0,04 | 0 | **6,2** | ossär, lymphogen, pleural und pulmonal metastasiertes Mamma-CA |
| P10 | 87 | 1098 | **3,6** | **0,12** | 1,5 | sekundär ossär metastasiertes Mamma-CA mit Tumorprogression |
| P11 | 73 | 106,8 | 0 | 0,011 | **6,7** | sekundär ossär, lymphogen, pleural und pulmonal metastasiertes Mamma-CA |
| P12 | 64 | 130,7 | **11,6** | 0 | 0,69 | sekundär ossär und hepatisch metastasiertes Mamma-CA |
| P13 | 60 | 157,5 | **5,3** | 0 | **3,4** | sekundär pulmonal, pleural und ossär metastasiertes Mamma-CA |
| P14 | 76 | 145,2 | **8,8** | 0 | 0,32 | sekundär lymphogen, pleural und in die Nebeniere metastasiertes Mamma-CA |
| P15 | 30 | 178,5 | **18,4** | 0,005 | **50,1** | sekundär ossär, lymphogen, pleural, hepatisch und meningeal metastasiertes Mamma-CA |
| P16 | 56 | 650 | **1,39** | 0 | 2,3 | sekundär pleural und hepatisch metastasiertes Mamma-CA |
| | | **CA125** | | | | |
| P17 | 70 | 57,2 | **1,12** | 0 | 1,02 | seröses high-grade Ovarial-CA |
| P18 | 76 | 1871 | **2,7** | 0 | 1,1 | lymphogen und hepatisch metastasiertes Ovarialkarzinom |
| P19 | 57 | 23,8 | 0 | **6,1** | **9,0** | maligne mesodermale Mischtumor im Bereich des Ovars |
| P20 | 51 | 188,6 | 0 | 0 | **6,0** | spätrezidivierendes Ovarialkarzinom mit hepatischer und lymphogener Metastasierung |

(fortgesetzt)

| | | | | | | |
|---|---|---|---|---|---|---|
| | | **CA125** | | | | |
| P21 | 54 | 903,2 | 0 | **1,3** | **7,0** | lymphogen und peritoneal metastasiertes Ovarialkarzinom |
| P22 | 73 | 56,2 | **0,12** | 0,005 | 2,5 | frührezidivierendes Ovarialkarzinom mit Lebermetastasen |
| P23 | 49 | 196,7 | **1,1** | **5,8** | 0 | spätrezidivierendes Ovarialkarzinom |
| | | | | | | |
| | | **CA15-3** | | | | |
| P8b | 63 | 133 | 0 | 0 | 2 | Serumprobe der Patientin P8a nach 8-wöchiger Chemotherapie |
| P24 | 32 | 9,8 | **0,54** | 0 | 1,1 | pathogene BRCA1-Mutation, prophylaktische Mastektomie |

**Literaturverzeichnis**

[1] Mikeska T, Candiloro IL, DobrovicA. Epigenomics. 2010; 2:561-73.
[2] Kristensen LS and Hansen LL. Clin Chem. 2009; 8: 1471-83.
[3] Shen L and Waterland RA. Curr Opin Clin Nutr Metab Care 2007; 5: 576-81.
[4] Hernández HG, Tse MY, Pang SC, et al. BioTechniques. 2013; 4: 181-97.
[5] Herman JG, Graff JR, Myöhänen S, et al. Proc Nat Acad Sci USA. 1996; 18: 9821-26.
[6] Kristensen LS, Wojdacz TK, Thestrup BB, et al. BMC Cancer 2009; 9: 453.
[7] Candiloro IL, Mikeska T, Hokland P, et al. Epigenetics Chromatin. 2008; 1: 7.
[8] Kraytsberg Y and Khrapko K. Expert Review of Molecular Diagnostics. 2005; 5: 809-15.
[9] Applications Guide dPCR, Biorad. http://www.biorad.com/webroot/web/pdf/lsr/literature/Bulletin_6407.pdf
[10] Grützmann R, Molnar B, Pilarsky C, et al. PLoS one. 2008; 3:e3759.
[11] How Kit A, Nielsen HM, Tost J. Biochimie. 2012 Nov;94(11):2314-37.
[12] Stewart GD, Van Neste L, Delvenne P, et al. J Urol. 2013; 189: 110-1116.
[13] Tombal B. EurUrol. 2012;62:997-8.
[14] Tombal B. Eur Urology Suppl. 2006; 5:511-513.
[15] Castellanos-Rizaldos E, Milbury CA, Karatza E, et al., PLoS One. 2014; 9(4):e94103.
[16] Warnecke PM, Stirzaker C, Melki JR, Millar DS, Paul CL, Clark SJ. Nucleic Acids Res. 1997 Nov 1;25(21):4422-6.
[17] Wojdacz TK, Dobrovic A, Hansen LL. Nat Protoc. 2008;3(12):1903-8. doi: 10.1038/nprot.2008.191.
[18] Wojdacz TK, Hansen LL, Dobrovic A. BMC Res Notes. 2008 Jul 28; 1:54. doi: 10.1186/1756-0500-1-54.
[19] Wojdacz TK, Hansen LL: BioTechnique. 2006; 41: 274-8.
[20] Hubbard RA. Annals of Internal Medicine. 2011; 155: 481-92.
[21] Redshaw N. et al. BMC Genomics 2014; 15:1174.
[22] Okino ST et al. Mol. Carcinogenesis 2007; 46: 839-846.
[23] Wojdacz T et al. Epigenetics. 2009 May 16;4(4):231-4.


**Patentansprüche**

1. Verfahren zur Diagnose einer Tumorerkrankung in einer isolierten Probe mit den Schritten

a) Bisulfit-Umwandlung der DNA in der Probe,
b) Amplifikation von methylierten und nicht-methylierten DNA-Sequenzen mittels PCR,
wobei jeweils die Annealingtemperatur, $MgCl_2$-Konzentration und Zyklenanzahl, sowie 5'-CG-3'-Gehalt der Primer so eingestellt werden, dass
die methylierten DNA-Sequenzen stärker amplifiziert werden als die nicht-methylierten DNA-Sequenzen, sofern die methylierten DNA-Sequenzen tumorspezifisch sind, oder
die nicht-methylierten DNA-Sequenzen stärker amplifiziert werden als die methylierten DNA-Sequenzen, sofern die nicht-methylierte DNA-Sequenzen tumorspezifisch sind,
c) Quantifizierung der methylierten und nicht-methylierten DNA mittels digitaler PCR, wobei die in der digitalen PCR eingesetzte Anzahl der in Schritt b) erhaltenen DNA-Kopien gemittelt mindestens 8 Kopien pro Kompartiment beträgt.

2. Verfahren nach Anspruch 1, **dadurch gekennzeichnet, dass** die isolierte Probe eine Körperflüssigkeit ist.

3. Verfahren nach Anspruch 1 oder 2, **dadurch gekennzeichnet, dass** im Schritt b) Primer eingesetzt werden, welche methylierte und nicht-methylierte DNA-Sequenzen der Gene PLA2R1, RASSF1A und GSTP1 amplifizieren, wobei methylierte DNA stärker amplifiziert wird als die nicht-methylierte DNA.

4. Verfahren nach Anspruch 3, **dadurch gekennzeichnet, dass** die in Schritt b) zur Prä-Amplifikation von methylierten und nicht-methylierten DNA-Sequenzen der Gene PLA2R1, RASSF1A und GSTP1 verwendeten Primer ausgewählt sind aus den Sequenzen 1 bis 13, 53, 61, 62, 91 bis 98 und 104 bis 111.

5. Verfahren nach einem der Ansprüche 1 bis 4, **dadurch gekennzeichnet, dass** in Schritt b) zusätzlich methylierte und nicht-methylierte Sequenzen des/der Gene AOX-1, SERPINE-1, Thrombomodulin und/oder Septin-9 prä-amplifiziert werden und die Primer entsprechend ausgewählt sind und in Schritt c) zusätzlich eine Quantifizierung der methylierten und nicht-methylierten DNA-Sequenzen dieser Gene erfolgt.

6. Verfahren nach Anspruch 5, **dadurch gekennzeichnet, dass** die in Schritt b) zur Amplifikation von methylierten und nicht-methylierten DNA-Sequenzen des/der Gene AOX-1, SERPINE-1, Thrombomodulin und/oder Septin-9 verwendeten Primer ausgewählt sind aus den Sequenzen 14 bis 19, 63 bis 76, 99 bis 103 und 126 bis 144.

7. Verfahren nach einem der Ansprüche 1 bis 6, **dadurch gekennzeichnet, dass** in Schritt b) zur Prä-Amplifikation die Primerpaare gleichzeitig in der PCR (multiplex PCR) eingesetzt werden.

8. Verfahren nach einem der Ansprüche 1 bis 7, **dadurch gekennzeichnet, dass** die Quantifizierung der methylierten und nicht-methylierten DNA-Sequenzen in Schritt c) mittels Sonden erfolgt, wobei die Sonden für die methylierten DNA-Sequenzen insgesamt für jedes Gen mindestens drei 5'-CG-3' Dinukleotide aufweisen und/oder die Sonden für die nicht-methylierten DNA-Sequenzen insgesamt für jedes Gen mindestens drei 5'-CA-3' Dinukleotide oder mindestens drei 5'-TG-3' Dinukleotide aufweisen, bevorzugt sind die Sonden ausgewählt aus den Sequenzen 20 bis 52, 54 bis 60, 77 bis 90, 112 bis 125 und 145 bis 184.

9. Verwendung eines Kits zur Diagnose einer Tumorerkrankung in einer isolierten Probe enthaltend:

i) Primer zur Prä-Amplifikation von methylierten und nicht-methylierten DNA-Sequenzen der Gene PLA2R1, RASSF1A und GSTP1 mittels PCR, wobei die Primer jeweils so ausgewählt werden, dass Forward- und Reverse-Primer keine bis sieben 5'-CG-3' Dinukleotidsequenzen pro Primerpaar, bevorzugt zwei bis sechs, vorzugsweise drei bis fünf, besonders bevorzugt ein bis maximal drei, weiter bevorzugt ein oder zwei 5'-CG-3' Dinukleotidsequenzen pro Primerpaar, 5'-CG-3' Dinukleotide aufweisen,
ii) Sonden zur Quantifizierung der methylierten und nicht-methylierten DNA-Sequenzen der Gene PLA2R1, RASSF1A und GSTP1.

zur Diagnose einer Tumorerkrankung in einer isolierten Probe mit einem Verfahren nach einem der Ansprüche 1 bis 8.

10. Verwendung nach Anspruch 9, wobei das Kit Primer ausgewählt aus den Sequenzen 1 bis 13, 53, 61, 62, 91 bis 98 und 104 bis 111 enthält.

**11.** Verwendung nach Anspruch 9 oder 10, wobei das Kit zusätzlich Primer zur Amplifikation von methylierten und nicht-methylierten DNA-Sequenzen des/der Gene AOX-1, SERPINE-1, Thrombomodulin und/oder Septin-9 enthält.

**12.** Verwendung nach einem der Ansprüche 9 bis 11, **dadurch gekennzeichnet, dass** die Sonden zur Quantifizierung der methylierten DNA-Sequenzen für jedes Gen insgesamt mindestens drei 5'-CG-3' Dinukleotide aufweisen.

**13.** Verwendung nach einem der Ansprüche 9 bis 12, **dadurch gekennzeichnet, dass** die Sonden zur Quantifizierung der nicht methylierten DNA-Sequenzen für jedes Gen insgesamt mindestens drei 5'-CA-3' Dinukleotide oder mindestens drei 5'-TG-3' Dinukleotide aufweisen.

**14.** Verwendung eines Primers mit einer Nukleinsäuresequenz ausgewählt aus den Sequenzen gemäß 1 bis 19, 53, 61 bis 76, 91 bis 111 sowie 126 bis 144 oder einer Sonde mit einer Nukleinsäuresequenz ausgewählt aus den Sequenzen gemäß 20 bis 52, 54 bis 60, 77 bis 90, 112 bis 125 und 145 bis 184 zur Diagnose einer Tumorerkrankung in einer isolierten Probe mit einem Verfahren nach einem der Ansprüche 1 bis 8.

**15.** Verwendung eines Verfahrens nach einem der Ansprüche 1 bis 8

i.) zur Differentialdiagnose von benigner Prostata-Hyperplasie, Prostatitis und Prostatakarzinom in einer isolierten Probe insbesondere bei erhöhten PSA-Werten oder bei einem anderweitig begründeten Verdacht auf ein Prostatakarzinom,
ii.) zur Diagnose eines Mamma- und Ovarialkarzinoms in einer isolierten Probe insbesondere bei nicht eindeutigen Befunden in der Mammografie oder Sonografie,
iii.) zur Diagnose eines Mamma- und Ovarialkarzinoms in einer isolierten Probe insbesondere bei Vorliegen einer pathologischen Genmutation verbunden mit einem erhöhten familiären Risiko für Mamma- und Ovarialkarzinomen zur Risikoabschätzung (Zeitpunkt) und Entscheidung für eine prophylaktische Mastektomie und/oder Ovariektomie, insbesondere wenn die Familienplanung der betroffenen Patientin noch nicht beendet ist, oder
iv.) zur Verlaufskontrolle einer Tumorerkrankung, insbesondere zum Ausschluss einer minimalen Resterkrankung (MRD).

## Claims

**1.** Method for diagnosing a tumor disease in an isolated sample, comprising the steps of

a) bisulphite conversion of the DNA in the sample,
b) amplifying methylated and unmethylated DNA sequences by means of PCR,
wherein each the annealing temperature, $MgCl_2$ concentration and number of cycles as well as 5'-CG-3' content are set such that
the methylated DNA sequences being amplified to a greater extent than the unmethylated DNA sequences, if the methylated DNA sequences are tumourspecific, or
the unmethylated DNA sequences being amplified to a greater extent than the methylated DNA sequences, if the unmethylated DNA sequences are tumourspecific,
c) quantifying the methylated and unmethylated DNA by means of digital PCR, wherein the average number of DNA copies obtained in step b) and used in the digital PCR being at least 8 copies per compartment.

**2.** Method according to claim 1, **characterised in that** the isolated sample is a body fluid.

**3.** Method according to either claim 1 or claim 2, **characterised in that** primers that amplify methylated and unmethylated DNA sequences in the genes PLA2RA, RASSF1A and GSTP1 are used in step b), wherein methylated DNA being amplified to a greater extent than unmethylated DNA.

**4.** Method according to claim 3, **characterised in that** the primers used in step b) for pre-amplifying methylated and unmethylated DNA sequences in the genes PLA2R1, RASSF1A and GSTP1 are selected from sequences 1 to 13, 53, 61, 62, 91 to 98, and 104 to 111

5. Method according to any of claims 1 to 4, **characterised in that** methylated and unmethylated sequences in the gene(s) AOX-1, SERPINE-1, thrombomodulin and/or septin-9 are additionally pre-amplified in step b) and the primers are selected accordingly, and the methylated and unmethylated DNA sequences in these genes are additionally quantified in step c).

6. Method according to claim 5, **characterised in that** the primers used in step b) for amplifying methylated and unmethylated DNA sequences in the genes AOX-1, SERPINE-1, thrombomodulin and/or septin-9 are selected from sequences 14 to 19, 63 to 76, 99 to 103, and 126 to 144.

7. Method according to any of claims 1 to 6, **characterised in that** in step b) for preamplification, the primer pairs are simultaneously used in the PCR (multiplex PCR).

8. Method according to any of claims 1 to 7, **characterised in that** the methylated and unmethylated DNA sequences are quantified in step c) using probes,
wherein the probes for the methylated DNA sequences comprise a total of at least three 5'-CG-3' dinucleotides for each gene and/or the probes for the unmethylated DNA sequences comprise a total of at least three 5'-CA-3' dinucleotides or at least three 5'-TG-3' dinucleotides for each gene, preferably the probes being selected from the sequences 20 to 52, 54 to 60, 77 to 90, 112 to 125, and 145 to 184.

9. Use of a kit for diagnosing a tumor disease in an isolated sample, comprising:

   i) primers for pre-amplifying methylated and unmethylated DNA sequences in the genes PLA2R1, RASSF1A and GSTP1 by means of PCR, wherein each primer is selected such that forward and reverse primers comprise zero to seven 5'-CG-3' dinucleotide sequences per primer pair, preferably two to six, especially three to five, particularly preferably one to at most three, more preferably either one or two 5'-CG-3' dinucleotide sequences per primer pair,
   ii) probes for quantifying the methylated and unmethylated DNA sequences in the genes PLA2R1, RASSF1A and GSTP1,

   for diagnosing a tumor disease in an isolated sample with a method according to any of claims 1 to 8.

10. Use according claim 9, wherein the kit comprises primers selected from sequences 1 to 13, 53, 61, 62, 91 to 98, and 104 to 111.

11. Use according claim 9 or 10, wherein the kit further comprises primers for amplifying methylated and unmethylated DNA sequences for the gene(s) AOX-1, SERPINE-1, thrombomodulin and/or septin-9.

12. Use according to any of claims 9 to 11, **characterised in that** the probes for quantifying the methylated DNA sequences comprise a total of at least three 5'-CG-3' dinucleotides for each gene.

13. Use according to any of claims 9 to 12, **characterised in that** the probes for quantifying the unmethylated DNA sequences comprise a total of at least three 5'-CA-3' dinucleotides or at least three 5'-TG-3' dinucleotides for each gene.

14. Use of a primer comprising a nucleic acid sequence selected from the sequences according to 1 to 19, 53, 61 to 76, 91 to 111, and 126 to 144, or of a probe comprising a nucleic acid sequence selected from the sequences according to 20 to 52, 54 to 60, 77 to 90, 112 to 125, and 145 to 184 for diagnosing a tumor disease in an isolated sample with a method according to any of claims 1 to 8.

15. Use of a method according to any of claims 1 to 8

   i) for the differential diagnosis of benign prostatic hyperplasia, prostatitis and prostate cancer in an isolated sample, in particular in the event of elevated PSA values or in other cases where prostate cancer is reasonably suspected,
   ii) for diagnosing breast and ovarian cancer in an isolated sample, in particular when the mammogram or ultrasound have not produced clear findings,
   iii) for diagnosing breast and ovarian cancer in an isolated sample, in particular in the event of a pathological gene mutation in combination with a higher family risk of breast and ovarian cancer, in order to estimate the risk (point in time) and opt for a prophylactic mastectomy and/or ovariectomy, in particular if the patient in question is still planning a family, or
iv) for monitoring the development of a tumor disease, in particular to rule out a minimal residual disease (MRD).

## Revendications

1. Procédé de diagnostic d'une maladie tumorale dans un échantillon isolé, comportant les étapes

   a) de conversion au bisulfite de l'ADN dans l'échantillon,
   b) d'amplification de séquences d'ADN méthylées et non méthylées par PCR,
   la température d'hybridation, la concentration en $MgCl_2$ et le nombre de cycles, ainsi que la teneur en 5'-CG-3' des amorces, étant respectivement réglés de sorte que les séquences d'ADN méthylées sont amplifiées davantage que les séquences d'ADN non méthylées, à condition que les séquences d'ADN méthylées soient spécifiques à une tumeur, ou de sorte que
   les séquences d'ADN non méthylées sont amplifiées davantage que les séquences d'ADN méthylées, à condition que les séquences d'ADN non méthylées soient spécifiques à une tumeur,
   c) de quantification de l'ADN méthylé et non méthylé par PCR numérique, le nombre de copies d'ADN utilisées dans la PCR numérique, lesquelles copies d'ADN ont été obtenues à l'étape b), étant en moyenne d'au moins 8 copies par compartiment.

2. Procédé selon la revendication 1, **caractérisé en ce que** l'échantillon isolé est un fluide corporel.

3. Procédé selon la revendication 1 ou 2, **caractérisé en ce que** des amorces sont utilisées à l'étape b), lesquelles amplifient des séquences d'ADN méthylées et non méthylées des gènes PLA2R1, RASSF1A et GSTP1, l'ADN méthylé étant amplifié davantage que l'ADN non méthylé.

4. Procédé selon la revendication 3, **caractérisé en ce que** les amorces utilisées à l'étape b) pour la pré-amplification de séquences d'ADN méthylées et non méthylées des gènes PLA2R1, RASSF1A et GSTP1 sont choisies parmi les séquences 1 à 13, 53, 61, 62, 91 à 98 et 104 à 111.

5. Procédé selon l'une des revendications 1 à 4, **caractérisé en ce que** des séquences méthylées et non méthylées du/des gène(s) AOX-1, SERPINE-1, thrombomoduline et/ou septine-9 sont également pré-amplifiées à l'étape b), et que les amorces sont choisies en conséquence et que, à l'étape c), les séquences d'ADN méthylées et non méthylées de ces gènes sont également quantifiées.

6. Procédé selon la revendication 5, **caractérisé en ce que** les amorces utilisées à l'étape b) pour l'amplification de séquences d'ADN méthylées et non méthylées du/des gène(s) AOX-1, SERPINE-1, thrombomoduline et/ou septine-9 sont choisies parmi les séquences 14 à 19, 63 à 76, 99 à 103 et 126 à 144.

7. Procédé selon l'une des revendications 1 à 6, **caractérisé en ce qu'**à l'étape b), les paires d'amorces sont utilisées simultanément dans la PCR (PCR multiplexe) pour la pré-amplification.

8. Procédé selon l'une des revendications 1 à 7, **caractérisé en ce que** les séquences d'ADN méthylées et non méthylées sont quantifiées à l'étape c) au moyen de sondes, les sondes destinées aux séquences d'ADN méthylées présentant au total au moins trois dinucléotides 5'-CG-3' pour chaque gène, et/ou les sondes destinées aux séquences d'ADN non méthylées présentant au total au moins trois dinucléotides 5'-CA-3' ou au moins trois dinucléotides 5'-TG-3' pour chaque gène, les sondes étant de préférence choisies parmi les séquences 20 à 52, 54 à 60, 77 à 90, 112 à 125 et 145 à 184.

9. Utilisation d'un kit servant au diagnostic d'une maladie tumorale dans un échantillon isolé contenant :

   i) des amorces servant à la pré-amplification de séquences d'ADN méthylées et non méthylées des gènes PLA2R1, RASSF1A et GSTP1 par PCR, les amorces étant respectivement choisies de sorte que les amorces sens et antisens présentent zéro à sept séquences de dinucléotides 5'-CG-3' par paire d'amorces, de préférence deux à six, préférablement trois à cinq, de manière particulièrement préférée une à trois au plus, de manière davantage préférée une ou deux séquences de dinucléotides 5'-CG-3' par paire d'amorces,
   ii) des sondes servant à la quantification des séquences d'ADN méthylées et non méthylées des gènes PLA2R1, RASSF1A et GSTP1.

pour le diagnostic d'une maladie tumorale dans un échantillon isolé au moyen d'un procédé selon l'une des revendications 1 à 8.

**10.** Utilisation selon la revendication 9, dans laquelle le kit contient des amorces choisies parmi les séquences 1 à 13, 53, 61, 62, 91 à 98 et 104 à 111.

**11.** Utilisation selon la revendication 9 ou 10, dans laquelle le kit contient également des amorces servant à l'amplification de séquences d'ADN méthylées et non méthylées du/des gène(s) AOX-1, SERPINE-1, thrombomoduline et/ou septine-9.

**12.** Utilisation selon l'une des revendications 9 à 11, **caractérisée en ce que** les sondes servant à la quantification des séquences d'ADN méthylées présentent au total au moins trois dinucléotides 5'-CG-3' pour chaque gène.

**13.** Utilisation selon l'une des revendications 9 à 12, **caractérisée en ce que** les sondes servant à la quantification des séquences d'ADN non méthylées présentent au total au moins trois dinucléotides 5'-CA-3' ou au moins trois dinucléotides 5'-TG-3' pour chaque gène.

**14.** Utilisation d'une amorce avec une séquence d'acide nucléique choisie parmi les séquences selon 1 à 19, 53, 61 à 76, 91 à 111 ainsi que 126 à 144, ou d'une sonde avec une séquence d'acide nucléique choisie parmi les séquences selon 20 à 52, 54 à 60, 77 à 90, 112 à 125 et 145 à 184, pour diagnostiquer une maladie tumorale dans un échantillon isolé au moyen d'un procédé selon l'une des revendications 1 à 8.

**15.** Utilisation d'un procédé selon l'une des revendications 1 à 8

i.) pour le diagnostic différentiel d'une hyperplasie bénigne de la prostate, d'une prostatite et d'un cancer de la prostate dans un échantillon isolé, en particulier lors de valeurs APS élevées ou lors de toute autre suspicion justifiée de cancer de la prostate,
ii.) pour le diagnostic d'un cancer du sein et de l'ovaire dans un échantillon isolé, en particulier lors de résultats ambigus de la mammographie ou de l'échographie,
iii.) pour le diagnostic d'un cancer du sein et de l'ovaire dans un échantillon isolé, en particulier s'il existe une mutation génique pathologique associée à un risque familial accru de cancer du sein et de l'ovaire, pour l'évaluation du risque (temps) et la décision d'une mastectomie et/ou ovariectomie prophylactiques, en particulier si la planification familiale de la patiente concernée n'est pas encore terminée, ou
iv.) pour le suivi de l'évolution d'une maladie tumorale, en particulier pour exclure une maladie résiduelle minime (MRD).

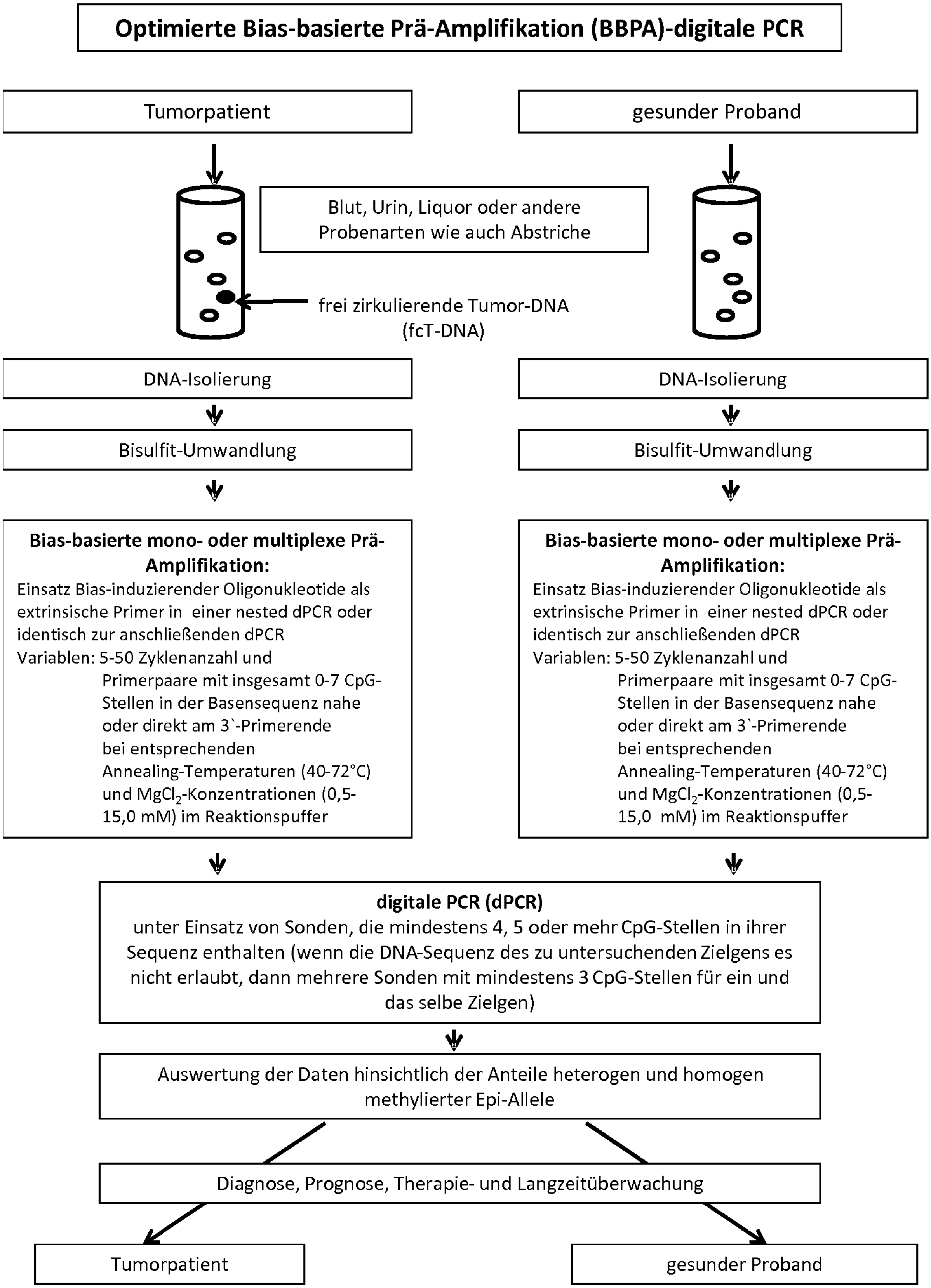
Optimierte Bias-basierte Prä-Amplifikation (BBPA)-digitale PCR
Tumorpatient
gesunder Proband
Blut, Urin, Liquor oder andere Probenarten wie auch Abstriche
frei zirkulierende Tumor-DNA (fcT-DNA)
DNA-Isolierung
DNA-Isolierung
Bisulfit-Umwandlung
Bisulfit-Umwandlung
Bias-basierte mono- oder multiplexe Prä-Amplifikation:
Einsatz Bias-induzierender Oligonukleotide als extrinsische Primer in einer nested dPCR oder identisch zur anschließenden dPCR
Variablen: 5-50 Zyklenanzahl und Primerpaare mit insgesamt 0-7 CpG-Stellen in der Basensequenz nahe oder direkt am 3`-Primerende bei entsprechenden Annealing-Temperaturen (40-72°C) und $MgCl_2$-Konzentrationen (0,5-15,0 mM) im Reaktionspuffer
Bias-basierte mono- oder multiplexe Prä-Amplifikation:
Einsatz Bias-induzierender Oligonukleotide als extrinsische Primer in einer nested dPCR oder identisch zur anschließenden dPCR
Variablen: 5-50 Zyklenanzahl und Primerpaare mit insgesamt 0-7 CpG-Stellen in der Basensequenz nahe oder direkt am 3`-Primerende bei entsprechenden Annealing-Temperaturen (40-72°C) und $MgCl_2$-Konzentrationen (0,5-15,0 mM) im Reaktionspuffer
digitale PCR (dPCR)
unter Einsatz von Sonden, die mindestens 4, 5 oder mehr CpG-Stellen in ihrer Sequenz enthalten (wenn die DNA-Sequenz des zu untersuchenden Zielgens es nicht erlaubt, dann mehrere Sonden mit mindestens 3 CpG-Stellen für ein und das selbe Zielgen)
Auswertung der Daten hinsichtlich der Anteile heterogen und homogen methylierter Epi-Allele
Diagnose, Prognose, Therapie- und Langzeitüberwachung
Tumorpatient
gesunder Proband

**Fig. 1**

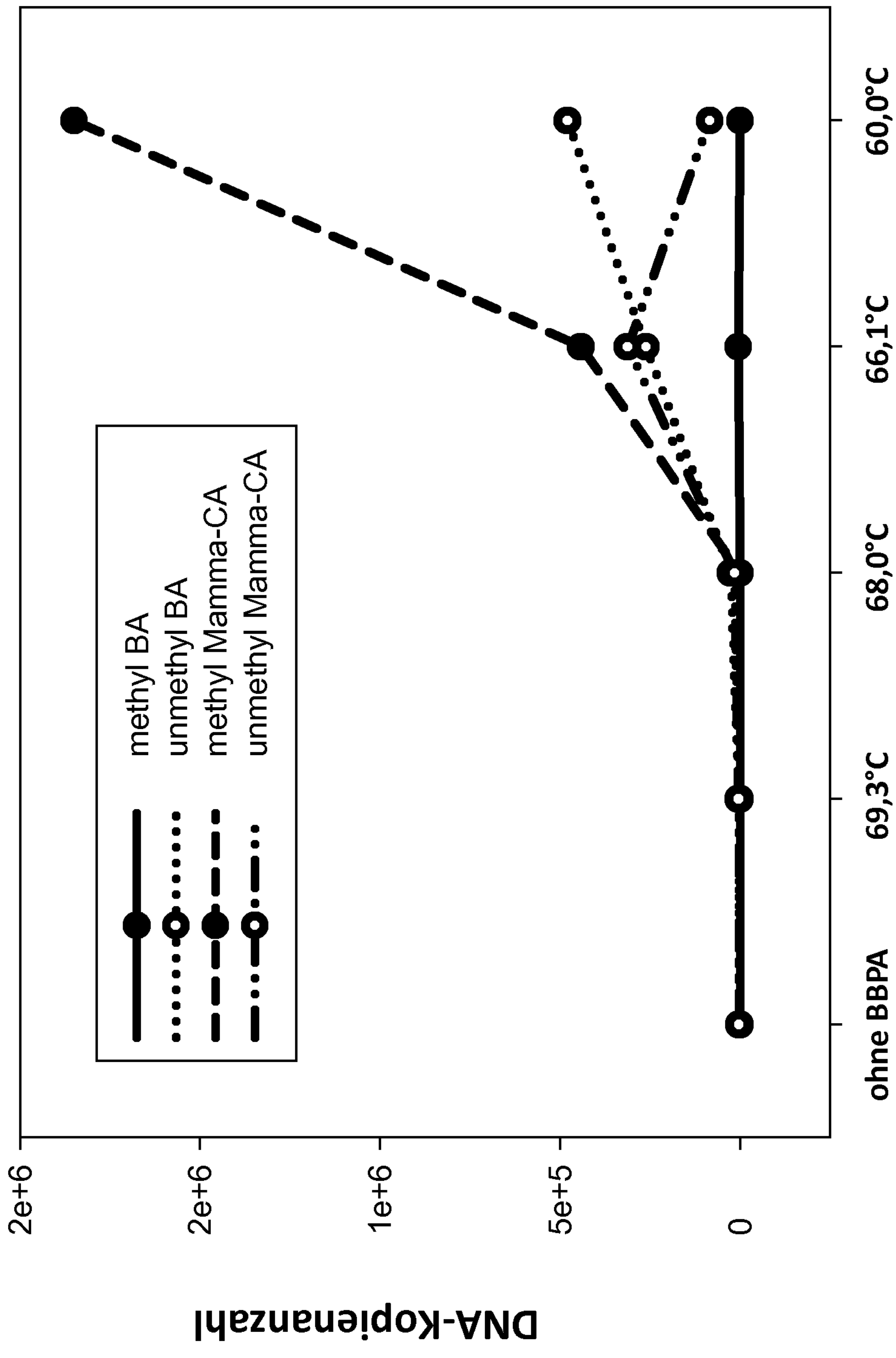
DNA-Kopienanzahl
0
5e+5
1e+6
2e+6
2e+6
ohne BBPA
69,3°C
68,0°C
66,1°C
60,0°C
methyl BA
unmethyl BA
methyl Mamma-CA
unmethyl Mamma-CA

Fig. 2

Fig. 3

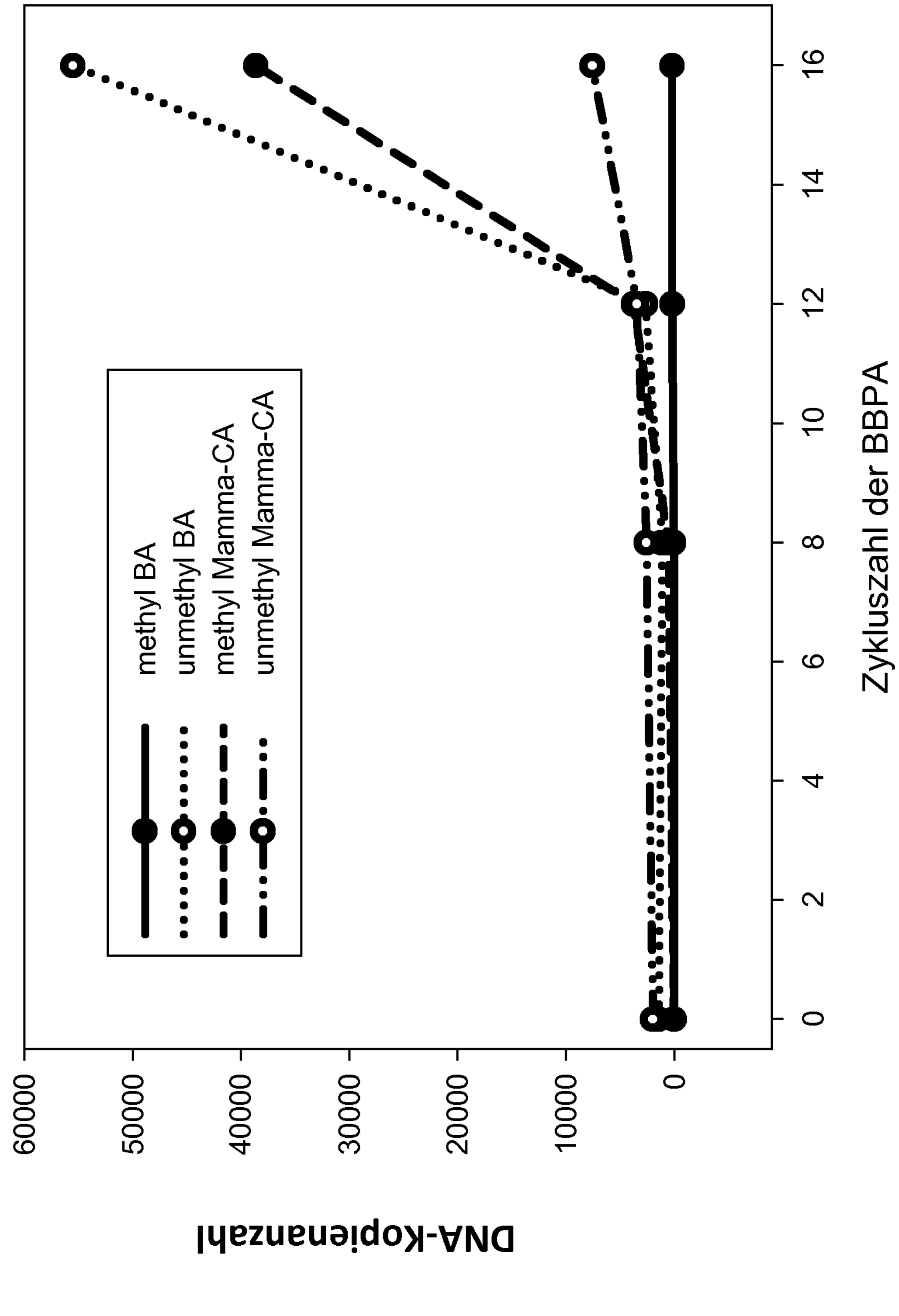
methyl BA
unmethyl BA
methyl Mamma-CA
unmethyl Mamma-CA
DNA-Kopienanzahl
60000
50000
40000
30000
20000
10000
0
0
2
4
6
8
10
12
14
16
Zykluszahl der BBPA

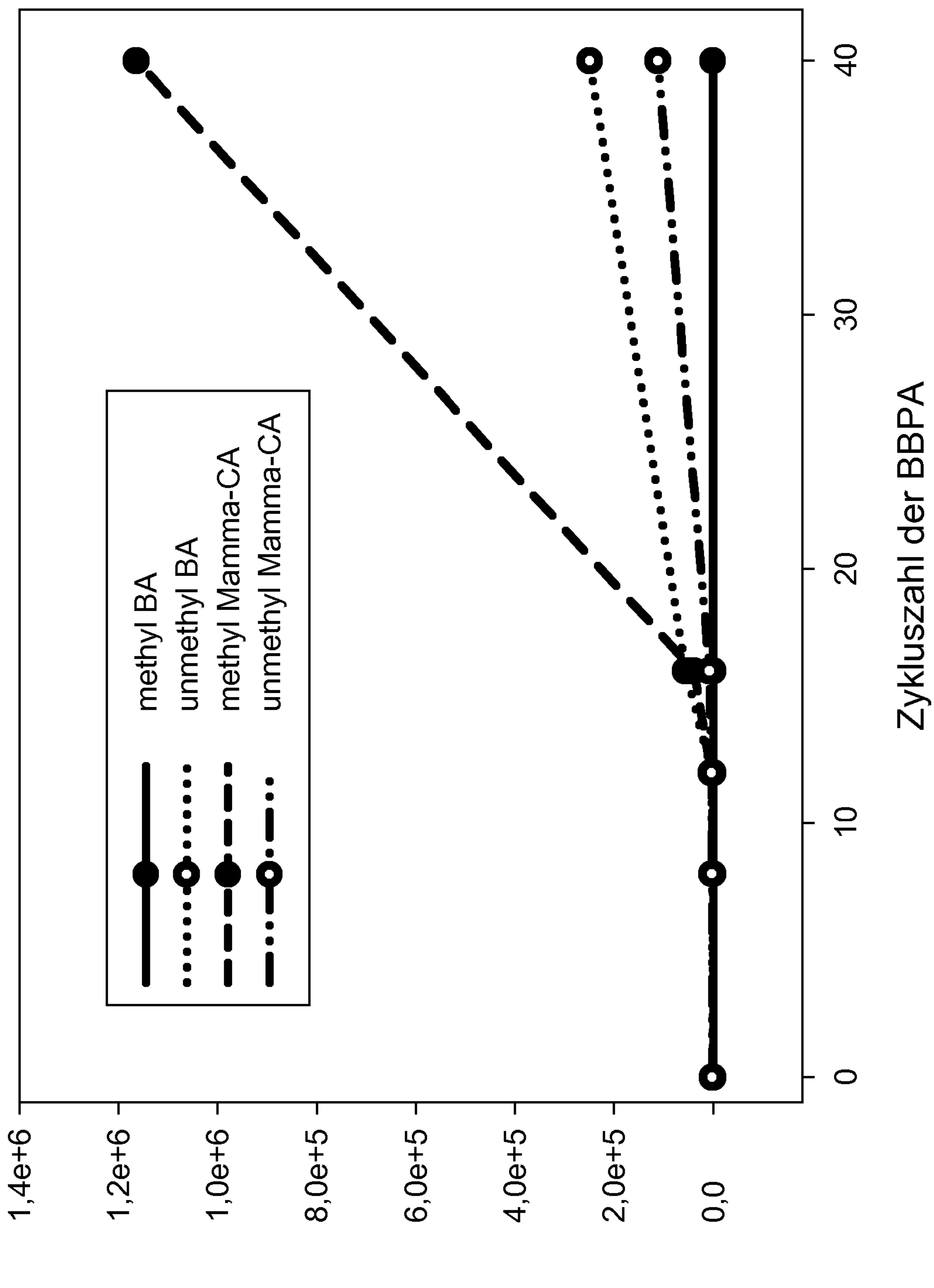
methyl BA
unmethyl BA
methyl Mamma-CA
unmethyl Mamma-CA
DNA-Kopienanzahl
0,0
2,0e+5
4,0e+5
6,0e+5
8,0e+5
1,0e+6
1,2e+6
1,4e+6
0
10
20
30
40
Zykluszahl der BBPA

Fig. 4

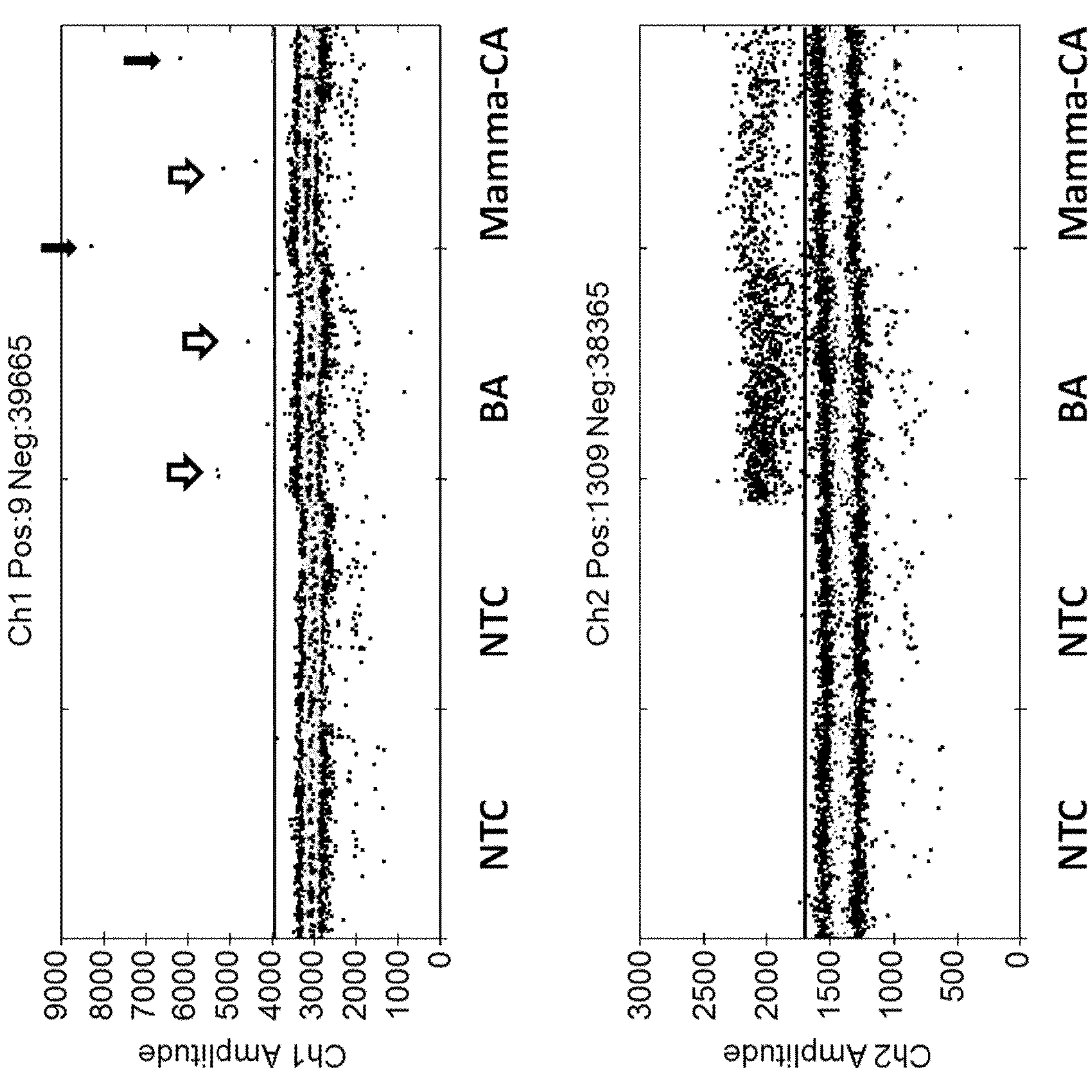
Ch1 Pos:9 Neg:39665
Ch1 Amplitude
9000
8000
7000
6000
5000
4000
3000
2000
1000
0
Ch2 Pos:1309 Neg:38365
Ch2 Amplitude
3000
2500
2000
1500
1000
500
0
NTC
NTC
BA
Mamma-CA

Fig. 5

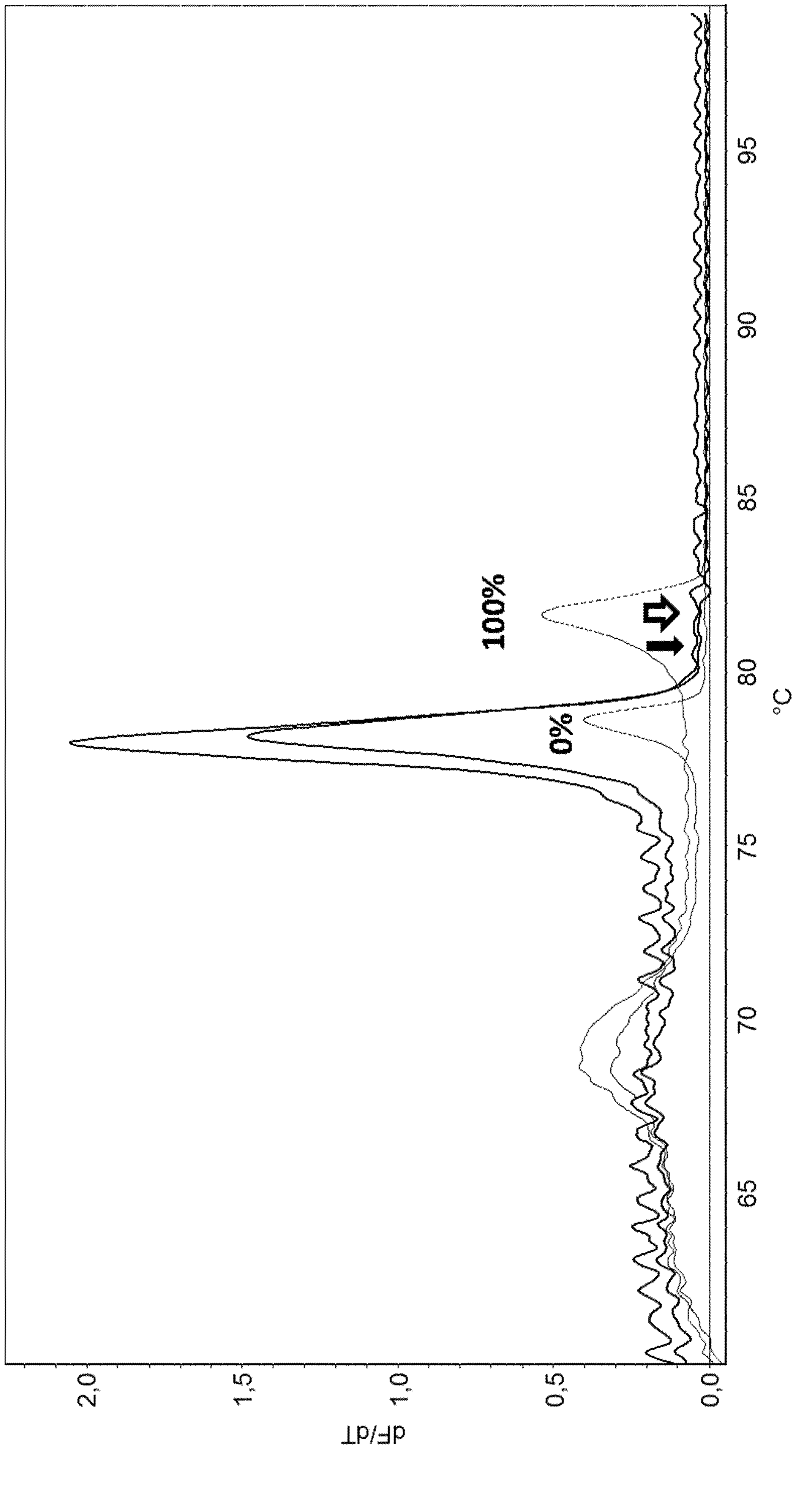


Fig. 6

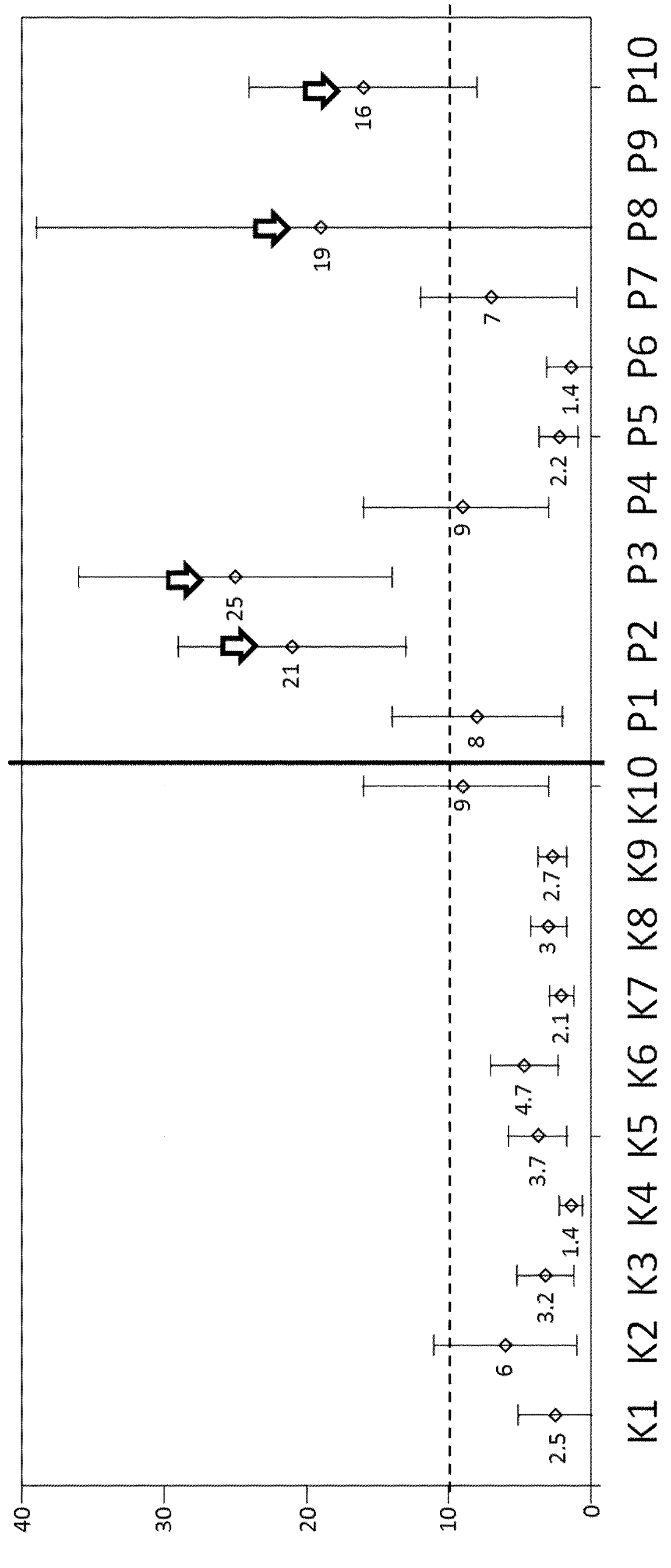
0
10
20
30
40
K1
K2
K3
K4
K5
K6
K7
K8
K9
K10
P1
P2
P3
P4
P5
P6
P7
P8
P9
P10
2.5
6
3.2
1.4
3.7
4.7
2.1
3
2.7
9
8
21
25
9
2.2
1.4
7
19
16

Fig. 7

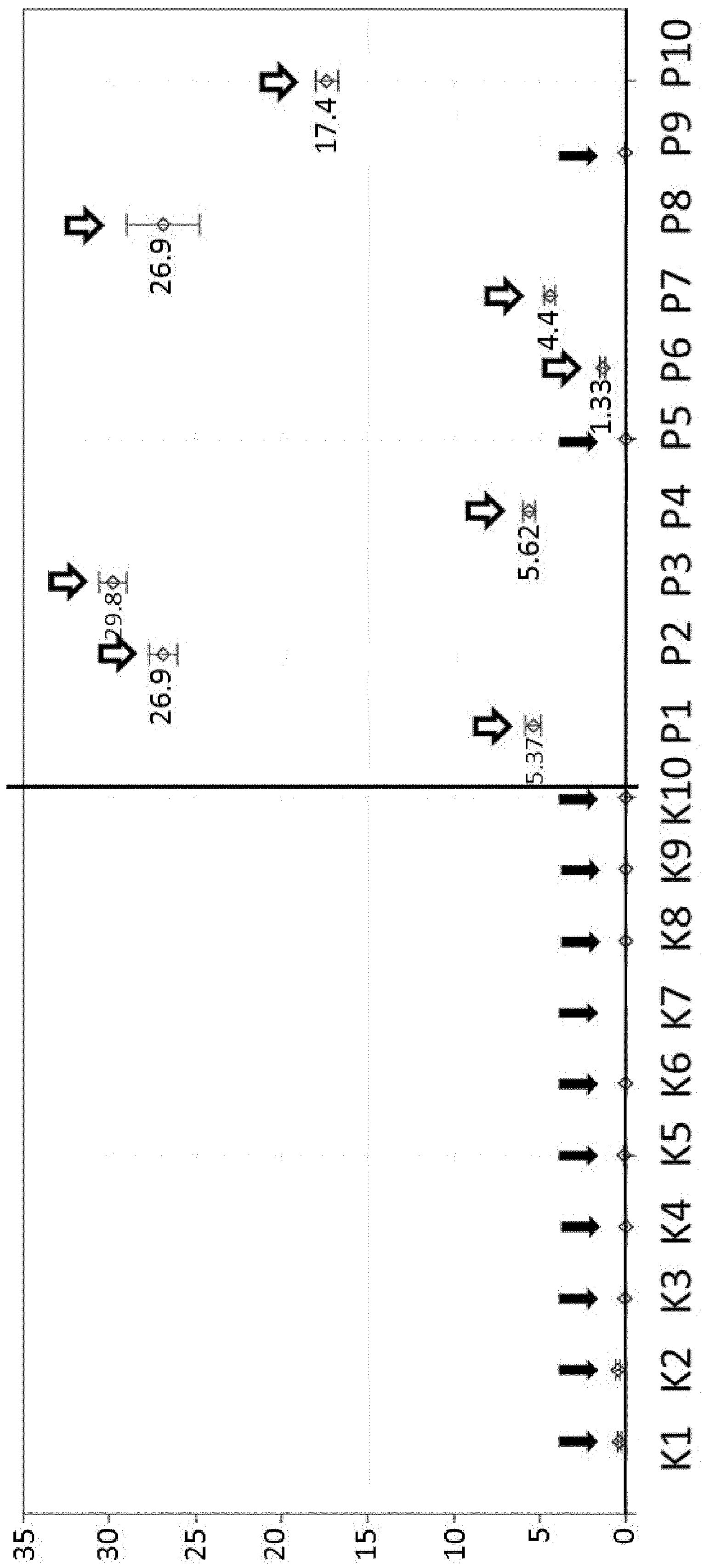
35
30
25
20
15
10
5
0
K1
K2
K3
K4
K5
K6
K7
K8
K9
K10
P1
P2
P3
P4
P5
P6
P7
P8
P9
P10
5.37
26.9
29.8
5.62
1.33
4.4
26.9
17.4

Fig. 8

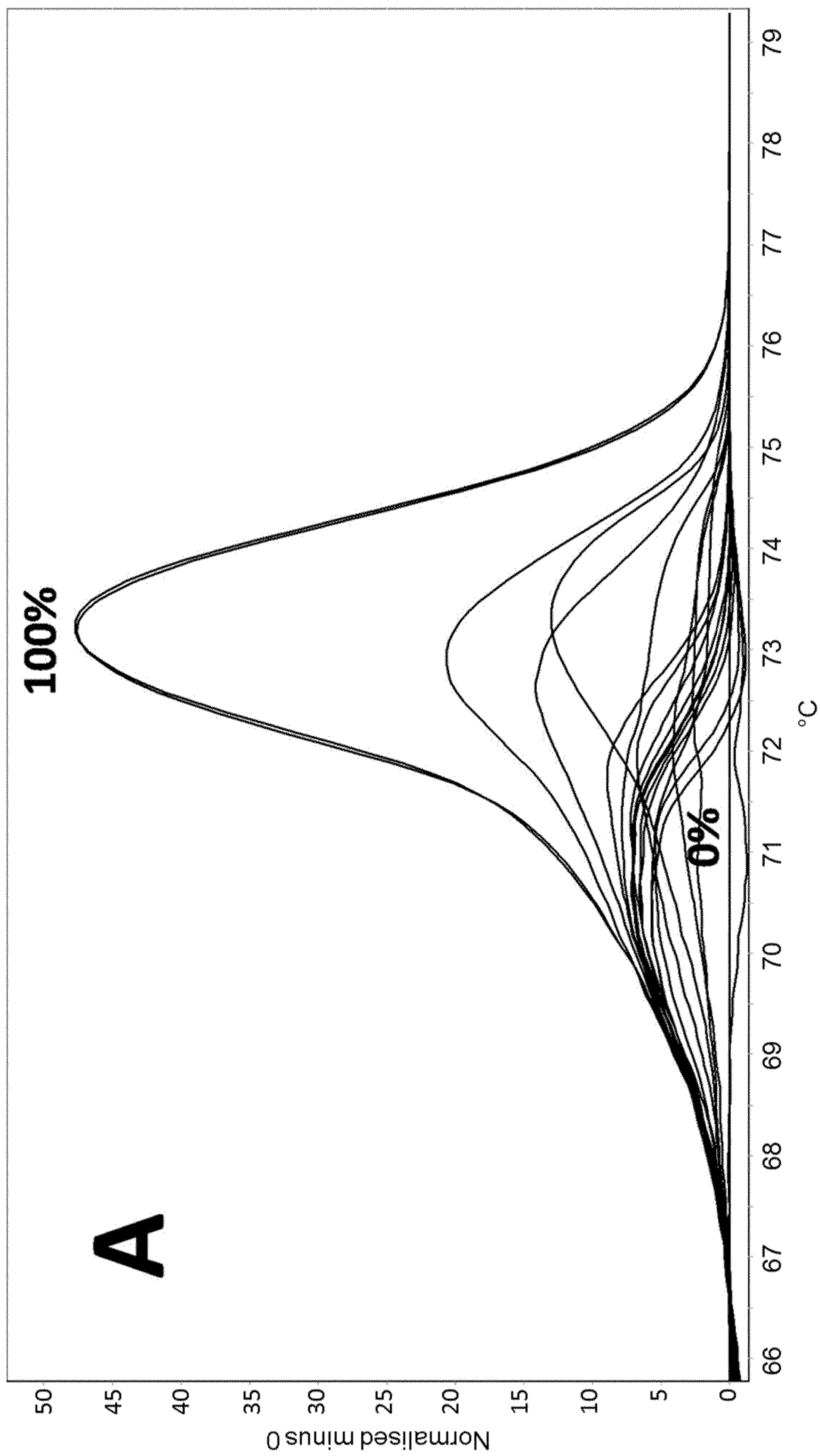
A
100%
0%
Normalised minus 0
50
45
40
35
30
25
20
15
10
5
0
66
67
68
69
70
71
72
73
74
75
76
77
78
79
°C

Fig. 9A

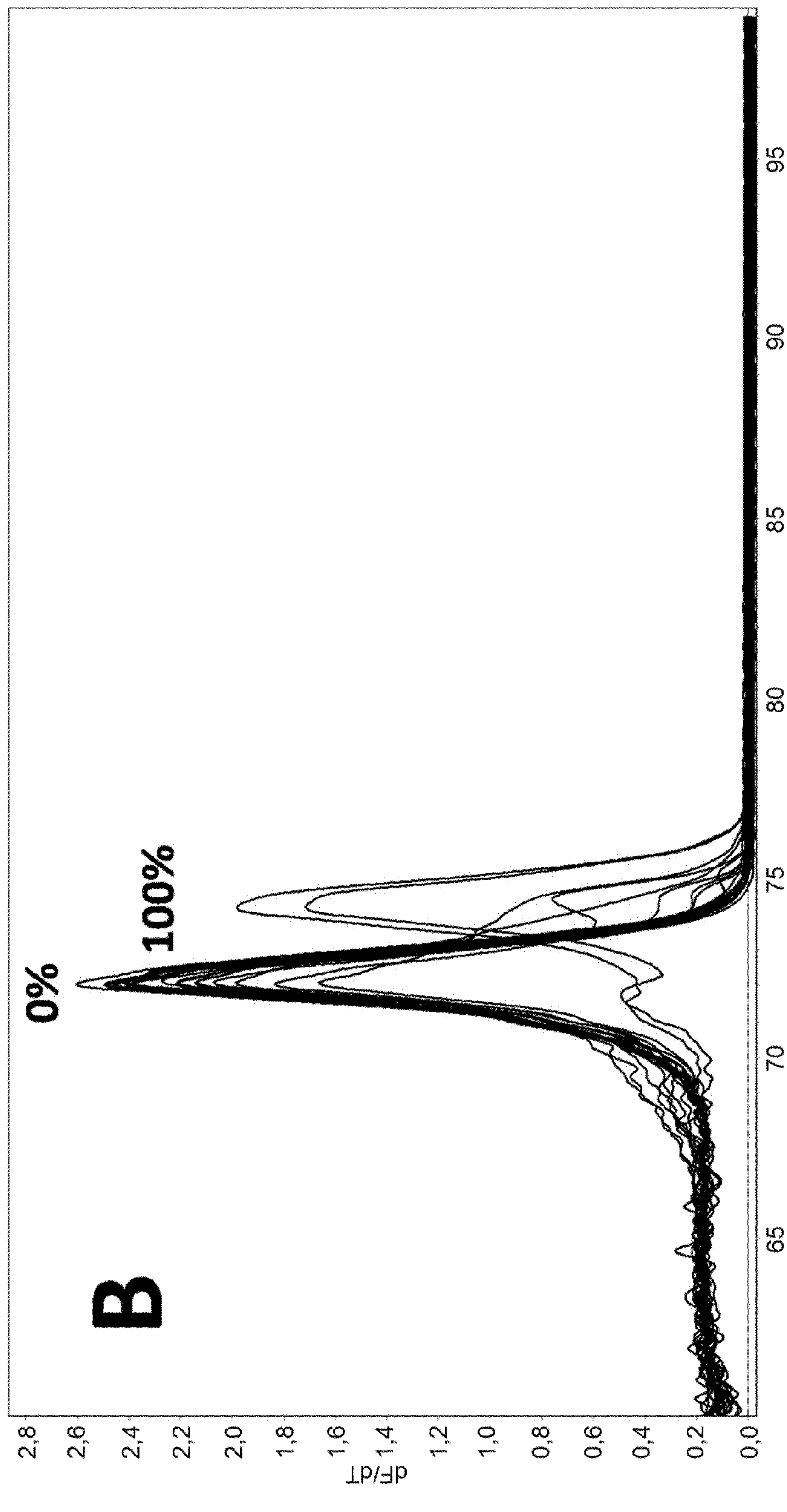
B
0%
100%
dF/dT
°C
0,0
0,2
0,4
0,6
0,8
1,0
1,2
1,4
1,6
1,8
2,0
2,2
2,4
2,6
2,8
65
70
75
80
85
90
95

Fig. 9B

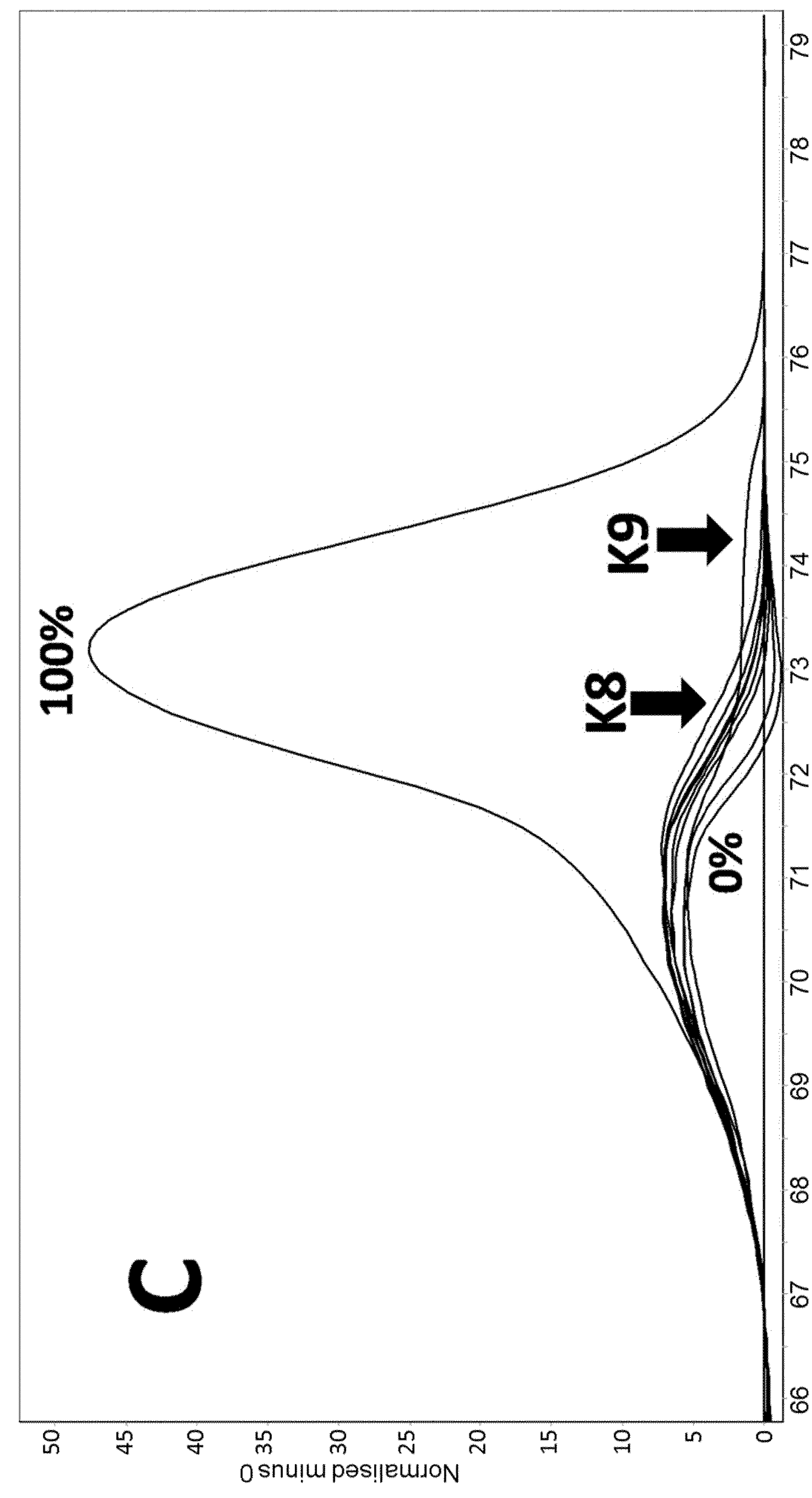
C
100%
K8
K9
0%
Normalised minus 0
°C
0
5
10
15
20
25
30
35
40
45
50
66
67
68
69
70
71
72
73
74
75
76
77
78
79

Fig. 9C

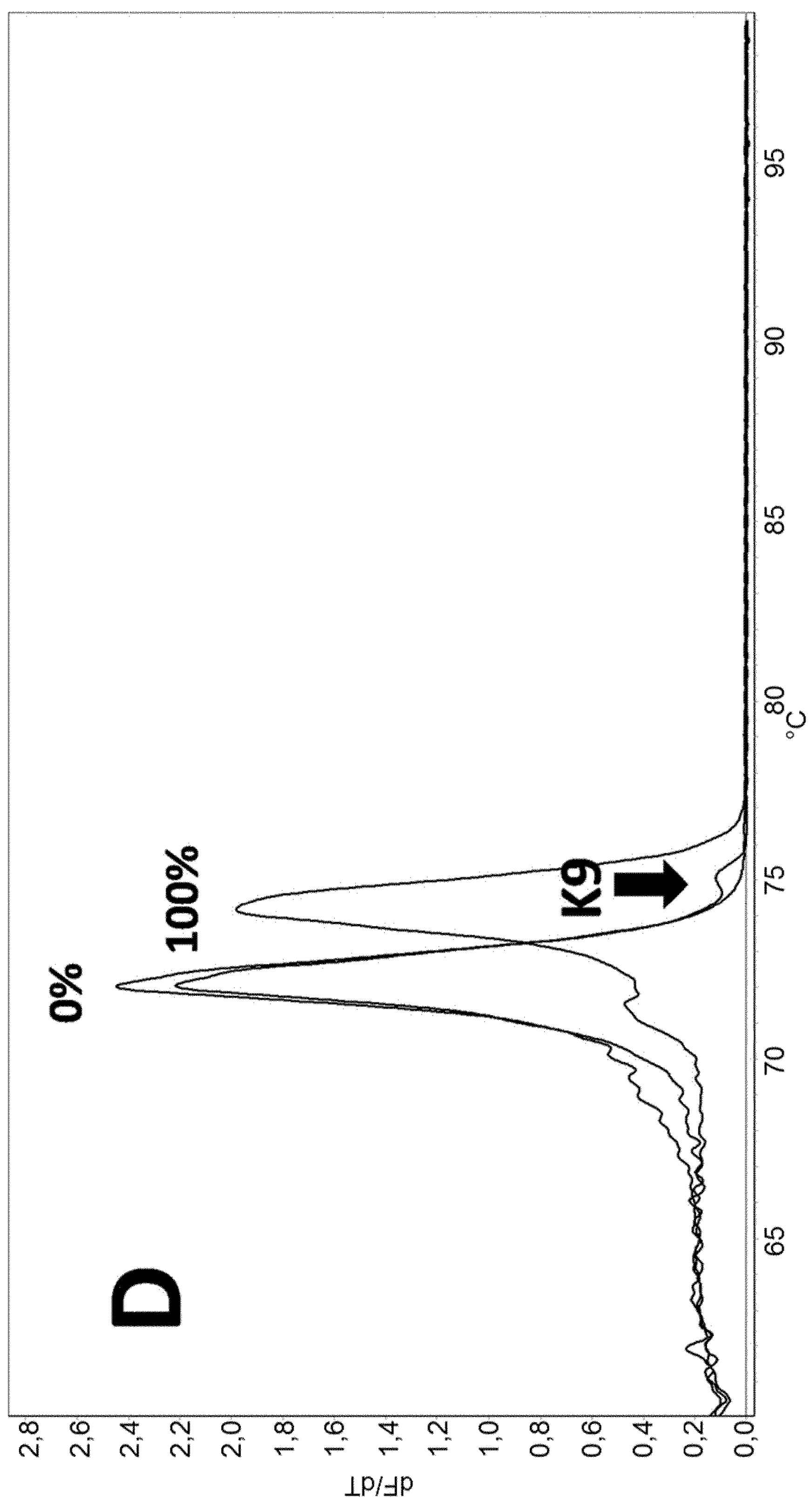
D
0%
100%
K9
dF/dT
2,8
2,6
2,4
2,2
2,0
1,8
1,6
1,4
1,2
1,0
0,8
0,6
0,4
0,2
0,0
65
70
75
80
85
90
95
°C

Fig. 9D

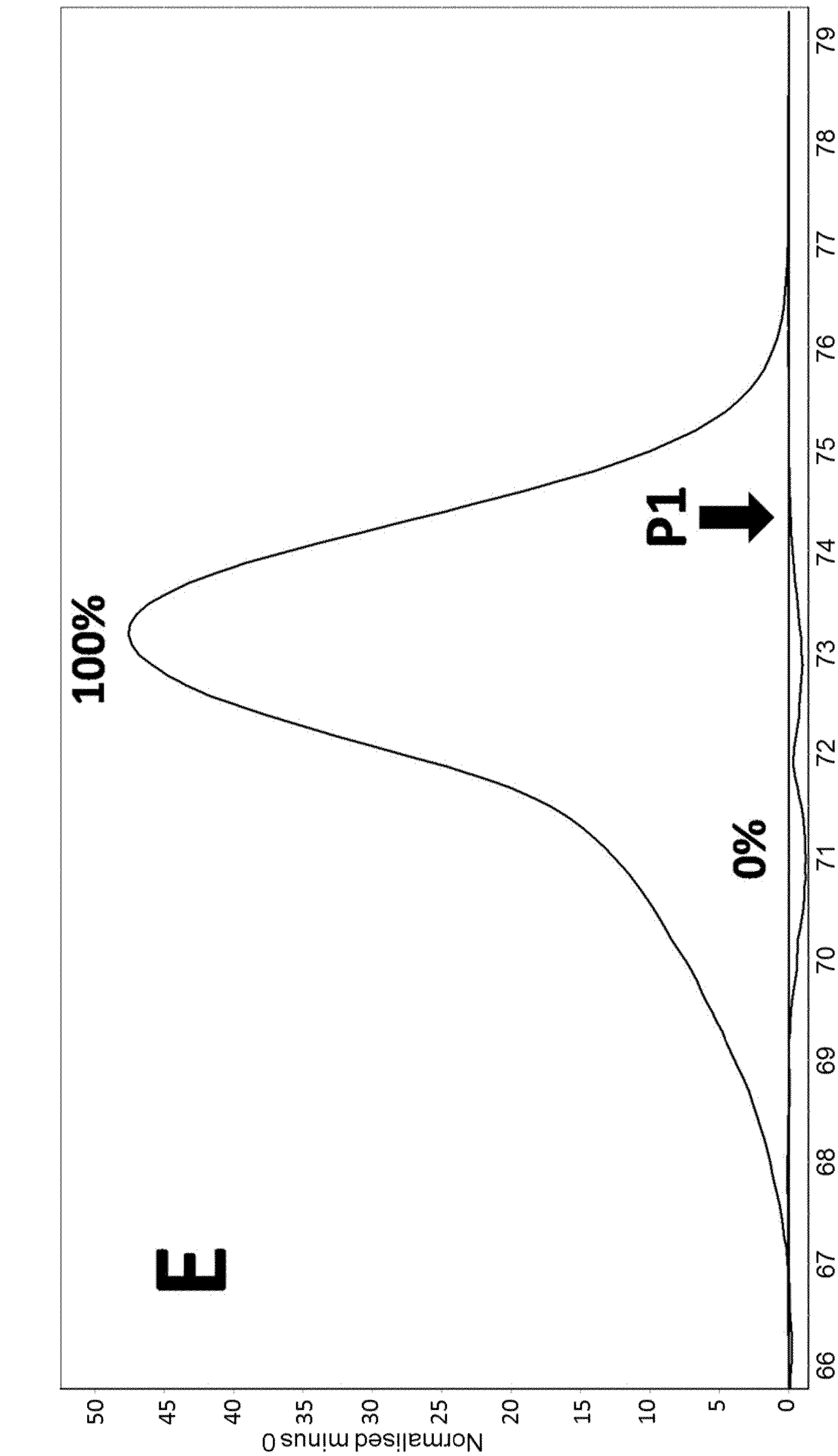
E
100%
0%
P1
Normalised minus 0
°C
0
5
10
15
20
25
30
35
40
45
50
66
67
68
69
70
71
72
73
74
75
76
77
78
79

Fig. 9E

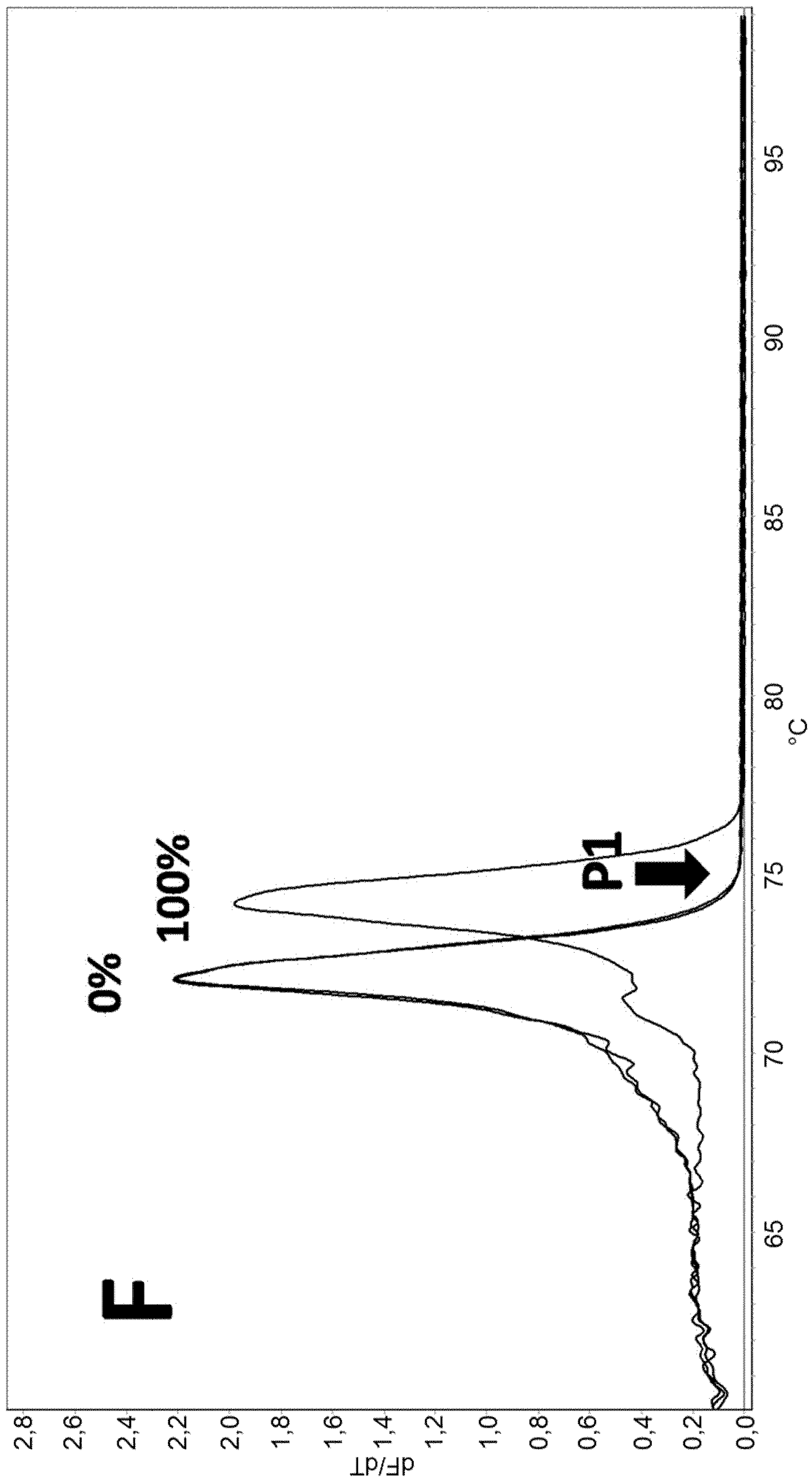
F
0%
100%
P1
dF/dT
2,8
2,6
2,4
2,2
2,0
1,8
1,6
1,4
1,2
1,0
0,8
0,6
0,4
0,2
0,0
65
70
75
80
85
90
95
°C

Fig. 9F

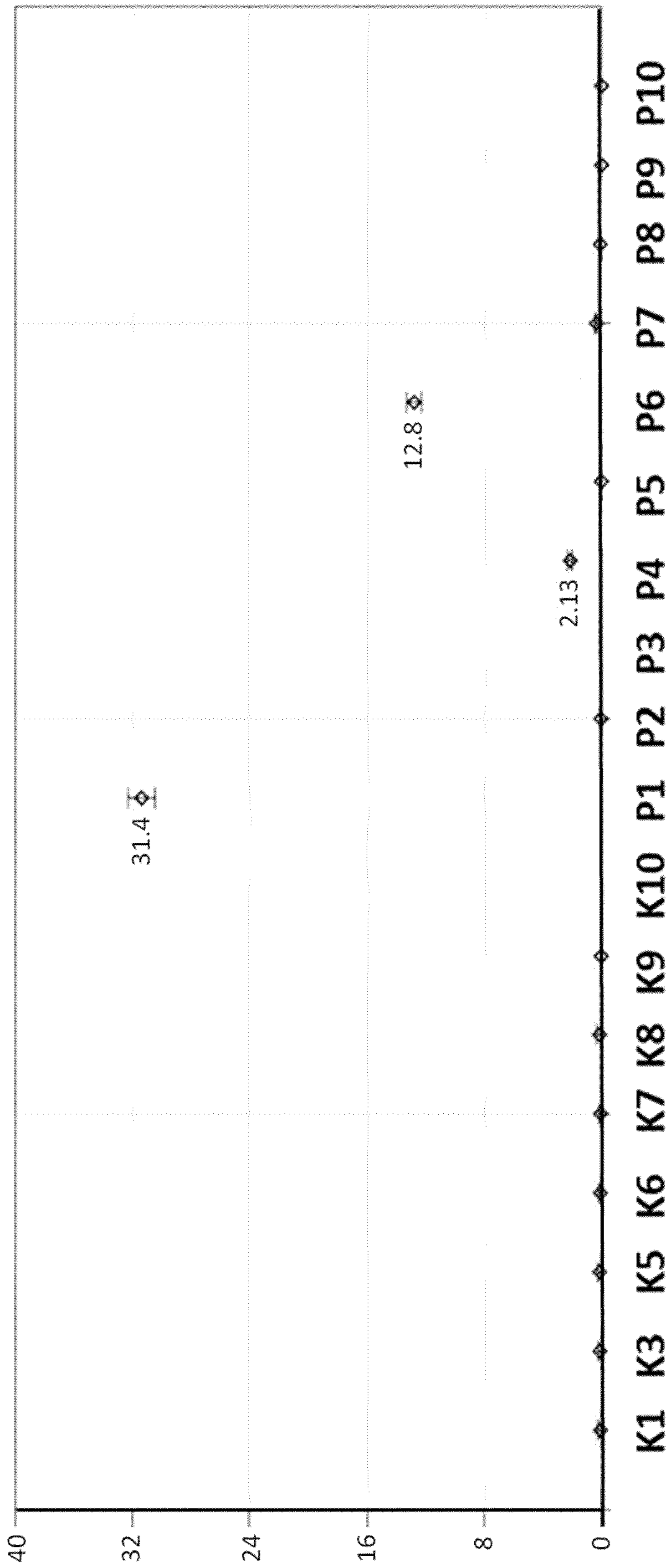
40
32
24
16
8
0
K1 K3 K5 K6 K7 K8 K9 K10 P1 P2 P3 P4 P5 P6 P7 P8 P9 P10
31.4
2.13
12.8

Fig. 10

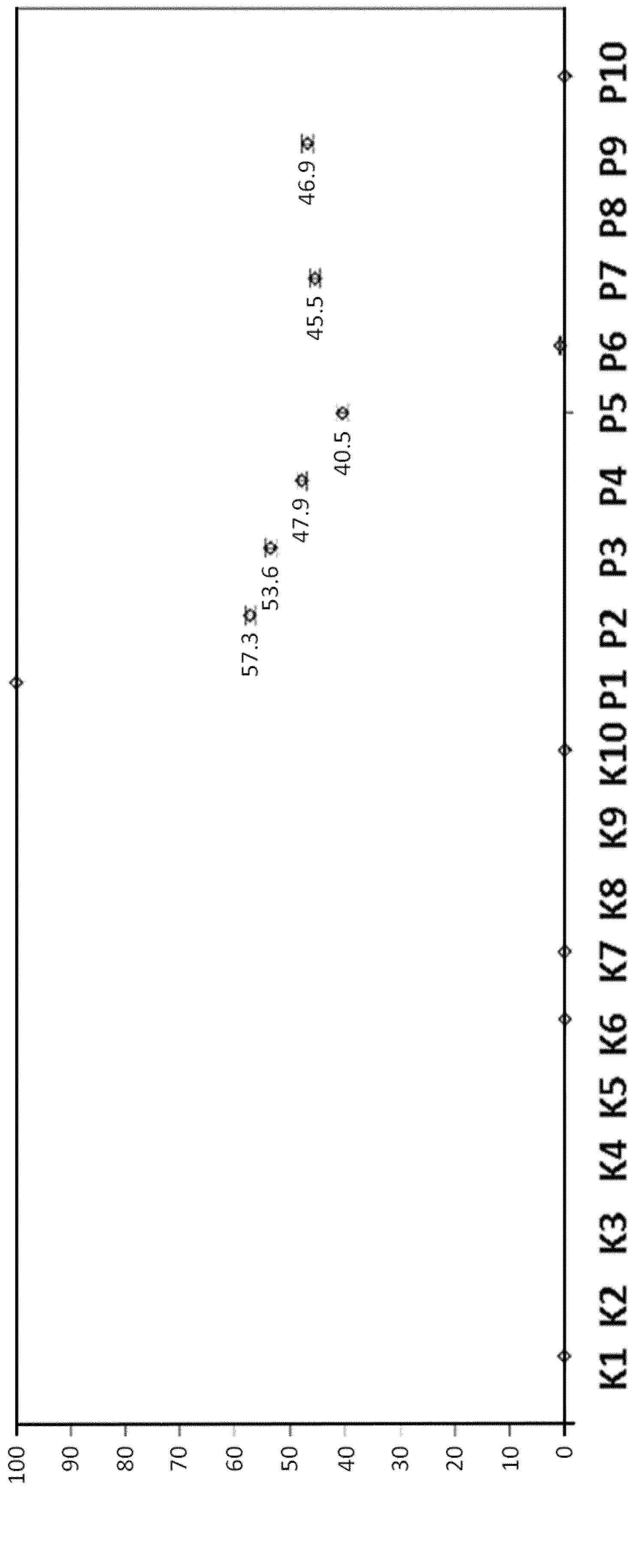
100
90
80
70
60
50
40
30
20
10
0
K1 K2 K3 K4 K5 K6 K7 K8 K9 K10 P1 P2 P3 P4 P5 P6 P7 P8 P9 P10
57.3
53.6
47.9
40.5
45.5
46.9

Fig. 11

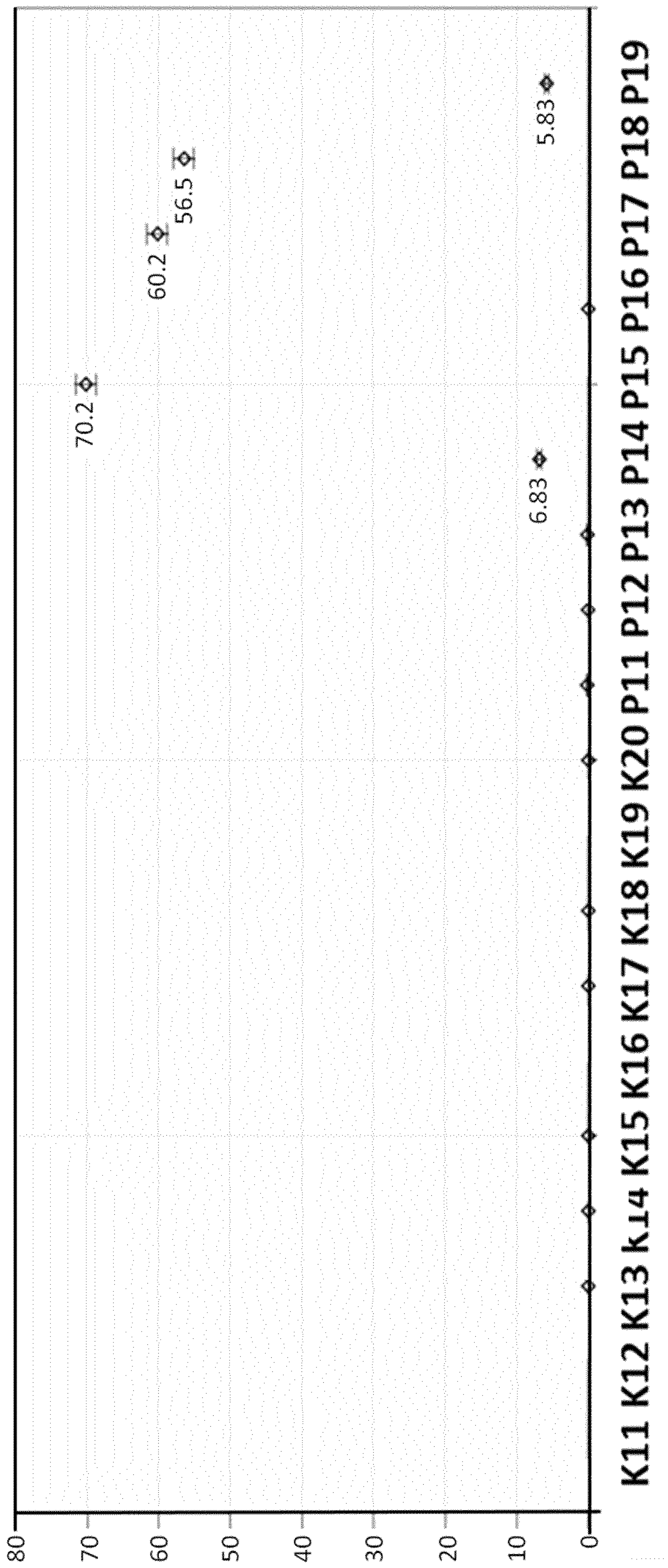
80
70
60
50
40
30
20
10
0
K11 K12 K13 K14 K15 K16 K17 K18 K19 K20 P11 P12 P13 P14 P15 P16 P17 P18 P19
70.2
60.2
56.5
6.83
5.83

Fig. 12

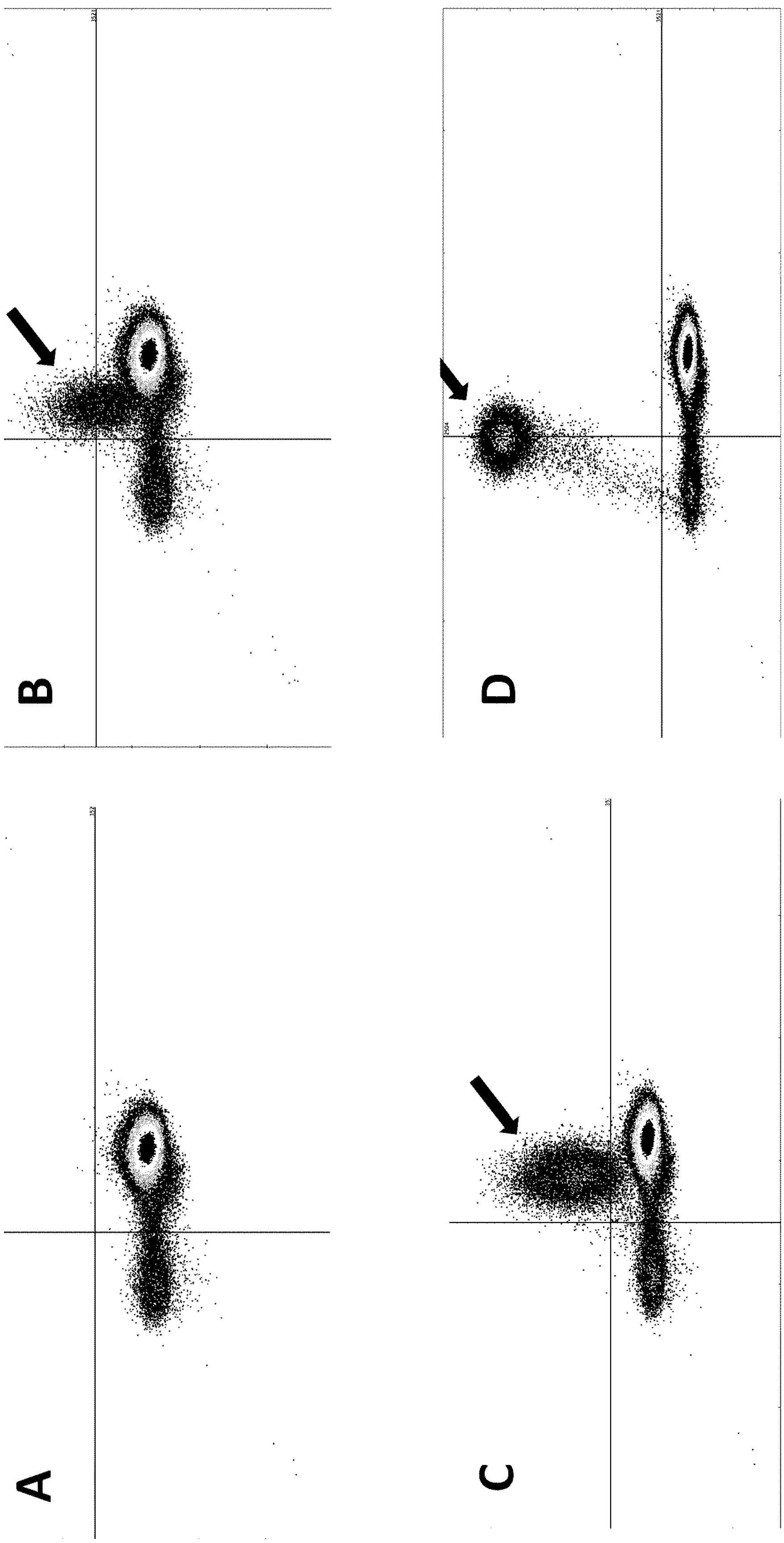


Fig. 13

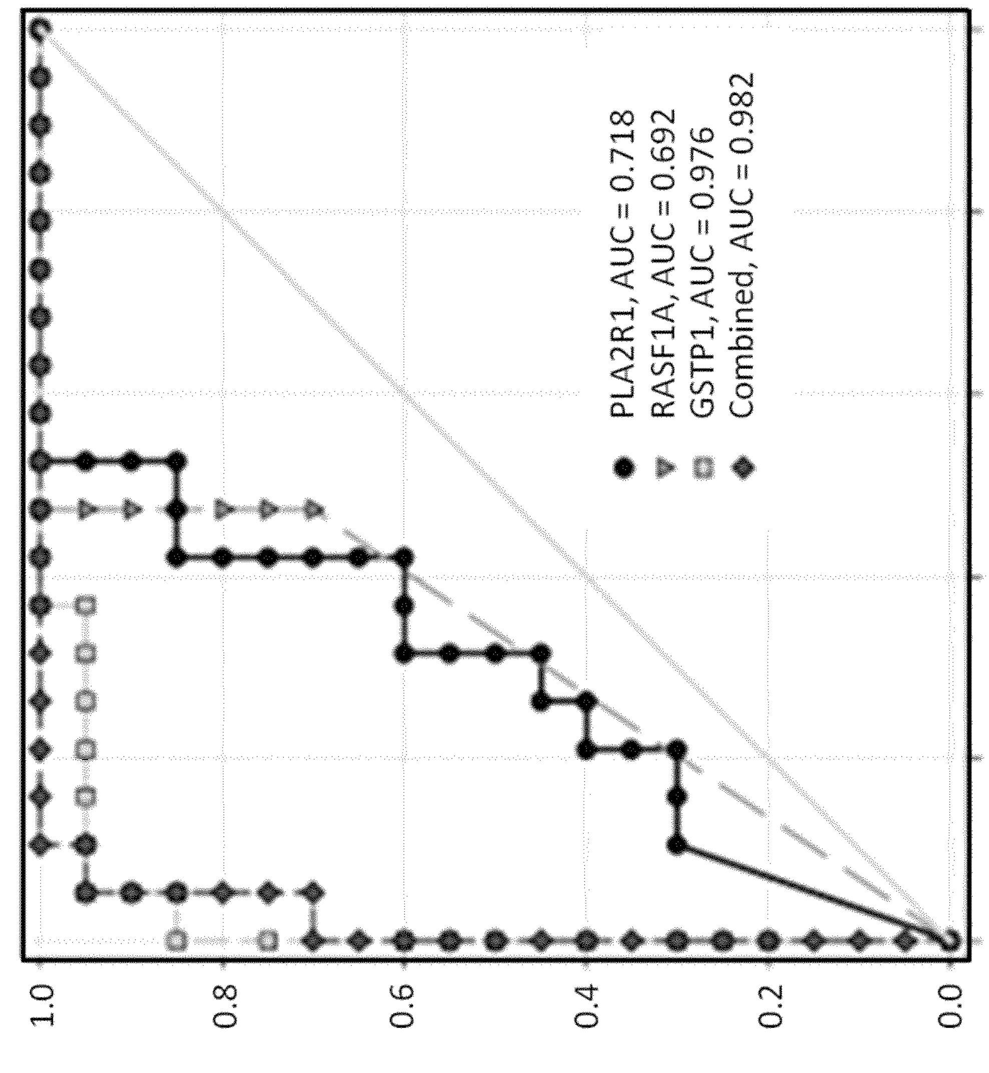
PLA2R1, AUC = 0.718
RASF1A, AUC = 0.692
GSTP1, AUC = 0.976
Combined, AUC = 0.982
0.0
0.2
0.4
0.6
0.8
1.0
0.0
0.2
0.4
0.6
0.8
1.0

Fig. 14

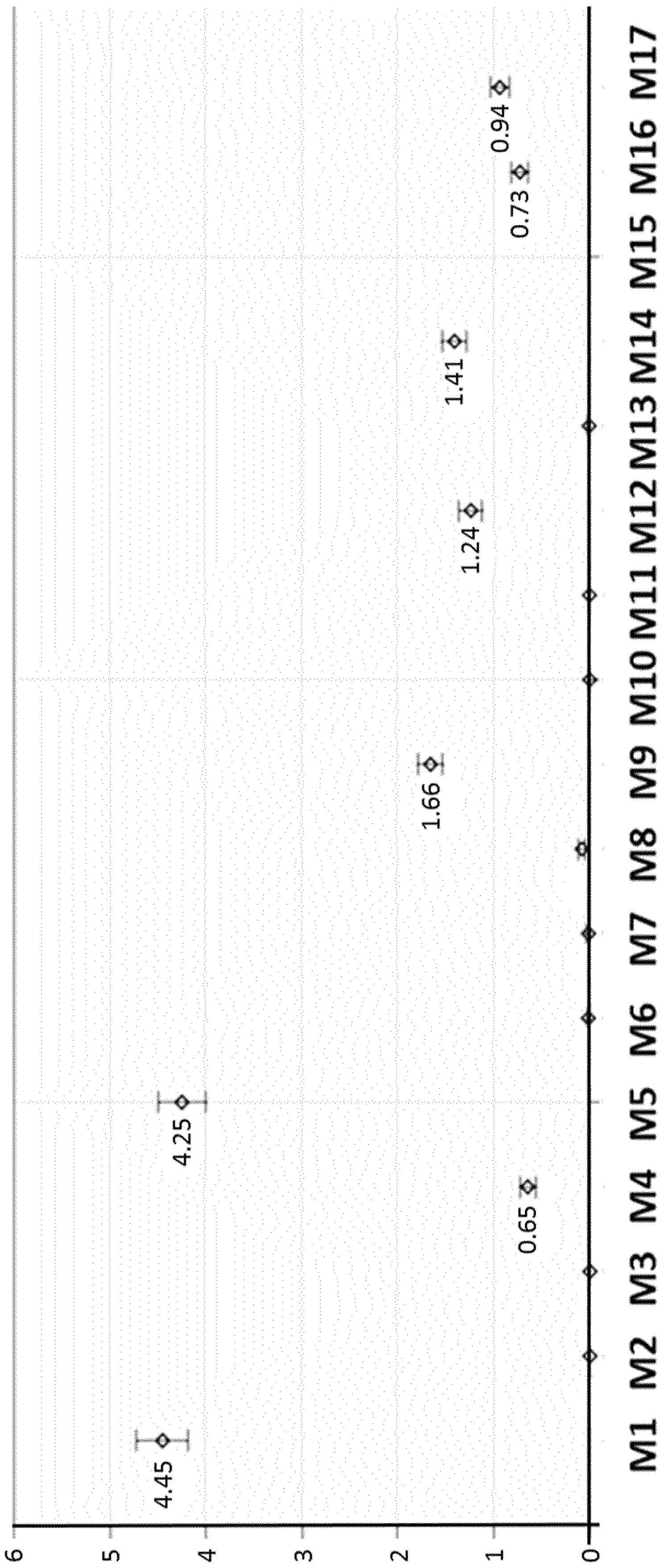

6
5
4
3
2
1
0
M1 M2 M3 M4 M5 M6 M7 M8 M9 M10 M11 M12 M13 M14 M15 M16 M17
4.45
0.65
4.25
1.66
1.24
1.41
0.73
0.94


Fig. 15

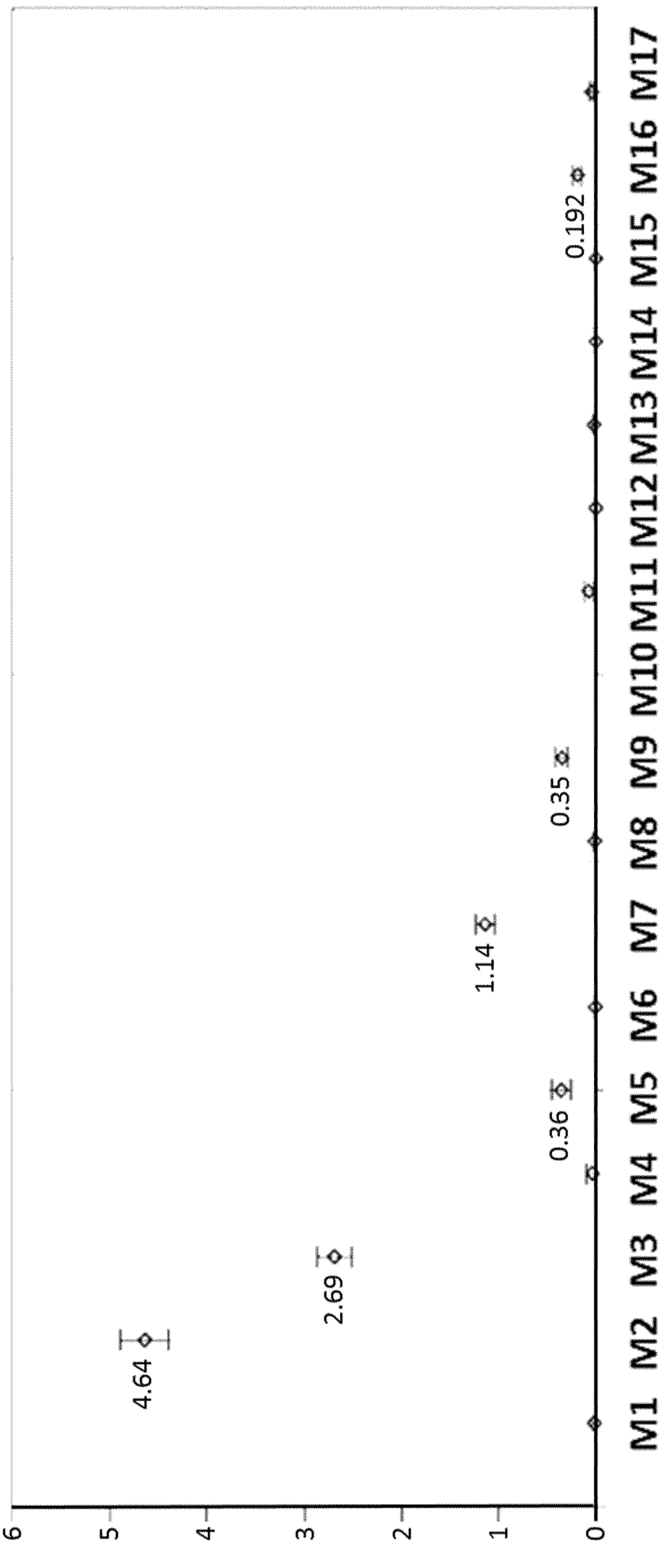
6
5
4
3
2
1
0
M1
M2
M3
M4
M5
M6
M7
M8
M9
M10
M11
M12
M13
M14
M15
M16
M17
4.64
2.69
0.36
1.14
0.35
0.192

**Fig. 16**

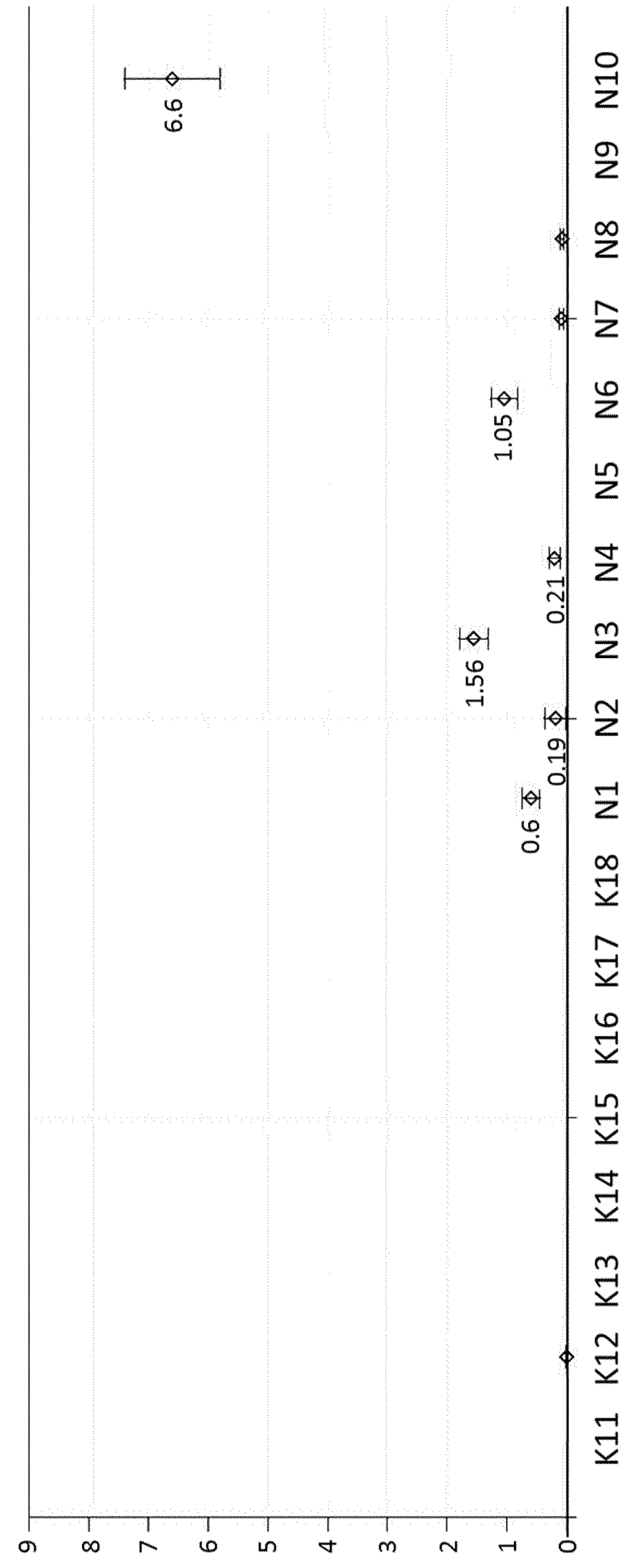
0
1
2
3
4
5
6
7
8
9
K11
K12
K13
K14
K15
K16
K17
K18
N1
N2
N3
N4
N5
N6
N7
N8
N9
N10
0.6
0.19
1.56
0.21
1.05
6.6

Fig. 17

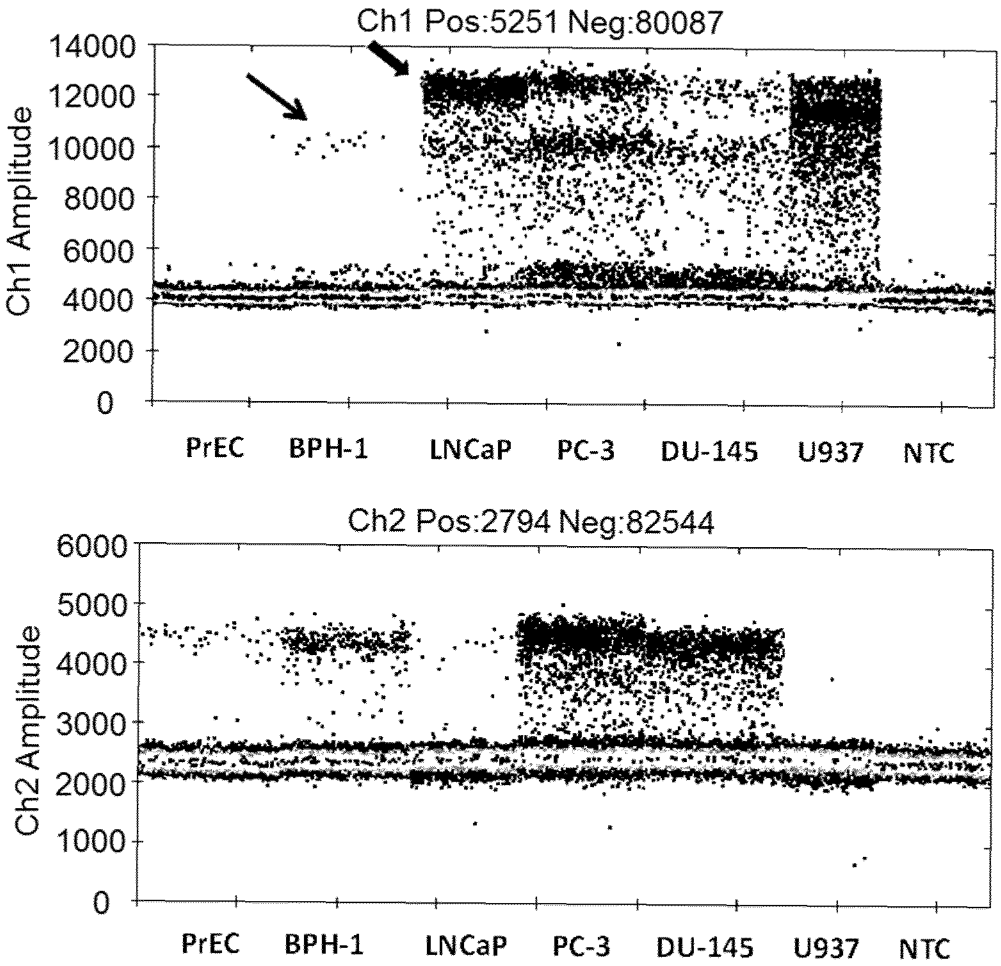
Ch1 Pos:5251 Neg:80087
Ch1 Amplitude
14000
12000
10000
8000
6000
4000
2000
0
PrEC
BPH-1
LNCaP
PC-3
DU-145
U937
NTC
Ch2 Pos:2794 Neg:82544
Ch2 Amplitude
6000
5000
4000
3000
2000
1000
0
PrEC
BPH-1
LNCaP
PC-3
DU-145
U937
NTC

Fig. 18

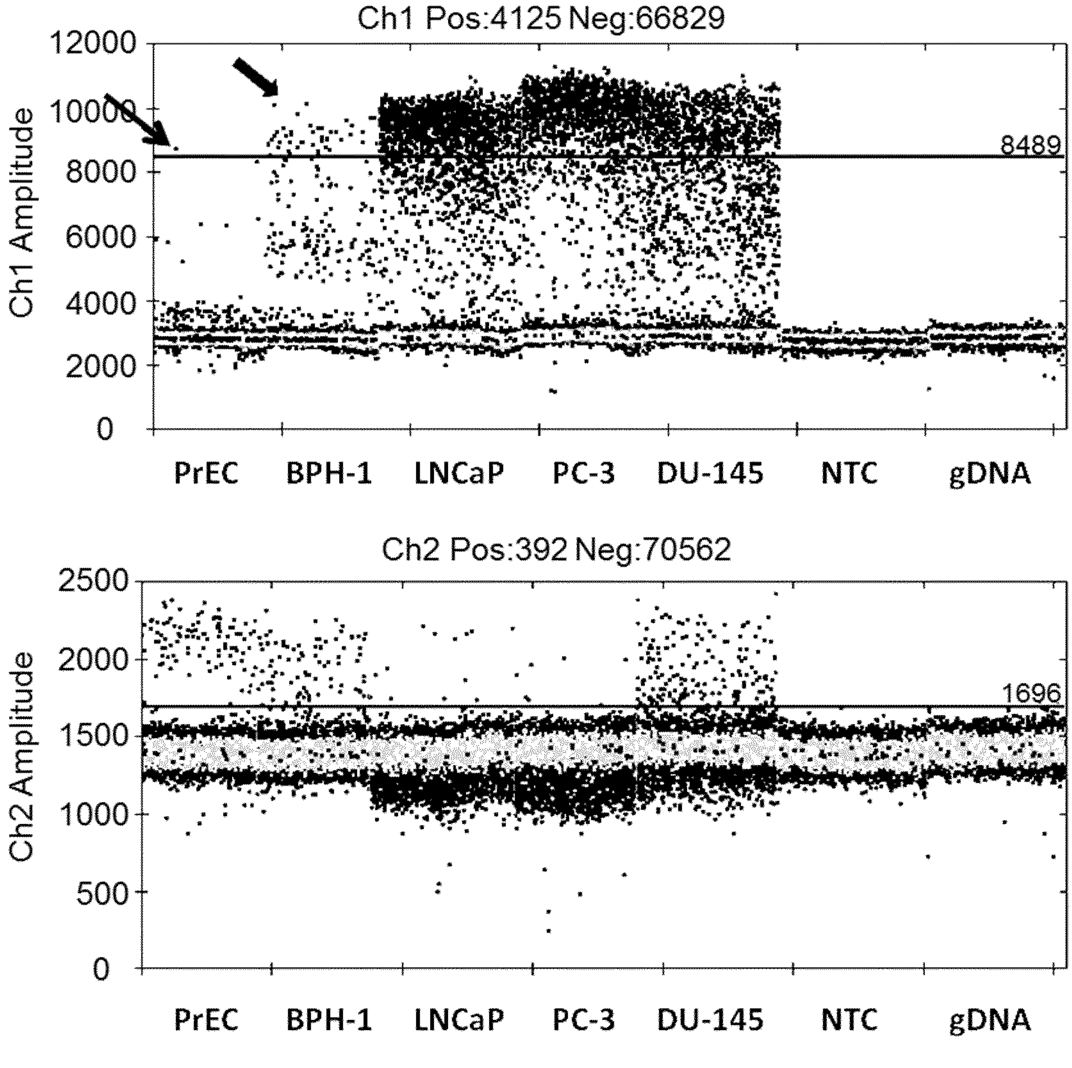
Ch1 Pos:4125 Neg:66829
12000
10000
8000
6000
4000
2000
0
Ch1 Amplitude
8489
PrEC
BPH-1
LNCaP
PC-3
DU-145
NTC
gDNA
Ch2 Pos:392 Neg:70562
2500
2000
1500
1000
500
0
Ch2 Amplitude
1696
PrEC
BPH-1
LNCaP
PC-3
DU-145
NTC
gDNA

Fig. 19

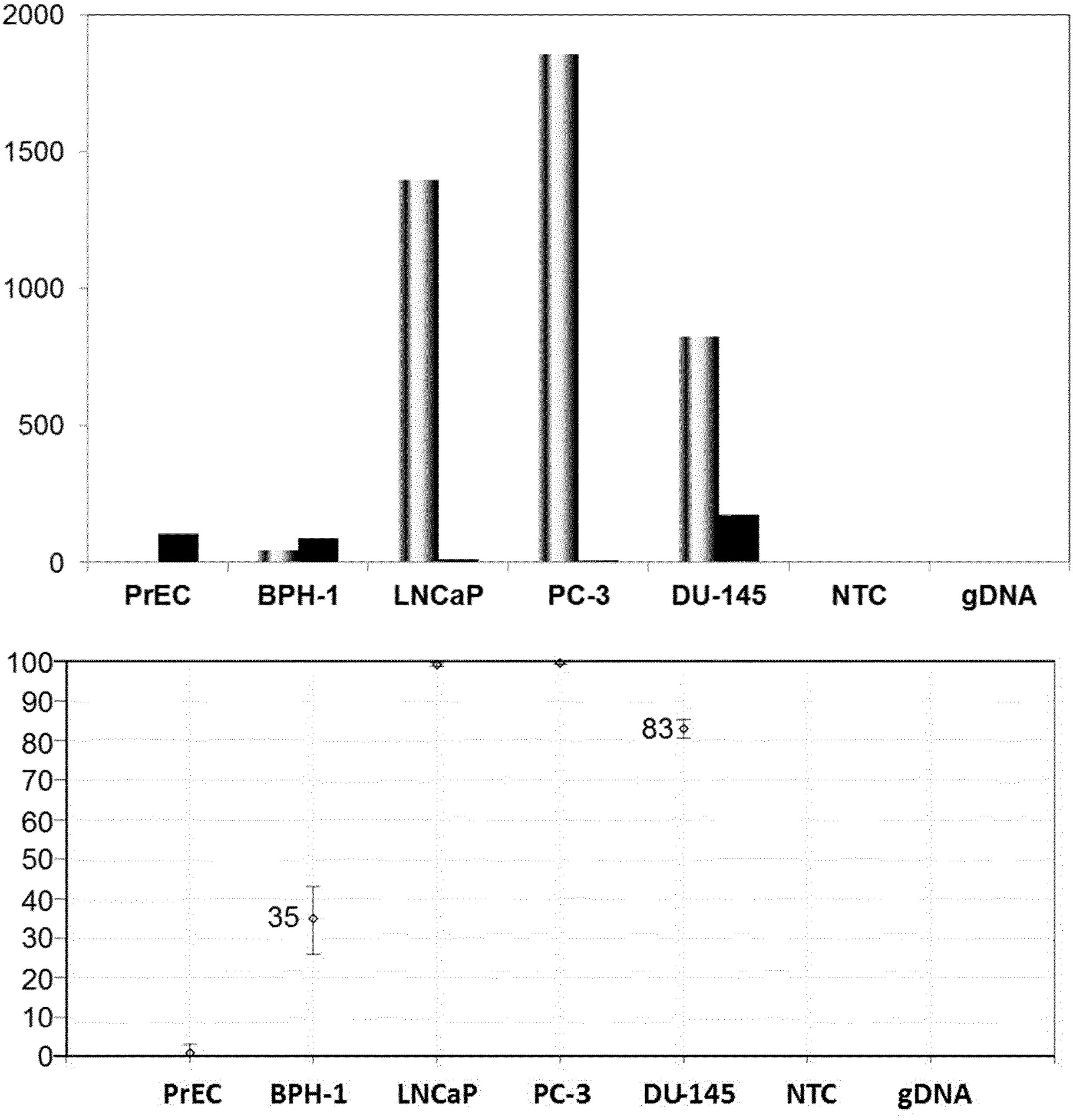
2000
1500
1000
500
0
PrEC
BPH-1
LNCaP
PC-3
DU-145
NTC
gDNA
100
90
80
70
60
50
40
30
20
10
0
35
83
PrEC
BPH-1
LNCaP
PC-3
DU-145
NTC
gDNA

Fig. 20

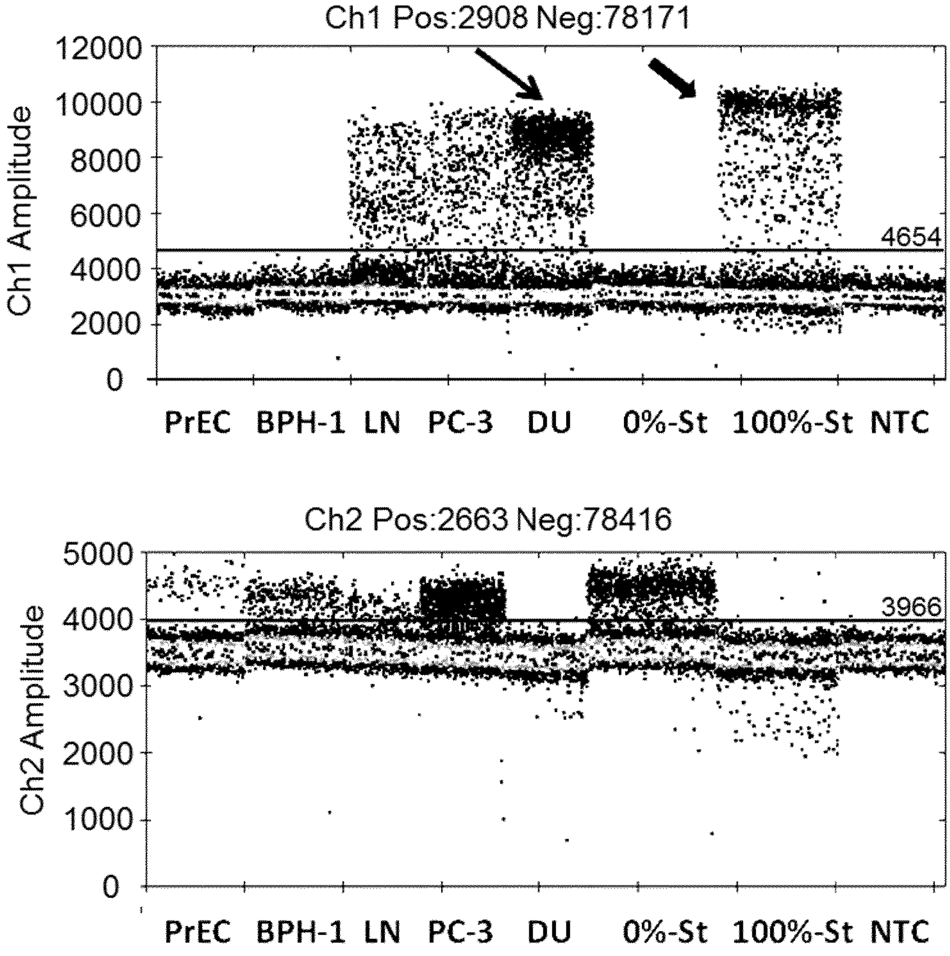
Ch1 Pos:2908 Neg:78171
Ch1 Amplitude
12000
10000
8000
6000
4000
2000
0
4654
PrEC BPH-1 LN PC-3 DU 0%-St 100%-St NTC
Ch2 Pos:2663 Neg:78416
Ch2 Amplitude
5000
4000
3000
2000
1000
0
3966
PrEC BPH-1 LN PC-3 DU 0%-St 100%-St NTC

Fig. 21

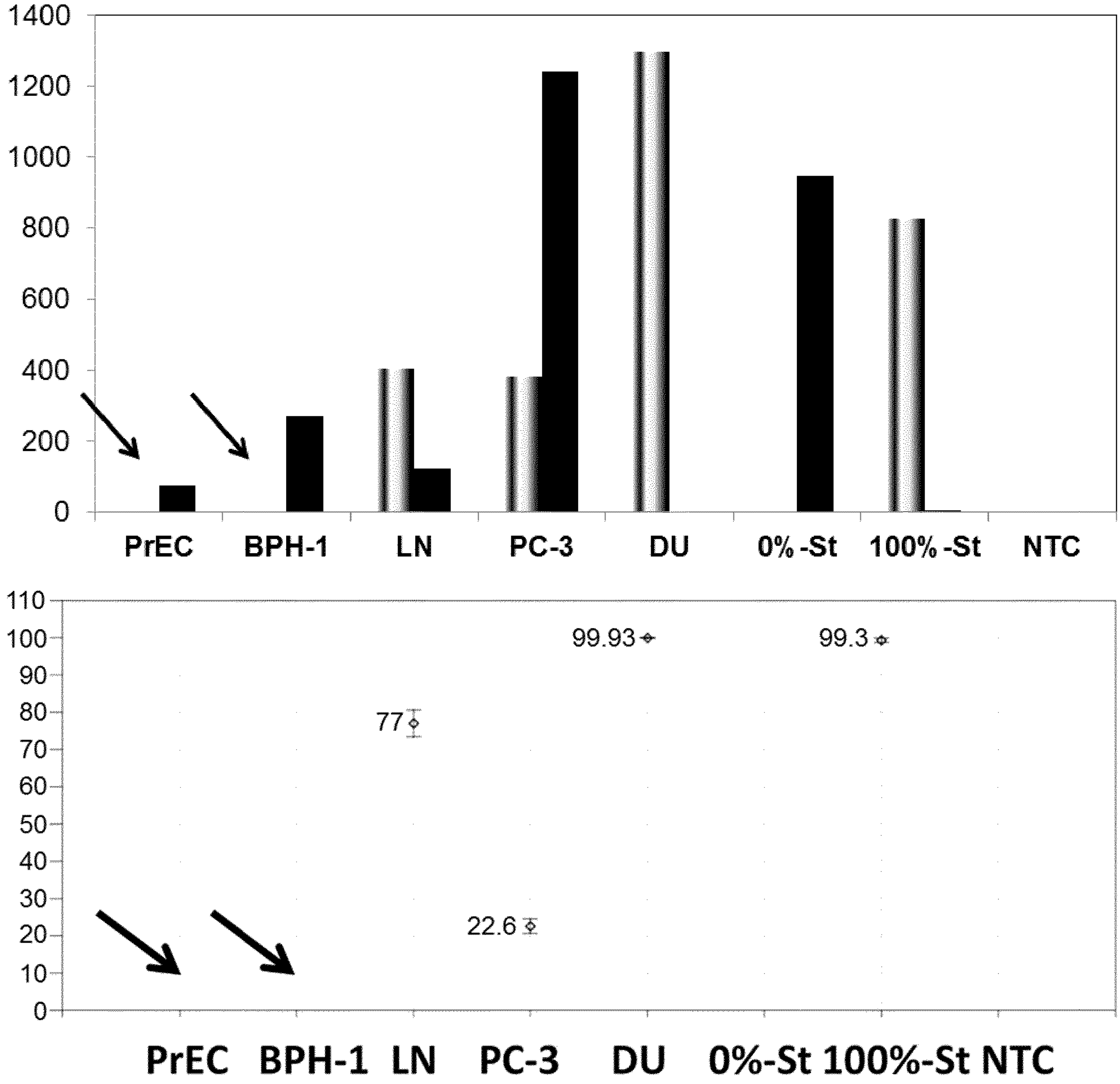
1400
1200
1000
800
600
400
200
0
PrEC
BPH-1
LN
PC-3
DU
0% -St
100% -St
NTC
110
100
90
80
70
60
50
40
30
20
10
0
99.93
99.3
77
22.6
PrEC BPH-1 LN PC-3 DU 0%-St 100%-St NTC

Fig. 22

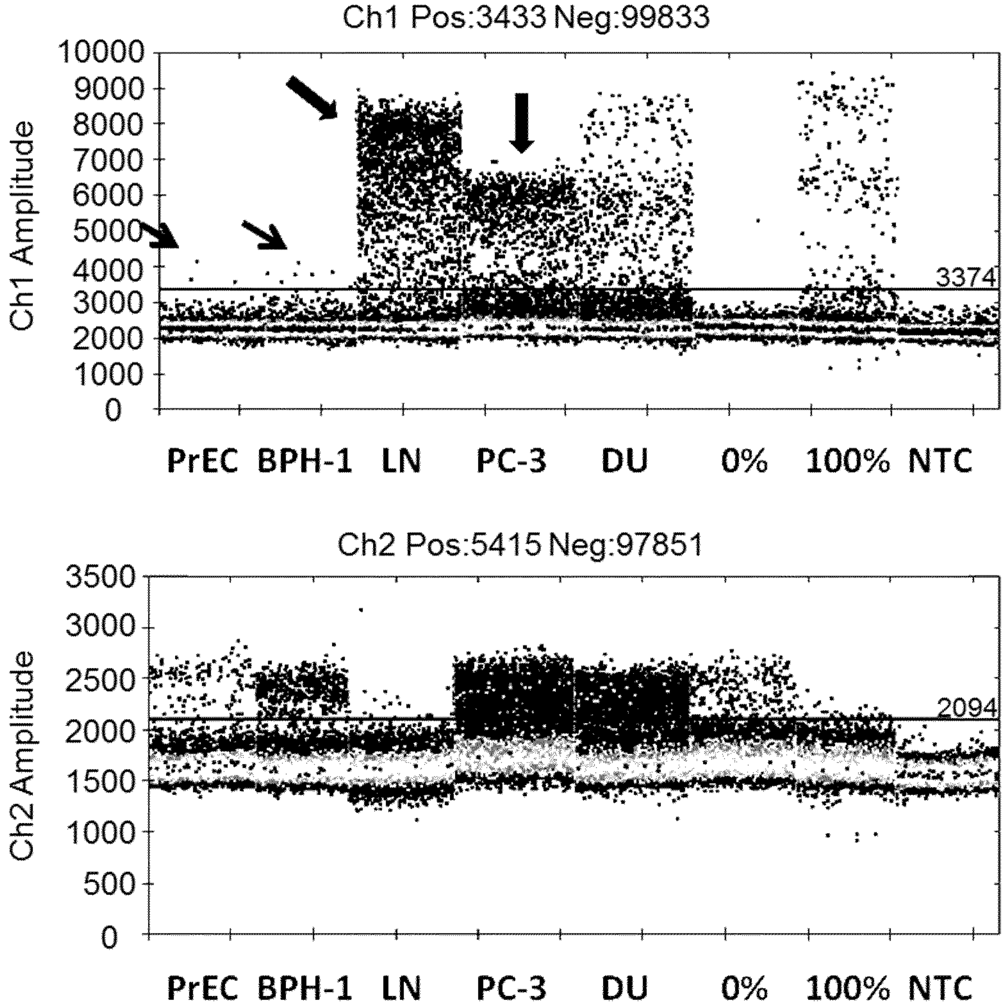
Ch1 Pos:3433 Neg:99833
Ch1 Amplitude
10000
9000
8000
7000
6000
5000
4000
3000
2000
1000
0
3374
PrEC BPH-1 LN PC-3 DU 0% 100% NTC
Ch2 Pos:5415 Neg:97851
Ch2 Amplitude
3500
3000
2500
2000
1500
1000
500
0
2094
PrEC BPH-1 LN PC-3 DU 0% 100% NTC

Fig. 23

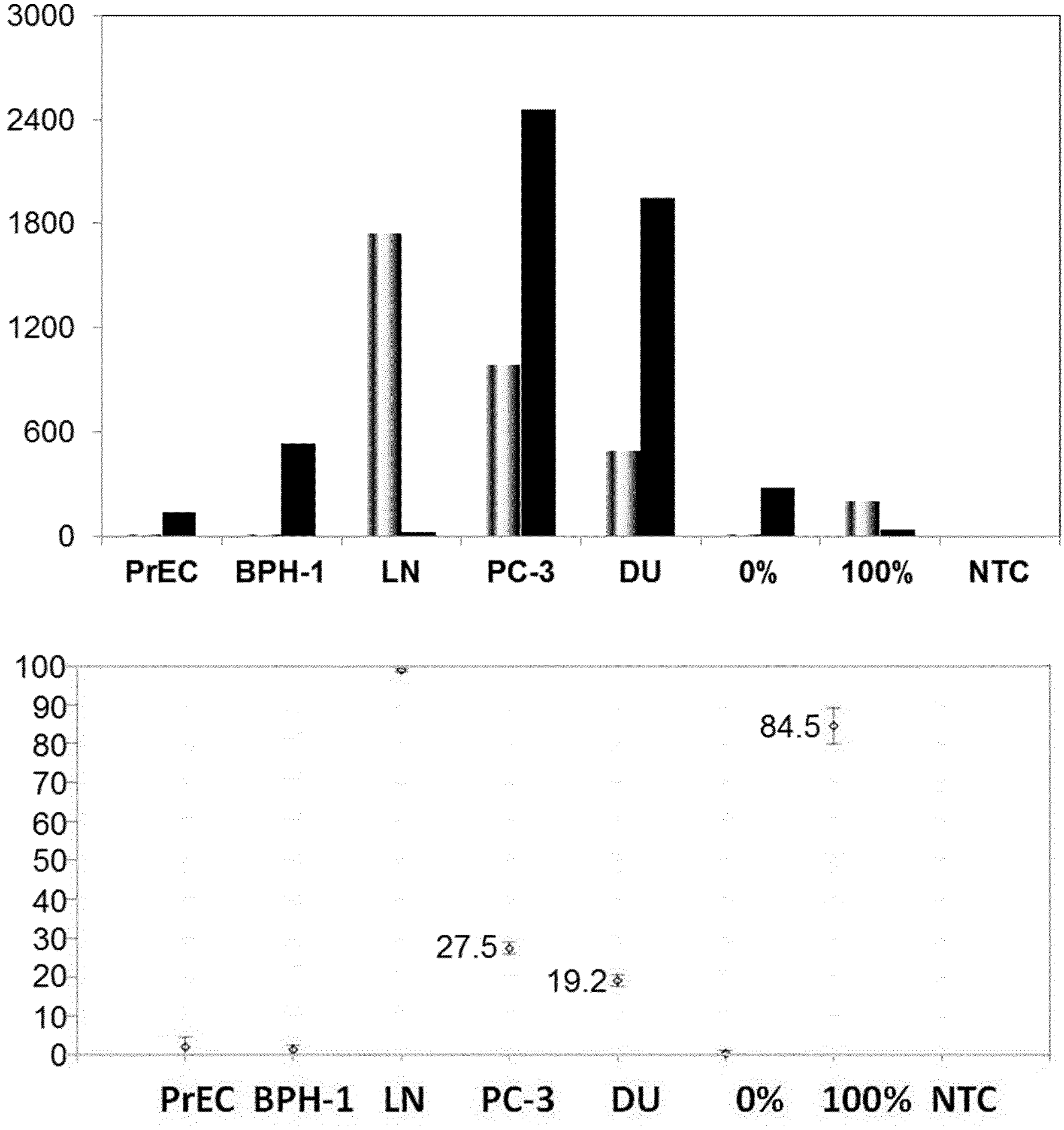
3000
2400
1800
1200
600
0
PrEC
BPH-1
LN
PC-3
DU
0%
100%
NTC
100
90
80
70
60
50
40
30
20
10
0
84.5
27.5
19.2
PrEC
BPH-1
LN
PC-3
DU
0%
100%
NTC

Fig. 24

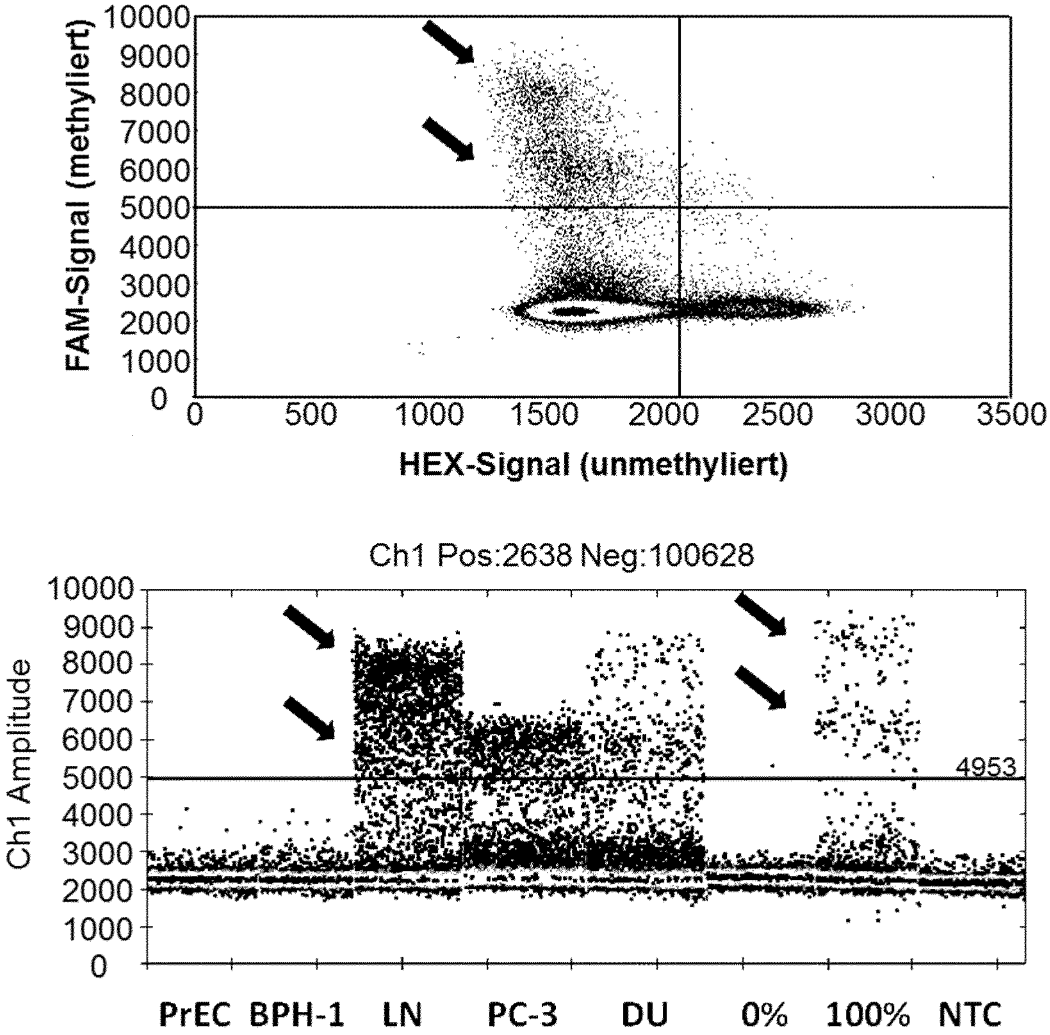
FAM-Signal (methyliert)
HEX-Signal (unmethyliert)
10000
9000
8000
7000
6000
5000
4000
3000
2000
1000
0
0
500
1000
1500
2000
2500
3000
3500
Ch1 Pos:2638 Neg:100628
Ch1 Amplitude
4953
PrEC BPH-1 LN PC-3 DU 0% 100% NTC

Fig. 25

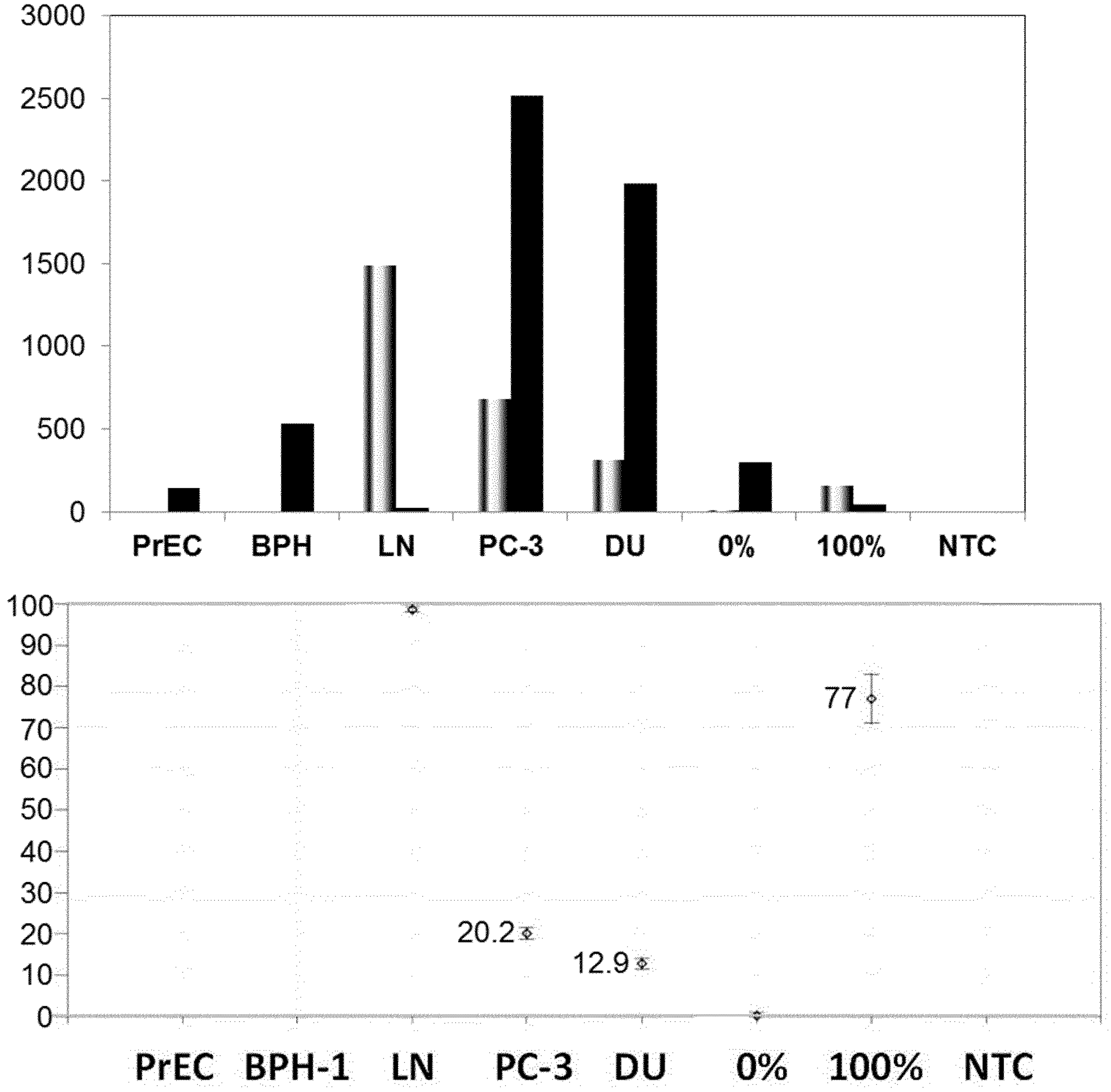
3000
2500
2000
1500
1000
500
0
PrEC
BPH
LN
PC-3
DU
0%
100%
NTC
100
90
80
70
60
50
40
30
20
10
0
77
20.2
12.9
PrEC
BPH-1
LN
PC-3
DU
0%
100%
NTC

Fig. 26

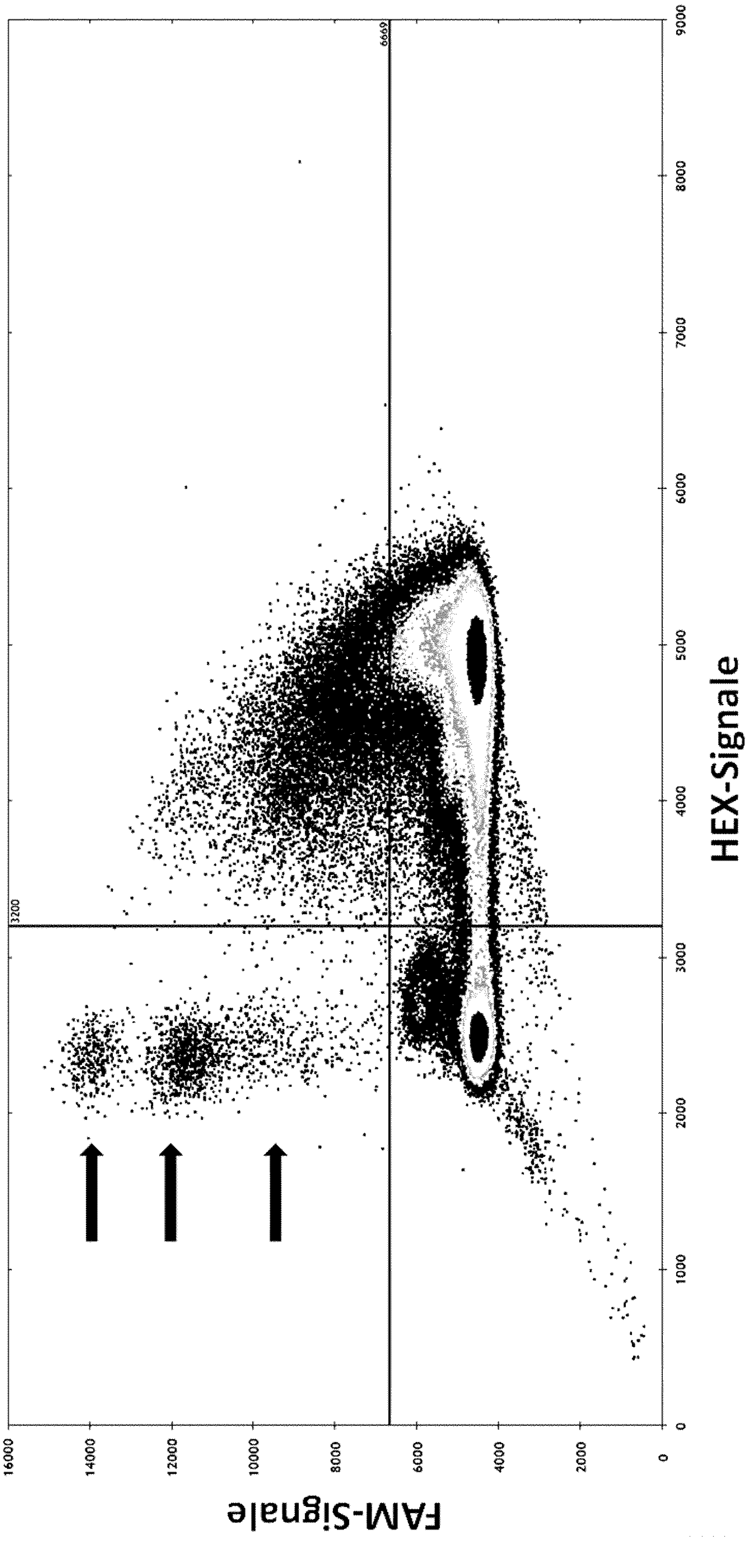
FAM-Signale
HEX-Signale
0
2000
4000
6000
8000
10000
12000
14000
16000
1000
3000
5000
7000
9000
3200
6669

Fig. 27

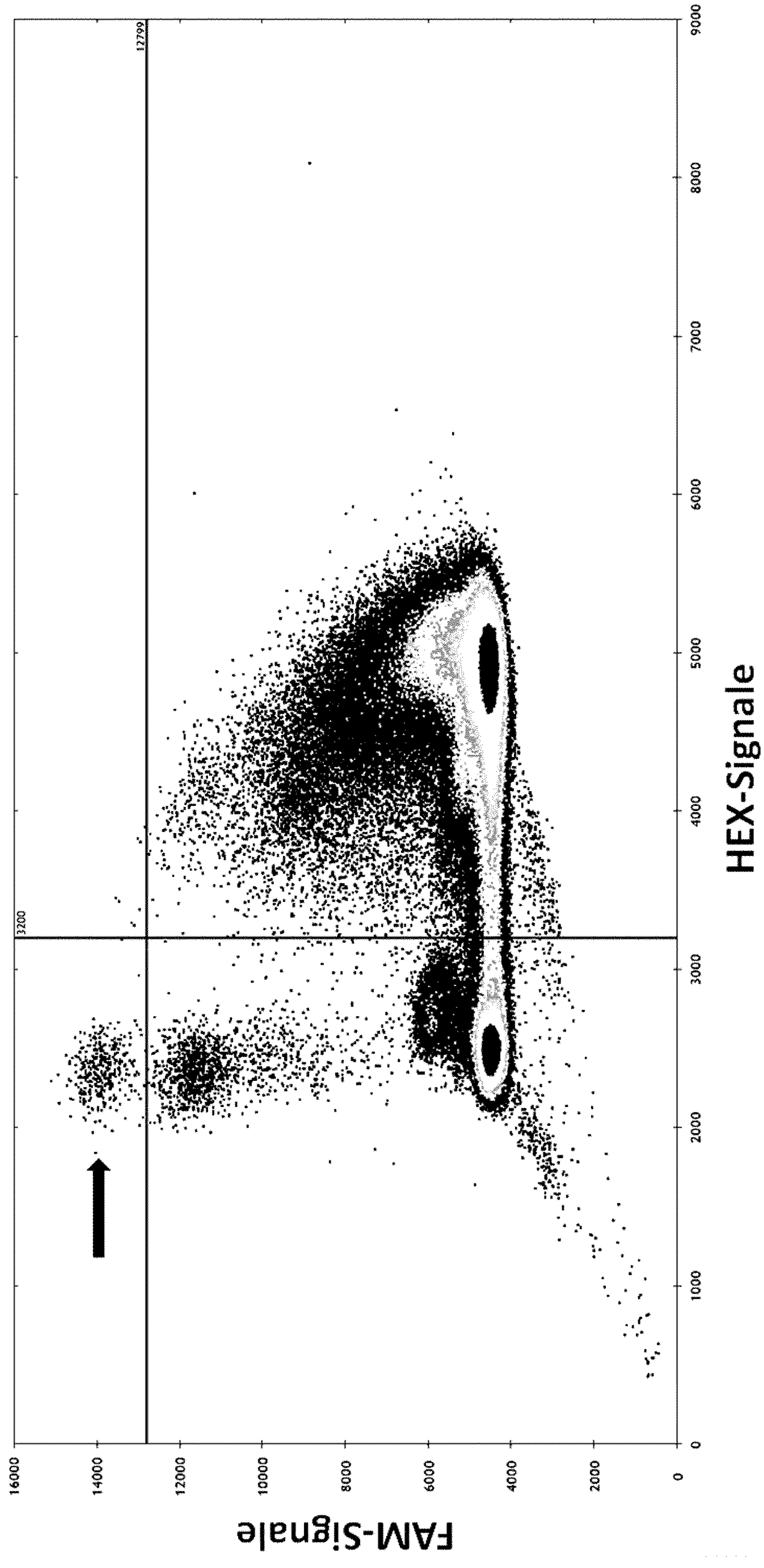
HEX-Signale
FAM-Signale
3200
12799
0
1000
2000
3000
4000
5000
6000
7000
8000
9000
0
2000
4000
6000
8000
10000
12000
14000
16000

Fig. 28

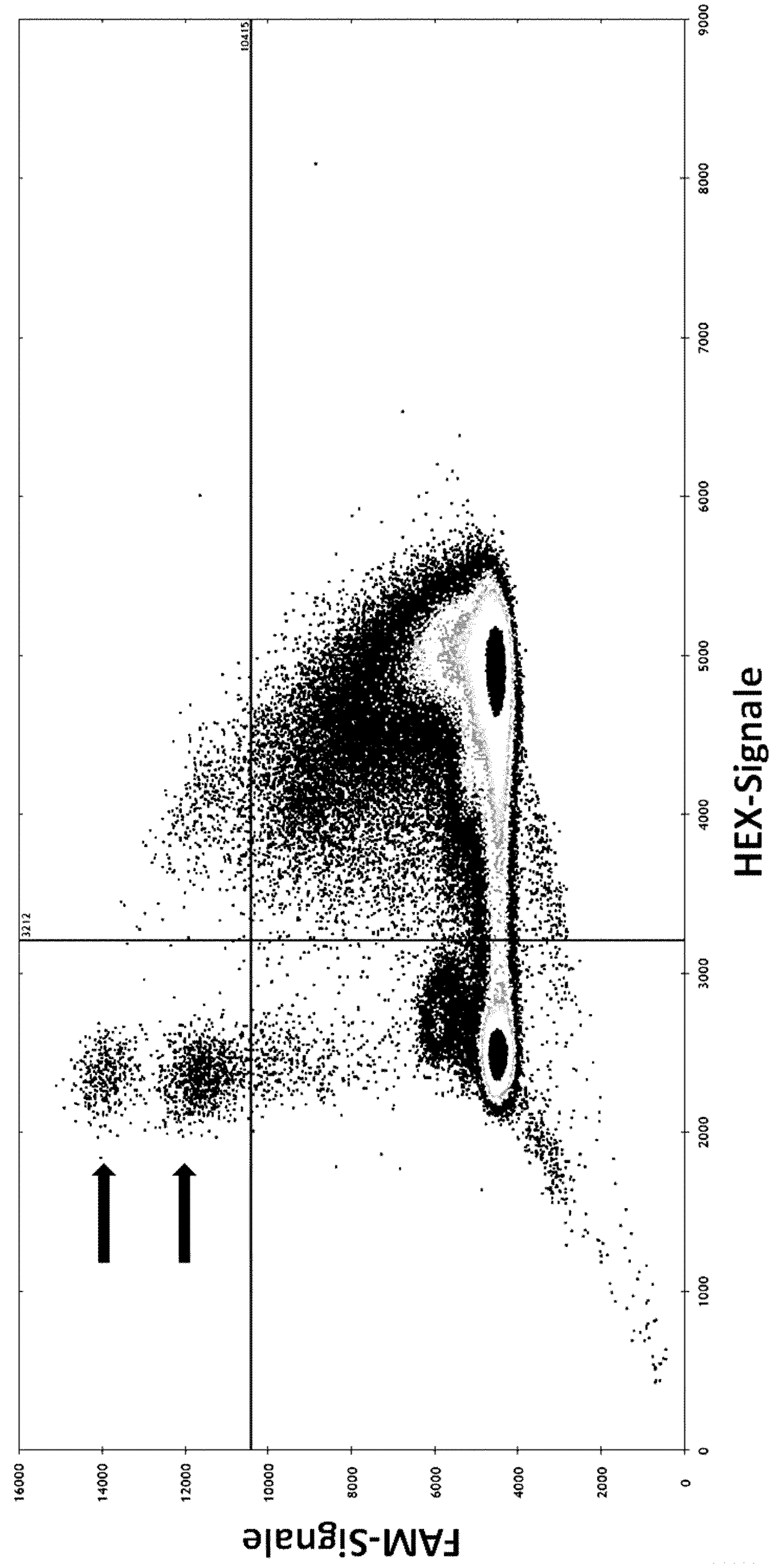
HEX-Signale
FAM-Signale
3212
10415
0
1000
2000
3000
4000
5000
6000
7000
8000
9000
0
2000
4000
6000
8000
10000
12000
14000
16000

Fig. 29

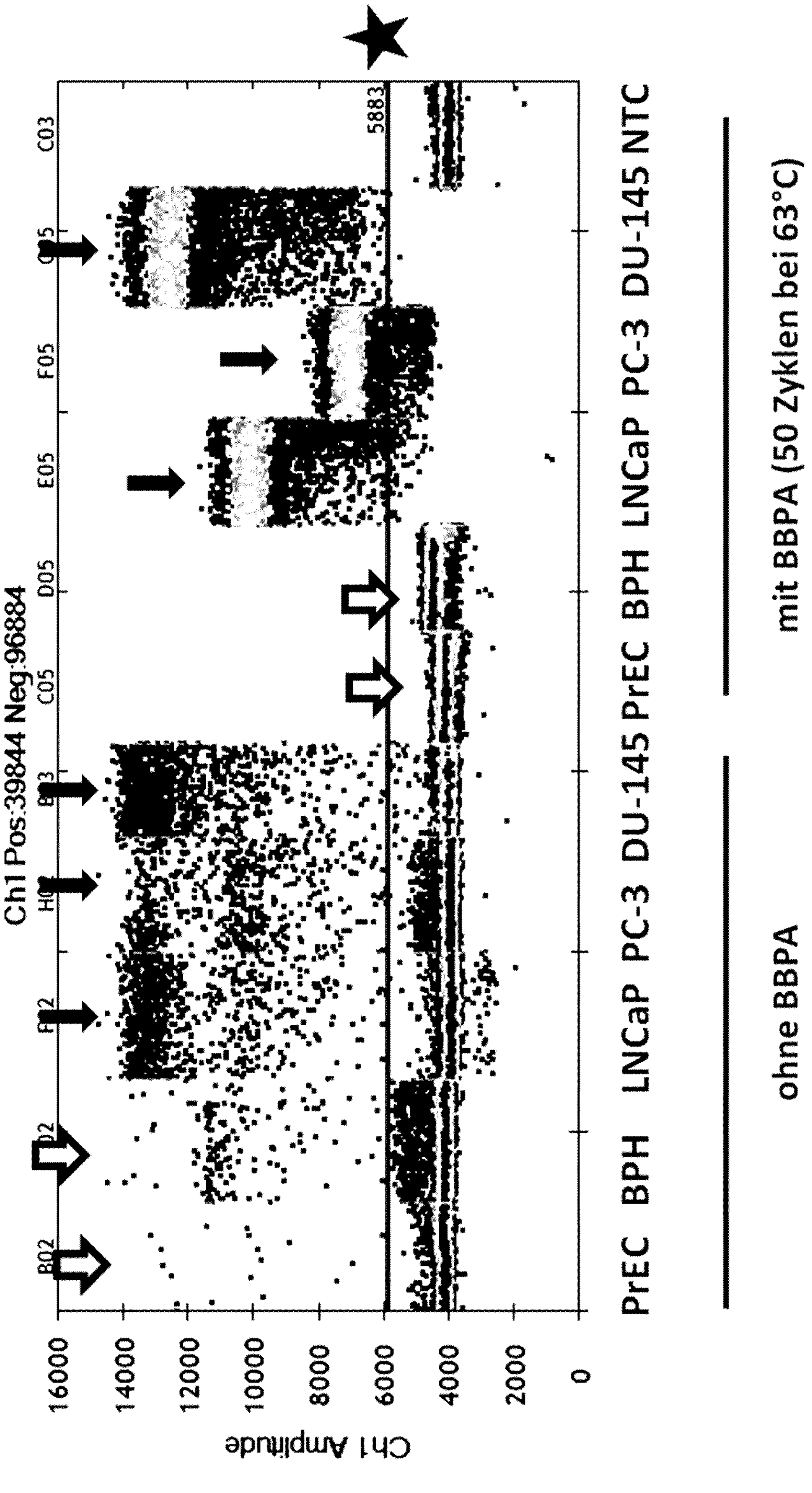
Ch1 Pos:39844 Neg:96884
Ch1 Amplitude
16000
14000
12000
10000
8000
6000
4000
2000
0
B02
C05
D05
E05
F05
C03
5883
PrEC BPH LNCaP PC-3 DU-145 PrEC BPH LNCaP PC-3 DU-145 NTC
ohne BBPA
mit BBPA (50 Zyklen bei 63°C)

Fig. 30

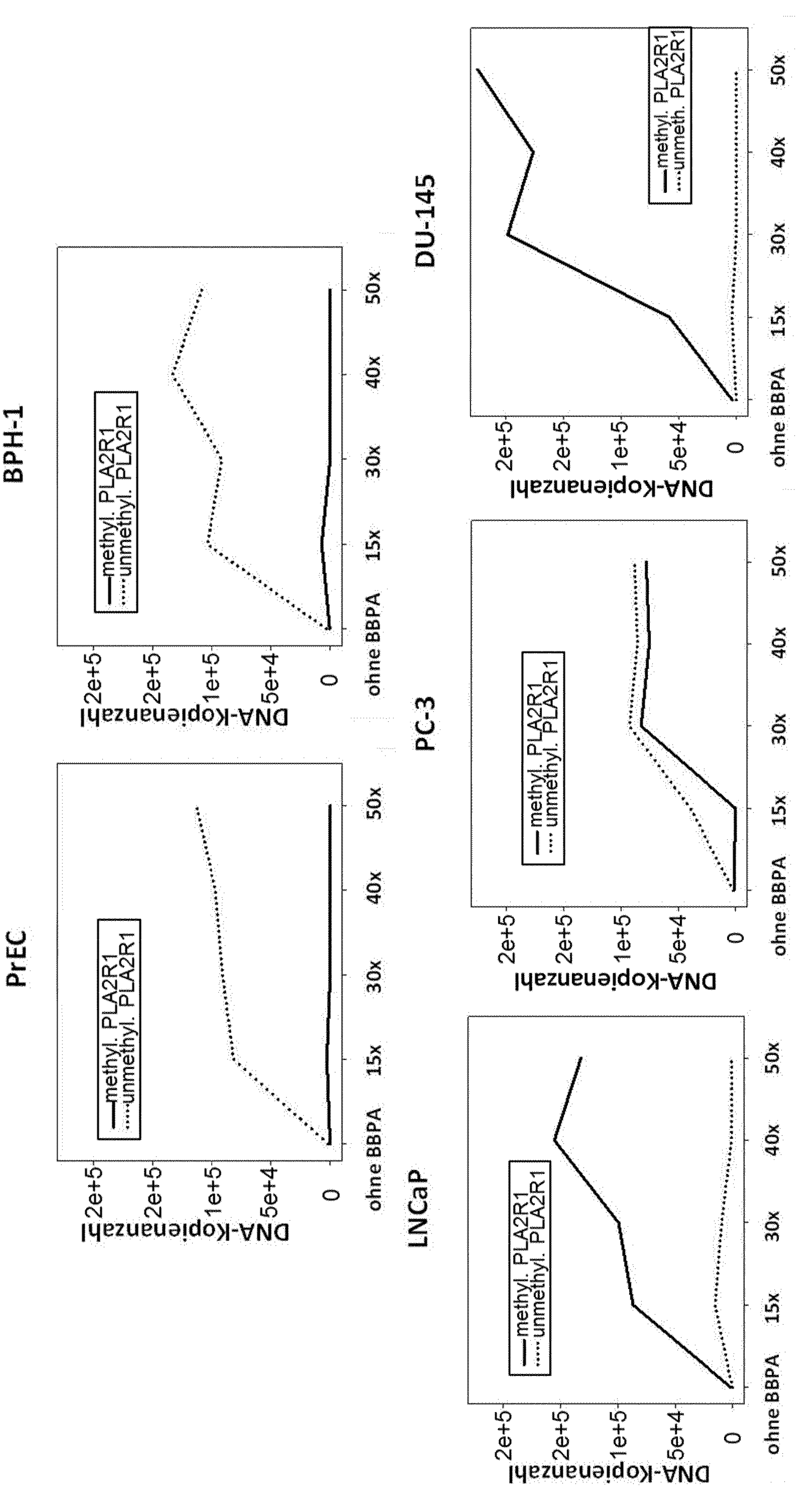
PrEC
BPH-1
LNCaP
PC-3
DU-145
DNA-Kopienanzahl
0
5e+4
1e+5
2e+5
2e+5
ohne BBPA
15x
30x
40x
50x
methyl. PLA2R1
unmethyl. PLA2R1
unmeth. PLA2R1

Fig. 31

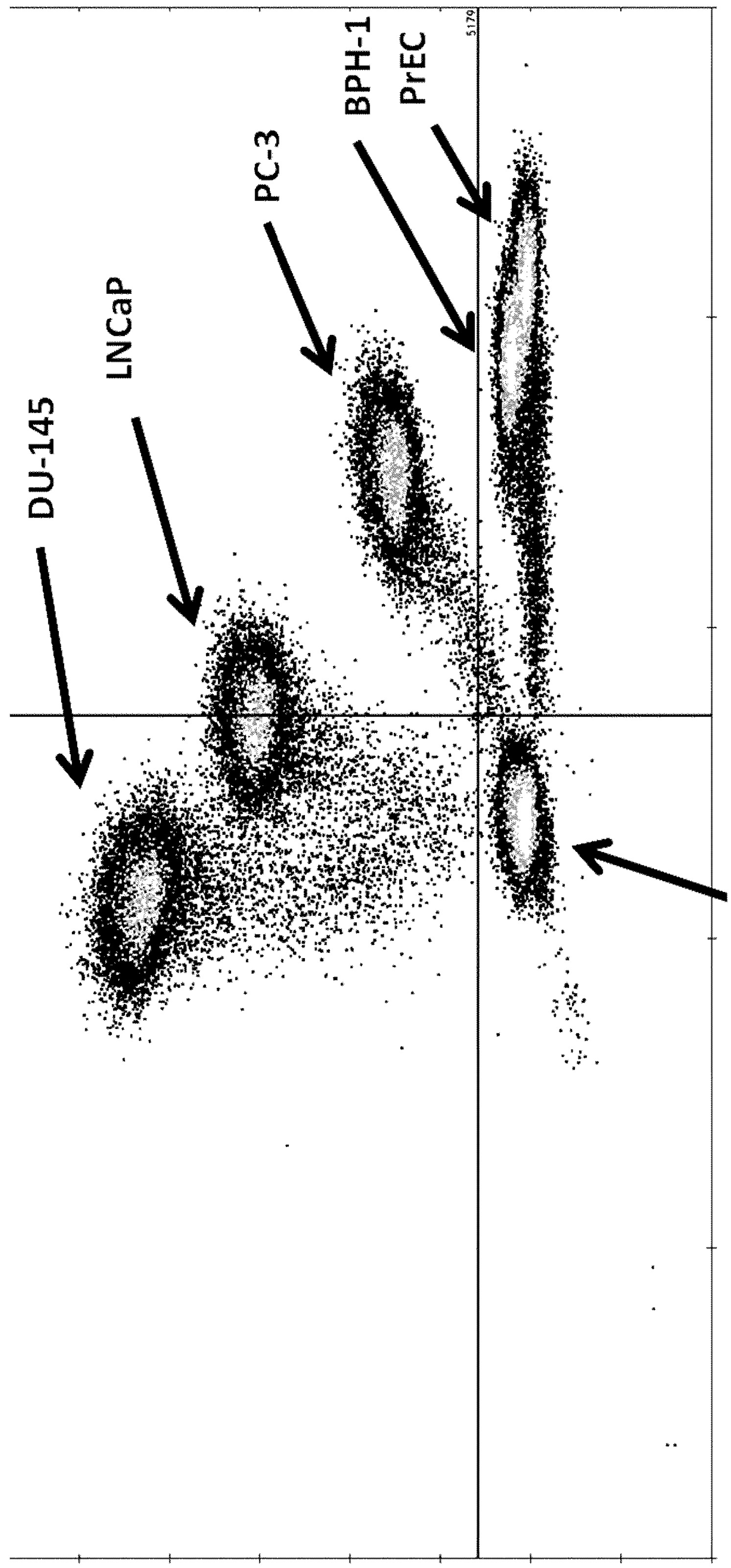


Fig. 32

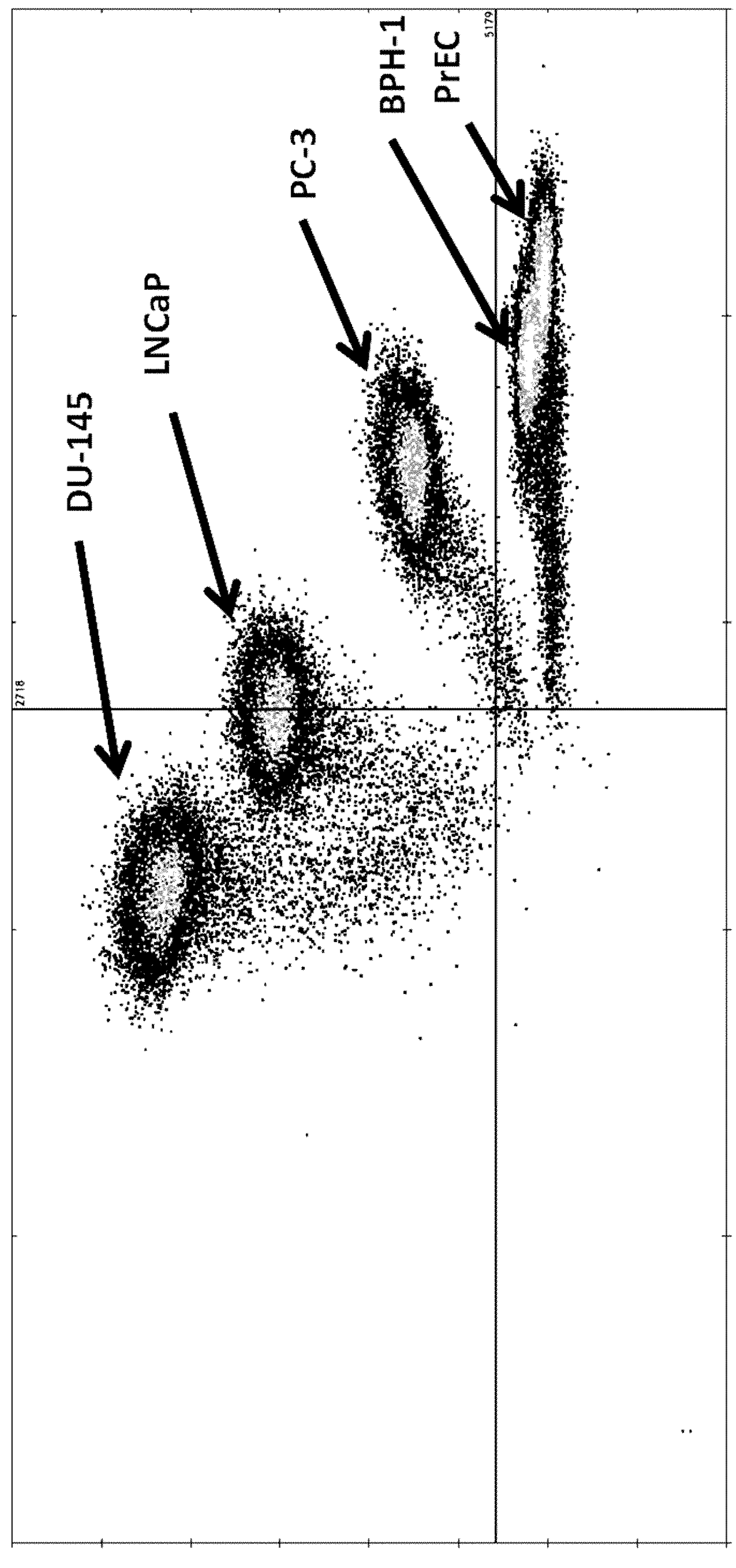


Fig. 33

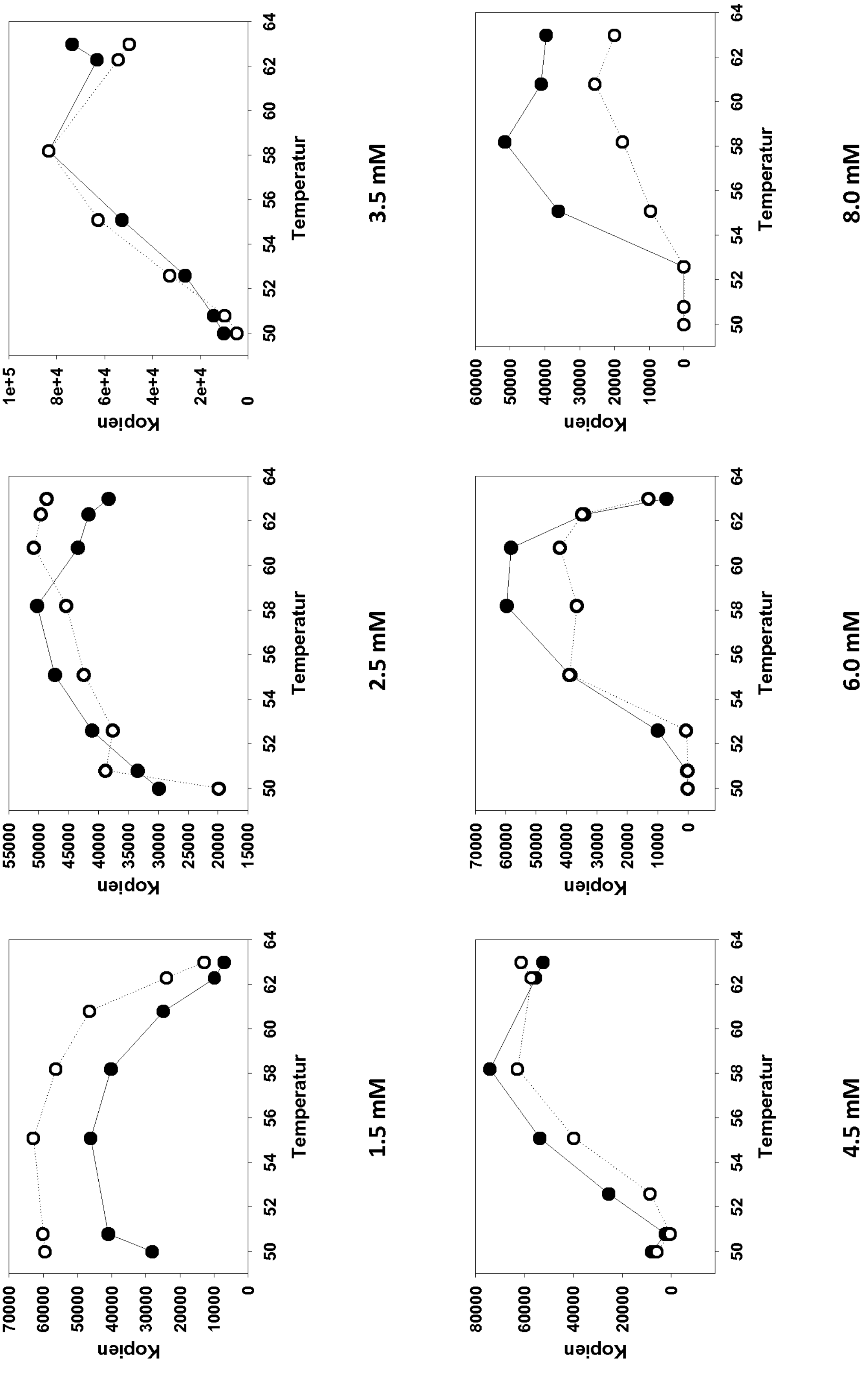


Fig. 34

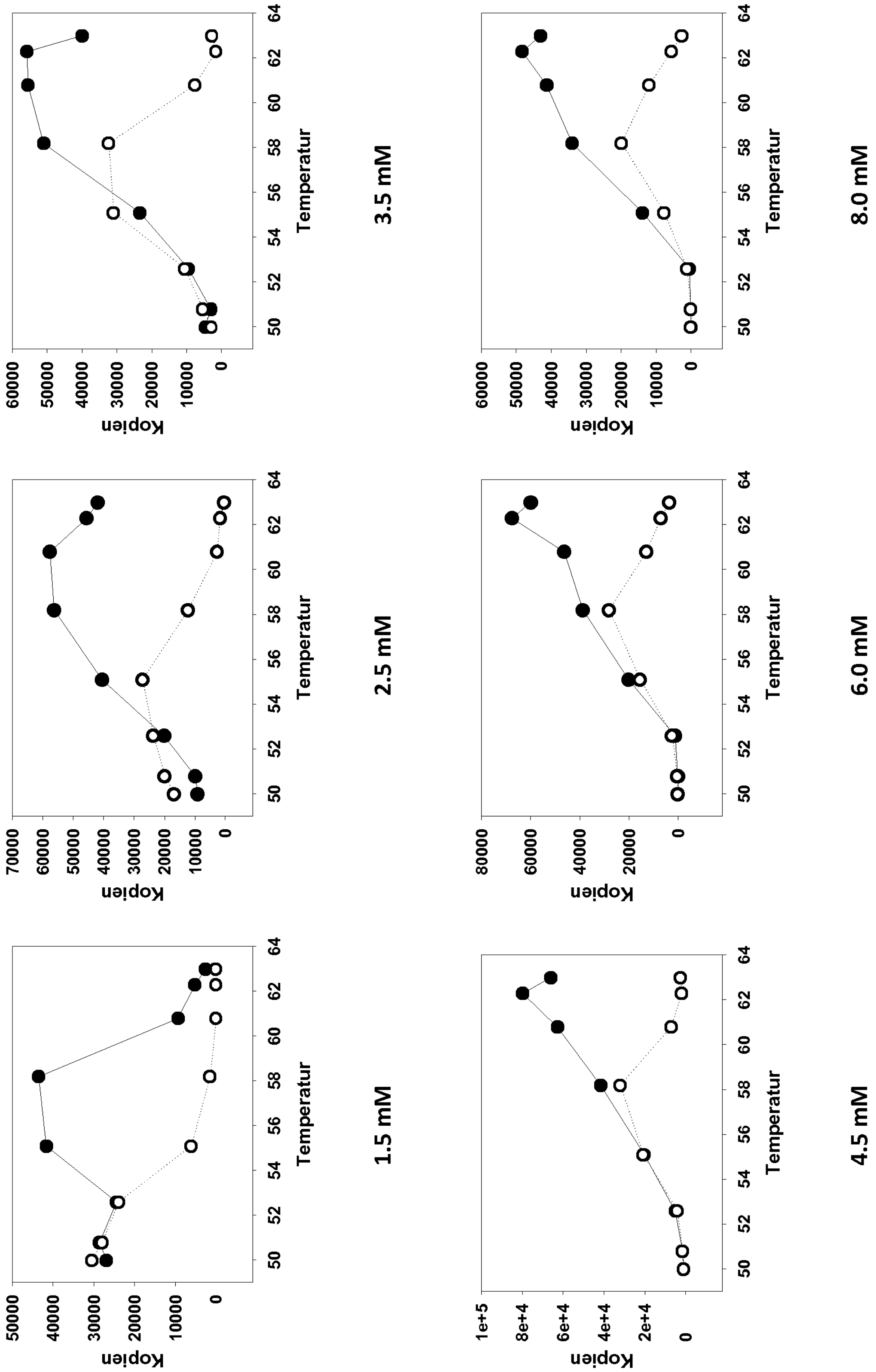


Fig. 35

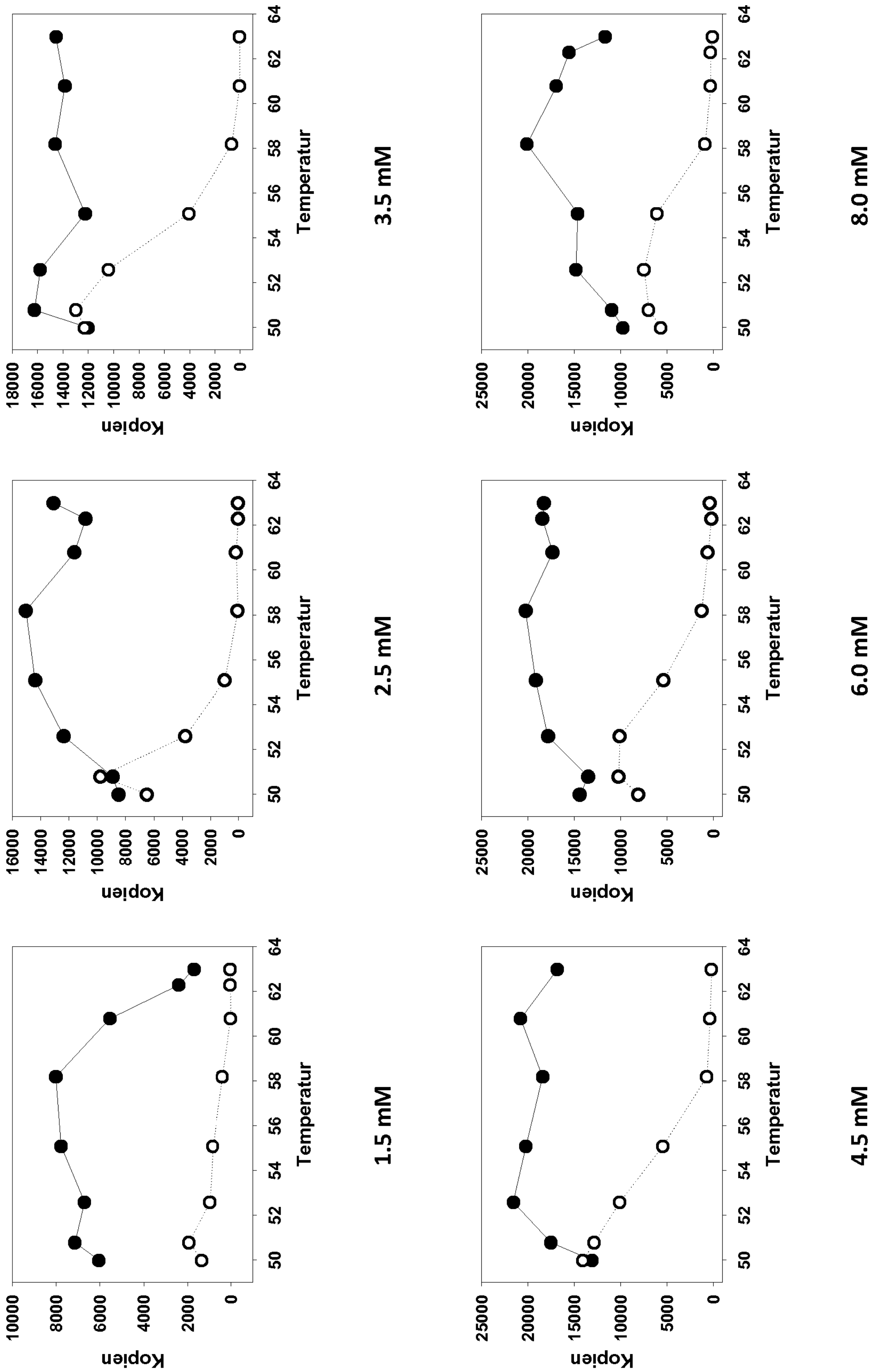


Fig. 36

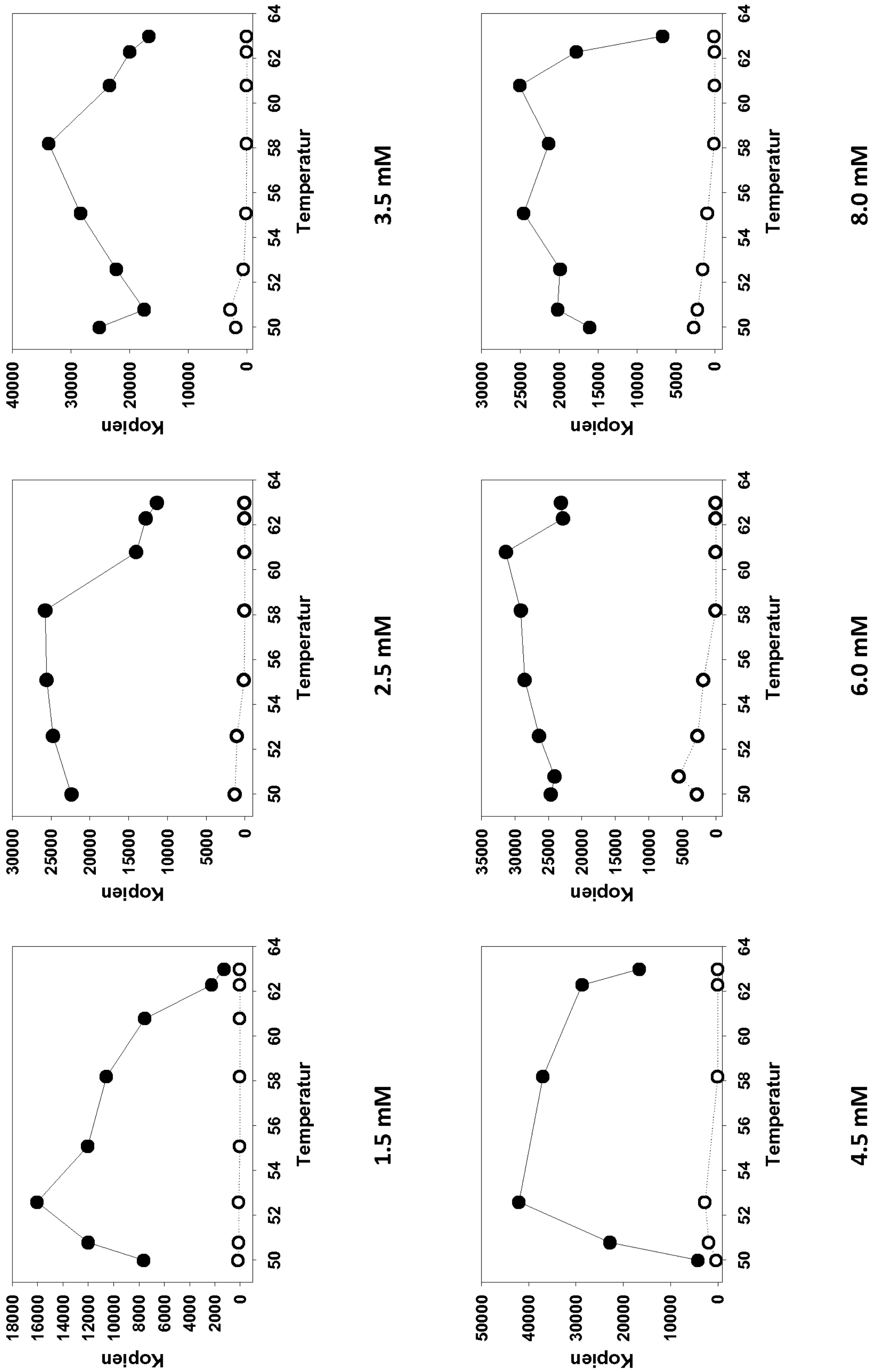


Fig. 37

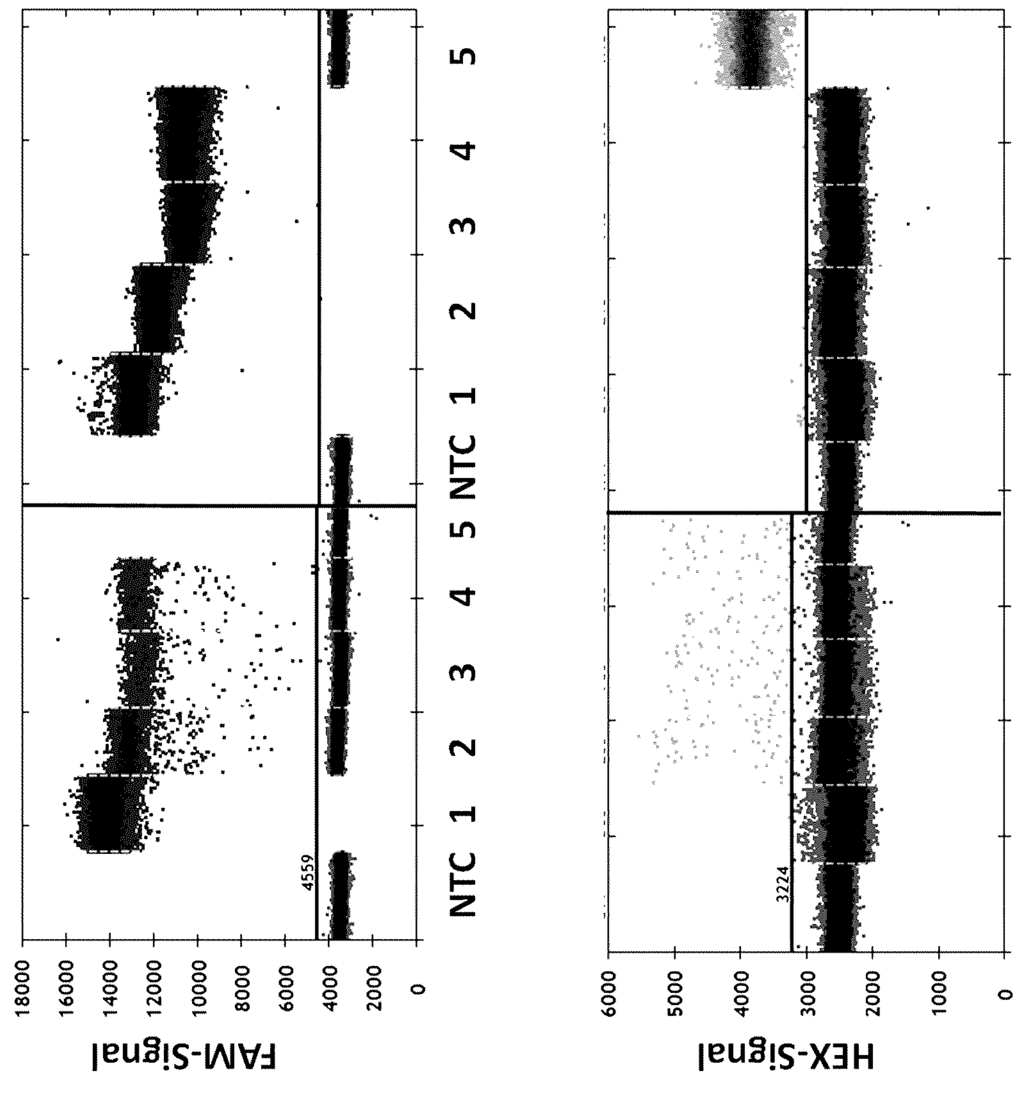
FAM-Signal
18000
16000
14000
12000
10000
8000
6000
4000
2000
0
4559
NTC 1 2 3 4 5 NTC 1 2 3 4 5
HEX-Signal
6000
5000
4000
3000
2000
1000
0
3224
NTC 1 2 3 4 5 NTC 1 2 3 4 5

Fig. 38

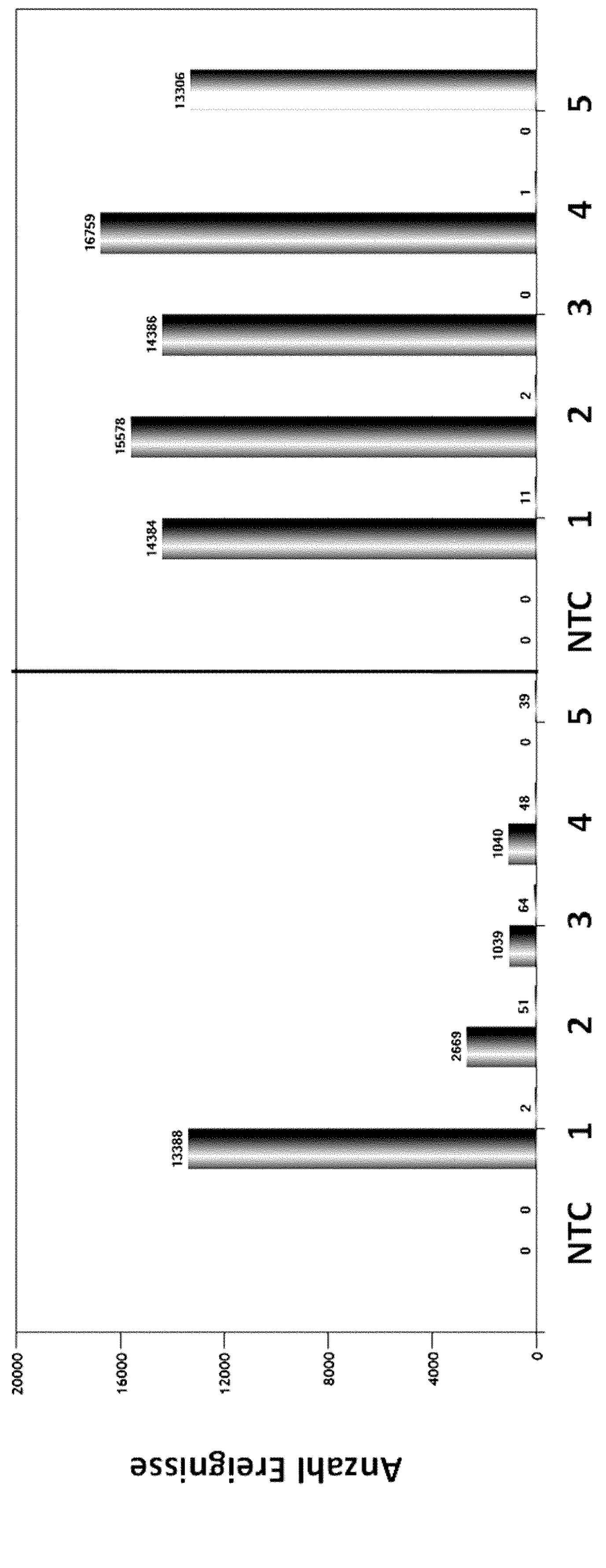
Anzahl Ereignisse
20000
16000
12000
8000
4000
0
NTC
0
0
1
13388
2
2
2669
51
3
1039
64
4
1040
48
5
0
39
NTC
0
0
1
14384
11
2
15578
2
3
14386
0
4
16759
1
5
0
13306

Fig. 39

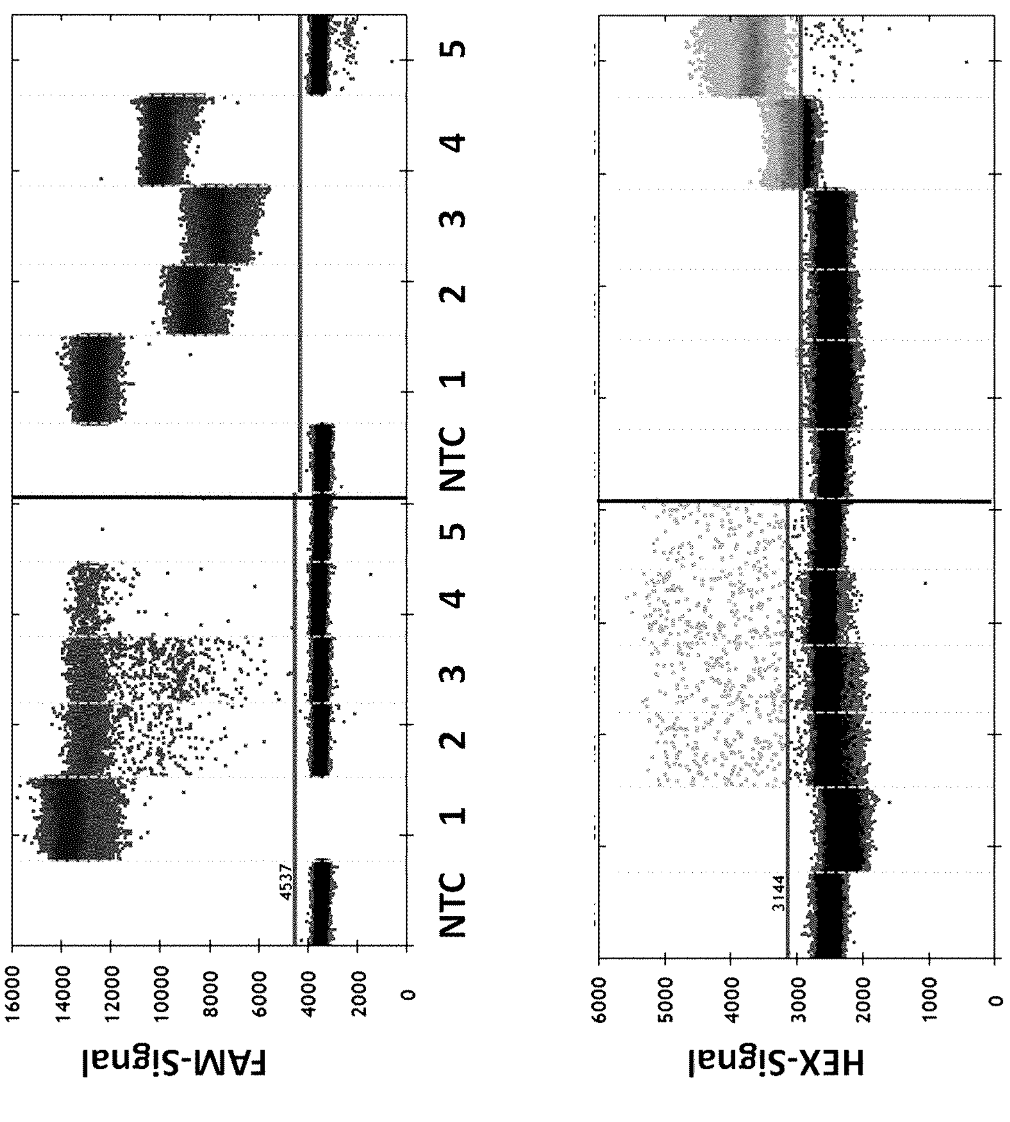
FAM-Signal
16000
14000
12000
10000
8000
6000
4000
2000
0
4537
NTC 1 2 3 4 5 NTC 1 2 3 4 5
HEX-Signal
6000
5000
4000
3000
2000
1000
0
3144
NTC 1 2 3 4 5 NTC 1 2 3 4 5

Fig. 40

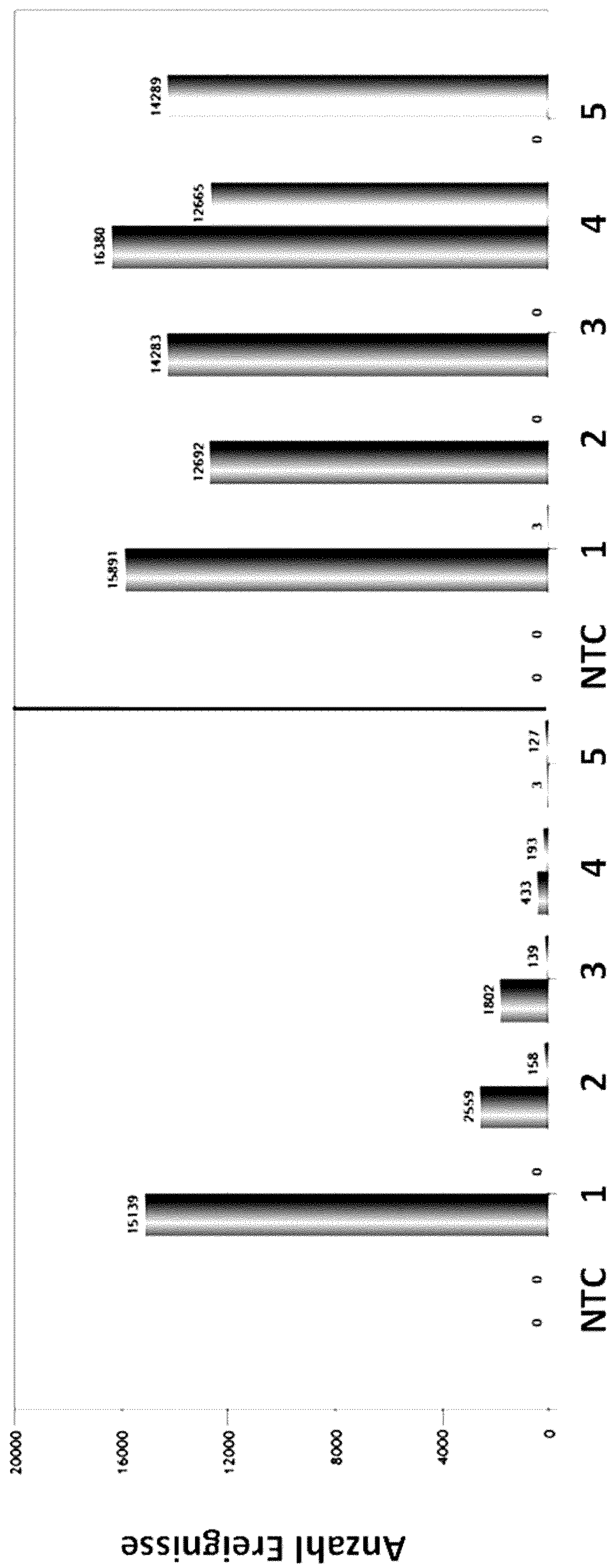
Anzahl Ereignisse
0
4000
8000
12000
16000
20000
NTC
1
2
3
4
5
0
0
15139
0
2559
158
1802
139
433
193
3
127
0
0
15891
3
12692
0
14283
0
16380
12665
0
14289

Fig. 41

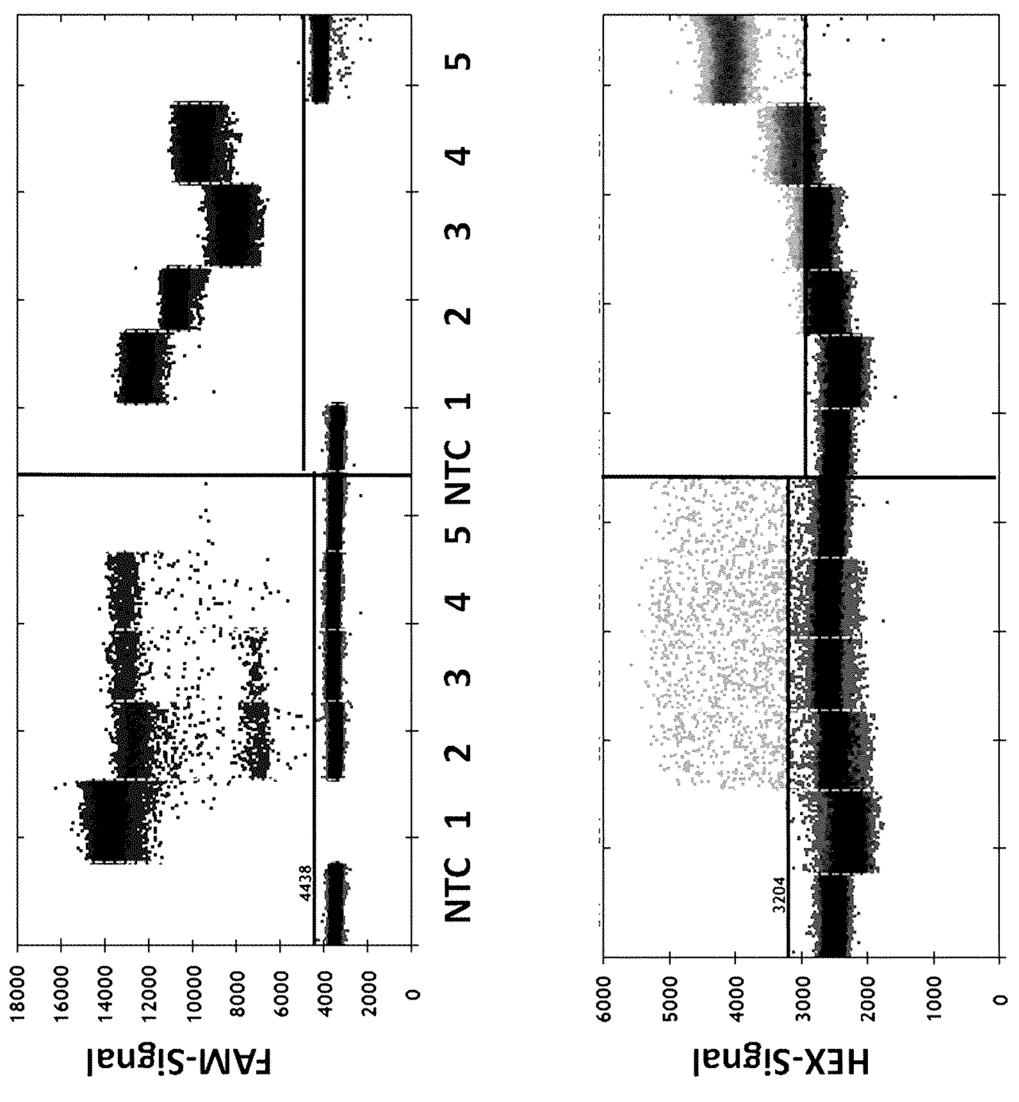
FAM-Signal
18000
16000
14000
12000
10000
8000
6000
4000
2000
0
4438
NTC 1 2 3 4 5 NTC 1 2 3 4 5
HEX-Signal
6000
5000
4000
3000
2000
1000
0
3204
NTC 1 2 3 4 5 NTC 1 2 3 4 5

Fig. 42

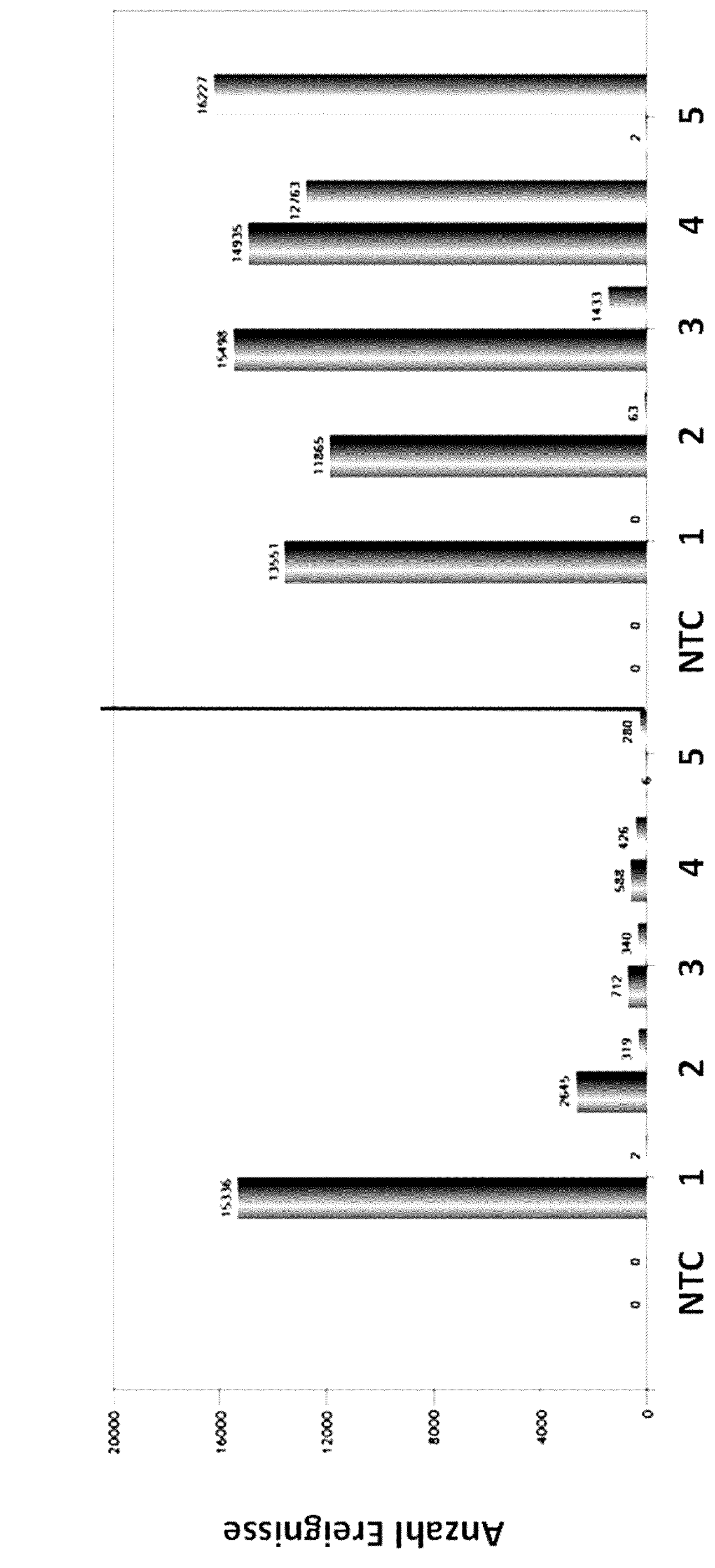
Anzahl Ereignisse
0
4000
8000
12000
16000
20000
NTC
1
2
3
4
5
0
0
15336
2
2645
319
712
340
588
426
6
280
0
0
13551
0
11865
63
15498
1433
14935
12763
2
16227

Fig. 43

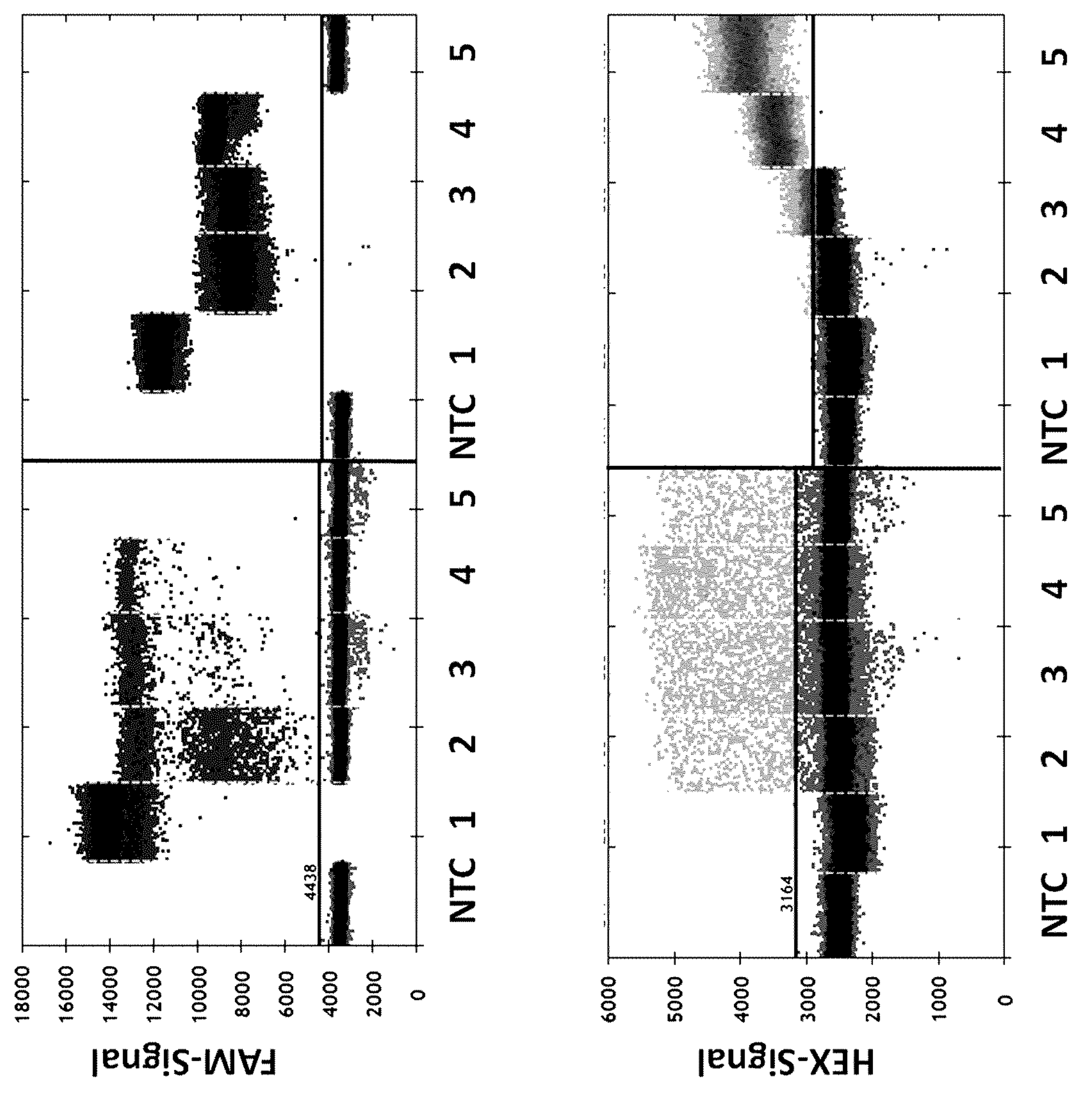
FAM-Signal
18000
16000
14000
12000
10000
8000
6000
4000
2000
0
4438
NTC 1 2 3 4 5 NTC 1 2 3 4 5
HEX-Signal
6000
5000
4000
3000
2000
1000
0
3164
NTC 1 2 3 4 5 NTC 1 2 3 4 5

Fig. 44

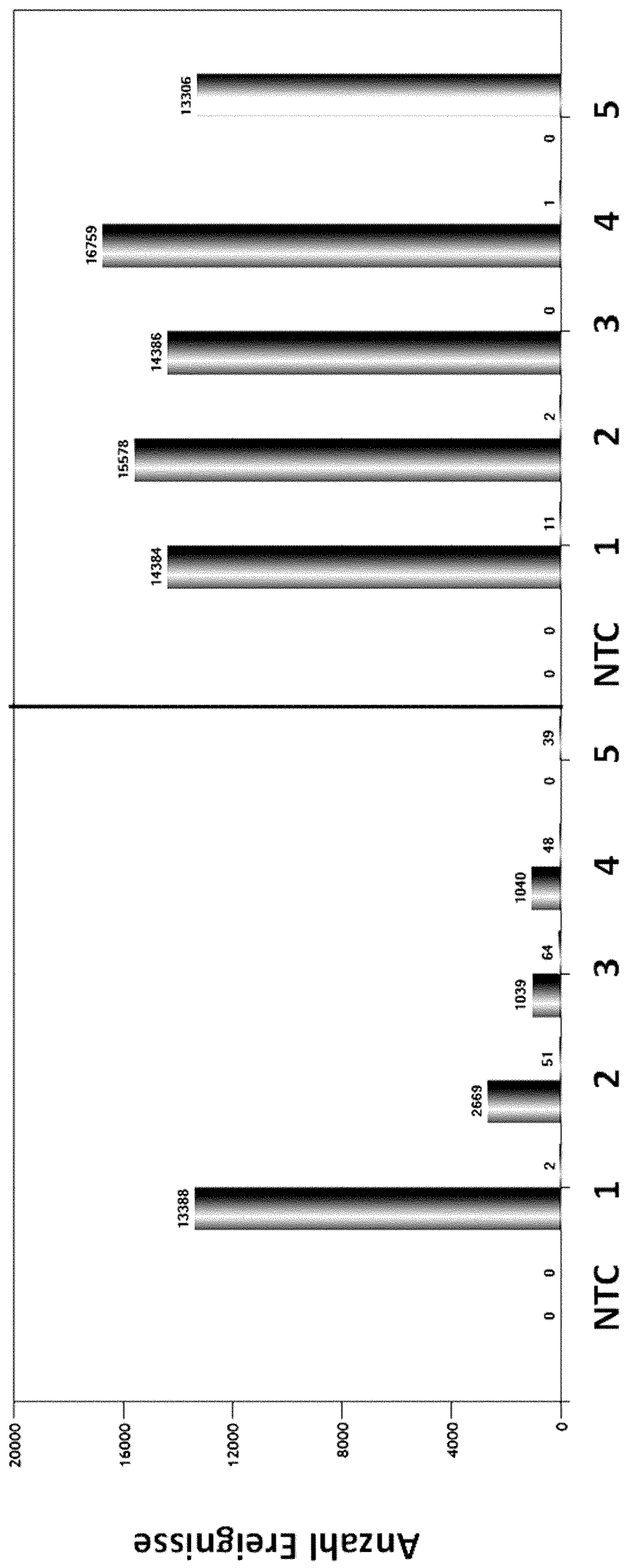
Anzahl Ereignisse
0
4000
8000
12000
16000
20000
NTC
1
2
3
4
5
0
0
13388
2
2669
51
1039
64
1040
48
0
39
14384
11
15578
2
14386
0
16759
1
0
13306

Fig. 45

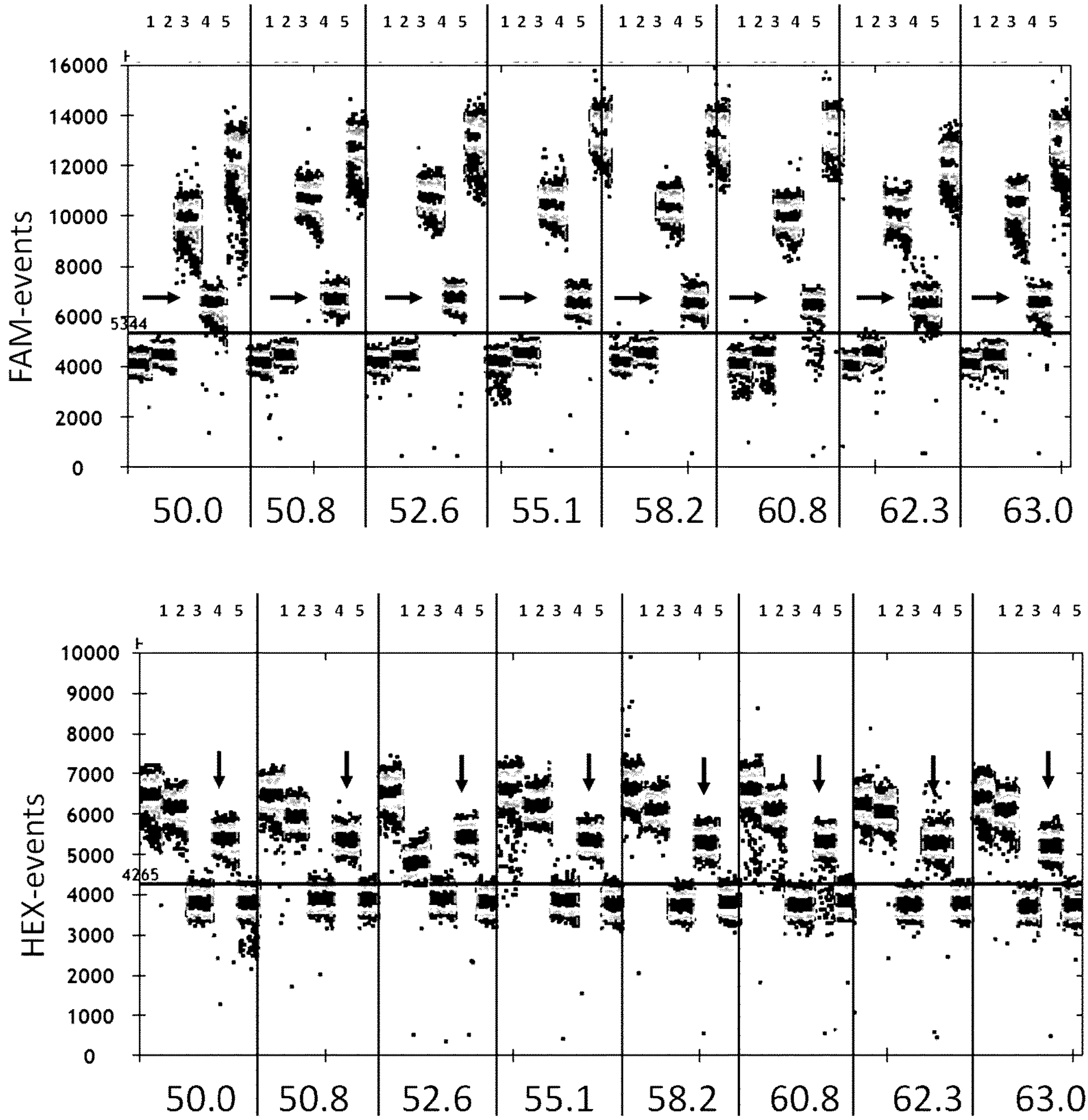
1 2 3 4 5
16000
14000
12000
10000
8000
6000
4000
2000
0
5344
FAM-events
50.0
50.8
52.6
55.1
58.2
60.8
62.3
63.0
10000
9000
8000
7000
6000
5000
4000
3000
2000
1000
0
4265
HEX-events

Fig. 46

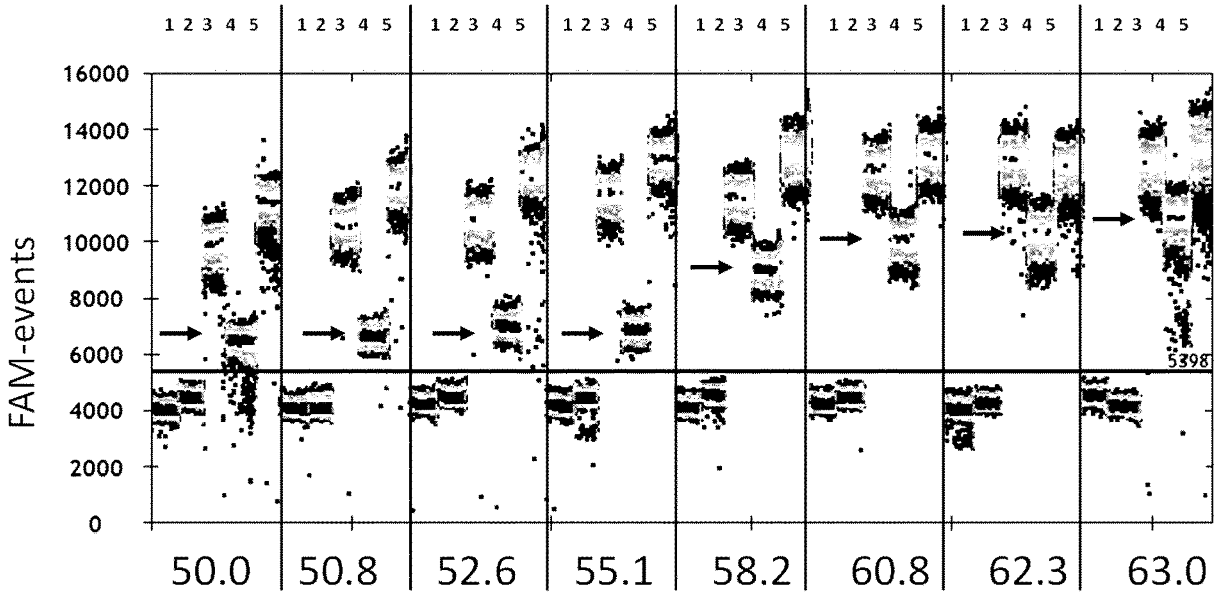
1 2 3 4 5
FAM-events
16000
14000
12000
10000
8000
6000
4000
2000
0
5398
50.0
50.8
52.6
55.1
58.2
60.8
62.3
63.0

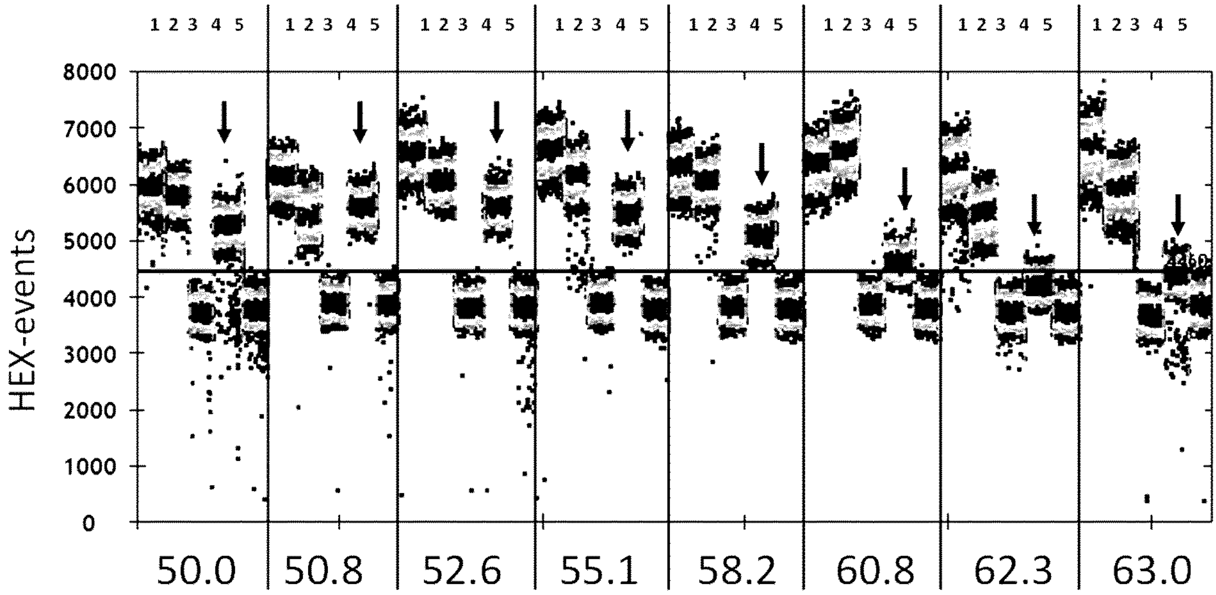
1 2 3 4 5
HEX-events
8000
7000
6000
5000
4000
3000
2000
1000
0
4460
50.0
50.8
52.6
55.1
58.2
60.8
62.3
63.0

Fig. 47

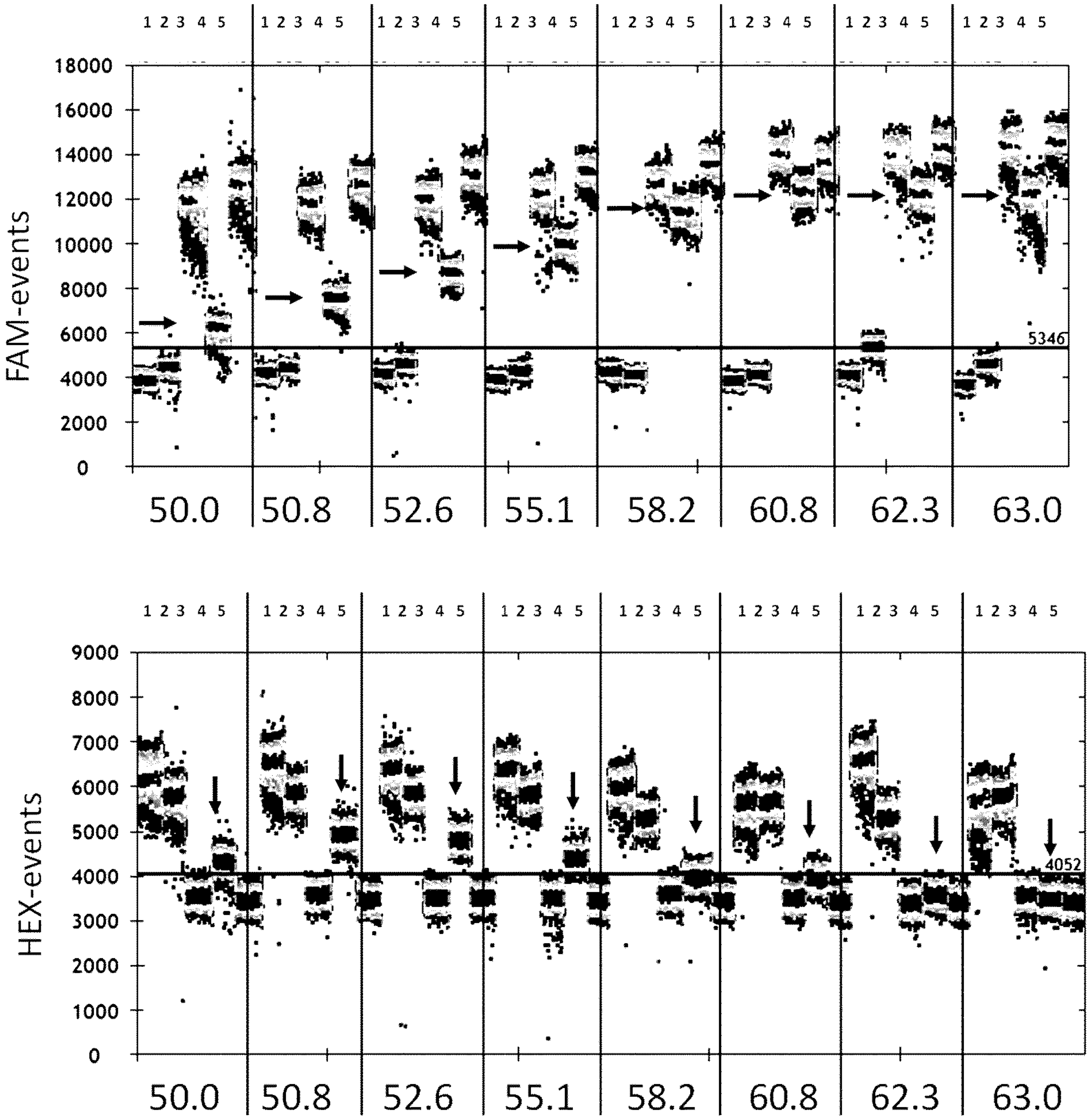
1 2 3 4 5
FAM-events
18000
16000
14000
12000
10000
8000
6000
4000
2000
0
5346
50.0
50.8
52.6
55.1
58.2
60.8
62.3
63.0
HEX-events
9000
8000
7000
6000
5000
4000
3000
2000
1000
0
4052

Fig. 48

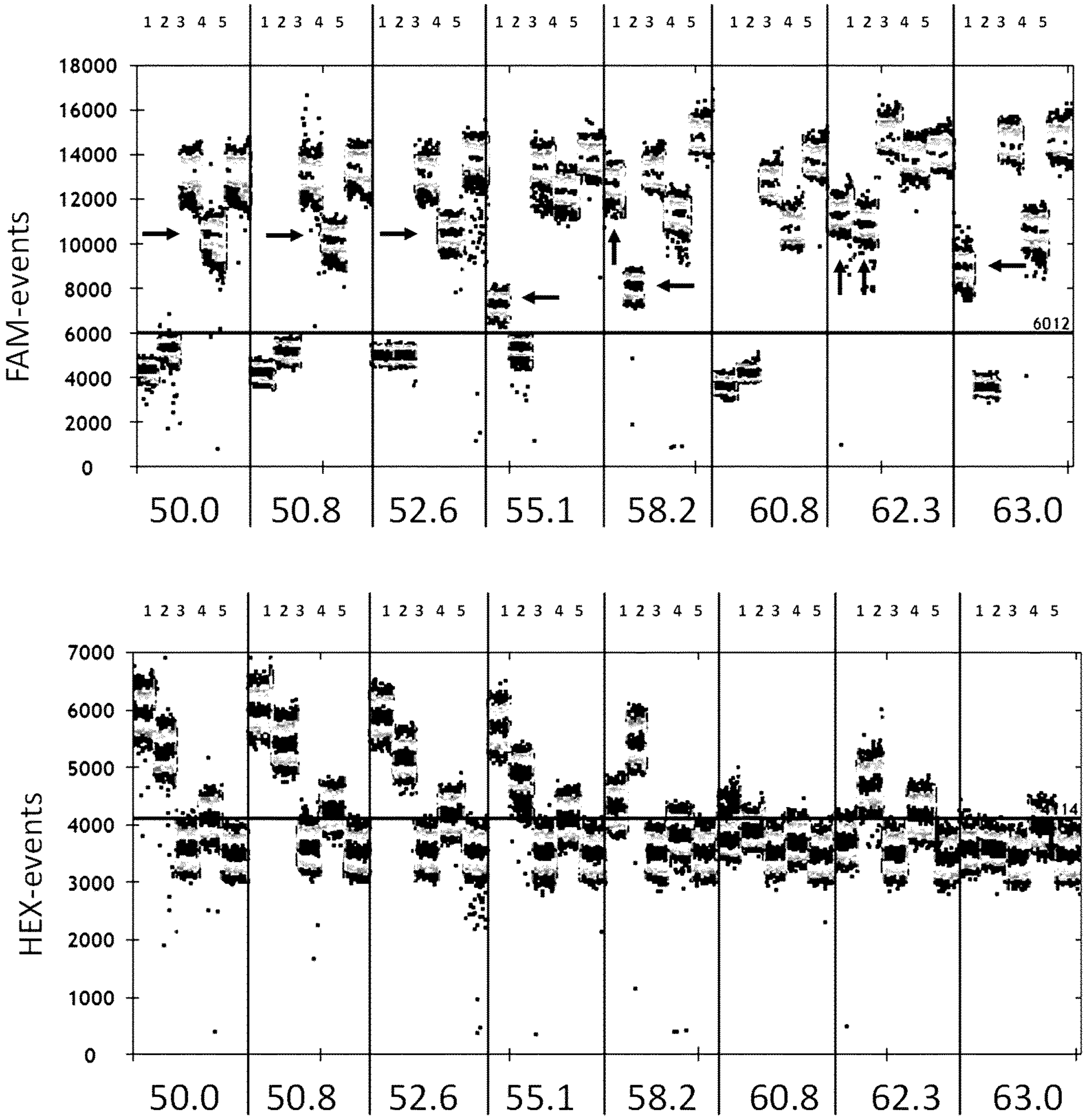

1 2 3 4 5
FAM-events
18000
16000
14000
12000
10000
8000
6000
4000
2000
0
6012
50.0
50.8
52.6
55.1
58.2
60.8
62.3
63.0
HEX-events
7000
6000
5000
4000
3000
2000
1000
0
4114


Fig. 49

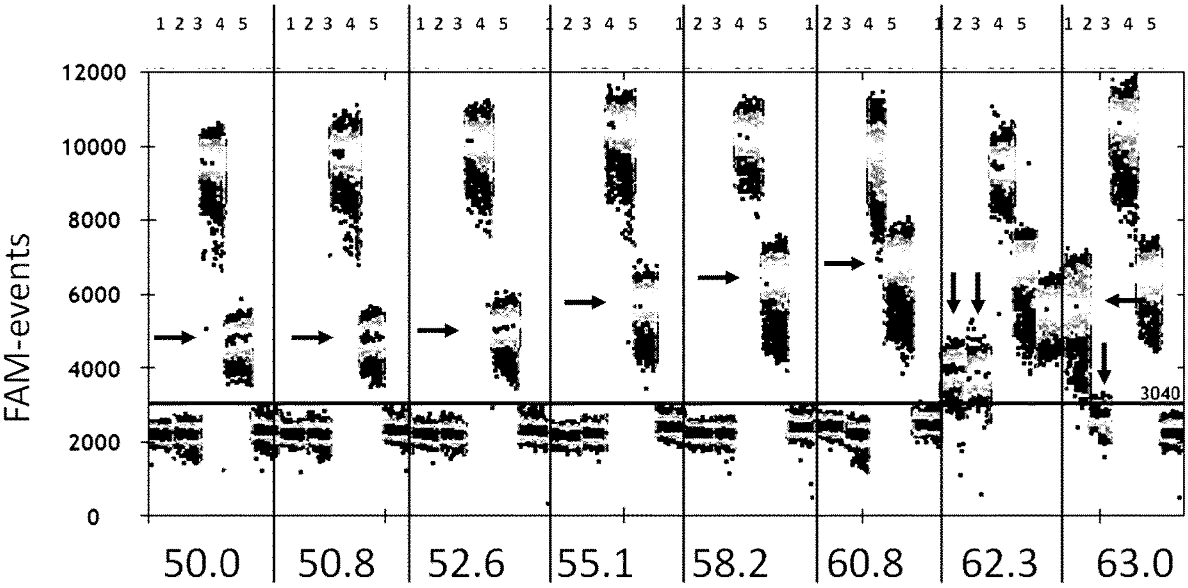
1 2 3 4 5
12000
10000
8000
6000
4000
2000
0
FAM-events
3040
50.0
50.8
52.6
55.1
58.2
60.8
62.3
63.0

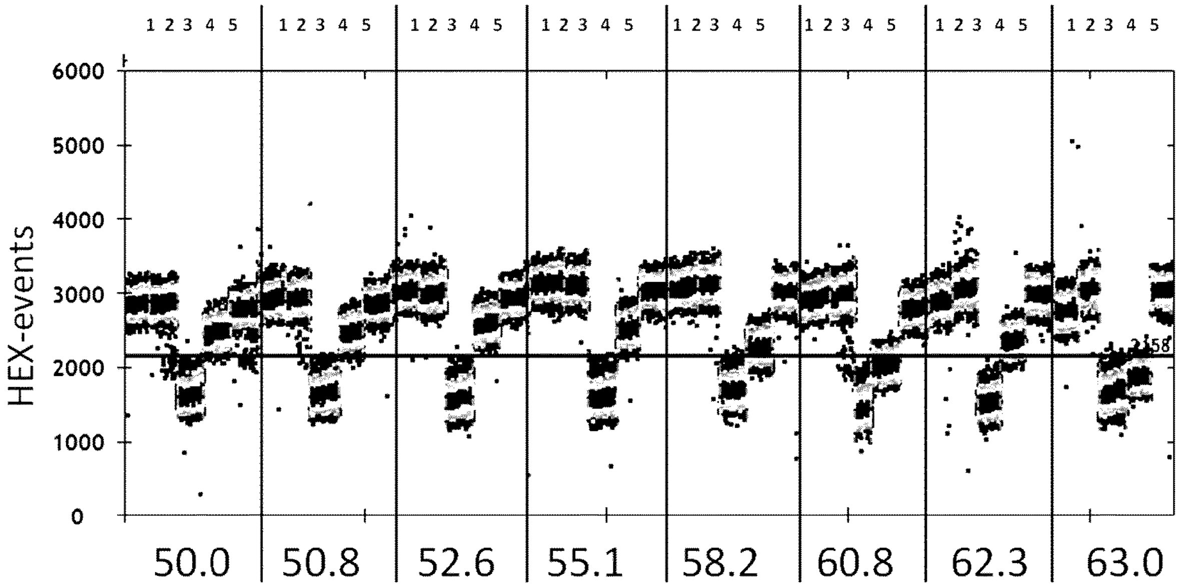
1 2 3 4 5
6000
5000
4000
3000
2000
1000
0
HEX-events
2158
50.0
50.8
52.6
55.1
58.2
60.8
62.3
63.0

**Fig. 50**

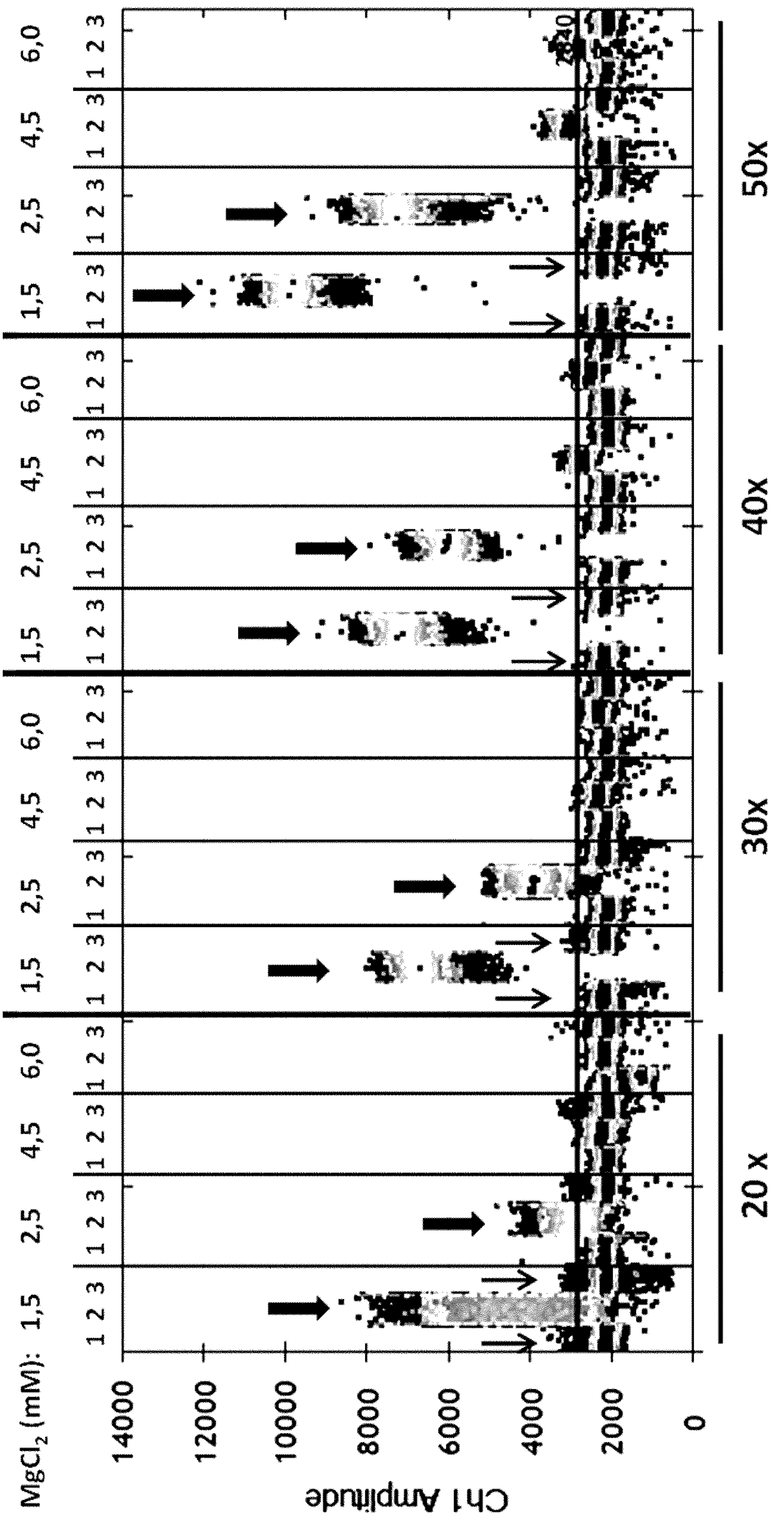
MgCl$_2$ (mM):
1,5
2,5
4,5
6,0
1 2 3
Ch1 Amplitude
14000
12000
10000
8000
6000
4000
2000
0
20 x
30x
40x
50x

Fig. 51

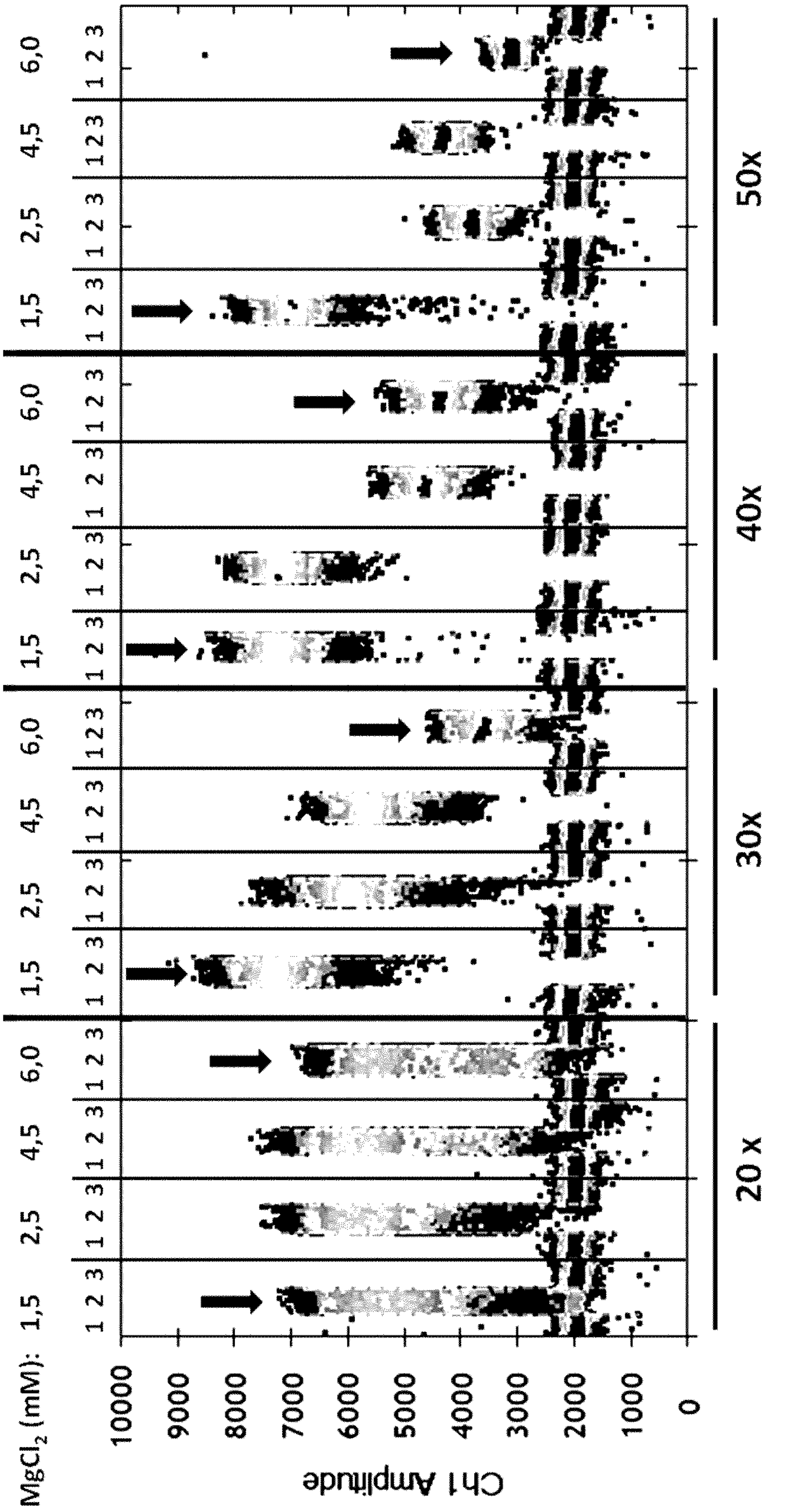
MgCl$_2$ (mM):
1,5
2,5
4,5
6,0
1 2 3
Ch1 Amplitude
10000
9000
8000
7000
6000
5000
4000
3000
2000
1000
0
20 x
30x
40x
50x

Fig. 52

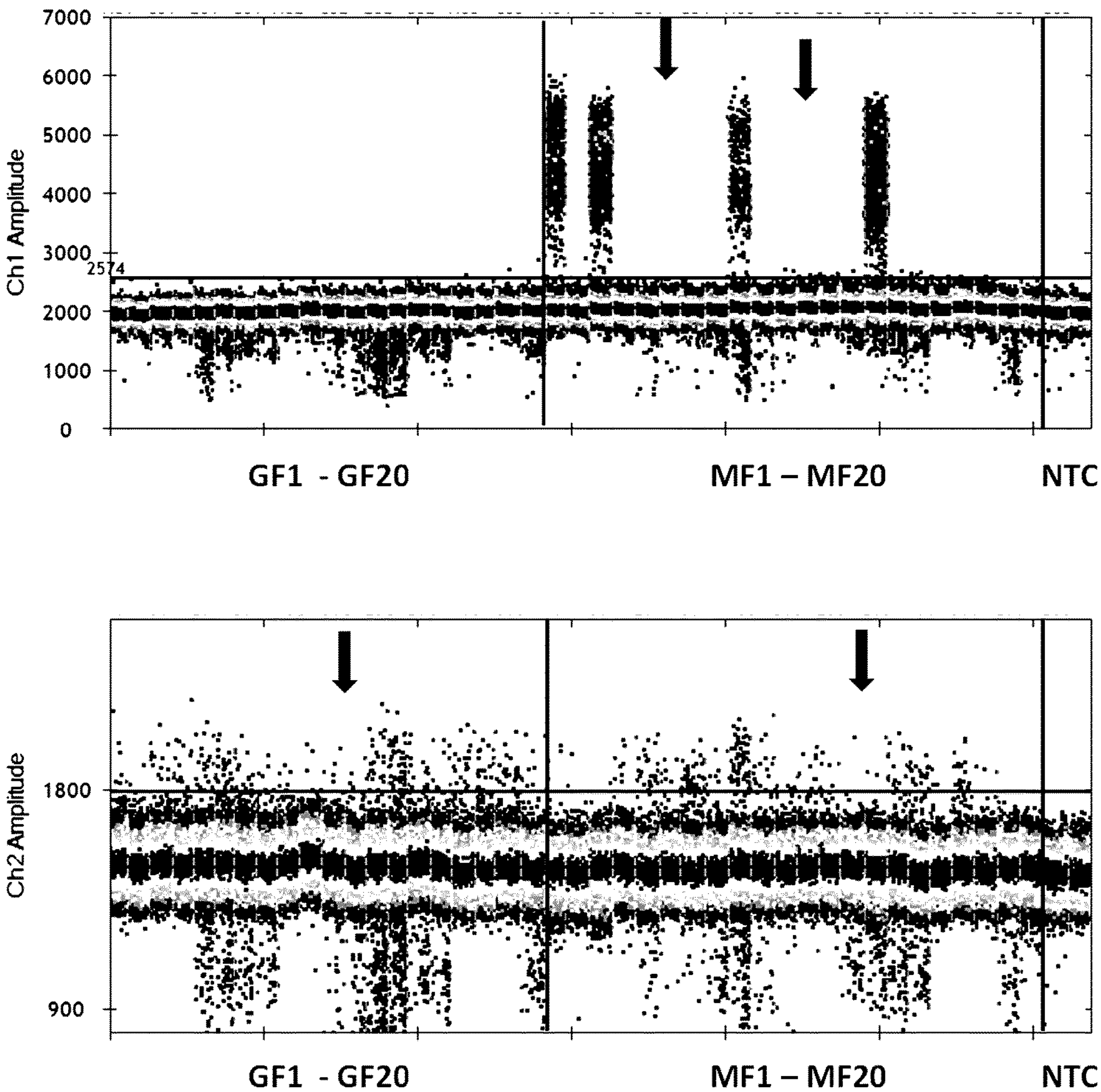
7000
6000
5000
4000
3000
2574
2000
1000
0
Ch1 Amplitude
GF1 - GF20
MF1 – MF20
NTC
1800
900
Ch2 Amplitude
GF1 - GF20
MF1 – MF20
NTC

Fig. 53

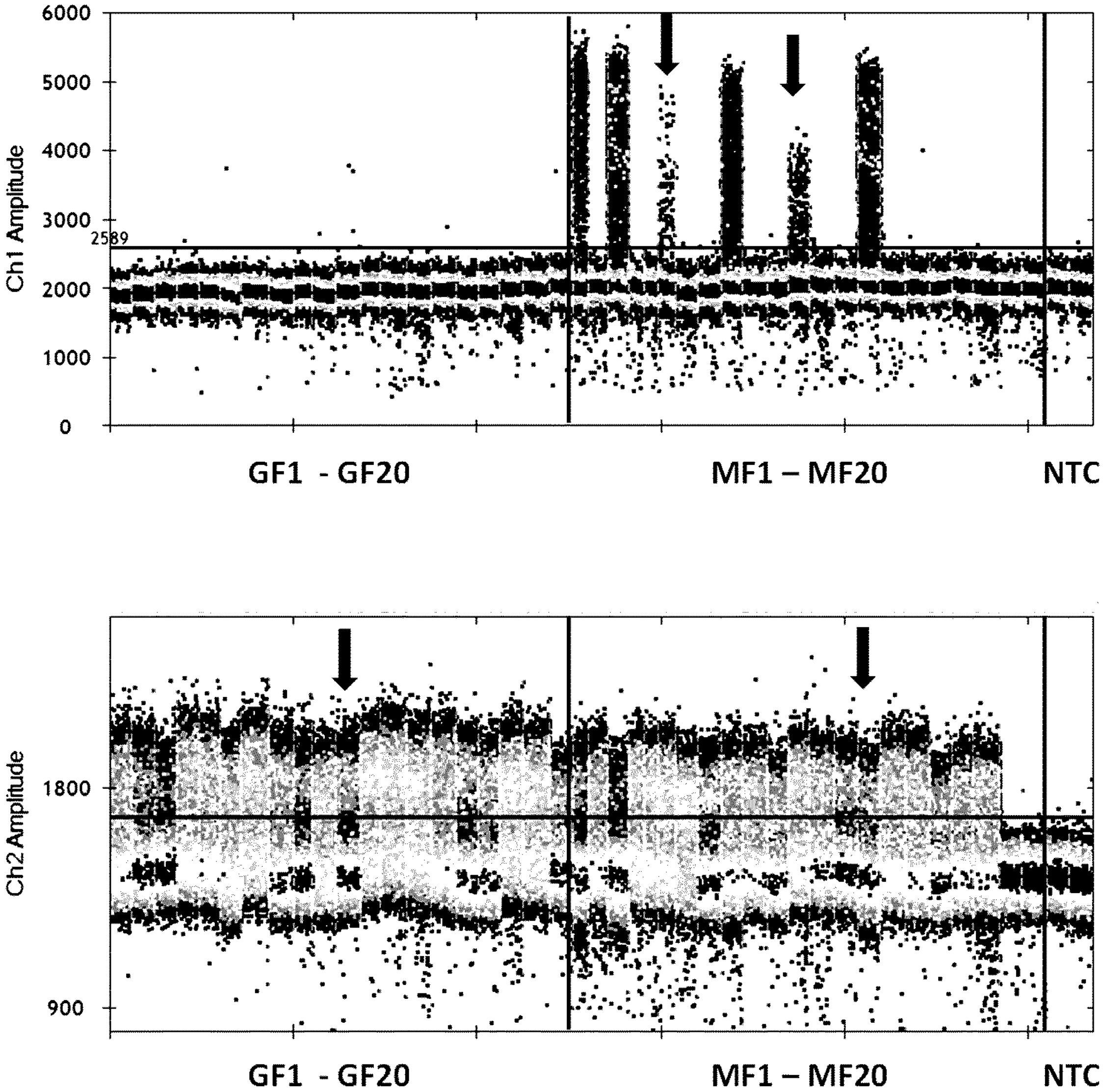
6000
5000
4000
3000
2589
2000
1000
0
Ch1 Amplitude
GF1 - GF20
MF1 – MF20
NTC
1800
900
Ch2 Amplitude
GF1 - GF20
MF1 – MF20
NTC

Fig. 54

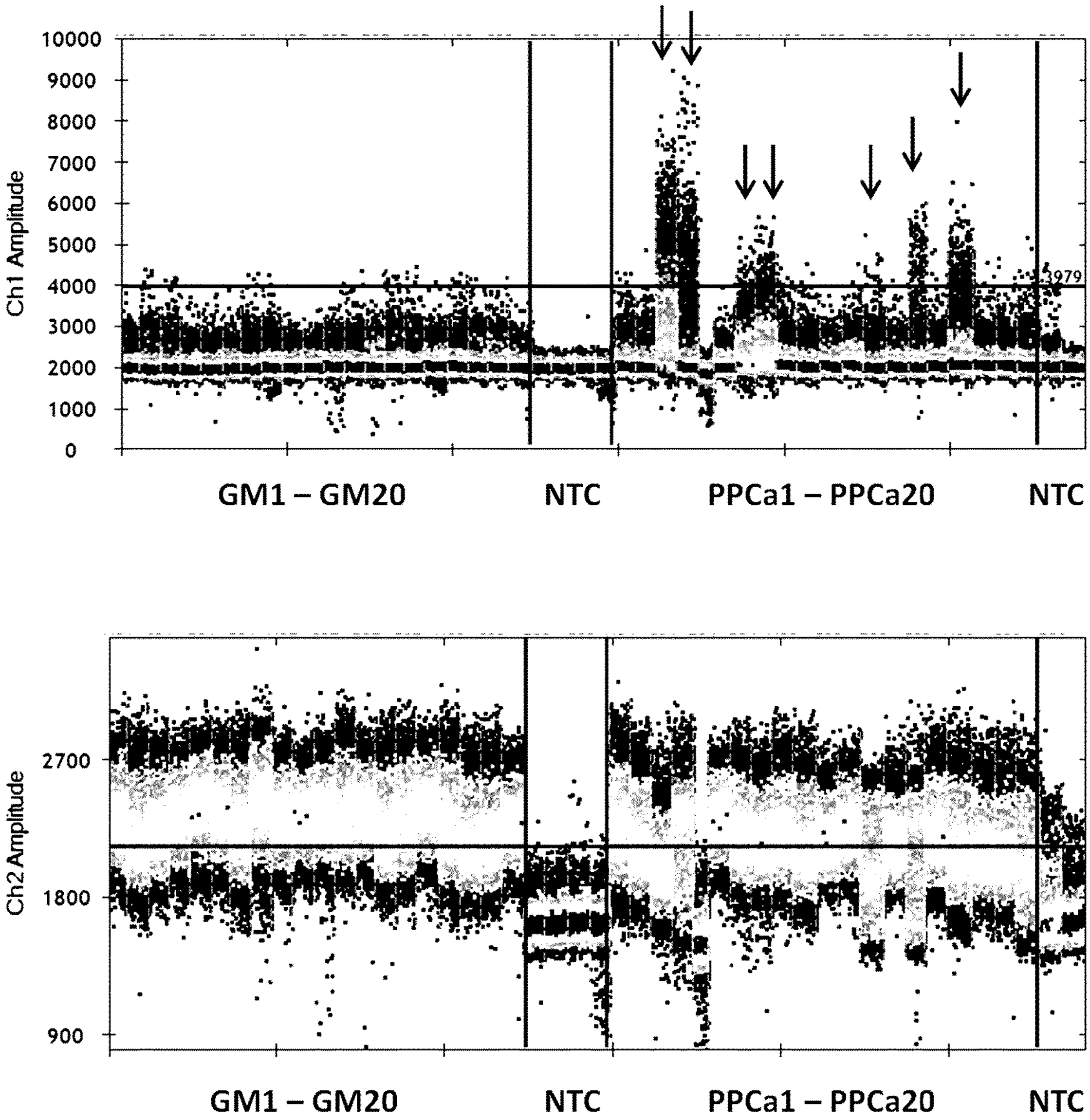
10000
9000
8000
7000
6000
5000
4000
3000
2000
1000
0
Ch1 Amplitude
3979
GM1 – GM20
NTC
PPCa1 – PPCa20
NTC
2700
1800
900
Ch2 Amplitude
GM1 – GM20
NTC
PPCa1 – PPCa20
NTC

Fig. 55

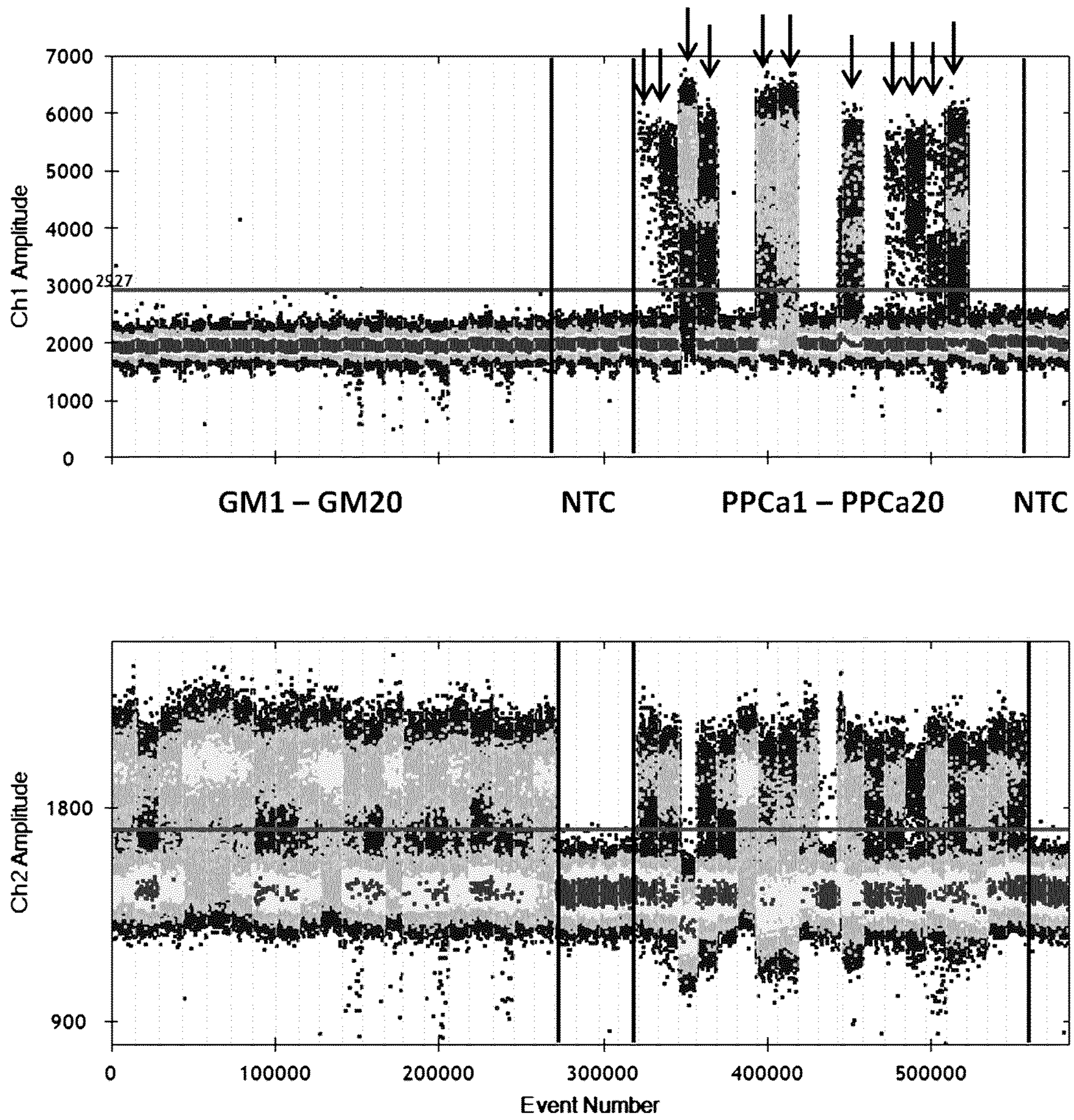
7000
6000
5000
4000
3000
2000
1000
0
2927
Ch1 Amplitude
GM1 – GM20
NTC
PPCa1 – PPCa20
NTC
1800
900
Ch2 Amplitude
0
100000
200000
300000
400000
500000
Event Number

Fig. 56

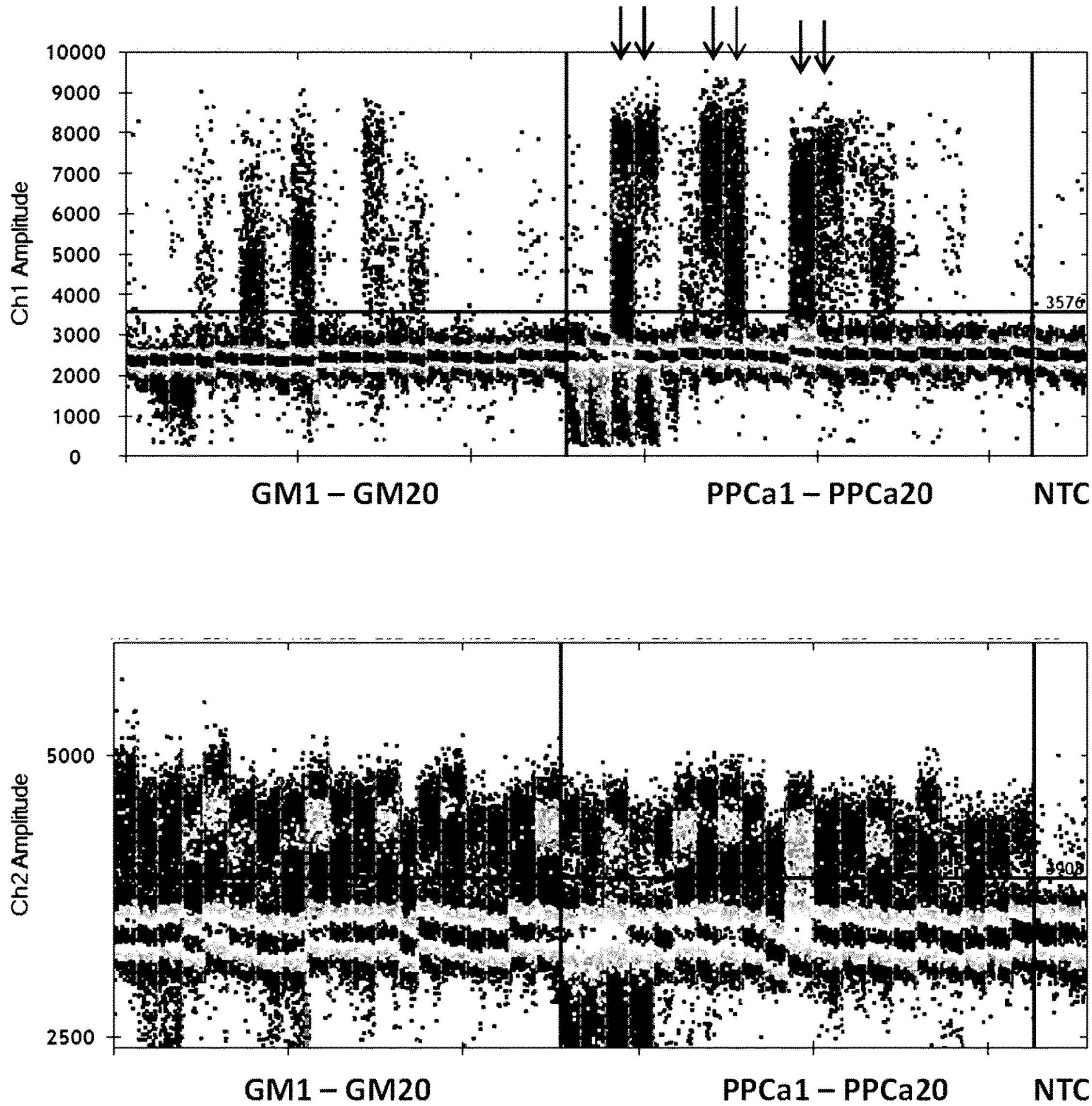
10000
9000
8000
7000
6000
5000
4000
3000
2000
1000
0
Ch1 Amplitude
3576
GM1 – GM20
PPCa1 – PPCa20
NTC
5000
2500
Ch2 Amplitude
3908
GM1 – GM20
PPCa1 – PPCa20
NTC

Fig. 57

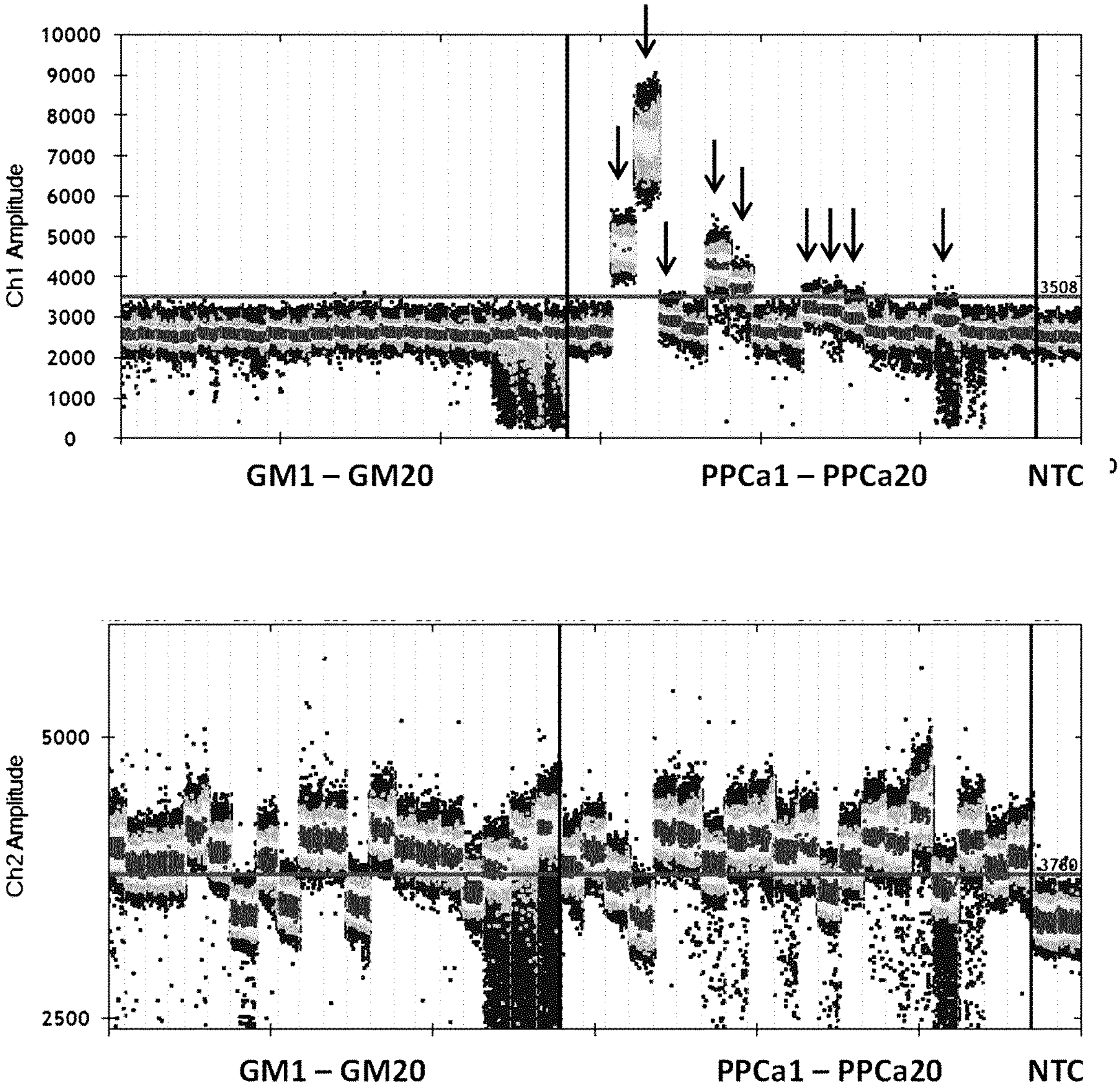
10000
9000
8000
7000
6000
5000
4000
3000
2000
1000
0
Ch1 Amplitude
3508
GM1 – GM20
PPCa1 – PPCa20
NTC
5000
2500
Ch2 Amplitude
3760
GM1 – GM20
PPCa1 – PPCa20
NTC

Fig. 58

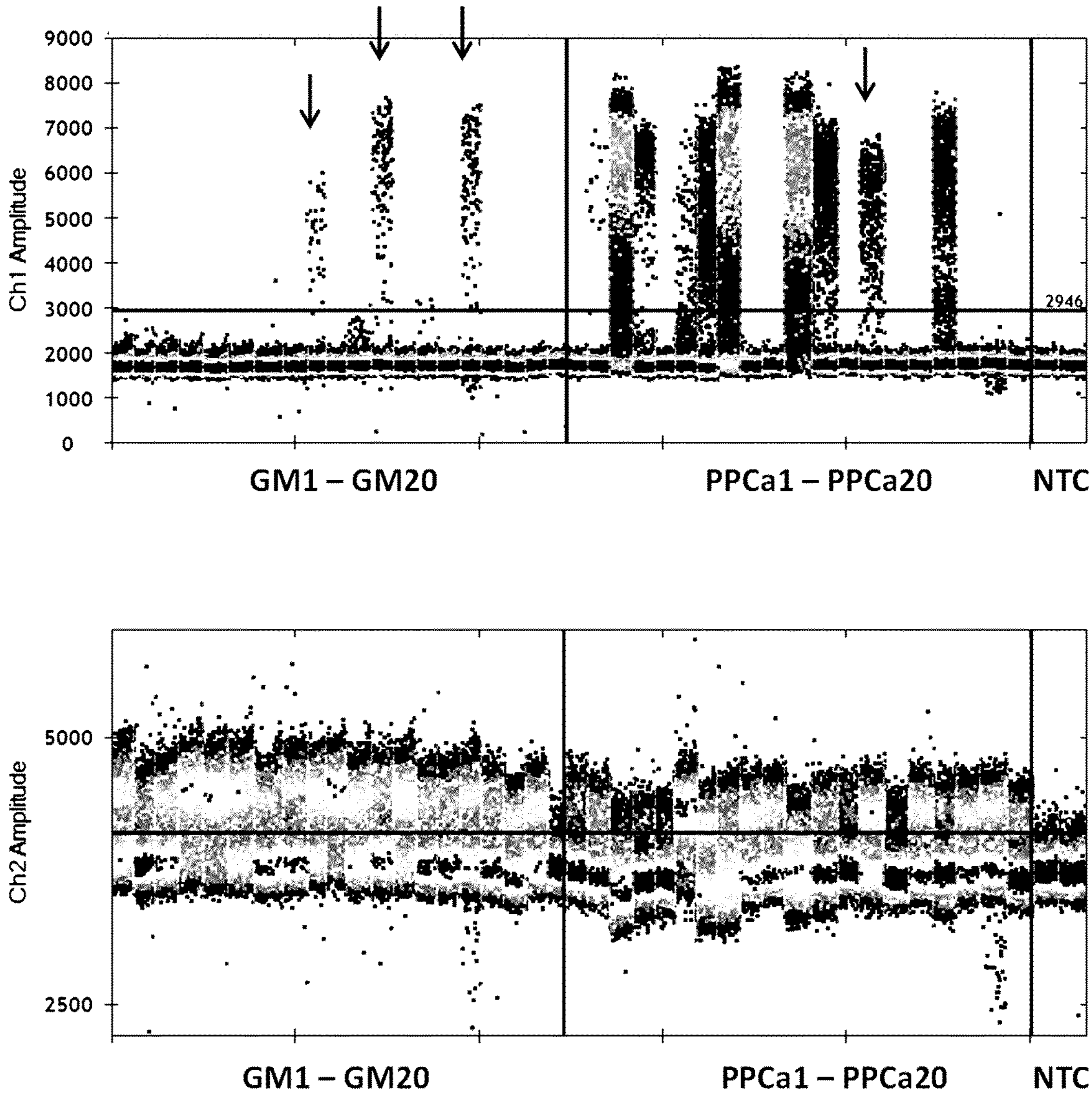
9000
8000
7000
6000
5000
4000
3000
2000
1000
0
Ch1 Amplitude
2946
GM1 – GM20
PPCa1 – PPCa20
NTC
5000
2500
Ch2 Amplitude
GM1 – GM20
PPCa1 – PPCa20
NTC

Fig. 59

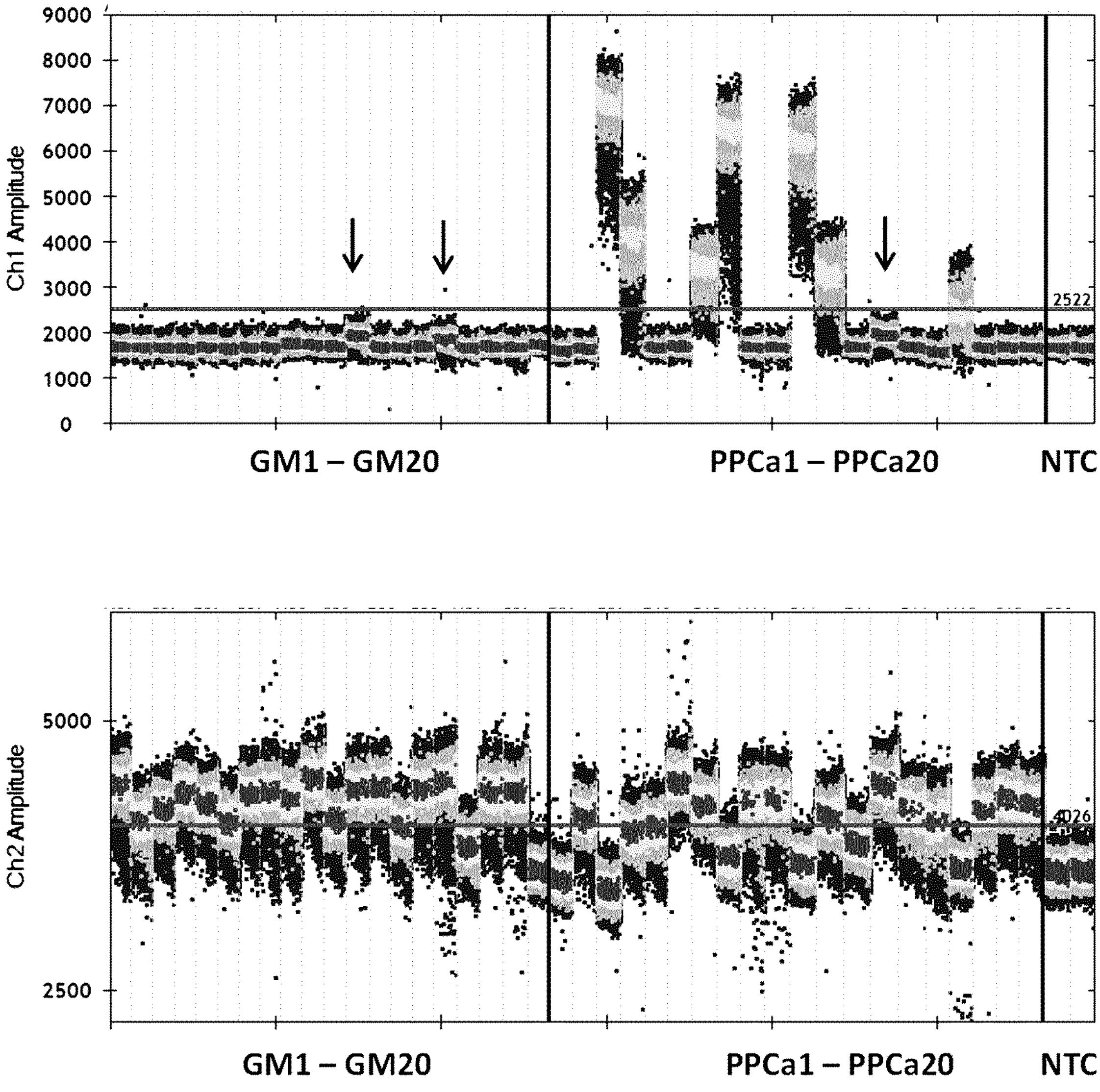
9000
8000
7000
6000
5000
4000
3000
2000
1000
0
Ch1 Amplitude
2522
GM1 – GM20
PPCa1 – PPCa20
NTC
5000
2500
Ch2 Amplitude
4026
GM1 – GM20
PPCa1 – PPCa20
NTC

Fig. 60

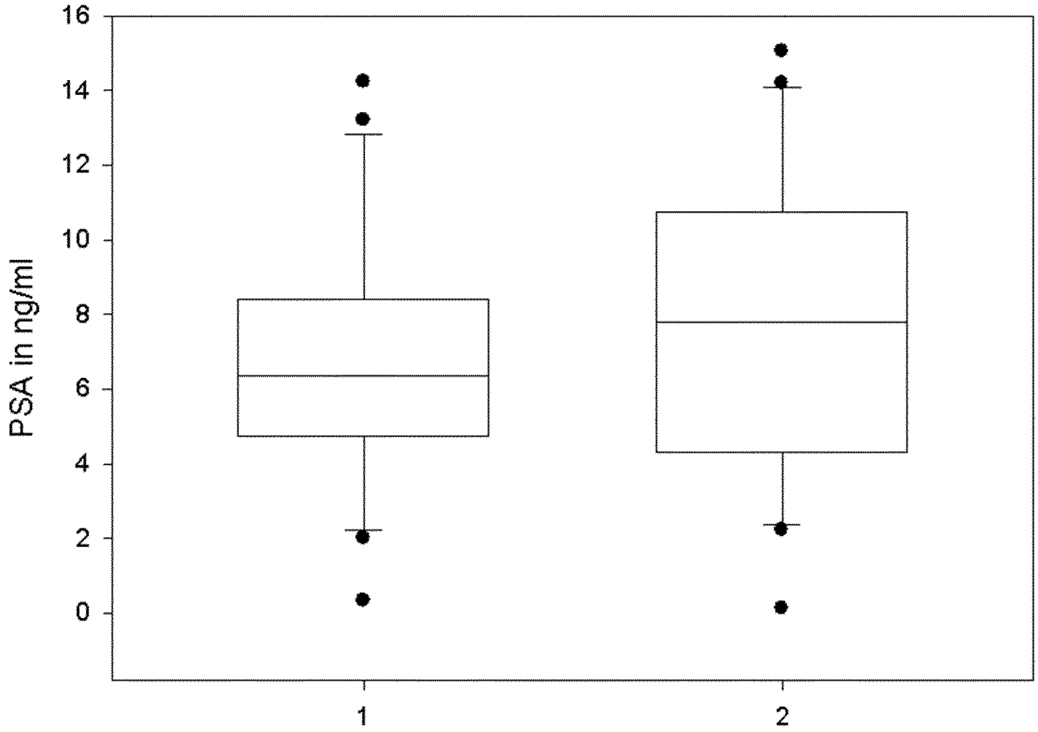


Fig. 61

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2012007462 A1
- WO 2013064163 A1
- US 20130022974 A
- US 20110301050 A
- WO 2012092259 A1
- WO 2013192351 A1
- WO 2013033714 A1
- WO 2013041731 A1
- US 20100233707 A1
- WO 102015226843 A
- WO 102016216438 A


### In der Beschreibung aufgeführte Nicht-Patentliteratur


- **MIKESKA T ; CANDILORO IL ; DOBROVICA.** *Epigenomics,* 2010, vol. 2, 561-73
- **KRISTENSEN LS ; HANSEN LL.** *Clin Chem.,* 2009, vol. 8, 1471-83
- **SHEN L ; WATERLAND RA.** *Curr Opin Clin Nutr Metab Care,* 2007, vol. 5, 576-81
- **HERNÁNDEZ HG ; TSE MY ; PANG SC et al.** *BioTechniques,* 2013, vol. 4, 181-97
- **HERMAN JG ; GRAFF JR ; MYÖHÄNEN S et al.** *Proc Nat Acad Sci USA.,* 1996, vol. 18, 9821-26
- **KRISTENSEN LS ; WOJDACZ TK ; THESTRUP BB et al.** *BMC Cancer,* 2009, vol. 9, 453
- **CANDILORO IL ; MIKESKA T ; HOKLAND P et al.** *Epigenetics Chromatin,* 2008, vol. 1, 7
- **KRAYTSBERG Y ; KHRAPKO K.** *Expert Review of Molecular Diagnostics,* 2005, vol. 5, 809-15
- **GRÜTZMANN R ; MOLNAR B ; PILARSKY C et al.** *PLoS one,* 2008, vol. 3, e3759
- **HOW KIT A ; NIELSEN HM.** *Tost J. Biochimie.,* November 2012, vol. 94 (11), 2314-37
- **STEWART GD ; VAN NESTE L ; DELVENNE P et al.** *J Urol.,* 2013, vol. 189, 110-1116
- **TOMBAL B.** *EurUrol.,* 2012, vol. 62, 997-8
- **TOMBAL B.** *Eur Urology Suppl.,* 2006, vol. 5, 511-513
- **CASTELLANOS-RIZALDOS E ; MILBURY CA ; KARATZA E et al.** *PLoS One,* 2014, vol. 9 (4), e94103
- **WARNECKE PM ; STIRZAKER C ; MELKI JR ; MILLAR DS ; PAUL CL ; CLARK SJ.** *Nucleic Acids Res.,* 01. November 1997, vol. 25 (21), 4422-6
- **WOJDACZ TK ; DOBROVIC A ; HANSEN LL.** *Nat Protoc.,* 2008, vol. 3 (12), 1903-8
- **WOJDACZ TK ; HANSEN LL ; DOBROVIC A.** *BMC Res Notes,* 28. Juli 2008, vol. 1, 54
- **WOJDACZ TK ; HANSEN LL.** *BioTechnique,* 2006, vol. 41, 274-8
- **HUBBARD RA.** *Annals of Internal Medicine,* 2011, vol. 155, 481-92
- **REDSHAW N. et al.** *BMC Genomics,* 2014, vol. 15, 1174
- **OKINO ST et al.** *Mol. Carcinogenesis,* 2007, vol. 46, 839-846
- **WOJDACZ T et al.** *Epigenetics,* 16. Mai 2009, vol. 4 (4), 231-4